# EUROPEAN PATENT SPECIFICATION

(11) **EP 3 846 803 B1**
(45) Date of publication and mention of the grant of the patent: **18.11.2026**
(21) Application number: 19857913.8
(22) Date of filing: 06.09.2019
(51) Int. Cl.: A61K 31/416, C07D 403/14, A61P 1/16, A61P 37/00, C07D 487/06, C07D 487/16, C07D 487/20, C07D 487/22, C07D 498/18, C07D 498/20, C07D 519/00, C07D 471/04, C07D 401/12, C07D 401/14, C07D 403/12, A61K 45/06

(54) **MACROCYCLIC COMPOUNDS FOR THE TREATMENT OF MEDICAL DISORDERS**
MAKROCYCLISCHE VERBINDUNGEN ZUR BEHANDLUNG VON MEDIZINISCHEN ERKRANKUNGEN
COMPOSÉS MACROCYCLIQUES POUR LE TRAITEMENT DE TROUBLES MÉDICAUX

(30) Priority: 06.09.2018 US 201862727857 P
(43) Date of publication of application: 14.07.2021
(73) Proprietor: Achillion Pharmaceuticals, Inc., Boston, MA 02210 (US)
(72) Inventor: WILES, Jason, Allan, Boston, MA 02210 (US); GADHACHANDA, Venkat, Rao, Boston, MA 02210 (US); EASTMAN, Kyle, J., Boston, MA 02210 (US); PAIS, Godwin, Boston, MA 02210 (US)
(74) Representative: J A Kemp LLP
(86) International application number: PCT/US2019/050065
(87) International publication number: WO 2020/051532

(56) References cited:
- WO-A1-2012/093101
- WO-A1-2017/035405
- WO-A1-2017/035411
- WO-A1-2018/160892
- WO-A1-2018/160892
- WO-A2-2014/002051
- WO-A2-2017/035413
- WO-A2-2017/035413
- WO-A2-2017/098328
- US-A1- 2018 179 186
- QU ET AL.: "Recent Developments in Low Molecular Weight Complement Inhibitors", MOL IMMUNOL., vol. 47, no. 2-3, 2009, pages 185 - 195, XP026886936, DOI: 10.1016/j.molimm.2009.08.032

## Description

### Field of the Invention

This invention provides a group of compounds for use in treating medical disorders, such as complement-mediated disorders, including Complement Factor D mediated disorders

### Background of the Invention

The complement system is a part of the innate immune system which does not adapt to changes over the course of the host's life, but is recruited and used by the adaptive immune system. For example, it assists, or complements, the ability of antibodies and phagocytic cells to clear pathogens. This sophisticated regulatory pathway allows rapid reaction to pathogenic organisms while protecting host cells from destruction. Over thirty proteins and protein fragments make up the complement system. These proteins act through opsonization (enhancing phagocytosis of antigens), chemotaxis (attracting macrophages and neutrophils), cell lysis (rupturing membranes of foreign cells), and agglutination (clustering and binding of pathogens together).

The complement system has three pathways: classical, alternative, and lectin. Complement Factor D plays an early and central role in activation of the alternative pathway of the complement cascade. Activation of the alternative complement pathway is initiated by spontaneous hydrolysis of a thioester bond within the C3 protein to produce C3(H₂O), which associates with Factor B to form the C3(H₂O)B complex. Complement Factor D acts to cleave Factor B within the C3(H₂O)B complex to form Ba and Bb. The Bb fragment remains associated with C3(H₂O) to form the alternative pathway C3 convertase C3(H₂O)Bb. Additionally, C3b generated by any of the C3 convertases also associates with Factor B to form C3bB, which Factor D cleaves to generate the later stage alternative pathway C3 convertase C3bBb. This latter form of the alternative pathway C3 convertase may provide important downstream amplification within all three of the defined complement pathways, leading ultimately to the recruitment and assembly of additional factors in the complement cascade pathway, including the cleavage of C5 to C5a and C5b. C5b acts in the assembly of factors C6, C7, C8, and C9 into the membrane attack complex, which can destroy pathogenic cells by lysing the cell.

The dysfunction of or excessive activation of complement has been linked to certain autoimmune, inflammatory, and neurodegenerative diseases, as well as ischemia-reperfusion injury and cancer. For example, activation of the alternative pathway of the complement cascade contributes to the production of C3a and C5a, both potent anaphylatoxins, which also have roles in a number of inflammatory disorders. Therefore, in some instances, it is desirable to decrease the response of the complement pathway, including the alternative complement pathway. Some examples of disorders mediated by the complement pathway include age-related macular degeneration (AMD), paroxysmal nocturnal hemoglobinuria (PNH), multiple sclerosis, and rheumatoid arthritis.

BioCryst Pharmaceuticals Inc. US Pat. No. 6,653,340 titled "Compounds useful in the complement, coagulate and kallikrein pathways and method for their preparation" describes fused bicyclic ring compounds that are inhibitors of Factor D. US Patent Application 2019-0142802 assigned to BioCryst Pharmaceuticals describes benzopyrazole compounds for the treatment of aberrant complement disorders. Additional patents assigned to BioCryst for the treatment and prevention of complement disorders include granted US patent 10,125,102 and US Applications US2018-0362458. Development of BioCryst's Factor D inhibitor BCX1470 was discontinued due to lack of specificity and short half-life of the compound.

Novartis PCT patent publication WO2012/093101 titled "Indole compounds or analogues thereof useful for the treatment of age-related macular degeneration" describes certain Factor D inhibitors. Additional Factor D inhibitors are described in Novartis PCT patent publications WO2012093101, WO2013/164802, WO2013/192345, WO2014/002051, WO2014/002052, WO2014/002053, WO2014/002054, WO2014/002057, WO2014/002058, WO2014/002059, WO2014/005150, WO2014/009833, WO2014/143638, WO2015/009616, WO2015/009977, WO2015/066241, and WO2016088082.

Lifesci Pharmaceuticals PCT patent publication WO2017/098328 titled "Therapeutic Inhibitory Compounds" describes various Factor D inhibitors with variations in the central core heterocyclic ring. PCT patent publication WO2018/015818 is also titled "Therapeutic Inhibitory Compounds" and describes Factor D inhibitors without a cyclic central core.

Bristol-Myers Squibb PCT patent publication WO2004/045518 titled "Open chain prolyl urea-related modulators of androgen receptor function" describes open chain prolyl urea and thiourea related compounds for the treatment of androgen receptor-associated conditions, such as age-related diseases, for example, sarcopenia.

Additional complement factor D inhibitors are described in U.S. Patent Nos. 9,598,446; 9,643,986; 9,663,543; 9,695,205; 9,732,103; 9,732,104; 9,758,537; 9,796,741; 9,828,396; 10,000,516; 10,005,802; 10,011,612; 10,081,645; 10,087,203; 10,092,584; 10,100,072; 10,138,225; 10,189,869; 10,106,563; 10,301,336; and 10,287,301; International Publication Nos. WO2019/028284; WO2018/160889; WO2018/160891; WO2018/160892; WO2017/035348; WO2017/035349; WO 2017/035351; WO 2017/035352; WO 2017/035353; WO 2017/035355; WO2017/035357; WO2017/035360; WO2017/035361; WO2017//035362; WO2017/035415; WO2017/035401; WO2017/035405; WO2017/035413; WO2017/035409; WO2017/035411; WO2017/035417; WO2017/035408 WO2015/130784; WO2015/130795; WO2015/130806; WO2015/130830; WO2015/130838; WO2015/130842; WO2015/130845; and WO2015/130854; and U.S. Patent Publication Nos. US 2016-0361329; US 2016-0362432; US 2016-0362433; US 2016-0362399; US 2017-0056428; US 2017-0057950; US 2017-0057993; US 2017-0189410; US 2017-0226142; US 2017-0260219; US 2017-0298084; US 2017-0298085; US 2018-0022766; US 2018-0022767; US 2018-0072762; US 2018-0030075; US 2018-0169109; US 2018-0177761; US 2018-0179185; US 2018-0179186; US 2018-0179236; US 2018-0186782; US 2018-0201580; US 2019-0031692; US 2019-0048033; US 2019-0144473; and US 2019-0211033 all owned by Achillion Pharmaceuticals, Inc.

Given the wide variety of medical disorders that are caused by detrimental immune or inflammatory responses, new compounds are needed for medical treatment.

### SUMMARY

This invention provides a compound selected from: 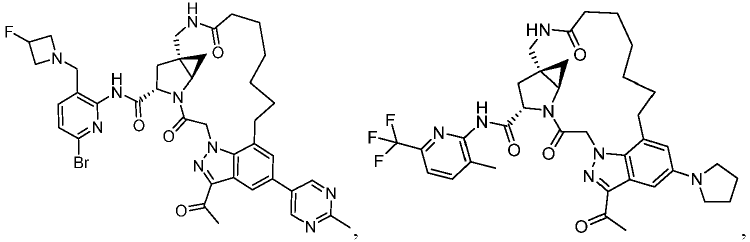 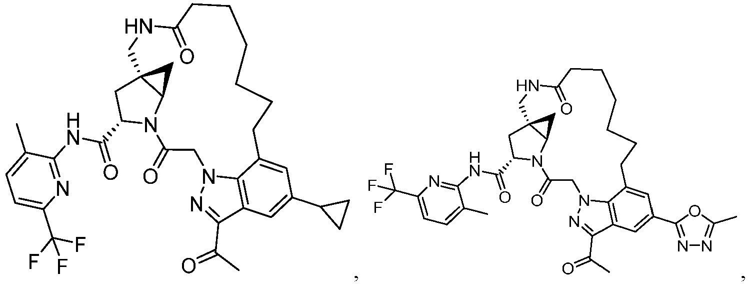 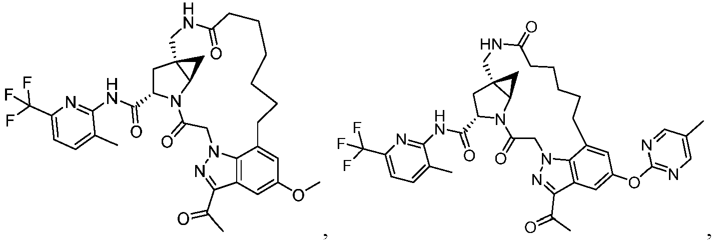 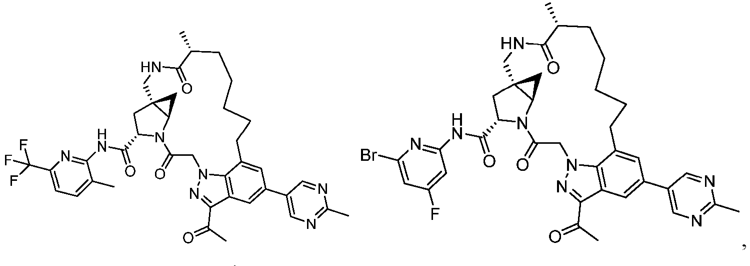 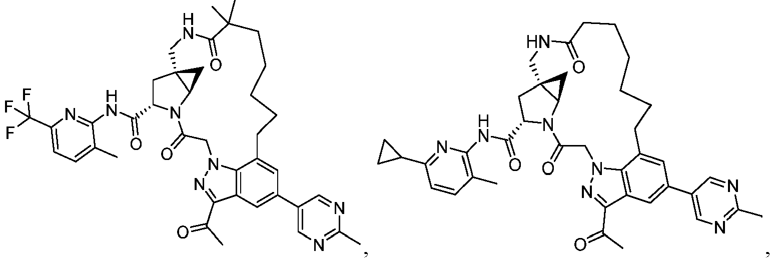 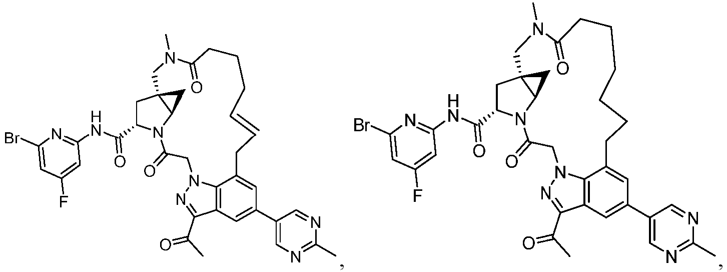 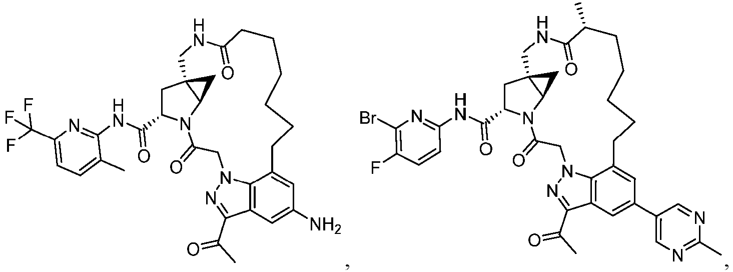 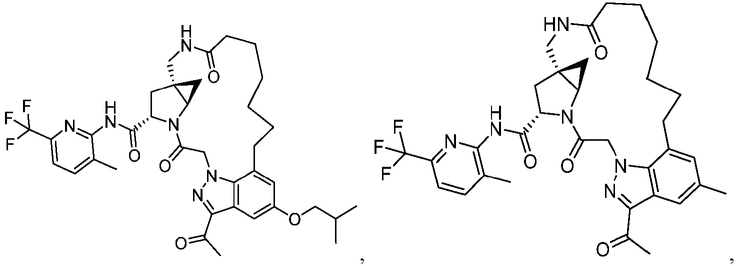 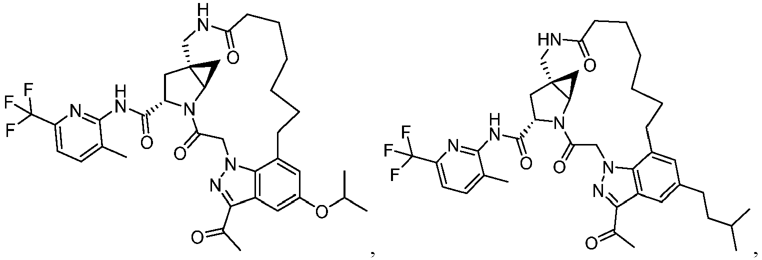 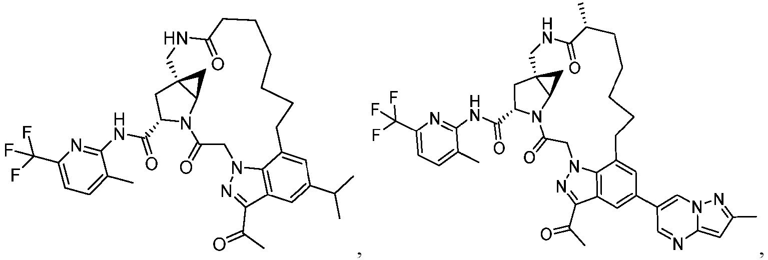 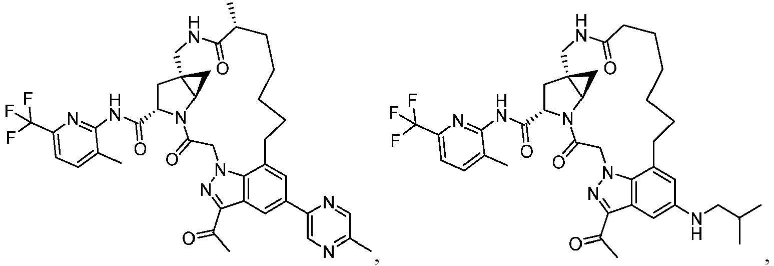 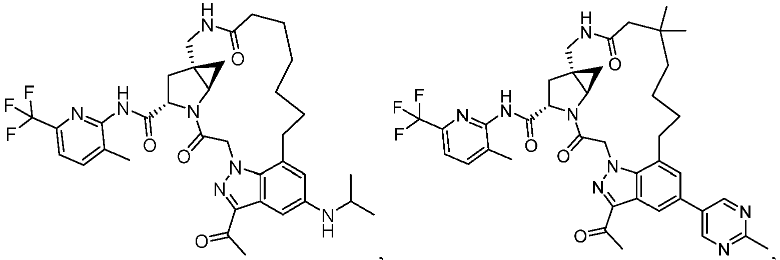 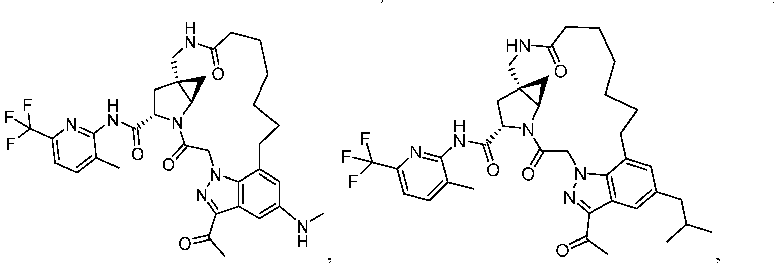 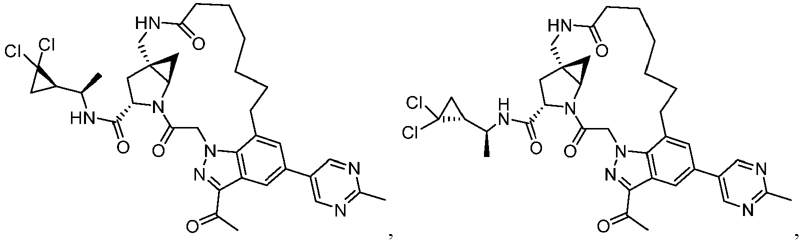 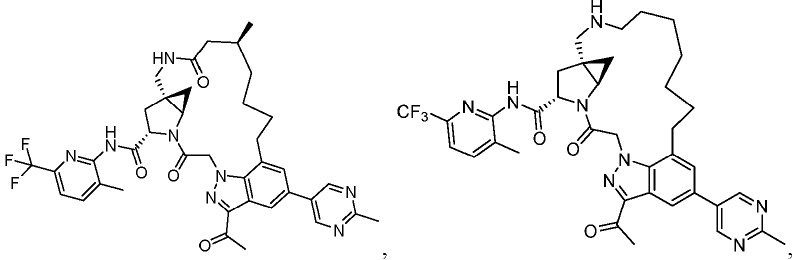 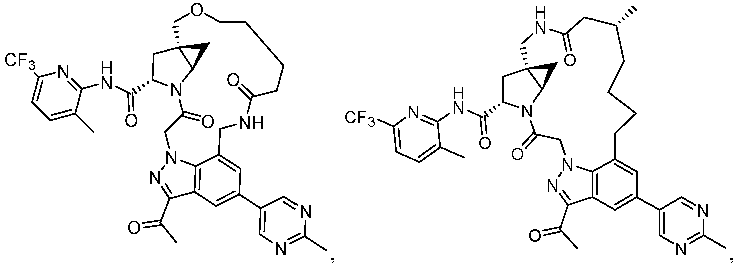 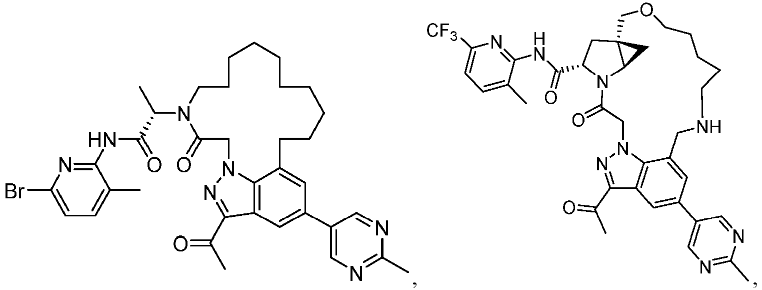 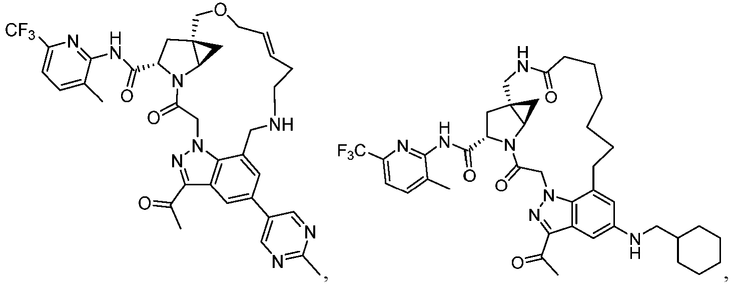 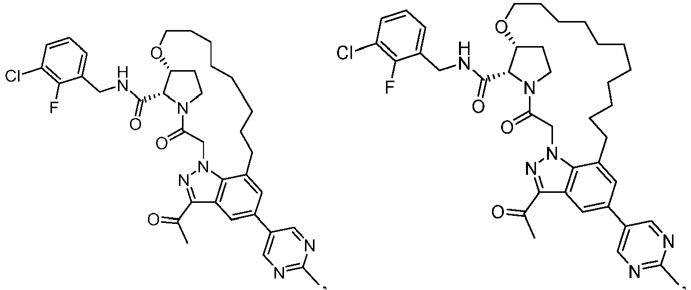 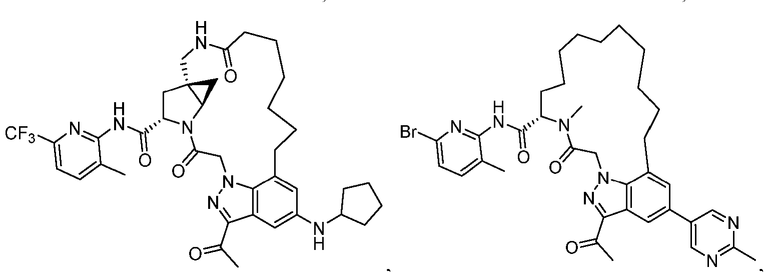 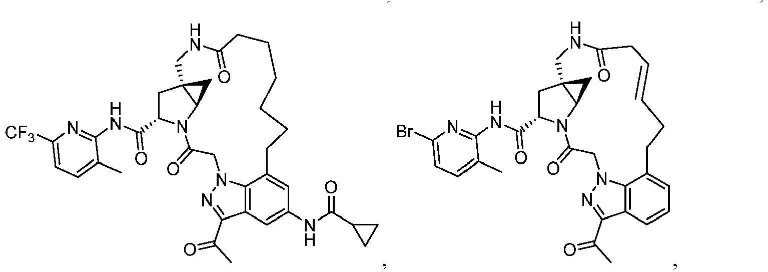 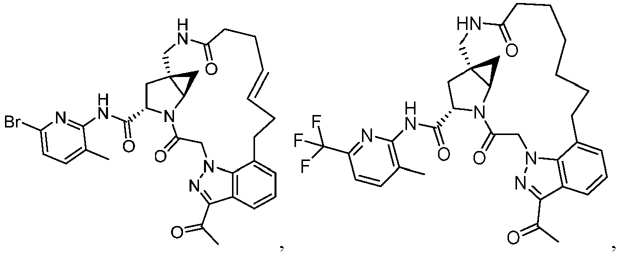 and 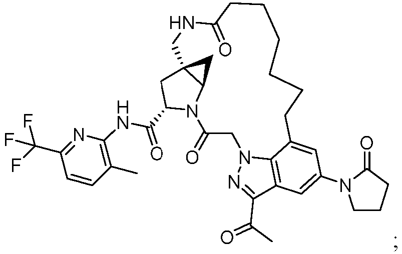 or a pharmaceutically acceptable salt thereof.

Pharmaceutical compositions comprising a compound or salt as described hereintogether with a pharmaceutically acceptable carrier are also disclosed. Further aspects of the invention are set out in the appended set of claims.

### DETAILED DESCRIPTION

### TERMINOLOGY

Compounds are described using standard nomenclature. Unless defined otherwise, all technical and scientific terms used herein have the same meaning as is commonly understood by one of skill in the art to which this invention belongs.

The compounds in any of the Formulas described herein include enantiomers, mixtures of enantiomers, diastereomers, tautomers, racemates and other isomers, such as rotamers, as if each is specifically described, unless otherwise indicated or otherwise excluded by context.

The terms "a" and "an" do not denote a limitation of quantity, but rather denote the presence of at least one of the referenced items. The term "or" means "and/or". Recitation of ranges of values are merely intended to serve as a shorthand method of referring individually to each separate value falling within the range, unless otherwise indicated herein, and each separate value is incorporated into the specification as if it were individually recited herein. The endpoints of all ranges are included within the range and independently combinable. All methods described herein can be performed in a suitable order unless otherwise indicated herein or otherwise clearly contradicted by context. The use of examples, or exemplary language (e.g., "such as"), is intended merely to better illustrate the invention and does not pose a limitation on the scope of the invention unless otherwise claimed. Unless defined otherwise, technical and scientific terms used herein have the same meaning as is commonly understood by one of skill in the art to which this invention belongs.

The present invention includes compounds as described herein with at least one desired isotopic substitution of an atom, at an amount above the natural abundance of the isotope, i.e., enriched.

Examples of isotopes that can be incorporated into compounds of the invention include isotopes of hydrogen, carbon, nitrogen, oxygen, phosphorous, fluorine, and chlorine, such as ²H, ³H, ¹¹C, ¹³C, ¹⁴C, ¹⁵N, ¹⁷O, ¹⁸O, ¹⁸F ³¹P, ³²P, ³⁵S, ³⁶CI, and ¹²⁵I respectively. In one embodiment, isotopically labelled compounds can be used in metabolic studies (with ¹⁴C), reaction kinetic studies (with, for example ²H or ³H), detection or imaging techniques, such as positron emission tomography (PET) or single-photon emission computed tomography (SPECT) including drug or substrate tissue distribution assays, or in radioactive treatment of patients. In particular, an ¹⁸F labeled compound may be particularly desirable for PET or SPECT studies. Isotopically labeled compounds of this invention can generally be prepared by carrying out the procedures disclosed in the schemes or in the examples and preparations described below by substituting a readily available isotopically labeled reagent for a non-isotopically labeled reagent.

By way of general example and without limitation, isotopes of hydrogen, for example, deuterium (²H) and tritium (³H) may optionally be used anywhere in described structures that achieves the desired result. Alternatively or in addition, isotopes of carbon, e.g., ¹³C and ¹⁴C, may be used. In one embodiment, the isotopic substitution is replacing hydrogen with a deuterium at one or more locations on the molecule to improve the performance of the drug, for example, the pharmacodynamics, pharmacokinetics, biodistribution, half-life, stability, AUC, Tmax, Cmax, etc. For example, the deuterium can be bound to carbon in a location of bond breakage during metabolism (an α-deuterium kinetic isotope effect) or next to or near the site of bond breakage (a β-deuterium kinetic isotope effect).

Isotopic substitutions, for example deuterium substitutions, can be partial or complete. Partial deuterium substitution means that at least one hydrogen is substituted with deuterium. In certain embodiments, the isotope is 80, 85, 90, 95 or 99% or more enriched in an isotope at any location of interest. In one embodiments deuterium is 80, 85, 90, 95 or 99% enriched at a desired location. Unless otherwise stated, the enrichment at any point is above natural abundance, and in an embodiment is enough to alter a detectable property of the drug in a human.

The compound of the present invention may form a solvate with solvents (including water). Therefore, in one embodiment, the invention includes a solvated form of the active compound. The term "solvate" refers to a molecular complex of a compound of the present invention (including a salt thereof) with one or more solvent molecules. Nonlimiting examples of solvents are water, ethanol, dimethyl sulfoxide, acetone and other common organic solvents. The term "hydrate" refers to a molecular complex comprising a compound of the invention and water. Pharmaceutically acceptable solvates in accordance with the invention include those wherein the solvent of crystallization may be isotopically substituted, *e.g*. D₂O, d₆-acetone, d₆-DMSO. A solvate can be in a liquid or solid form.

A dash ("-") that is not between two letters or symbols is used to indicate a point of attachment for a substituent. For example, -(C=O)NH₂ is attached through carbon of the keto (C=O) group.

The term "substituted", as used herein, means that any one or more hydrogens on the designated atom or group is replaced with a moiety selected from the indicated group, provided that the designated atom's normal valence is not exceeded and the resulting compound is stable. For example, when the substituent is oxo (i.e., =O) then two hydrogens on the atom are replaced. For example a pyridyl group substituted by oxo is a pyridone. Combinations of substituents and/or variables are permissible only if such combinations result in stable compounds or useful synthetic intermediates.

A stable active compound refers to a compound that can be isolated and can be formulated into a dosage form with a shelf life of at least one month. A stable manufacturing intermediate or precursor to an active compound is stable if it does not degrade within the period needed for reaction or other use. A stable moiety or substituent group is one that does not degrade, react or fall apart within the period necessary for use. Nonlimiting examples of unstable moieties are those that combine heteroatoms in an unstable arrangement, as typically known and identifiable to those of skill in the art.

Any suitable group may be present on a "substituted" or "optionally substituted" position that forms a stable molecule and meets the desired purpose of the invention and includes, but is not limited to, e.g., halogen (which can independently be F, Cl, Br or I); cyano; hydroxyl; nitro; azido; alkanoyl (such as a C₂-C₆ alkanoyl group); carboxamide; alkyl, cycloalkyl, alkenyl, alkynyl, alkoxy, aryloxy such as phenoxy; thioalkyl including those having one or more thioether linkages; alkylsulfinyl; alkylsulfonyl groups including those having one or more sulfonyl linkages; aryl (e.g., phenyl, biphenyl, naphthyl, or the like, each ring either substituted or unsubstituted); arylalkyl having for example, 1 to 3 separate or fused rings and from 6 to about 14 or 18 ring carbon atoms, with benzyl being an exemplary arylalkyl group; arylalkoxy, for example, having 1 to 3 separate or fused rings with benzyloxy being an exemplary arylalkoxy group; or a saturated or partially unsaturated heterocycle having 1 to 3 separate or fused rings with one or more N, O or S atoms, or a heteroaryl having 1 to 3 separate or fused rings with one or more N, O or S atoms, e.g. coumarinyl, quinolinyl, isoquinolinyl, quinazolinyl, pyridyl, pyrazinyl, pyrimidinyl, furanyl, pyrrolyl, thienyl, thiazolyl, triazinyl, oxazolyl, isoxazolyl, imidazolyl, indolyl, benzofuranyl, benzothiazolyl, tetrahydrofuranyl, tetrahydropyranyl, piperidinyl, morpholinyl, piperazinyl, and pyrrolidinyl. Such groups may be further substituted, e.g. with hydroxy, alkyl, alkoxy, halogen and amino. In certain embodiments "optionally substituted" includes one or more substituents independently selected from halogen, hydroxyl, amino, cyano, -CHO, -COOH, -CONH₂, alkyl including C₁-C₆alkyl, alkenyl including C₂-C₆alkenyl, alkynyl including C₂-C₆alkynyl, -C₁-C₆alkoxy, alkanoyl including C₂-C₆alkanoyl, (mono- and di-C₁-C₆alkylamino)C₀-C₂alkyl, haloalkyl including C₁-C₆haloalkyl, hydoxyC₁-C₆alkyl, ester, carbamate, urea, sulfonamide,-C₁-C₆alkyl(heterocyclo), C₁-C₆alkyl(heteroaryl), -C₁-C₆alkyl(C₃-C₇cycloalkyl), O-C₁-C₆alkyl(C₃-C₇cycloalkyl), B(OH)₂, phosphate, phosphonate and haloalkoxy including C₁-C₆haloalkoxy.

"Alkyl" is a branched or straight chain saturated aliphatic hydrocarbon group. In one embodiment, the alkyl contains from 1 to about 12 carbon atoms, more generally from 1 to about 6 carbon atoms or from 1 to about 4 carbon atoms. In one embodiment, the alkyl contains from 1 to about 8 carbon atoms. In certain embodiments, the alkyl is C₁-C₂, C₁-C₃, C₁-C₄, C₁-C₅ or C₁-C₆. The specified ranges as used herein indicate an alkyl group having each member of the range described as an independent species. For example, the term C₁-C₆ alkyl as used herein indicates a straight or branched alkyl group having from 1, 2, 3, 4, 5, or 6 carbon atoms and is intended to mean that each of these is described as an independent species. For example, the term C₁-C₄alkyl as used herein indicates a straight or branched alkyl group having from 1, 2, 3, or 4 carbon atoms and is intended to mean that each of these is described as an independent species. When C₀-Cₙ alkyl is used herein in conjunction with another group, for example, (C₃₋C₇cycloalkyl)C₀-C₄ alkyl, or -C₀-C₄alkyl(C₃-C₇cycloalkyl), the indicated group, in this case cycloalkyl, is either directly bound by a single covalent bond (C₀alkyl), or attached by an alkyl chain in this case 1, 2, 3, or 4 carbon atoms. Alkyls can also be attached via other groups such as heteroatoms as in -O-C₀-C₄alkyl(C₃-C₇cycloalkyl). Examples of alkyl include, but are not limited to, methyl, ethyl, n-propyl, isopropyl, n-butyl, isobutyl, sec-butyl, t-butyl, n-pentyl, isopentyl, *tert*-pentyl, neopentyl, n-hexyl, 2-methylpentane, 3-methylpentane, 2,2-dimethylbutane, 2,3-dimethylbutane, and hexyl.

"Aliphatic" refers to a saturated or unsaturated, straight, branched, or cyclic hydrocarbon. "Aliphatic" is intended herein to include, but is not limited to, alkyl, alkenyl, alkynyl, cycloalkyl, cycloalkenyl, and cycloalkynyl moieties, and thus incorporates each of these definitions. In one embodiment, "aliphatic" is used to indicate those aliphatic groups having 1-20 carbon atoms. The aliphatic chain can be, for example, mono-unsaturated, di-unsaturated, tri-unsaturated, polyunsaturated, or alkynyl. Unsaturated aliphatic groups can be in a cis or trans configuration. In one embodiment, the aliphatic group contains from 1 to about 12 carbon atoms, more generally from 1 to about 6 carbon atoms or from 1 to about 4 carbon atoms. In one embodiment, the aliphatic group contains from 1 to about 8 carbon atoms. In certain embodiments, the aliphatic group is C₁-C₂, C₁-C₃, C₁-C₄, C₁-C₅ or C₁-C₆. The specified ranges as used herein indicate an aliphatic group having each member of the range described as an independent species. For example, the term C₁-C₆ aliphatic as used herein indicates a straight or branched alkyl, alkenyl, or alkynyl group having from 1, 2, 3, 4, 5, or 6 carbon atoms and is intended to mean that each of these is described as an independent species. For example, the term C₁-C₄ aliphatic as used herein indicates a straight or branched alkyl, alkenyl, or alkynyl group having from 1, 2, 3, or 4 carbon atoms and is intended to mean that each of these is described as an independent species. In one embodiment, the aliphatic group is substituted with one or more functional groups that results in the formation of a stable moiety.

The term "heteroaliphatic" refers to an aliphatic moiety that contains at least one heteroatom in the chain, for example, an amine, carbonyl, carboxy, oxo, thio, phosphate, phosphonate, nitrogen, phosphorus, silicon, or boron atoms in place of a carbon atom. In one embodiment, the only heteroatom is nitrogen. In one embodiment, the only heteroatom is oxygen. In one embodiment, the only heteroatom is sulfur. "Heteroaliphatic" is intended herein to include, but is not limited to, heteroalkyl, heteroalkenyl, heteroalkynyl, heterocycle, and heterocycloalkynyl moieties. In one embodiment, "heteroaliphatic" is used to indicate a heteroaliphatic group (cyclic, acyclic, substituted, unsubstituted, branched or unbranched) having 1-20 carbon atoms. In one embodiment, the heteroaliphatic group is optionally substituted in a manner that results in the formation of a stable moiety. Nonlimiting examples of heteroaliphatic moieties are polyethylene glycol, polyalkylene glycol, amide, polyamide, polylactide, polyglycolide, thioether, ether, alkyl-heterocycle-alkyl, -O-alkyl-O-alkyl, alkyl-O-haloalkyl, etc. When a term is used that includes "alk" it should be understood that "cycloalkyl" or "carbocyclic" can be considered part of the definition, unless unambiguously excluded by the context. For example and without limitation, the terms alkyl, alkenyl, alkynyl, alkoxy, alkanoyl, alkenloxy, haloalkyl, etc. can all be considered to include the cyclic forms of alkyl, unless unambiguously excluded by context.

"Alkenyl" is a branched or straight chain aliphatic hydrocarbon group having one or more carbon-carbon double bonds that may occur at a stable point along the chain. Nonlimiting examples are C₂-C₈alkenyl, C₂-C₇alkenyl, C₂-C₆alkenyl, C₂-C₅alkenyl and C₂-C₄alkenyl. The specified ranges as used herein indicate an alkenyl group having each member of the range described as an independent species, as described above for the alkyl moiety. Examples of alkenyl include, but are not limited to, ethenyl and propenyl.

"Alkynyl" is a branched or straight chain aliphatic hydrocarbon group having one or more carbon-carbon triple bonds that may occur at any stable point along the chain, for example, C₂-C₈alkynyl or C₂-C₆alkynyl. The specified ranges as used herein indicate an alkynyl group having each member of the range described as an independent species, as described above for the alkyl moiety. Examples of alkynyl include, but are not limited to, ethynyl, propynyl, 1-butynyl, 2-butynyl, 3-butynyl, 1-pentynyl, 2-pentynyl, 3-pentynyl, 4-pentynyl, 1-hexynyl, 2-hexynyl, 3-hexynyl, 4-hexynyl and 5-hexynyl.

"Alkoxy" is an alkyl group as defined above covalently bound through an oxygen bridge (-O-). Examples of alkoxy include, but are not limited to, methoxy, ethoxy, n-propoxy, i-propoxy, n-butoxy, 2-butoxy, t-butoxy, n-pentoxy, 2-pentoxy, 3-pentoxy, isopentoxy, neopentoxy, n-hexoxy, 2-hexoxy, 3-hexoxy, and 3-methylpentoxy. Similarly an "alkylthio" or a "thioalkyl" group is an alkyl group as defined above with the indicated number of carbon atoms covalently bound through a sulfur bridge (-S-). In one embodiment, the alkoxy group is optionally substituted as described above.

"Alkenyloxy" is an alkenyl group as defined covalently bound to the group it substitutes by an oxygen bridge (-O-).

"Alkanoyl" is an alkyl group as defined above covalently bound through a carbonyl (C=O) bridge. The carbonyl carbon is included in the number of carbons, that is C₂alkanoyl is a CH₃(C=O)- group. In one embodiment, the alkanoyl group is optionally substituted as described

"Haloalkyl" indicates both branched and straight-chain alkyl groups substituted with 1 or more halogen atoms, up to the maximum allowable number of halogen atoms. Examples of haloalkyl include, but are not limited to, trifluoromethyl, monofluoromethyl, difluoromethyl, 2-fluoroethyl, and penta-fluoroethyl.

"Haloalkoxy" indicates a haloalkyl group as defined herein attached through an oxygen bridge (oxygen of an alcohol radical).

"Halo" or "halogen" indicates independently, any of fluoro, chloro, bromo or iodo.

"Aryl" indicates an aromatic group containing only carbon in the aromatic ring or rings. In one embodiment, the aryl group contains 1 to 3 separate or fused rings and is 6 to 14 or 18 ring atoms, without heteroatoms as ring members. When indicated, such aryl groups may be further substituted with carbon or non-carbon atoms or groups. Such substitution may include fusion to a 4 to 7 or a 5 to 7-membered saturated or partially unsaturated cyclic group that optionally contains 1, 2 or 3 heteroatoms independently selected from N, O, B, P, Si and S, to form, for example, a 3,4-methylenedioxyphenyl group. Aryl groups include, for example, phenyl and naphthyl, including 1-naphthyl and 2-naphthyl. In one embodiment, aryl groups are pendant. An example of a pendant ring is a phenyl group substituted with a phenyl group.

The term "heterocycle" refers to saturated and partially saturated heteroatom-containing ring radicals, where the heteroatoms may be selected from N, S, and O. The term "heterocycle" includes monocyclic 3-12 membered rings, as well as bicyclic 5-16 membered ring systems (which can include fused, bridged, or spiro, bicyclic ring systems). It does not include rings containing - O-O-. -O-S-, or -S-S- portions. Examples of saturated heterocycle groups include saturated 4- to 7-membered monocyclic groups containing 1 to 4 nitrogen atoms [e.g. pyrrolidinyl, imidazolidinyl, piperidinyl, pyrrolinyl, azetidinyl, piperazinyl, and pyrazolidinyl]; saturated 4 to 6-membered monocyclic groups containing 1 to 2 oxygen atoms and 1 to 3 nitrogen atoms [e.g. morpholinyl]; saturated 3 to 6-membered heteromonocyclic group containing 1 to 2 sulfur atoms and 1 to 3 nitrogen atoms [e.g., thiazolidinyl]. Examples of partially saturated heterocycle radicals include but are not limited to, dihydrothienyl, dihydropyranyl, dihydrofuryl, and dihydrothiazolyl. Examples of partially saturated and saturated heterocycle groups include but are not limited to, pyrrolidinyl, imidazolidinyl, piperidinyl, pyrrolinyl, pyrazolidinyl, piperazinyl, morpholinyl, tetrahydropyranyl, thiazolidinyl, dihydrothienyl, 2,3-dihydro-benzo[1,4]dioxanyl, indolinyl, isoindolinyl, dihydrobenzothienyl, dihydrobenzofuryl, isochromanyl, chromanyl, 1,2-dihydroquinolyl, 1,2,3,4- tetrahydro-isoquinolyl, 1 ,2,3,4-tetrahydro-quinolyl, 2,3,4,4a,9,9a-hexahydro-lH-3-aza-fluorenyl, 5,6,7- trihydro-1,2,4-triazolo[3,4-a]isoquinolyl, 3,4-dihydro-2H-benzo[l,4]oxazinyl, benzo[l,4]dioxanyl, 2,3- dihydro-lH-lλ'-benzo[d]isothiazol-6-yl, dihydropyranyl, dihydrofuryl and dihydrothiazolyl. "Bicyclic heterocycle" includes groups wherein the heterocyclic radical is fused with an aryl radical wherein the point of attachment is the heterocycle ring. "Bicyclic heterocycle" also includes heterocyclic radicals that are fused with a carbocycle radical. For example partially unsaturated condensed heterocyclic group containing 1 to 5 nitrogen atoms, for example, indoline, isoindoline, partially unsaturated condensed heterocyclic group containing 1 to 2 oxygen atoms and 1 to 3 nitrogen atoms, partially unsaturated condensed heterocyclic group containing 1 to 2 sulfur atoms and 1 to 3 nitrogen atoms, and saturated condensed heterocyclic group containing 1 to 2 oxygen or sulfur atoms.

Non-limiting examples of bicyclic heterocycles include:

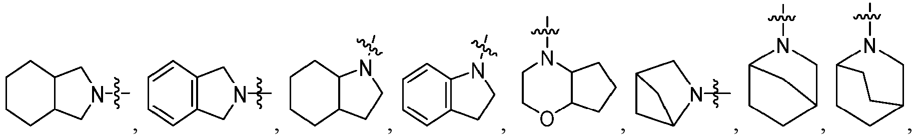

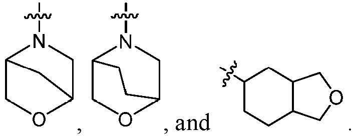

Unless otherwise drawn or clear from the context, the term "bicyclic heterocycle" includes cis and trans diastereomers. Non-limiting examples of chiral bicyclic heterocycles include:

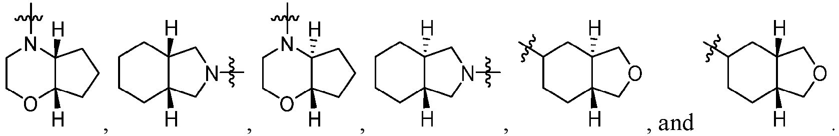

"Heteroaryl" refers to a stable monocyclic, bicyclic, or multicyclic aromatic ring which contains from 1 to 3, or in some embodiments from 1, 2, or 3 heteroatoms selected from N, O, S, B, and P (and typically selected from N, O, and S) with remaining ring atoms being carbon, or a stable bicyclic or tricyclic system containing at least one 5, 6, or 7 membered aromatic ring which contains from 1 to 3, or in some embodiments from 1 to 2, heteroatoms selected from N, O, S, B or P with remaining ring atoms being carbon. In one embodiment, the only heteroatom is nitrogen. In one embodiment, the only heteroatom is oxygen. In one embodiment, the only heteroatom is sulfur. Monocyclic heteroaryl groups typically have from 5 or 6 ring atoms. In some embodiments bicyclic heteroaryl groups are 8- to 10-membered heteroaryl groups, that is, groups containing 8 or 10 ring atoms in which one 5, 6, or 7 member aromatic ring is fused to a second aromatic or non-aromatic ring wherein the point of attachment is the aromatic ring. When the total number of S and O atoms in the heteroaryl group exceeds 1, these heteroatoms are not adjacent to one another. In one embodiment, the total number of S and O atoms in the heteroaryl group is not more than 2. In another embodiment, the total number of S and O atoms in the aromatic heterocycle is not more than 1. Examples of heteroaryl groups include, but are not limited to, pyridinyl (including, for example, 2-hydroxypyridinyl), imidazolyl, imidazopyridinyl, pyrimidinyl (including, for example, 4-hydroxypyrimidinyl), pyrazolyl, triazolyl, pyrazinyl, furyl, thienyl, isoxazolyl, thiazolyl, oxadiazolyl, oxazolyl, isothiazolyl, pyrrolyl, quinolinyl, isoquinolinyl, tetrahydroisoquinolinyl, indolyl, benzimidazolyl, benzofuranyl, cinnolinyl, indazolyl, indolizinyl, phthalazinyl, pyridazinyl, triazinyl, isoindolyl, pteridinyl, purinyl, oxadiazolyl, triazolyl, thiadiazolyl, thiadiazolyl, furazanyl, benzofurazanyl, benzothiophenyl, benzothiazolyl, benzoxazolyl, quinazolinyl, quinoxalinyl, naphthyridinyl, tetrahydrofuranyl, and furopyridinyl. Heteroaryl groups are optionally substituted independently with one or more substituents described herein. "Heteroaryloxy" is a heteroaryl group as described bound to the group it substituted via an oxygen, -O-, linker.

"Heterocycloalkyl" is a fully saturated heterocycle as defined herein. It may, for example, include 1, 2, 3, or 4 heteroatoms independently selected from N, S, O, Si and B with the remaining ring atoms being carbon. In a typical embodiment, nitrogen is the heteroatom. Monocyclic heterocycloalkyl groups typically have from 3 to about 8 ring atoms or from 4 to 6 ring atoms.

A "dosage form" means a unit of administration of an active agent. Examples of dosage forms include tablets, capsules, injections, suspensions, liquids, emulsions, implants, particles, spheres, creams, ointments, suppositories, inhalable forms, transdermal forms, buccal, sublingual, topical, gel, mucosal, and the like. A "dosage form" can also include an implant, for example an optical implant.

"Pharmaceutical compositions" are compositions comprising at least one active agent, and at least one other substance, such as a carrier. "Pharmaceutical combinations" are combinations of at least two active agents which may be combined in a single dosage form or provided together in separate dosage forms with instructions that the active agents are to be used together to treat any disorder described herein.

A "pharmaceutically acceptable salt" is a derivative of the disclosed compound in which the parent compound is modified by making inorganic and organic, pharmaceutically acceptable, acid or base addition salts thereof. The salts of the present compounds can be synthesized from a parent compound that contains a basic or acidic moiety by conventional chemical methods. Generally, such salts can be prepared by reacting free acid forms of these compounds with a stoichiometric amount of the appropriate base (such as Na, Ca, Mg, or K hydroxide, carbonate, bicarbonate, or the like), or by reacting free base forms of these compounds with a stoichiometric amount of the appropriate acid. Such reactions are typically carried out in water or in an organic solvent, or in a mixture of the two. Generally, non-aqueous media like ether, ethyl acetate, ethanol, isopropanol, or acetonitrile are typical, where practicable. Salts of the present compounds further include solvates of the compounds and of the compound salts.

Examples of pharmaceutically acceptable salts include, but are not limited to, mineral or organic acid salts of basic residues such as amines; alkali or organic salts of acidic residues such as carboxylic acids; and the like. The pharmaceutically acceptable salts include salts which are acceptable for human consumption and the quaternary ammonium salts of the parent compound formed, for example, from inorganic or organic acids. Examples of such salts include those derived from inorganic acids such as hydrochloric, hydrobromic, sulfuric, sulfamic, phosphoric, nitric and the like; and the salts prepared from organic acids such as acetic, propionic, succinic, glycolic, stearic, lactic, malic, tartaric, citric, ascorbic, pamoic, maleic, hydroxymaleic, phenylacetic, glutamic, benzoic, salicylic, mesylic, esylic, besylic, sulfanilic, 2-acetoxybenzoic, fumaric, toluenesulfonic, methanesulfonic, ethane disulfonic, oxalic, isethionic, HOOC-(CH₂)₁₋₄-COOH, and the like, or using a different acid that produces the same counterion. Lists of additional suitable salts may be found, e.g., in Remington's Pharmaceutical Sciences, 17th ed., Mack Publishing Company, Easton, Pa., p. 1418 (1985).

The term "carrier" applied to pharmaceutical compositions/combinations of the invention refers to a diluent, excipient, or vehicle with which an active compound is provided.

A "pharmaceutically acceptable excipient" means an excipient that is useful in preparing a pharmaceutical composition/combination that is generally safe, acceptable for human consumption, and neither biologically nor otherwise inappropriate for administration to a host, typically a human. In one embodiment, an excipient is used that is acceptable for veterinary use.

A "patient" or "host" or "subject" is a human or non-human animal in need of treatment or prevention of any of the disorders as specifically described herein, including but not limited to by modulation of the complement Factor D pathway or with a condition that is treatable with one of the compounds described herein. Typically the host is a human. A "patient" or "host" or "subject" also refers to for example, a mammal, primate (e.g., human), cows, sheep, goat, horse, dog, cat, rabbit, rat, mice, bird and the like.

"Providing a compound with at least one additional active agent," for example, in one embodiment can mean that the compound and the additional active agent(s) are provided simultaneously in a single dosage form, provided concomitantly in separate dosage forms, or provided in separate dosage forms for administration. In one embodiment, the compound administrations are separated by some amount of time that is within the time in which both the compound and the at least one additional active agent are within the blood stream of a patient. In certain embodiments the compound and the additional active agent need not be prescribed for a patient by the same medical care worker. In certain embodiments the additional active agent or agents need not require a prescription. Administration of the compound or the at least one additional active agent can occur via any appropriate route, for example, oral tablets, oral capsules, oral liquids, inhalation, injection, suppositories, parenteral, sublingual, buccal, intravenous, intraaortal, transdermal, polymeric controlled delivery, non-polymeric controlled delivery, nano or microparticles, liposomes, and/or topical contact. In one embodiment, the instructions for administration in a form of combination therapy is provided in the drug labeling.

A "therapeutically effective amount" of a pharmaceutical composition/combination of this invention means an amount effective, when administered to a host, provides a therapeutic benefit such as an amelioration of symptoms or reduction or dimunition of the disease itself. In one embodiment, a therapeutically effective amount is an amount sufficient to prevent a significant increase or will significantly reduce the detectable level of complement Factor D in the patient's blood, serum, or tissues.

### N-Oxides

In certain embodiments, any of the active compounds can be provided in its N-oxide form to a patient in need thereof. In one embodiment, an N-oxide of an active compound or a precursor of the active compound is used in a manufacturing scheme. In yet another embodiment, the N-oxide is a metabolite of administration of one of the active compounds herein, and may have independent activity. The N-oxide can be formed by treating the compound of interest with an oxidizing agent, for example a suitable peroxyacid or peroxide, to generate an N-oxide compound. For example, a heteroaryl group, for example a pyridyl group, can be treated with an oxidizing agent such as sodium percarbonate in the presence of a rhenium-based catalyst under mild reaction conditions to generate an N-oxide compound. A person skilled in the art will understand that appropriate protecting groups may be necessary to carry out the chemistry. See, Jain, S.L. et al., "Rhenium-Catalyzed Highly Efficient Oxidations of Tertiary Nitrogen Compounds to N-Oxides Using Sodium Percarbonate as Oxygen Source, Synlett, 2261-2663, 2006.

In one embodiment the N-oxide is in the A-Ring. In one embodiment the N-oxide is in the B-Ring. In one embodiment the N-oxide is on the R³² group.
In other aspects of the present invention, any of the active compounds with a sulfur can be provided in its sulfoxide or sulfone form to a patient in need thereof. In a different embodiment, a sulfoxide or sulfone of one of the active compounds or a precursor of the active compound is used in a manufacturing scheme. A sulfur atom in a selected compound as described herein can be oxidized to form a sulfoxide 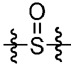 or a sulfone 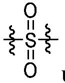 using known methods. For example, the compound 1,3,5-triazo-2,4,6-triphosphorine-2,2,4,4,6,6-tetrachloride (TAPC) is an efficient promoter for the oxidation of sulfides to sulfoxides. *See,* Bahrami, M. et al., "TAPC-Promoted Oxidation of sulfides and Deoxygenation of Sulfoxides", J. Org. Chem., 75, 6208-6213 (2010). Oxidation of sulfides with 30% hydrogen peroxide catalyzed by tantalum carbide provides sulfoxides in high yields, see, Kirihara, A., et al., "Tantalum Carbide or Niobium Carbide Catalyzed Oxidation of Sulfides with Hydrogen Peroxide: Highly Efficient and Chemoselective Syntheses of Sulfoxides and Sulfones", Synlett, 1557-1561 (2010). Sulfides can be oxidized to sulfones using, for example, niobium carbide as the catalyst, see, Kirihara, A., et al., "Tantalum Cardide or Niobium Carbide Catalyzed Oxidation of Sulfides with Hydrogen Peroxide: Highly Efficient and Chemoselective Syntheses of Sulfoxides and Sulfones", Synlett, 1557-1561 (2010). Urea-hydrogen peroxide adduct is a stable inexpensive and easily handled reagent for the oxidation of sulfides to sulfones, see Varma, R.S. and Naicker, K.P., "The Urea-Hydrogen Peroxide Complex: Solid-State Oxidative Protocols for Hydroxylated Aldehydes and Ketones (Dakin Reaction), Nitriles, Sulfides, and Nitrogen Heterocycles", Org. Lett., 1, 189-191 (1999). One skilled in the art will appreciate that other heteroatoms, such as nitrogen, may need to be protected and then deprotected while carrying out the oxidation of a sulfur atom to produce the desired compound.

### COMPOUNDS

The invention provides a compound selected from: 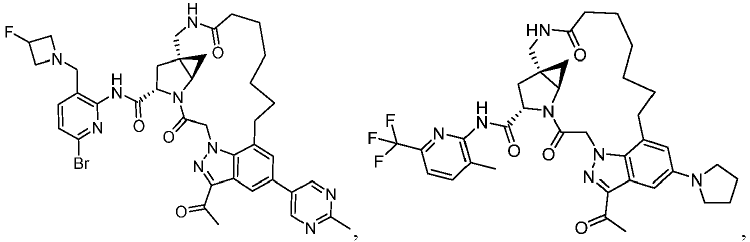 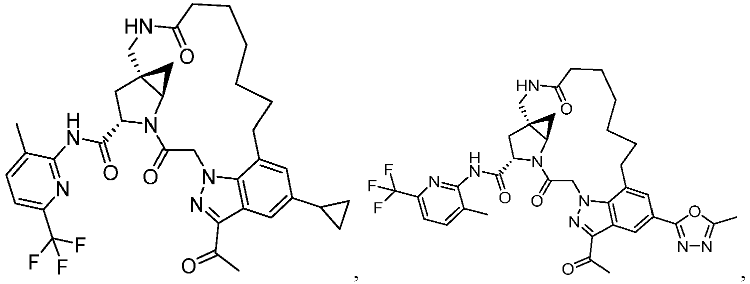 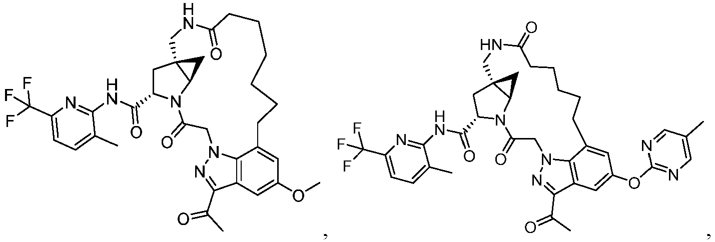 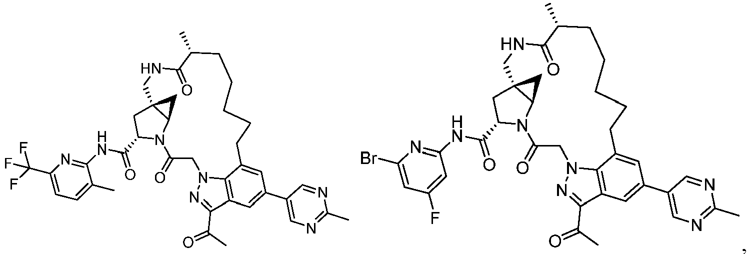 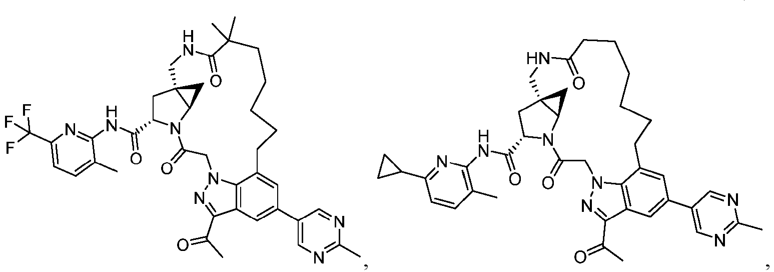 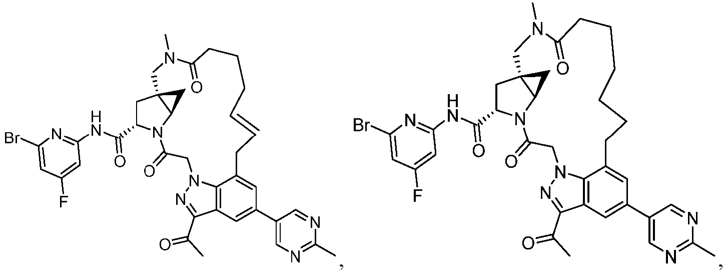 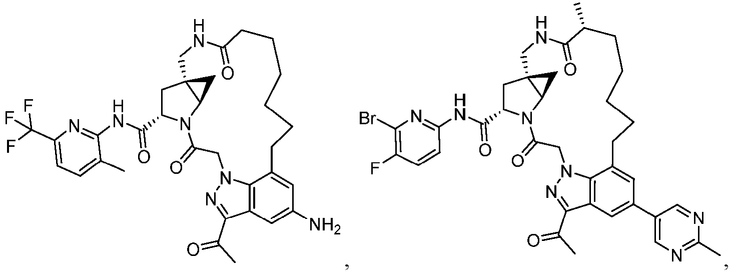 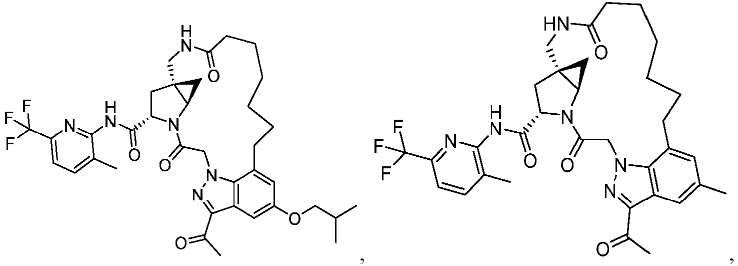 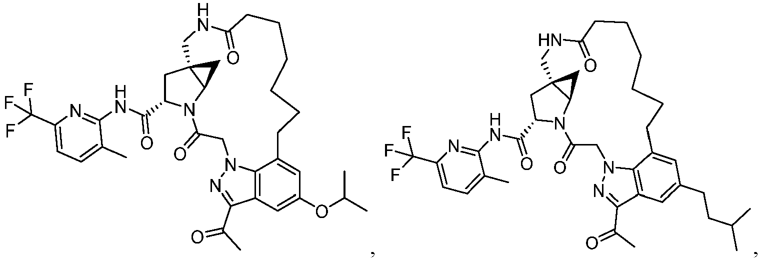 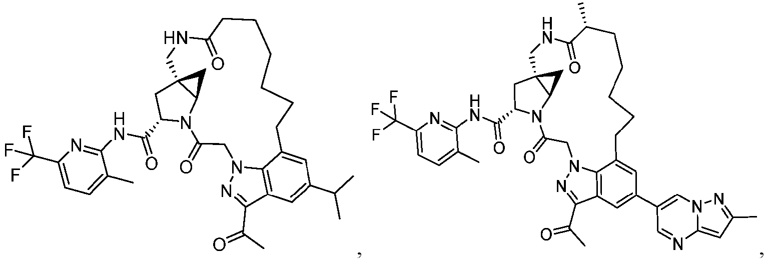 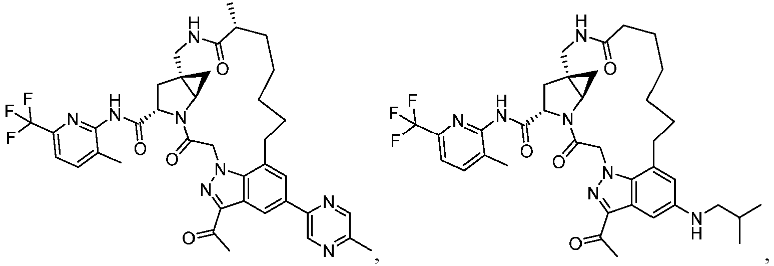 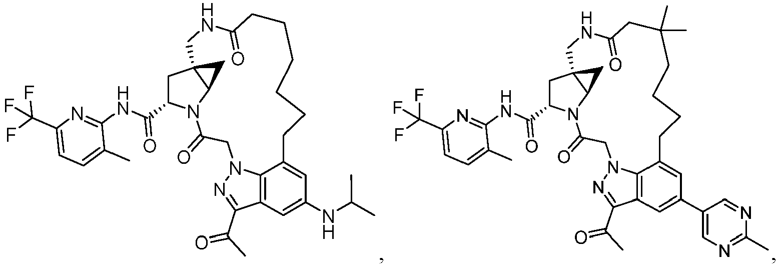 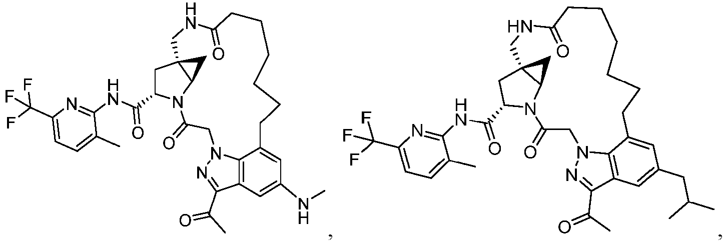 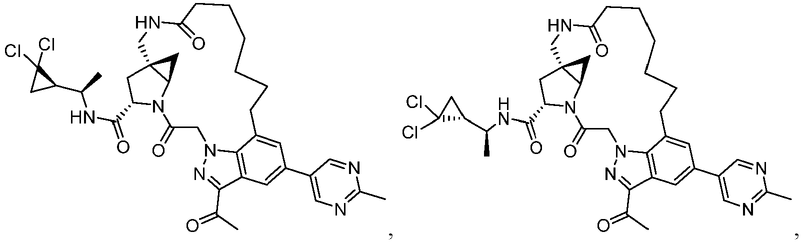 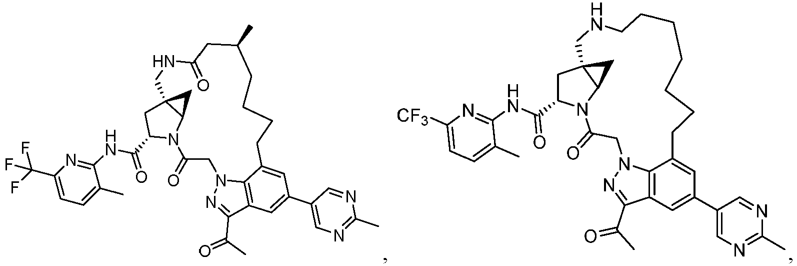 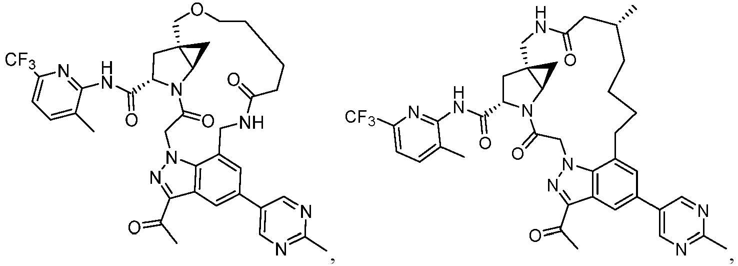 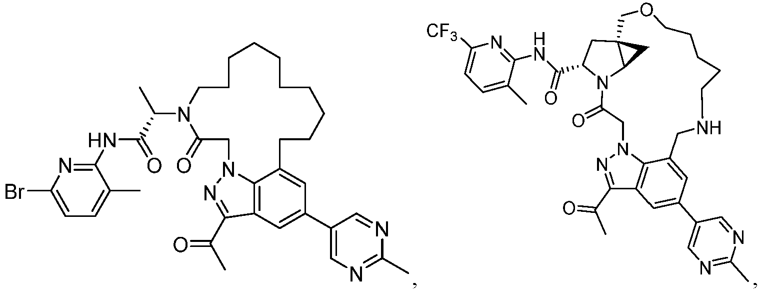 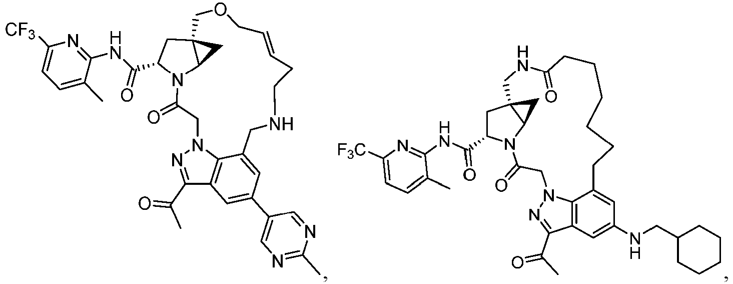 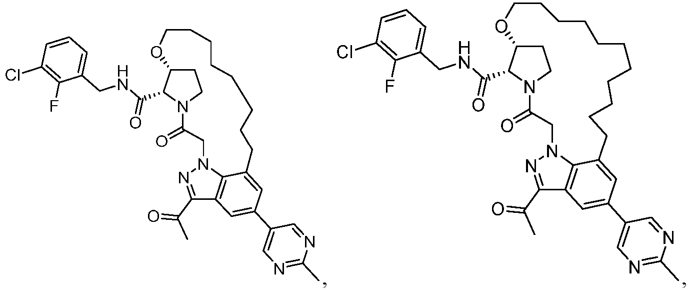 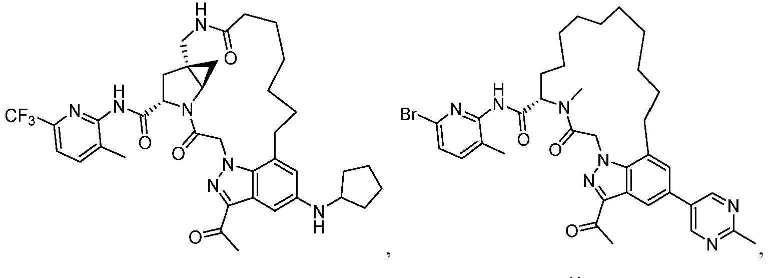 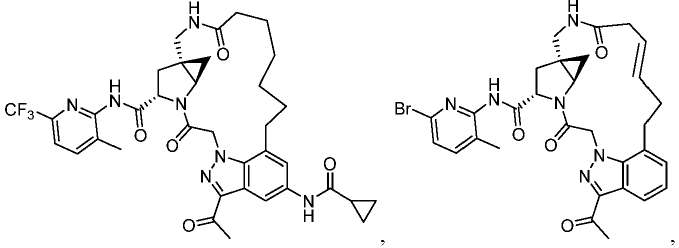 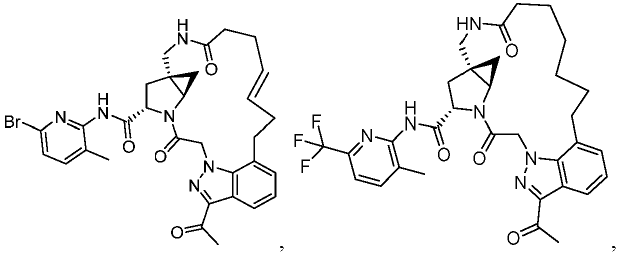 and 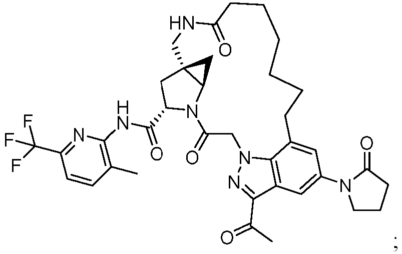 or a pharmaceutically acceptable salt thereof.

### PHARMACEUTICAL PREPARATIONS

Active compounds described herein can be administered to a host in need thereof as the neat chemical, but are more typically administered as a pharmaceutical composition that includes an effective amount for a host, typically a human, in need of such treatment of an active compound as described herein or its pharmaceutically acceptable salt. Thus, in one embodiment, the disclosure provides pharmaceutical compositions comprising an effective amount of compound or pharmaceutically acceptable salt thereof together with at least one pharmaceutically acceptable carrier for any of the uses described herein. The pharmaceutical composition may contain a compound or salt as the only active agent, or, in an alternative embodiment, the compound and at least one additional active agent.

An effective amount of an active compound as described herein, or the active compound described herein in combination or alternation with, or preceded by, concomitant with or followed by another active agent, can be used in an amount sufficient to (a) inhibit the progression of a disorder mediated by the complement pathway, including an inflammatory, immune, including an autoimmune, disorder or complement Factor D related disorder; (b) cause a regression of an inflammatory, immune, including an autoimmune, disorder or complement Factor D related disorder; (c) cause a cure of an inflammatory, immune, including an autoimmune, disorder or complement Factor D related disorder; or inhibit or prevent the development of an inflammatory, immune, including an autoimmune, disorder or complement Factor D related disorder.

Accordingly, an effective amount of an active compound or its salt or composition described herein will provide a sufficient amount of the active agent when administered to a patient to provide a clinical benefit.

The exact amount of the active compound or pharmaceutical composition described herein to be delivered to the host, typically a human, in need thereof, will be determined by the health care provider to achieve the desired clinical benefit.

In certain embodiments the pharmaceutical composition is in a dosage form that contains from about 0.1 mg to about 2000 mg, from about 10 mg to about 1000 mg, from about 100 mg to about 800 mg, or from about 200 mg to about 600 mg of the active compound and optionally from about 0.1 mg to about 2000 mg, from about 10 mg to about 1000 mg, from about 100 mg to about 800 mg, or from about 200 mg to about 600 mg of an additional active agent in a unit dosage form. Examples are dosage forms with at least about 0.5, 1, 1.5, 2, 2.5, 3, 3.5, 4, 4.5, 5, 10, 15, 20, 25, 50, 75, 100, 150, 200, 250, 300, 350, 400, 450, 500, 550, 600, 650, 700, 750, 800, 900, 1000, 1100, 1200, 1250, 1300, 1400, 1500, or 1600 mg of active compound, or its salt. In one embodiment, the dosage form has at least about 1mg, 5 mg, 10 mg, 25 mg, 50 mg, 75 mg, 100 mg, 200 mg, 400 mg, 500 mg, 600 mg, 1000mg, 1200 mg, or 1600 mg of active compound, or its salt. The amount of active compound in the dosage form is calculated without reference to the salt. The dosage form can be administered, for example, once a day (q.d.), twice a day (b.i.d.), three times a day (t.i.d.), four times a day (q.i.d.), once every other day (Q2d), once every third day (Q3d), as needed, or any dosage schedule that provides treatment of a disorder described herein.

The pharmaceutical composition may for example include a molar ratio of the active compound and an additional active agent that achieves the desired result. For example, the pharmaceutical composition may contain a molar ratio of about 0.5:1, about 1:1, about 2:1, about 3:1 or from about 1.5:1 to about 4:1 of an additional active agent in combination with the active compound (additional active agent: active compound), or its salt, described herein. In one embodiment, the additional active agent is an anti-inflammatory or immunosuppressing agent.

Compounds disclosed herein or used as described herein may be administered orally, topically, parenterally, by inhalation or spray, sublingually, via implant, including ocular implant, transdermally, via buccal administration, rectally, as an ophthalmic solution, injection, including ocular injection, intravenous, intra-aortal, intracranial, subdermal, intraperitoneal, subcutaneous, transnasal, sublingual, intrathecal, or rectal or by other means, in dosage unit formulations containing conventional pharmaceutically acceptable carriers. For ocular delivery, the compound can be administered, as desired, for example, as a solution, suspension, or other formulation via intravitreal, intrastromal, intracameral, sub-tenon, sub-retinal, retro-bulbar, peribulbar, suprachorodial, subchorodial, chorodial, conjunctival, subconjunctival, episcleral, periocular, transscleral, retrobulbar, posterior juxtascleral, circumcorneal, or tear duct injections, or through a mucus, mucin, or a mucosal barrier, in an immediate or controlled release fashion or via an ocular device, injection, or topically administered formulation, for example a solution or suspension provided as an eye drop.

The pharmaceutical composition may be formulated as any pharmaceutically useful form, e.g., as an aerosol, a cream, a gel, a gel cap, a pill, a microparticle, a nanoparticle, an injection or infusion solution, a capsule, a tablet, a syrup, a transdermal patch, a subcutaneous patch, a dry powder, an inhalation formulation, in a medical device, suppository, buccal, or sublingual formulation, parenteral formulation, or an ophthalmic solution or suspension. Some dosage forms, such as tablets and capsules, are subdivided into suitably sized unit doses containing appropriate quantities of the active components, e.g., an effective amount to achieve the desired purpose.

Pharmaceutical compositions, and methods of manufacturing such compositions, suitable for administration as contemplated herein are known in the art. Examples of known techniques include, for example, US Patent Nos. 4,983,593, 5,013,557, 5,456,923, 5,576,025, 5,723,269, 5,858,411, 6,254,889, 6,303,148, 6,395,302, 6,497,903, 7,060,296, 7,078,057, 7,404,828, 8,202,912, 8,257,741, 8,263,128, 8,337,899, 8,431,159, 9,028,870, 9,060,938, 9,211,261, 9,265,731, 9,358,478, and 9,387,252.

The pharmaceutical compositions contemplated here can optionally include a carrier. Carriers must be of sufficiently high purity and sufficiently low toxicity to render them suitable for administration to the patient being treated. The carrier can be inert or it can possess pharmaceutical benefits of its own. The amount of carrier employed in conjunction with the compound is sufficient to provide a practical quantity of material for administration per unit dose of the compound. Classes of carriers include, but are not limited to binders, buffering agents, coloring agents, diluents, disintegrants, emulsifiers, fillers, flavorants, glidents, lubricants, pH modifiers, preservatives, stabilizers, surfactants, solubilizers, tableting agents, and wetting agents. Some carriers may be listed in more than one class, for example vegetable oil may be used as a lubricant in some formulations and a diluent in others. Exemplary pharmaceutically acceptable carriers include sugars, starches, celluloses, powdered tragacanth, malt, gelatin; talc, and vegetable oils. Examples of other matrix materials, fillers, or diluents include lactose, mannitol, xylitol, microcrystalline cellulose, calcium diphosphate, and starch. Examples of surface active agents include sodium lauryl sulfate and polysorbate 80. Examples of drug complexing agents or solubilizers include the polyethylene glycols, caffeine, xanthene, gentisic acid and cylodextrins. Examples of disintegrants include sodium starch gycolate, sodium alginate, carboxymethyl cellulose sodium, methyl cellulose, colloidal silicon dioxide, and croscarmellose sodium. Examples of binders include methyl cellulose, microcrystalline cellulose, starch, and gums such as guar gum, and tragacanth. Examples of lubricants include magnesium stearate and calcium stearate. Examples of pH modifiers include acids such as citric acid, acetic acid, ascorbic acid, lactic acid, aspartic acid, succinic acid, phosphoric acid, and the like; bases such as sodium acetate, potassium acetate, calcium oxide, magnesium oxide, trisodium phosphate, sodium hydroxide, calcium hydroxide, aluminum hydroxide, and the like, and buffers generally comprising mixtures of acids and the salts of said acids. Optional other active agents may be included in a pharmaceutical composition, which do not substantially interfere with the activity of the compound of the present invention.

In certain embodiments, the pharmaceutical composition for administration further includes a compound or salt as described herein and optionally comprises one or more of a phosphoglyceride; phosphatidylcholine; dipalmitoyl phosphatidylcholine (DPPC); dioleylphosphatidyl ethanolamine (DOPE); dioleyloxypropyltriethylammonium (DOTMA); dioleoylphosphatidylcholine; cholesterol; cholesterol ester; diacylglycerol; diacylglycerolsuccinate; diphosphatidyl glycerol (DPPG); hexanedecanol; fatty alcohol such as polyethylene glycol (PEG); polyoxyethylene-9-lauryl ether; a surface active fatty acid, such as palmitic acid or oleic acid; fatty acid; fatty acid monoglyceride; fatty acid diglyceride; fatty acid amide; sorbitan trioleate (Span^{®}85) glycocholate; sorbitan monolaurate (Span^{®}20); polysorbate 20 (Tween^{®}20); polysorbate 60 (Tween^{®}60); polysorbate 65 (Tween^{®}65); polysorbate 80 (Tween^{®}80); polysorbate 85 (Tween^{®}85); polyoxyethylene monostearate; surfactin; a poloxomer; a sorbitan fatty acid ester such as sorbitan trioleate; lecithin; lysolecithin; phosphatidylserine; phosphatidylinositol; sphingomyelin; phosphatidylethanolamine (cephalin); cardiolipin; phosphatidic acid; cerebroside; dicetylphosphate; dipalmitoylphosphatidylglycerol; stearylamine; dodecylamine; hexadecyl-amine; acetyl palmitate; glycerol ricinoleate; hexadecyl sterate; isopropyl myristate; tyloxapol; poly(ethylene glycol)5000-phosphatidylethanolamine; poly(ethylene glycol)400-monostearate; phospholipid; synthetic and/or natural detergent having high surfactant properties; deoxycholate; cyclodextrin; chaotropic salt; ion pairing agent; glucose, fructose, galactose, ribose, lactose, sucrose, maltose, trehalose, cellbiose, mannose, xylose, arabinose, glucoronic acid, galactoronic acid, mannuronic acid, glucosamine, galatosamine, and neuramic acid; pullulan, cellulose, microcrystalline cellulose, hydroxypropyl methylcellulose (HPMC), hydroxycellulose (HC), methylcellulose (MC), dextran, cyclodextran, glycogen, hydroxyethylstarch, carageenan, glycon, amylose, chitosan, N,O-carboxylmethylchitosan, algin and alginic acid, starch, chitin, inulin, konjac, glucommannan, pustulan, heparin, hyaluronic acid, curdlan, and xanthan, mannitol, sorbitol, xylitol, erythritol, maltitol, and lactitol, a pluronic polymer, polyethylene, polycarbonate (e.g. poly(1,3-dioxan-2one)), polyanhydride (e.g. poly(sebacic anhydride)), polypropylfumerate, polyamide (e.g. polycaprolactam), polyacetal, polyether, polyester (e.g., polylactide, polyglycolide, polylactide-co-glycolide, polycaprolactone, polyhydroxyacid (e.g. poly((β-hydroxyalkanoate))), poly(orthoester), polycyanoacrylate, polyvinyl alcohol, polyurethane, polyphosphazene, polyacrylate, polymethacrylate, polyurea, polystyrene, and polyamine, polylysine, polylysine-PEG copolymer, and poly(ethyleneimine), poly(ethylene imine)-PEG copolymer, glycerol monocaprylocaprate, propylene glycol, Vitamin E TPGS (also known as d-α-Tocopheryl polyethylene glycol 1000 succinate), gelatin, titanium dioxide, polyvinylpyrrolidone (PVP), hydroxypropyl methyl cellulose (HPMC), hydroxypropyl cellulose (HPC), methyl cellulose (MC), block copolymers of ethylene oxide and propylene oxide (PEO/PPO), polyethyleneglycol (PEG), sodium carboxymethylcellulose (NaCMC), hydroxypropylmethyl cellulose acetate succinate (HPMCAS).

In some embodiments, the pharmaceutical preparation may include polymers for controlled delivery of the described compounds, including, but not limited to pluronic polymers, polyesters (e.g., polylactic acid, poly(lactic-co-glycolic acid), polycaprolactone, polyvalerolactone, poly(1,3-dioxan-2one)); polyanhydrides (e.g., poly(sebacic anhydride)); polyethers (e.g., polyethylene glycol); polyurethanes; polymethacrylates; polyacrylates; and polycyanoacrylates. In some embodiments, polymers may be modified with polyethylene glycol (PEG), with a carbohydrate, and/or with acyclic polyacetals derived from polysaccharides. See, e.g., Papisov, 2001, ACS Symposium Series, 786:301.

The compounds of the present invention can be formulated as particles. In one embodiment the particles are or include microparticles. In an alternative embodiment the particles are or include nanoparticles.

In an additional alternative embodiment, common techniques for preparing particles include, but are not limited to, solvent evaporation, solvent removal, spray drying, phase inversion, coacervation, and low temperature casting. Suitable methods of particle formulation are briefly described below. Pharmaceutically acceptable excipients, including pH modifying agents, disintegrants, preservatives, and antioxidants, can optionally be incorporated into the particles during particle formation.

In one embodiment, the particles are derived through a solvent evaporation method. In this method, a compound described herein (or polymer matrix and one or more compounds described herein) is dissolved in a volatile organic solvent, such as methylene chloride. The organic solution containing a compound described herein is then suspended in an aqueous solution that contains a surface active agent such as poly(vinyl alcohol). The resulting emulsion is stirred until most of the organic solvent evaporated, leaving solid nanoparticles or microparticles. The resulting nanoparticles or microparticles are washed with water and dried overnight in a lyophilizer. Nanoparticles with different sizes and morphologies can be obtained by this method.

Pharmaceutical compositions which contain labile polymers, such as certain polyanhydrides, may degrade during the fabrication process due to the presence of water. For these polymers, methods which are performed in completely or substantially anhydrous organic solvents can be used to make the particles.

Solvent removal can also be used to prepare particles from a compound that is hydrolytically unstable. In this method, the compound (or polymer matrix and one or more compounds) is dispersed or dissolved in a volatile organic solvent such as methylene chloride. This mixture is then suspended by stirring in an organic oil (such as silicon oil) to form an emulsion. Solid particles form from the emulsion, which can subsequently be isolated from the supernatant. The external morphology of spheres produced with this technique is highly dependent on the identity of the drug.

In one embodiment an active compound as described herein is administered to a patient in need thereof as particles formed by solvent removal. In another embodiment the present invention provides particles formed by solvent removal comprising a compound of the present invention and one or more pharmaceutically acceptable excipients as defined herein. In another embodiment the particles formed by solvent removal comprise a compound of the present invention and an additional therapeutic agent. In a further embodiment the particles formed by solvent removal comprise a compound of the present invention, an additional therapeutic agent, and one or more pharmaceutically acceptable excipients. In another embodiment any of the described particles formed by solvent removal can be formulated into a tablet and then coated to form a coated tablet. In an alternative embodiment the particles formed by solvent removal are formulated into a tablet but the tablet is uncoated.

In one embodiment, the particles are derived by spray drying. In this method, a compound (or polymer matrix and one or more compounds) is dissolved in an organic solvent such as methylene chloride. The solution is pumped through a micronizing nozzle driven by a flow of compressed gas, and the resulting aerosol is suspended in a heated cyclone of air, allowing the solvent to evaporate from the micro droplets, forming particles. Microparticles and nanoparticles can be obtained using this method.

In one embodiment an active compound as described herein is administered to a patient in need thereof as a spray dried dispersion (SDD). In another embodiment the present invention provides a spray dried dispersion (SDD) comprising a compound of the present invention and one or more pharmaceutically acceptable excipients as defined herein. In another embodiment the SDD comprises a compound of the present invention and an additional therapeutic agent. In a further embodiment the SDD comprises a compound of the present invention, an additional therapeutic agent, and one or more pharmaceutically acceptable excipients. In another embodiment any of the described spray dried dispersions can be coated to form a coated tablet. In an alternative embodiment the spray dried dispersion is formulated into a tablet but is uncoated. Particles can be formed from the active compound as described herein using a phase inversion method. In this method, the compound (or polymer matrix and one or more active compounds) is dissolved in a suitable solvent, and the solution is poured into a strong non-solvent for the compound to spontaneously produce, under favorable conditions, microparticles or nanoparticles. The method can be used to produce nanoparticles in a wide range of sizes, including, for example, from nanoparticles to microparticles, typically possessing a narrow particle size distribution.

In one embodiment, an active compound as described herein is administered to a patient in need thereof as particles formed by phase inversion. In another embodiment the present invention provides particles formed by phase inversion comprising a compound of the present invention and one or more pharmaceutically acceptable excipients as defined herein. In another embodiment the particles formed by phase inversion comprise a compound of the present invention and an additional therapeutic agent. In a further embodiment the particles formed by phase inversion comprise a compound of the present invention, an additional therapeutic agent, and one or more pharmaceutically acceptable excipients. In another embodiment any of the described particles formed by phase inversion can be formulated into a tablet and then coated to form a coated tablet. In an alternative embodiment the particles formed by phase inversion are formulated into a tablet but the tablet is uncoated.

Techniques for particle formation using coacervation are known in the art, for example, as described in GB-B-929 406; GB-B-929 40 1; and U.S. Patent Nos. 3,266,987, 4,794,000, and 4,460,563. Coacervation involves the separation of a compound (or polymer matrix and one or more compounds) solution into two immiscible liquid phases. One phase is a dense coacervate phase, which contains a high concentration of the compound, while the second phase contains a low concentration of the compound. Within the dense coacervate phase, the compound forms nanoscale or microscale droplets, which harden into particles. Coacervation may be induced by a temperature change, addition of a non-solvent or addition of a micro-salt (simple coacervation), or by the addition of another polymer thereby forming an interpolymer complex (complex coacervation).

In one embodiment an active compound as described herein is administered to a patient in need thereof as particles formed by coacervation. In another embodiment the present invention provides particles formed by coacervation comprising a compound of the present invention and one or more pharmaceutically acceptable excipients as defined herein. In another embodiment the particles formed by coacervation comprise a compound of the present invention and an additional therapeutic agent. In a further embodiment the particles formed by coacervation comprise a compound of the present invention, an additional therapeutic agent, and one or more pharmaceutically acceptable excipients. In another embodiment any of the described particles formed by coacervation can be formulated into a tablet and then coated to form a coated tablet. In an alternative embodiment the particles formed by coacervation are formulated into a tablet but the tablet is uncoated.

Methods for very low temperature casting of controlled release microspheres are described in U.S. Patent No. 5,019,400 to Gombotz et al. In this method, the compound is dissolved in a solvent. The mixture is then atomized into a vessel containing a liquid non-solvent at a temperature below the freezing point of the drug solution which freezes the compound droplets. As the droplets and non-solvent for the compound are warmed, the solvent in the droplets thaws and is extracted into the non-solvent, hardening the microspheres.

In one embodiment, a compound of the present invention is administered to a patient in need thereof as particles formed by low temperature casting. In another embodiment the present invention provides particles formed by low temperature casting comprising a compound of the present invention and one or more pharmaceutically acceptable excipients as defined herein. In another embodiment the particles formed by low temperature casting comprise a compound of the present invention and an additional therapeutic agent. In a further embodiment the particles formed by low temperature casting comprise a compound of the present invention, an additional therapeutic agent, and one or more pharmaceutically acceptable excipients. In another embodiment any of the described particles formed by low temperature casting can be formulated into a tablet and then coated to form a coated tablet. In an alternative embodiment the particles formed by low temperature casting are formulated into a tablet but the tablet is uncoated.

The pharmaceutical compositions can be formulated for oral administration. These compositions can contain any amount of active compound that achieves the desired result, for example between 0.1 and 99 weight % (wt.%) of the compound and usually at least about 5 wt.% of the compound. Some embodiments contain at least about 10%, 15%, 20%, 25 wt.% to about 50 wt. % or from about 5 wt.% to about 75 wt.% of the compound.

Pharmaceutical compositions suitable for rectal administration are typically presented as unit dose suppositories. These may be prepared by admixing the active compound with one or more conventional solid carriers, for example, cocoa butter, and then shaping the resulting mixture.

Pharmaceutical compositions suitable for topical application to the skin preferably take the form of an ointment, cream, lotion, paste, gel, spray, aerosol, or oil. Carriers which may be used include petroleum jelly, lanoline, polyethylene glycols, alcohols, transdermal enhancers, and combinations of two or more thereof.

Pharmaceutical compositions suitable for transdermal administration may be presented as discrete patches adapted to remain in intimate contact with the epidermis of the recipient for a prolonged period of time. Pharmaceutical compositions suitable for transdermal administration may also be delivered by iontophoresis (*see,* for example, Pharmaceutical Research 3 (6):318 (1986)) and typically take the form of an optionally buffered aqueous solution of the active compound. In one embodiment, microneedle patches or devices are provided for delivery of drugs across or into biological tissue, particularly the skin. The microneedle patches or devices permit drug delivery at clinically relevant rates across or into skin or other tissue barriers, with minimal or no damage, pain, or irritation to the tissue.

Pharmaceutical compositions suitable for administration to the lungs can be delivered by a wide range of passive breath driven and active power driven single/-multiple dose dry powder inhalers (DPI). The devices most commonly used for respiratory delivery include nebulizers, metered-dose inhalers, and dry powder inhalers. Several types of nebulizers are available, including jet nebulizers, ultrasonic nebulizers, and vibrating mesh nebulizers. Selection of a suitable lung delivery device depends on parameters, such as nature of the drug and its formulation, the site of action, and pathophysiology of the lung.

Many methods and devices for drug delivery to the eye are known in the art. Non-limiting examples are described in the following patents and patent applications. Examples are US 8,192,408 titled "Ocular trocar assembly" (Psivida Us, Inc.); US 7,585,517 titled "Transcleral delivery" (Macusight, Inc.); US 5,710,182 and US 5,795,913 titled "Ophthalmic composition" (Santen OY); US 8,663,639 titled "Formulations for treating ocular diseases and conditions", US 8,486,960 titled "Formulations and methods for vascular permeability-related diseases or conditions", US 8,367,097 and US 8,927,005 titled "Liquid formulations for treatment of diseases or conditions", US 7,455,855 titled "Delivering substance and drug delivery system using the same" (Santen Pharmaceutical Co., Ltd.); WO/2011/050365 titled "Conformable Therapeutic Shield For Vision and Pain" and WO/2009/145842 titled "Therapeutic Device for Pain Management and Vision" (Forsight Labs, LLC); US 9,066,779 and US 8,623,395 titled "Implantable therapeutic device", WO/2014/160884 titled "Ophthalmic Implant for Delivering Therapeutic Substances", US 8,399,006, US 8,277,830, US 8,795,712, US 8,808,727, US 8,298,578, and WO/2010/088548 titled "Posterior segment drug delivery", WO/2014/152959 and US20140276482 titled "Systems for Sustained Intraocular Delivery of Low Solubility Compounds from a Port Delivery System Implant", US 8,905,963 and US 9,033,911 titled "Injector apparatus and method for drug delivery", WO/2015/057554 titled "Formulations and Methods for Increasing or Reducing Mucus", US 8,715,712 and US 8,939,948 titled "Ocular insert apparatus and methods", WO/2013/116061 titled "Insertion and Removal Methods and Apparatus for Therapeutic Devices", WO/2014/066775 titled "Ophthalmic System for Sustained Release of Drug to the Eye", WO/2015/085234 and WO/2012/019176 titled "Implantable Therapeutic Device", WO/2012/065006 titled "Methods and Apparatus to determine Porous Structures for Drug Delivery", WO/2010/141729 titled "Anterior Segment Drug Delivery", WO/2011/050327 titled "Corneal Denervation for Treatment of Ocular Pain", WO/2013/022801 titled "Small Molecule Delivery with Implantable Therapeutic Device", WO/2012/019047 titled "Subconjunctival Implant for Posterior Segment Drug Delivery", WO/2012/068549 titled "Therapeutic Agent Formulations for Implanted Devices", WO/2012/019139 titled " Combined Delivery Methods and Apparatus", WO/2013/040426 titled "Ocular Insert Apparatus and Methods", WO/2012/019136 titled "Injector Apparatus and Method for Drug Delivery", WO/2013/040247 titled "Fluid Exchange Apparatus and Methods" (ForSight Vision4, Inc.).

Additional non-limiting examples of how to deliver the active compounds are provided in WO/2015/085251 titled "Intracameral Implant for Treatment of an Ocular Condition" (Envisia Therapeutics, Inc.); WO/2011/008737 titled "Engineered Aerosol Particles, and Associated Methods", WO/2013/082111 titled "Geometrically Engineered Particles and Methods for Modulating Macrophage or Immune Responses", WO/2009/132265 titled "Degradable compounds and methods of use thereof, particularly with particle replication in non-wetting templates", WO/2010/099321 titled "Interventional drug delivery system and associated methods", WO/2008/100304 titled "Polymer particle composite having high fidelity order, size, and shape particles", WO/2007/024323 titled "Nanoparticle fabrication methods, systems, and materials" (Liquidia Technologies, Inc. and the University of North Carolina at Chapel Hill); WO/2010/009087 titled "Iontophoretic Delivery of a Controlled-Release Formulation in the Eye", (Liquidia Technologies, Inc. and Eyegate Pharmaceuticals, Inc.) and WO/2009/132206 titled "Compositions and Methods for Intracellular Delivery and Release of Cargo", WO/2007/133808 titled "Nano-particles for cosmetic applications", WO/2007/056561 titled "Medical device, materials, and methods", WO/2010/065748 titled "Method for producing patterned materials", WO/2007/081876 titled "Nanostructured surfaces for biomedical/biomaterial applications and processes thereof" (Liquidia Technologies, Inc.).

Additional non-limiting examples of methods and devices for drug delivery to the eye include, for example, WO2011/106702 and US 8,889,193 titled "Sustained delivery of therapeutic agents to an eye compartment", WO2013/138343 and US 8,962,577 titled "Controlled release formulations for the delivery of HIF-1 inhibitors", WO/2013/138346 and US2013/0272994 titled "Non-Linear Multiblock Copolymer-Drug Conjugates for the Delivery of Active Agents", WO2005/072710 and US 8,957,034 titled "Drug and Gene Carrier Particles that Rapidly Move Through Mucus Barriers", WO2008/030557, US2010/0215580, US2013/0164343 titled "Compositions and Methods for Enhancing Transport Through Mucous", WO2012/061703, US2012/0121718, and US2013/0236556 titled "Compositions and Methods Relating to Reduced Mucoadhesion", WO2012/039979 and US2013/0183244 titled "Rapid Diffusion of Large Polymeric Nanoparticles in the Mammalian Brain", WO2012/109363 and US2013/0323313 titled "Mucus Penetrating Gene Carriers", WO 2013/090804 and US2014/0329913 titled "Nanoparticles with enhanced mucosal penetration or decreased inflammation", WO2013/110028 titled "Nanoparticle formulations with enhanced mucosal penetration", WO2013/166498 and US2015/0086484 titled "Lipid-based drug carriers for rapid penetration through mucus linings" (The Johns Hopkins University); WO2013/166385 titled "Pharmaceutical Nanoparticles Showing Improved Mucosal Transport", US2013/0323179 titled "Nanocrystals, Compositions, And Methods that Aid Particle Transport in Mucus" (The Johns Hopkins University and Kala Pharmaceuticals, Inc.); WO/2015/066444 titled "Compositions and methods for ophthalmic and/or other applications", WO/2014/020210 and WO/2013/166408 titled "Pharmaceutical nanoparticles showing improved mucosal transport" (Kala Pharmaceuticals, Inc.); US 9,022,970 titled "Ophthalmic injection device including dosage control device", WO/2011/153349 titled "Ophthalmic compositions comprising pbo-peo-pbo block copolymers", WO/2011/140203 titled "Stabilized ophthalmic galactomannan formulations", WO/2011/068955 titled "Ophthalmic emulsion" , WO/2011/037908 titled "Injectable aqueous ophthalmic composition and method of use therefor", US2007/0149593 titled "Pharmaceutical Formulation for Delivery of Receptor Tyrosine Kinase Inhibiting (RTKi) Compounds to the Eye", US 8,632,809 titled "Water insoluble polymer matrix for drug delivery" (Alcon, Inc.).

Additional non-limiting examples of drug delivery devices and methods include, for example, US20090203709 titled "Pharmaceutical Dosage Form For Oral Administration Of Tyrosine Kinase Inhibitor" (Abbott Laboratories); US20050009910 titled "Delivery of an active drug to the posterior part of the eye via subconjunctival or periocular delivery of a prodrug", US 20130071349 titled "Biodegradable polymers for lowering intraocular pressure", US 8,481,069 titled "Tyrosine kinase microspheres", US 8,465,778 titled "Method of making tyrosine kinase microspheres", US 8,409,607 titled "Sustained release intraocular implants containing tyrosine kinase inhibitors and related methods", US 8,512,738 and US 2014/0031408 titled "Biodegradable intravitreal tyrosine kinase implants", US 2014/0294986 titled "Microsphere Drug Delivery System for Sustained Intraocular Release", US 8,911,768 titled "Methods For Treating Retinopathy With Extended Therapeutic Effect" (Allergan, Inc.); US 6,495,164 titled "Preparation of injectable suspensions having improved injectability" (Alkermes Controlled Therapeutics, Inc.); WO 2014/047439 titled "Biodegradable Microcapsules Containing Filling Material" (Akina, Inc.); WO 2010/132664 titled "Compositions And Methods For Drug Delivery" (Baxter International Inc. Baxter Healthcare SA); US20120052041 titled "Polymeric nanoparticles with enhanced drugloading and methods of use thereof" (The Brigham and Women's Hospital, Inc.); US20140178475, US20140248358, and US20140249158 titled "Therapeutic Nanoparticles Comprising a Therapeutic Agent and Methods of Making and Using Same" (BIND Therapeutics, Inc.); US 5,869,103 titled "Polymer microparticles for drug delivery" (Danbiosyst UK Ltd.); US 8628801 titled "Pegylated Nanoparticles" (Universidad de Navarra); US2014/0107025 titled "Ocular drug delivery system" (Jade Therapeutics, LLC); US 6,287,588 titled "Agent delivering system comprised of microparticle and biodegradable gel with an improved releasing profile and methods of use thereof", US 6,589,549 titled "Bioactive agent delivering system comprised of microparticles within a biodegradable to improve release profiles" (Macromed, Inc.); US 6,007,845 and US 5,578,325 titled "Nanoparticles and microparticles of non-linear hydrophilichydrophobic multiblock copolymers" (Massachusetts Institute of Technology); US20040234611, US20080305172, US20120269894, and US20130122064 titled "Ophthalmic depot formulations for periocular or subconjunctival administration (Novartis Ag); US 6,413,539 titled "Block polymer" (Poly-Med, Inc.); US 20070071756 titled "Delivery of an agent to ameliorate inflammation" (Peyman); US 20080166411 titled "Injectable Depot Formulations And Methods For Providing Sustained Release Of Poorly Soluble Drugs Comprising Nanoparticles" (Pfizer, Inc.); US 6,706,289 titled "Methods and compositions for enhanced delivery of bioactive molecules" (PR Pharmaceuticals, Inc.); and US 8,663,674 titled "Microparticle containing matrices for drug delivery" (Surmodics).

### USES OF ACTIVE COMPOUNDS FOR TREATMENT OF SELECTED DISORDERS

In one aspect, an effective amount of an active compound or its salt or composition as described herein is used to treat a medical disorder which is an inflammatory or immune condition, a disorder mediated by the complement cascade (including a dysfunctional cascade) including a complement-mediated disease or disorder including a complement factor D-related disorder or alternative complement pathway-related disorder, a disorder or abnormality of a cell that adversely affects the ability of the cell to engage in or respond to normal complement activity, or an undesired complement-mediated response to a medical treatment, such as surgery or other medical procedure or a pharmaceutical or biopharmaceutical drug administration, a blood transfusion, or other allogenic tissue or fluid administration.

A complement-mediated disease or disorder is a disease or disorder in which the amount or activity of complement is such as to cause disease or disorder in an individual. In some embodiments, the complement-mediated disease or disorder is selected from the group consisting of autoimmune disease, cancer, hematological disease, infectious disease, inflammatory disease, ischemia-reperfusion injury, neurodegenerative disease, neurodegenerative disorder, ocular disease, renal disease, transplant rejection, vascular disease, and vasculitis disease. In some embodiments, the complement-mediated disease or disorder is an autoimmune disease. In some embodiments, the complement-mediated disease or disorder is cancer. In some embodiments, the complement-mediated disease or disorder is an infectious disease. In some embodiments, the complement-mediated disease or disorder is an inflammatory disease. In some embodiments, the complement-mediated disease or disorder is a hematological disease. In some embodiments, the complement-mediated disease or disorder is an ischemia-reperfusion injury. In some embodiments, the complement-mediated disease or disorder is ocular disease. In some embodiments, the complement-mediated disease or disorder is a renal disease. In some embodiments, the complement-mediated disease or disorder is transplant rejection. In some embodiments, the complement-mediated disease or disorder is antibody-mediated transplant rejection. In some embodiments, the complement-mediated disease or disorder is a vascular disease. In some embodiments, the complement-mediated disease or disorder is a vasculitis disorder. In some embodiments, the complement-mediated disease or disorder is a neurodegenerative disease or disorder. In some embodiments, the complement-mediated disease is a neurodegenerative disease. In some embodiments, the complement-mediated disorder is a neurodegenerative disorder. In some embodiments, the complement-mediated disease or disorder is a tauopathy.

In some embodiments the compound as described herein is provided for use in a method for the treatment of C3 glomerulonephritis (C3G) , said method comprising the administration of an effective amount of the compound to a host , or a pharmaceutically acceptable salt thereof, optionally in a pharmaceutically acceptable composition. In some embodiments the compound as described herein is provided for use in a method for the treatment of paroxysmal nocturnal hemoglobinuria (PNH) , said method comprising the administration of an effective amount of the compound to a host, or a pharmaceutically acceptable salt thereof, optionally in a pharmaceutically acceptable composition. In another embodiment the compound as described herein is provided for use in a method for the treatment of wet or dry age-related macular degeneration (AMD) in a host, said method comprising the administration of an effective amount of the compound, or a pharmaceutically acceptable salt thereof, optionally in a pharmaceutically acceptable composition. In another embodiment the compound as described herein is provided for use in a method for the treatment of rheumatoid arthritis in a host, said method comprising the administration of an effective amount of the compound, or a pharmaceutically acceptable salt thereof, optionally in a pharmaceutically acceptable composition. In another embodiment the compound as described herein is provided for use in a method for the treatment of multiple sclerosis in a host, said method comprising the administration of an effective amount of the compound, or a pharmaceutically acceptable saltthereof, optionally in a pharmaceutically acceptable composition.

The active compound or its pharmaceutically acceptable salt, optionally in a pharmaceutically acceptable composition, as disclosed herein is also useful for administration in combination (in the same or a different dosage form) or alternation with a second pharmaceutical agent for use in ameliorating or reducing a side effect of the second pharmaceutical agent. For example, in some embodiments, the active compound may be used in combination with an adoptive cell transfer therapy to reduce an inflammatory response associated with such therapy, for example, a cytokine mediated response such as cytokine response syndrome. In some embodiments, the adoptive cell transfer therapy is a chimeric antigen receptor T-Cell (CAR T) or a dendritic cell used to treat a hematologic or solid tumor, for example, a B-cell related hematologic cancer. In some embodiments, the hematologic or solid tumor is acute lymphoblastic leukemia (ALL), acute myeloid leukemia (AML), non-Hodgkin's lymphoma, chronic lymphocytic leukemia (CLL), pancreatic cancer, glioblastoma, or a cancer that expresses CD19. In some embodiments, the associated inflammatory response is a cytokine mediated response.

Another embodiment is provided that includes the administration of an effective amount of an active compound or a pharmaceutically acceptable salt thereof, optionally in a pharmaceutically acceptable composition to a host to treat an ocular, pulmonary, gastrointestinal, or other disorder that can benefit from topical or local delivery.

Any of the compounds described herein can be administered to the eye in any desired form of administration, including via intravitreal, intrastromal, intracameral, sub-tenon, sub-retinal, retro-bulbar, peribulbar, suprachorodial, choroidal, subchoroidal, conjunctival, subconjunctival, episcleral, posterior juxtascleral, scleral, circumcorneal, and tear duct injections, or through a mucus, mucin, or a mucosal barrier, in an immediate or controlled release fashion. In certain embodiments, the active compound includes a lipophilic group, such as a lipophilic acyl group, which is delivered to the eye in a polymeric drug delivery system such as polylactic acid, polylactide-co-glycolide, polyglycolide or other erodible polymer, or a combination thereof, or in another type of lipophilic material for ocular delivery. In some embodiments, the lipophilic active molecule is more soluble in the polymeric or other form of delivery system than in ocular fluid.

In other embodiments of the invention, an active compound provided herein can be used to treat or prevent a disorder in a host mediated by complement factor D, or by an excessive or detrimental amount of the complement-C3 amplification loop of the complement pathway. As examples, the invention includes the compounds and their salts as described herein for use in methods to treat or prevent complement associated disorders that are induced by antibody-antigen interactions, a component of an immune or autoimmune disorder or by ischemic injury. The invention also provides the compounds and their salts as described herein for use in methods to decrease inflammation or an immune response, including an autoimmune response, where mediated or affected by factor D.

In some embodiments, the disorder is selected from fatty liver and conditions stemming from fatty liver, such as nonalcoholic steatohepatitis (NASH), liver inflammation, cirrhosis and liver failure. In some embodiments of the present invention, the compounds and their salts as described herein are provided for use in a method for treating fatty liver disease in a host by administering an effective amount of an active compound or its salt or composition as described herein.

In another embodiment, an active compound or its salt or composition as described herein is used to modulate an immune response prior to or during surgery or other medical procedure. One non-limiting example is use in connection with acute or chronic graft versus host disease, which is a common complication as a result of allogeneic tissue transplant, and can also occur as a result of a blood transfusion.

In some embodiments, the present invention provides an active compound or its salt or composition as described herein for use in a method of treating or preventing dermatomyositis by administering to a subject in need thereof an effective amount of the active compound or its salt or composition as described herein.

In some embodiments, the present invention provides an active compound or its salt or composition as described herein for use in a method of treating or preventing amyotrophic lateral sclerosis by administering to a subject in need thereof an effective amount of the active compound or its salt or composition as described herein.

In some embodiments, the present invention provides an active compound or its salt or composition as described herein for use in a method of treating or preventing abdominal aortic aneurysm, hemodialysis complications, hemolytic anemia, or hemodialysis by administering to a subject in need thereof an effective amount of the active compound or its salt or composition as described herein.

In another embodiment, the present invention provides an active compound or its salt or composition as described herein for use in a method for the treatment or prevention of cytokine or inflammatory reactions in response to the administration of pharmaceutical or biotherapeutic (e.g. CAR T-cell therapy or monoclonal antibody therapy) in a host by administering an effective amount of the active compound or its salt or composition as described herein. Various types of cytokine or inflammatory reactions may occur in response to a number of factors, such as the administrations of biotherapeutics. In some embodiments, the cytokine or inflammatory reaction is cytokine release syndrome. In some embodiments, the cytokine or inflammatory reaction is tumor lysis syndrome (which also leads to cytokine release). Symptoms of cytokine release syndrome range from fever, headache, and skin rashes to bronchospasm, hypotension and even cardiac arrest. Severe cytokine release syndrome is described as cytokine storm, and can be fatal.

Fatal cytokine storms have been observed in response to infusion with several monoclonal antibody therapeutics. See, Abramowicz D, et al. "Release of tumor necrosis factor, interleukin-2, and gamma-interferon in serum after injection of OKT3 monoclonal antibody in kidney transplant recipients" Transplantation (1989) 47(4):606-8; Chatenoud L, et al. "In vivo cell activation following OKT3 administration. Systemic cytokine release and modulation by corticosteroids" Transplantation (1990) 49(4):697-702; and Lim LC, Koh LP, and Tan P. "Fatal cytokine release syndrome with chimeric anti-CD20 monoclonal antibody rituximab in a 71-year-old patient with chronic lymphocytic leukemia" J. Clin Oncol. (1999) 17(6):1962-3.

Also contemplated herein, is the use of an active compound or its salt or composition as described herein to mediate an adverse immune response in patients receiving bi-specific T-cell engagers (BiTE). A bi-specific T-cell engager directs T-cells to target and bind with a specific antigen on the surface of a cancer cell. For example, Blinatumomab (Amgen), a BiTE has recently been approved as a second line therapy in Philadelphia chromosome-negative relapsed or refractory acute lymphoblastic leukemia. Blinatumomab is given by continuous intravenous infusion in 4-week cycles. The use of BiTE agents has been associated with adverse immune responses, including cytokine release syndrome. The most significantly elevated cytokines in the CRS associated with ACT include IL-10, IL-6, and IFN-γ (Klinger et al., Immunopharmacologic response of patients with B-lineage acute lymphoblastic leukemia to continuous infusion of T cell-engaging CD19/CD3-bispecific BiTE antibody blinatumomab. Blood (2012) 119:6226-6233).

In another embodiment, the disorder is episcleritis, idiopathic episcleritis, anterior episcleritis, or posterior episcleritis. In some embodiments, the disorder is idiopathic anterior uveitis, HLA-B27 related uveitis, herpetic keratouveitis, Posner Schlossman syndrome, Fuch's heterochromic iridocyclitis, or cytomegalovirus anterior uveitis.

In some embodiments, the present invention provides an active compound or its salt or composition as described herein for use in a method of treating or preventing a C3 glomurenopathy by administering to a subject in need thereof an effective amount of the active compound or its salt or composition as described herein. In some embodiments, the disorder is selected from dense deposit disease (DDD) and C3 glomerulonephritis (C3GN).

In some embodiments, the present invention provides an active compound or its salt or composition as described herein for use in a method of treating or preventing a IC-MPGN by administering to a subject in need thereof an effective amount of the active compound or its salt or composition as described herein.

In some embodiments, the present invention provides an active compound or its salt or composition as described herein for use in a method of treating or preventing a delayed graft function (DGF) by administering to a subject in need thereof an effective amount of the active compound or its salt or composition as described herein.

In some embodiments, the present invention provides an active compound or its salt or composition as described herein for use in a method of treating or promoting wound healing by administering to a subject in need thereof an effective amount of the active compound or its salt or composition as described herein

In some embodiments, the present invention provides an active compound or its salt or composition as described herein for use in a method of treating or preventing a HSCT-TMA by administering to a subject in need thereof an effective amount of the active compound or its salt or composition as described herein.

In some embodiments, the present invention provides an active compound or its salt or composition as described herein for use in a method of treating or preventing a paroxysmal nocturnal hemoglobinuria (PNH) by administering to a subject in need thereof an effective amount of the active compound or its salt or composition as described herein.

In some embodiments, the present invention provides an active compound or its salt or composition as described herein for use in a method of treating or preventing age-related macular degeneration (AMD) by administering to a subject in need thereof an effective amount of the active compound or its salt or composition as described herein.

In some embodiments, the present invention provides an active compound or its salt or composition as described herein for use in a method of treating or preventing macular dystrophy by administering to a subject in need thereof an effective amount of the active compound or its salt or composition as described herein.

In some embodiments, the present invention provides an active compound or its salt or composition as described herein for use in a method of treating or preventing a Crohn's disease by administering to a subject in need thereof an effective amount of the active compound or its salt or composition as described herein.

In some embodiments, the present invention provides an active compound or its salt or composition as described herein for use in a method of treating or preventing Stargardt's disease (Stargardt macular dystrophy) by administering to a subject in need thereof an effective amount of the active compound or its salt or composition as described herein.

In some embodiments, the present invention provides an active compound or its salt or composition as described herein for use in a method of treating or preventing acute pancreatitis by administering to a subject in need thereof an effective amount of the active compound or its salt or composition as described herein.

In some embodiments, the present invention provides an active compound or its salt or composition as described herein for use in a method of treating or preventing asthma (TH2) or asthma (non-TH2) by administering to a subject in need thereof an effective amount of the active compound or its salt or composition as described herein.

In some embodiments, the present invention provides an active compound or its salt or composition as described herein for use in a method of treating or preventing periodontitis by administering to a subject in need thereof an effective amount of the active compound or its salt or composition as described herein.

In some embodiments, the present invention provides an active compound or its salt or composition as described herein for use in a method of treating or preventing a diabetic retinopathy by administering to a subject in need thereof an effective amount of the active compound or its salt or composition as described herein.

In some embodiments, the present invention provides an active compound or its salt or composition as described herein for use in a method of treating or preventing a hidradenitis suppurativa by administering to a subject in need thereof an effective amount of the active compound or its salt or composition as described herein.

In some embodiments, the present invention provides an active compound or its salt or composition as described herein for use in a method of treating or preventing acute respiratory distress syndrome (ARDS) by administering to a subject in need thereof an effective amount of the active compound or its salt or composition as described herein.

In some embodiments, the present invention provides an active compound or its salt or composition as described herein for use in methods of treating or preventing a nephrology disorder selected from acute kidney injury (AKI), idiopathic membranous nephropathy, IgA nephropathy (IgAN) lupus nephritis (LN), and primary focal segmental glomerulosclerosis by administering to a subject in need thereof an effective amount of the active compound or its salt or composition as described herein.

In some embodiments, the present invention provides an active compound or its salt or composition as described herein for use in methods of treating or preventing preeclampsia by administering to a subject in need thereof an effective amount of the active compound or its salt or composition as described herein.

In some embodiments, the present invention provides an active compound or its salt or composition as described herein for use in a method of treating or preventing rheumatoid arthritis by administering to a subject in need thereof an effective amount of the active compound or its salt or composition as described herein.

In some embodiments, the present invention provides an active compound or its salt or composition as described herein for use in a method of treating or preventing multiple sclerosis by administering to a subject in need thereof an effective amount of the active compound or its salt or composition as described herein.

In some embodiments, the present invention provides an active compound or its salt or composition as described herein for use in a method of treating or preventing myasthenia gravis by administering to a subject in need thereof an effective amount of the active compound or its salt or composition as described herein.

In some embodiments, the present invention provides an active compound or its salt or composition as described herein for use in a method of treating or preventing atypical hemolytic uremic syndrome (aHUS) by administering to a subject in need thereof an effective amount of the active compound or its salt or composition as described herein.

In some embodiments, the present invention provides an active compound or its salt or composition as described herein for use in a method of treating or preventing neuromyelitis optica (NMO) by administering to a subject in need thereof an effective amount of the active compound or its salt or composition as described herein.

In yet another embodiment, the present invention provides an active compound or its salt or composition as described herein for use in a method of treating or preventing a disorder as described below by administering to a subject in need thereof an effective amount of the active compound or its salt or composition as described herein, including: vitritis, sarcoidosis, syphilis, tuberculosis, or Lyme disease; retinal vasculitis, Eales disease, tuberculosis, syphilis, or toxoplasmosis; neuroretinitis, viral retinitis, or acute retinal necrosis;varicella zoster virus, herpes simplex virus, cytomegalovirus, Epstein-Barr virus, lichen planus, or Dengue-associated disease (e.g., hemorraghic Dengue Fever); Masquerade syndrome, contact dermatitis, trauma induced inflammation, UVB induced inflammation, eczema, granuloma annulare, or acne.

In an additional embodiment, the disorder is selected from: acute myocardial infarction, aneurysm, cardiopulmonary bypass, dilated cardiomyopathy, complement activation during cardiopulmonary bypass operations, coronary artery disease, restenosis following stent placement, or percutaneous transluminal coronary angioplasty (PTCA); antibody-mediated transplant rejection, anaphylactic shock, anaphylaxis, allogenic transplant, humoral and vascular transplant rejection, graft dysfunction, graft-versus-host disease, Graves' disease, adverse drug reactions, or chronic graft vasculopathy; allergic bronchopulmonary aspergillosis, allergic neuritis, drug allergy, radiation- induced lung injury, eosinophilic pneumonia, radiographic contrast media allergy, bronchiolitis obliterans, or interstitial pneumonia; parkinsonism-dementia complex, sporadic frontotemporal dementia, frontotemporal dementia with Parkinsonism linked to chromosome 17, frontotemporal lobar degeneration, tangle only dementia, cerebral amyloid angiopathy, cerebrovascular disorder, certain forms of frontotemporal dementia, chronic traumatic encephalopathy (CTE), PD with dementia (PDD), argyrophilic grain dementia, dementia pugilistica, dementia with Lewy Bodies (DLB), or multi-infarct dementia; Creutzfeldt-Jakob disease, Huntington's disease, multifocal motor neuropathy (MMN), prion protein cerebral amyloid angiopathy, polymyositis, postencephalitic parkinsonism, subacute sclerosing panencephalitis, non-Guamanian motor neuron disease with neurofibrillary tangles, neural regeneration, or diffuse neurofibrillary tangles with calcification.

In some embodiments, the disorder is selected from: transplant-associated thrombotic microangiopathy (TMA); hematopoietic stem cell transplant-associated thrombotic microangiopathy (HSCT-TMA); atopic dermatitis, dermatitis, dermatomyositis bullous pemphigoid, scleroderma, sclerodermatomyositis, psoriatic arthritis, pemphigus vulgaris, Discoid lupus erythematosus, cutaneous lupus, chilblain lupus erythematosus, or lupus erythematosus-lichen planus overlap syndrome; cryoglobulinemic vasculitis, mesenteric/enteric vascular disorder, peripheral vascular disorder, antineutrophil cytoplasm antibody (ANCA)-associated vasculitis (AAV), IL-2 induced vascular leakage syndrome, or immune complex vasculitis;angioedema, low platelets (HELLP) syndrome, sickle cell disease, platelet refractoriness, red cell casts, or typical or infectious hemolytic uremic syndrome (tHUS); hematuria, hemorrhagic shock, drug-induced thrombocytopenia, autoimmune hemolytic anemia (AIHA), azotemia, blood vessel and/or lymph vessel inflammation, rotational atherectomy, or delayed hemolytic transfusion reaction; British type amyloid angiopathy, Buerger's disease, bullous pemphigoid, C1q nephropathy, cancer, or catastrophic antiphospholipid syndrome.

In another embodiment, the disorder is selected from:wet (exudative) AMD, dry (non-exudative) AMD, chorioretinal degeneration, choroidal neovascularization (CNV), choroiditis, loss of RPE function, loss of vision (including loss of visual acuity or visual field), loss of vision from AMD, retinal damage in response to light exposure, retinal degeneration, retinal detachment, retinal dysfunction, retinal neovascularization (RNV), retinopathy of prematurity, pathological myopia, or RPE degeneration; pseudophakic bullous keratopathy, symptomatic macular degeneration related disorder, optic nerve degeneration, photoreceptor degeneration, cone degeneration, loss of photoreceptor cells, pars planitis, scleritis, proliferative vitreoretinopathy, or formation of ocular drusen; chronic urticaria, Churg-Strauss syndrome, cold agglutinin disease (CAD), corticobasal degeneration (CBD), cryoglobulinemia, cyclitis, damage of the Bruch's membrane, Degos disease, diabetic angiopathy, elevated liver enzymes, endotoxemia, epidermolysis bullosa, or epidermolysis bullosa acquisita (EBA); essential mixed cryoglobulinemia, excessive blood urea nitrogen-BUN, focal segmental glomerulosclerosis, Gerstmann-Straussler-Scheinker disease, giant cell arteritis, gout, Hallervorden-Spatz disease, Hashimoto's thyroiditis, Henoch-Schonlein purpura nephritis, or abnormal urinary sediments; hidradenitis suppurative (HS); hepatitis, hepatitis A, hepatitis B, hepatitis C or human immunodeficiency virus (HIV), a viral infection more generally, for example selected from Flaviviridae, Retroviruses, Coronaviridae, Poxviridae, Adenoviridae, Herpesviridae, Caliciviridae, Reoviridae, Picornaviridae, Togaviridae, Orthomyxoviridae, Rhabdoviridae, or Hepadnaviridae; Neisseria meningitidis, shiga toxin E. coli-related hemolytic uremic syndrome (STEC-HUS), hemolytic uremic syndrome (HUS); Streptococcus, or poststreptococcal glomerulonephritis.

In a further embodiment, the disorder is selected from: hyperlipidemia, hypertension, hypoalbuminemia, hypobolemic shock, hypocomplementemic urticarial vasculitis syndrome, hypophosphastasis, hypovolemic shock, idiopathic pneumonia syndrome, or idiopathic pulmonary fibrosis; inclusion body myositis, intestinal ischemia, iridocyclitis, iritis, juvenile chronic arthritis, Kawasaki's disease (arteritis), or lipiduria; membranoproliferative glomerulonephritis (MPGN) I, microscopic polyangiitis, mixed cryoglobulinemia, molybdenum cofactor deficiency (MoCD) type A, pancreatitis, panniculitis, Pick's disease, polyarteritis nodosa (PAN), progressive subcortical gliosis, proteinuria, reduced glomerular filtration rate (GFR), or renovascular disorder; multiple organ failure, multiple system atrophy (MSA), myotonic dystrophy, Niemann-Pick disease type C, chronic demyelinating diseases, or progressive supranuclear palsy; spinal cord injury, spinal muscular atrophy, spondyloarthropathies, Reiter's syndrome, spontaneous fetal loss, recurrent fetal loss, pre-eclampsia, synucleinopathy, Takayasu's arteritis, post-partum thryoiditis, thyroiditis, Type I cryoglobulinemia, Type II mixed cryoglobulinemia, Type III mixed cryoglobulinemia, ulcerative colitis, uremia, urticaria, venous gas embolus (VGE), or Wegener's granulomatosis; von Hippel-Lindau disease, histoplasmosis of the eye, hard drusen, soft drusen, pigment clumping, or photoreceptor and/or retinal pigmented epithelia (RPE) loss,.

In some embodiments, an active compound or its salt or composition as described herein is useful for treating or preventing a disorder selected from autoimmune oophoritis, endometriosis, autoimmune orchitis, Ord's thyroiditis, autoimmune enteropathy, coeliac disease, Hashimoto's encephalopathy, antiphospholipid syndrome (APLS) (Hughes syndrome), aplastic anemia, autoimmune lymphoproliferative syndrome (Canale-Smith syndrome), autoimmune neutropenia, Evans syndrome, pernicious anemia, pure red cell aplasia, thrombocytopenia, adipose dolorosa (Dercum's disease), adult onset Still's disease, ankylosing spondylitis, CREST syndrome, drug-induced lupus, eosinophilic fasciitis (Shulman's syndrome), Felty syndrome, IgG4-related disease, mixed connective tissue disease (MCTD), palindromic rheumatism (Hench-Rosenberg syndrome), Parry-Romberg syndrome, Parsonage-Turner syndrome, relapsing polychondritis (Meyenburg-Altherr-Uehlinger syndrome), retroperitonial fibrosis, rheumatic fever, Schnitzler syndrome, fibromyalgia, neuromyotonia (Isaac's disease), paraneoplastic degeneration, autoimmune inner ear disease, Meniere's disease, interstitial cystitis, autoimmune pancreatitis, zika virus-related disorders, chikungunya virus-related disorders, subacute bacterial endocarditis (SBE), IgA nephropathy, IgA vasculitis, polymyalgia rheumatic, rheumatoid vasculitis, alopecia areata, autoimmune progesterone dermatitis, dermatitis herpetiformis, erythema nodosum, gestational pemphigoid, hidradenitis suppurativa, lichen sclerosus, linear IgA disease (LAD), morphea, myositis, pityriasis lichenoides et varioliformis acuta, vitiligo post-myocardial infarction syndrome (Dressler's syndrome), post-pericardiotomy syndrome, autoimmune retinopathy, Cogan syndrome, Graves opthalmopathy, ligneous conjunctivitis, Mooren's ulcer, opsoclonus myoclonus syndrome, optic neuritis, retinocochleocerebral vasculopathy (Susac's syndrome), sympathetic opthalmia, Tolosa-Hunt syndrome, interstitial lung disease, antisynthetase syndrome, Addison's disease, autoimmune polyendocrine syndrome (APS) type I, autoimmune polyendocrine syndrome (APS) type II, autoimmune polyendocrine syndrome (APS) type III, disseminated sclerosis (multiple sclerosis, pattern II), rapidly progressing glomerulonephritis (RPGN), juvenile rheumatoid arthritis, enthesitis-related arthritis, reactive arthritis (Reiter's syndrome), autoimmune hepatitis or lupoid hepatitis, primary biliary cirrhosis (PBS), primary sclerosing cholangitis, microscopic colitis, latent lupus (undifferentiated connective tissue disease (UCTD)), acute disseminated encephalomyelitis (ADEM), acute motor axonal neuropathy, anti-n-methyl-D-aspartate receptor encephalitis, Balo concentric sclerosis (Schilders disease), Bickerstaff's encephalitis, chronic inflammatory demyelinating polyneuropathy, idiopathic inflammatory demyelinating disease, Lambert-Eaton mysathenic syndrome, Oshtoran syndrome, pediatric autoimmune neuropsychiatric disorder associated with streptococcus (PANDAS), progressive inflammatory neuropathy, restless leg syndrome, stiff person syndrome, Sydenhem syndrome, transverse myelitis, lupus vasculitis, leukocytoclastic vasculitis, Microscopic Polyangiitis, polymyositis or ischemic-reperfusion injury of the eye.

Examples of eye disorders that may be treated according to the compositions and methods disclosed herein include amoebic keratitis, fungal keratitis, bacterial keratitis, viral keratitis, onchorcercal keratitis, bacterial keratoconjunctivitis, viral keratoconjunctivitis, corneal dystrophic diseases, Fuchs' endothelial dystrophy, Sjogren's syndrome, Stevens-Johnson syndrome, autoimmune dry eye diseases, environmental dry eye diseases, corneal neovascularization diseases, post-corneal transplant rejection prophylaxis and treatment, autoimmune uveitis, infectious uveitis, posterior uveitis (including toxoplasmosis), pan-uveitis, an inflammatory disease of the vitreous or retina, endophthalmitis prophylaxis and treatment, macular edema, macular degeneration, age related macular degeneration, proliferative and non-proliferative diabetic retinopathy, hypertensive retinopathy, an autoimmune disease of the retina, primary and metastatic intraocular melanoma, other intraocular metastatic tumors, open angle glaucoma, closed angle glaucoma, pigmentary glaucoma and combinations thereof.

In a further embodiment, the disorder is selected from glaucoma, diabetic retinopathy, blistering cutaneous diseases (including bullous pemphigoid, pemphigus, and epidermolysis bullosa), ocular cicatrical pemphigoid, uveitis, adult macular degeneration, diabetic retinopa retinitis pigmentosa, macular edema, diabetic macular edema, Behcet's uveitis, non-infectious uvietis, multifocal choroiditis, Vogt-Koyangi-Harada syndrome, intermediate uveitis, birdshot retino-chorioditis, sympathetic ophthalmia, ocular dicatricial pemphigoid, ocular pemphigus, nonartertic ischemic optic neuropathy, postoperative inflammation, and retinal vein occlusion, or central retinal vein occulusion (CVRO).

In some embodiments, complement mediated diseases include ophthalmic diseases (including early or neovascular age-related macular degeneration and geographic atrophy), autoimmune diseases (including arthritis, rheumatoid arthritis), respiratory diseases, cardiovascular diseases. In other embodiments, the compounds of the invention are suitable for use in the treatment of diseases and disorders associated with fatty acid metabolism, including obesity and other metabolic disorders.

Disorders that may be treated or prevented by an active compound or its salt or composition as described herein also include, but are not limited to: hereditary angioedema, capillary leak syndrome, hemolytic uremic syndrome (HUS), neurological disorders, Guillain Barre Syndrome, diseases of the central nervous system and other neurodegenerative conditions, glomerulonephritis (including membrane proliferative glomerulonephritis), SLE nephritis, proliferative nephritis, liver fibrosis, tissue regeneration and neural regeneration, or Barraquer-Simons Syndrome; inflammatory effects of sepsis, systemic inflammatory response syndrome (SIRS), disorders of inappropriate or undesirable complement activation, interleukin-2 induced toxicity during IL-2 therapy, inflammatory disorders, inflammation of autoimmune diseases, system lupus erythematosus (SLE), lupus nephritides, arthritis, immune complex disorders and autoimmune diseases, systemic lupus, or lupus erythematosus; ischemia/ reperfusion injury (I/R injury), myocardial infarction, myocarditis, post-ischemic reperfusion conditions, balloon angioplasty, atherosclerosis, post-pump syndrome in cardiopulmonary bypass or renal bypass, renal ischemia, mesenteric artery reperfusion after aortic reconstruction, antiphospholipid syndrome, autoimmune heart disease, ischemia-reperfusion injuries, obesity, or diabetes; Alzheimer's dementia, stroke, schizophrenia, traumatic brain injury, trauma, Parkinson's disease, epilepsy, transplant rejection, prevention of fetal loss, biomaterial reactions (e.g. in hemodialysis, inplants), hyperacute allograft rejection, xenograft rejection, transplantation, psoriasis, burn injury, thermal injury including burns or frostbite, or crush injury; asthma, allergy, acute respiratory distress syndrome (ARDS), cystic fibrosis, adult respiratory distress syndrome, dyspnea, hemoptysis, chronic obstructive pulmonary disease (COPD), emphysema, pulmonary embolisms and infarcts, pneumonia, fibrogenic dust diseases, inert dusts and minerals (e.g., silicon, coal dust, beryllium, and asbestos), pulmonary fibrosis, organic dust diseases, chemical injury (due to irritant gases and chemicals, e.g., chlorine, phosgene, sulfur dioxide, hydrogen sulfide, nitrogen dioxide, ammonia, and hydrochloric acid), smoke injury, thermal injury (e.g., burn, freeze), bronchoconstriction, hypersensitivity pneumonitis, parasitic diseases, Goodpasture's Syndrome (anti-glomerular basement membrane nephritis), pulmonary vasculitis, Pauci-immune vasculitis, or immune complex- associated inflammation.

In some embodiments, the invention provides an active compound or its salt or composition as described herein for use in a method for the treatment of sickle cell in a host that comprises the administration of an effective amount of the active compound or its salt or composition as described herein. In some embodiments, the invention provides an active compound or its salt or composition as described herein for use in a method for the treatment of immunothrombocytopenic purpura (ITP), thrombotic thrombocytopenic purpura (TTP), or idiopathic thrombocytopenic purpura (ITP) in a host that comprises the administration of an effective amount of the active compound or its salt or composition as described herein. In some embodiments, the invention provides an active compound or its salt or composition as described herein for use in a method for the treatment of ANCA-vasculitis in a host that comprises the administration of an effective amount of the active compound or its salt or composition as described herein. In some embodiments, the invention provides an active compound or its salt or composition as described herein for use in a method for the treatment of IgA nephropathy in a host that comprises the administration of an effective amount of the active compound or its salt or composition as described herein. In some embodiments, the invention provides an active compound or its salt or composition as described herein for use in a method for the treatment of rapidly progressing glomerulonephritis (RPGN), in a host that comprises the administration of an effective amount of the active compound or its salt or composition as described herein. In some embodiments, the invention provides an active compound or its salt or composition as described herein for use in a method for the treatment of lupus nephritis, in a host that comprises the administration of an effective amount of the active compound or its salt or composition as described herein. In some embodiments, the invention provides an active compound or its salt or composition as described herein for use in a method for the treatment of hemorraghic dengue fever, in a host that comprises the administration of an effective amount of the active compound or its salt or composition as described herein.

In an additional alternative embodiment, an active compound or its salt or composition as described herein is used in the treatment of an autoimmune disorder.

The complement pathway enhances the ability of antibodies and phagocytic cells to clear microbes and damaged cells from the body. It is part of the innate immune system and in healthy individuals is an essential process. Inhibiting the complement pathway will decrease the body's immune system response. Therefore, it is an object of the present invention to treat autoimmune disorders by administering an effective does of an active compound or its salt or composition as described herein to a subject in need thereof.

In some embodiments the autoimmune disorder is caused by activity of the complement system. In some embodiments the autoimmune disorder is caused by activity of the alternative complement pathway. In some embodiments the autoimmune disorder is caused by activity of the classical complement pathway. In another embodiment the autoimmune disorder is caused by a mechanism of action that is not directly related to the complement system, such as the over-proliferation of T-lymphocytes or the over-production of cytokines.

Non-limiting examples of autoimmune disorders include: lupus, allograft rejection, autoimmune thyroid diseases (such as Graves' disease and Hashimoto's thyroiditis), autoimmune uveoretinitis, giant cell arteritis, inflammatory bowel diseases (including Crohn's disease, ulcerative colitis, regional enteritis, granulomatous enteritis, distal ileitis, regional ileitis, and terminal ileitis), diabetes, multiple sclerosis, pernicious anemia, psoriasis, rheumatoid arthritis, sarcoidosis, and scleroderma.

In some embodiments, an active compound or its salt or composition as described herein is used in the treatment of lupus. Non-limiting examples of lupus include lupus erythematosus, cutaneous lupus, discoid lupus erythematosus, chilblain lupus erythematosus, or lupus erythematosus-lichen planus overlap syndrome.

Lupus erythematosus is a general category of disease that includes both systemic and cutaneous disorders. The systemic form of the disease can have cutaneous as well as systemic manifestations. However, there are also forms of the disease that are only cutaneous without systemic involvement. For example, SLE is an inflammatory disorder of unknown etiology that occurs predominantly in women, and is characterized by articular symptoms, butterfly erythema, recurrent pleurisy, pericarditis, generalized adenopathy, splenomegaly, as well as CNS involvement and progressive renal failure. The sera of most patients (over 98%) contain antinuclear antibodies, including anti-DNA antibodies. High titers of anti-DNA antibodies are essentially specific for SLE. Conventional treatment for this disease has been the administration of corticosteroids or immunosuppressants.

There are three forms of cutaneous lupus: chronic cutaneous lupus (also known as discoid lupus erythematosus or DLE), subacute cutaneous lupus, and acute cutaneous lupus. DLE is a disfiguring chronic disorder primarily affecting the skin with sharply circumscribed macules and plaques that display erythema, follicular plugging, scales, telangiectasia and atrophy. The condition is often precipitated by sun exposure, and the early lesions are erythematous, round scaling papules that are 5 to 10 mm in diameter and display follicular plugging. DLE lesions appear most commonly on the cheeks, nose, scalp, and ears, but they may also be generalized over the upper portion of the trunk, extensor surfaces of the extremities, and on the mucous membranes of the mouth. If left untreated, the central lesion atrophies and leaves a scar. Unlike SLE, antibodies against double-stranded DNA (e.g., DNA-binding test) are almost invariably absent in DLE.

Multiple Sclerosis is an autoimmune demyelinating disorder that is believed to be T lymphocyte dependent. MS generally exhibits a relapsing-remitting course or a chronic progressive course. The etiology of MS is unknown, however, viral infections, genetic predisposition, environment, and autoimmunity all appear to contribute to the disorder. Lesions in MS patients contain infiltrates of predominantly T lymphocyte mediated microglial cells and infiltrating macrophages. CD4+ T lymphocytes are the predominant cell type present at these lesions. The hallmark of the MS lesion is plaque, an area of demyelination sharply demarcated from the usual white matter seen in MRI scans. Histological appearance of MS plaques varies with different stages of the disease. In active lesions, the blood-brain barrier is damaged, thereby permitting extravasation of serum proteins into extracellular spaces. Inflammatory cells can be seen in perivascular cuffs and throughout white matter. CD4+ T-cells, especially Th1, accumulate around postcapillary venules at the edge of the plaque and are also scattered in the white matter. In active lesions, up-regulation of adhesion molecules and markers of lymphocyte and monocyte activation, such as IL2-R and CD26 have also been observed. Demyelination in active lesions is not accompanied by destruction of oligodendrocytes. In contrast, during chronic phases of the disease, lesions are characterized by a loss of oligodendrocytes and hence, the presence of myelin oligodendrocyte glycoprotein (MOG) antibodies in the blood.

Diabetes can refer to either type 1 or type 2 diabetes. In some embodiments an active compound or its salt or composition as described herein is provided at an effective dose to treat a patient with type 1 diabetes. In some embodiments an active compound or its salt or composition as described herein is provided at an effective dose to treat a patient with type 2 diabetes.
Type 1 diabetes is an autoimmune disease. An autoimmune disease results when the body's system for fighting infection (the immune system) attacks a part of the body. In the case of diabetes type 1, the pancreas then produces little or no insulin.

In certain aspects, an effective amount of an active compound described herein, or it pharmaceutically acceptable salt, is used to treat a medical disorder of the central nervous system (CNS) or peripheral nervous system disorders involving complement activation. In embodiments, the CNS disorder is an acquired brain or spinal cord injury, including, but not limited to ischaemic-reperfusion injury or stroke, traumatic brain injury (TBI) and spinal cord injury (SCI). In embodiments, the disorder is a neurodegeneration disorder. In embodiments, the disorder is a neuroinflammation disorder.

In certain aspects, active compound described herein, or it pharmaceutically acceptable salt is used to treat Alzheimer's disease (AD). AD is characterized by two hallmark pathologies; amyloid-β (Aβ) plaques and neurofibrillary tangles comprising hyperphosphorylated tau. Recent studies have implicated complement in AD pathogenesis, including genome wide association studies identifying single nucleotide polymorphisms (SNPs) associated with risk of late-onset AD in genes encoding complement proteins Clusterin (CLU) and CR1 (CR1). See Carpanini et al., Therapeutic Inhibition of the Complement System in Diseases of the Central Nervous System, Front. Immunol., 04 March 2019. Biomarker studies have also identified complement proteins and activation products in plasma and/or CSF that distinguish AD from controls and predict risk of progression to AD. (Id.)

In certain aspects, an effective amount of active compound described herein, or it pharmaceutically acceptable salt is used to treat certain forms of frontotemporal dementia including, but not limited to, Pick's disease, sporadic Frontotemporal dementia and Frontotemporal dementia with Parkinsonism linked to chromosome 17, Progressive supranuclear palsy (PSP), Corticobasal degeneration (CBD), and Subacute sclerosing panencephalitis.

In certain aspects, an effective amount of active compound described herein, or it pharmaceutically acceptable salt is used to treat multiple sclerosis (MS). Multiple sclerosis (MS) is the most common cause of neurological disability in young adults in northern European-Caucasian populations, with an approximate lifetime risk of one in 400. C3 has been shown to be deposited in the brains of MS patients. TCC has been shown to be in association with capillary endothelial cells, predominantly within plaques and adjacent white matter. Localization of C activation to areas of active myelin destruction has also been shown, with TCC deposited exclusively in such areas. C3d has been shown to be deposited in association with short segments of disrupted myelin in plaques with low-grade active demyelination and provides evidence for a C contribution to disease progression as well as acute inflammation. See Ingram et al., Complement in multiple sclerosis: its role in disease and potential as a biomarker. Clin Exp Immunol. 2009 Feb;155(2):128-39.

In certain aspects, active compound described herein, or it pharmaceutically acceptable salt is used to treat neuromyelitis optica (NMO). Neuromyelitis optica (NMO) is an inflammatory demyelinating disease affecting predominantly the optic nerves and spinal cord. Traditionally seen as a variant of MS, it has been redefined recently according to new criteria using a combination of phenotypic subtyping along with a newly developed biomarker of disease, NMO-immunoglobulin G (IgG) (reported sensitivity of 58-76% and specificity of 85-99% for NMO). NMO patients have higher levels of C3a and anti-C1q antibodies than healthy controls. C3a levels correlated with disease activity, neurological disability and aquaporin-4 IgG. Nytrova et al., Complement activation in patients with neuromyelitis optica. J Neuroimmunol. 2014 Sep 15;274(1-2):185-91.

In certain aspects, active compound described herein, or it pharmaceutically acceptable salt is used to treat amyotrophic lateral sclerosis (ALS). ALS is caused by progressive loss of upper and lower (α) motor neurons resulting in denervation of neuromuscular junctions in the peripheral nervous system, progressive muscle weakness, atrophy, spasticity, respiratory failure, and ultimately paralysis and death. Recent studies have shown increased C1q protein in motor cortex and spinal cord of ALS post-mortem tissue; C3 activation fragments and TCC in areas of pathology; C4d and TCC staining of degenerating neurons and glia in ALS motor cortex and spinal cord, and C5aR1 upregulation in areas of pathology. C3d and C4d have been found on oligodendroglia and degenerating neurites, surrounded by CR4-positive microglia, in spinal cord and motor cortex, and C1q, C3, and TCC have been shown to be present on motor end-plates in intercostal muscles in ALS donors even early in the disease process. See Carpanini et al., Therapeutic Inhibition of the Complement System in Diseases of the Central Nervous System, Front. Immunol., 04 March 2019.

In certain aspects, active compound described herein, or it pharmaceutically acceptable salt is used to treat Parkinson's disease (PD). PD is characterized by loss of dopaminergic neurons in the substantia nigra and deposits of the protein α-synuclein that form the pathological hallmarks of the disease, Lewy bodies. Patients present with resting tremor, bradykinesia, and rigidity. Complement activation has been associated with α-synuclein and Lewy bodies in Parkinson's disease; in vitro studies have demonstrated that the disease-associated splice variant α-synuclein 112, but not the full-length protein, cause activation of complement. In vivo, C3d, C4d, C7 and C9 localization in Lewy bodies has been reported. More recently, deposition of iC3b and C9 in Lewy bodies and melanized neurons has been reported, and iC3b immunoreactivity has been shown to be increased with normal ageing and was further elevated in PD vs. age-matched controls. Furthermore, correlation between the ratios of C3/Aβ42 or FH/Aβ42 in CSF and severity of Parkinson's disease motor and cognitive symptoms has been shown. See Carpanini et al., Therapeutic Inhibition of the Complement System in Diseases of the Central Nervous System, Front. Immunol., 04 March 2019. In some embodiments, the subject to be treated suffers from Parkinson's Disease with dementia (PDD).

In certain aspects, active compound described herein, or it pharmaceutically acceptable salt is used to treat Huntington's disease (HD). HD is an autosomal dominant, inherited neurodegenerative disease characterized by progressive motor symptoms, psychiatric disturbances, and dementia. It is caused by expansion of a three-base-pair (CAG) repeat (39-121 repeats vs. normal range 8-39 repeats) in exon 1 of the HTT gene that translates into a polyglutamine tract at the N-terminus of the protein. This results in a polyglutamine length-dependent misfolding and accumulation of huntingtin protein in the striatum and cortex (layers 3, 5, and 6) followed by neuronal loss in these areas which spreads to the hippocampus. It has been shown that neurons, astrocytes, and myelin sheaths in the HD caudate and striatum were immunoreactive for C1q, C4, C3 and neo-epitopes in iC3b and TCC. Expression of mRNA encoding early complement components C1q (c-chain), C1r, C3, and C4, complement regulators C1INH, Clusterin, MCP, DAF and CD59, a See Carpanini et al., Therapeutic Inhibition of the Complement System in Diseases of the Central Nervous System, Front. Immunol., 04 March 2019.nd complement receptors C3a and C5a has been shown to be upregulated in the HD striatum.

In certain aspects, active compound described herein, or it pharmaceutically acceptable salt is used to treat argyrophilic grain dementia, British type amyloid angiopathy, cerebral amyloid angiopathy, Creutzfeldt-Jakob disease, dementia pugilistica, diffuse neurofibrillary tangles with calcification, Down's syndrome, frontotemporal lobar degeneration, Gerstmann-Straussler-Scheinker disease, Hallervorden-Spatz disease, inclusion body myositis, multiple system atrophy (MSA), myotonic dystrophy, Niemann-Pick disease type C, non-Guamanian motor neuron disease with neurofibrillary tangles, postencephalitic parkinsonism, prion protein cerebral amyloid angiopathy, progressive subcortical gliosis, progressive supranuclear palsy, subacute sclerosing panencephalitis, Tangle only dementia, multi-infarct dementia, ischemic stroke, chronic traumatic encephalopathy (CTE), traumatic brain injury (TBI), and stroke.

In certain aspects active compound described herein, or it pharmaceutically acceptable salt is used to treat a hereditary motor and sensory neuropathy (HMSN). In some embodiments, the hereditary and sensory neuropathy is Charcot-Marie-Tooth (CMT) disease. In some embodiments, the HSMN is Charcot-Marie-Tooth disease type 1A or type 1B. In some embodiments, the HSMN is Charcot-Marie-Tooth disease type 2. In some embodiments, the HSMN is Dejerine-Sottas disease (Charcot-Marie-Tooth type 3). In some embodiments, the HSMN is Refsum disease. In some embodiments, the HSMN is Charcot-Marie-Tooth with pyramidal features. In some embodiments, the HSMN is Charcot-Marie-Tooth type 6. In some embodiments, the HSMN is HMSN+retinitis pigmentosa.

In some embodiments, an active compound as described herein is used to treat Churg-Strauss syndrome.

In some embodiments, an active compound as described herein is used to treat a peripheral artery disease (PAD).

In certain aspects, an effective amount of active compound described herein, or it pharmaceutically acceptable salt to treat myasthenia gravis with CNS involvement.

In certain aspects, an effective amount of active compound described herein, or it pharmaceutically acceptable salt is used to treat dementia with Lewy bodies.

In certain aspects, active compound described herein, or it pharmaceutically acceptable salt is used to treat an individual suffering from prion disease.

In certain aspects, active compound described herein, or it pharmaceutically acceptable salt is used to treat Behcet's Disease.

In certain aspects, active compound described herein, or it pharmaceutically acceptable salt is used to treat congenital myasthenia.

In certain aspects, active compound described herein, or it pharmaceutically acceptable salt is used to treat subacute sclerosing panencephalitis (SSPE).

In certain aspects, active compound described herein, or it pharmaceutically acceptable salt is used to treat Guillain-Barré syndrome.

In certain aspects, the CNS disorder to be treated is a demyelinating disease, including, but not limited to demyelinating myelinoclastic diseases and demyelinating leukostrophic disease.

In certain aspects, the disorder to be treated is a demyelinating myelonoclastic disease including, but not limited to, multiple sclerosis, neuromyelitis optica, neuromyelitis optica spectrum of disorders (NMOSD), idiopathic inflammatory demyelinating diseases (IIDD), anti-NMDA receptor encephalitis, acute disseminated encephalomyelitis, anti-MOG autoimmune encephalomyelitis, chronic relapsing inflammatory optic neuritis (CRION), acute disseminated encephalomyelitis (ADEM), immune-mediated encephalomyelitis, progressive multifocal leukoencephalopathy (PML); McDonalds-positive multiple sclerosis, acute hemorrhagic leukoencephalitis, Rasmussen's Encephalitis, Marburg multiple sclerosis, pseudotumefactive and tumefactive multiple sclerosis, Balo concentric sclerosis, diffuse myelinoclastic sclerosis, solitary sclerosis, multiple sclerosis with cavitary lesions, myelocortical multiple sclerosis (MCMS), atypical optic-spinal multiple sclerosis, pure spinal multiple sclerosis, HLA DRB3*02:02 multiple sclerosis, autoimmune GFAP astrocytopathy, Chronic inflammatory demyelinating polyneuropathy (CIDP), Guillain-Barré syndrome, progressive inflammatory neuropathy, Lewis-Sumner Syndrome, combined central and peripheral demyelination (CCPD), Bickerstaff brainstem encephalitis, Fisher syndrome, trigeminal neuralgia, NMDAR anti-NMDA receptor encephalitis, primary progressive MS (PPMS), OPA1 variant multiple sclerosis, KIR4.1 multiple sclerosis, aquaporine-related multiple sclerosis, chronic cerebrospinal venous insufficiency (CCSVI or CCVI), diffuse sclerosis or Schilder's disease,

In certain aspects, the disorder to be treated is a demyelinating leukostrophic disease including, but not limited to, myelitis, central pontine myelinolysis (CPM), extrapontine myelinolysis, tabes dorsalis, progressive multifocal leukoencephalopathy, leukoencephalopathy with vanishing white matter, leukoencephalopathy with neuroaxonal spheroids, reversible posterior leukoencephalopathy syndrome, megalencephalic leukoencephalopathy with subcortical cysts, megalencephalic leukoencephalopathy with subcortical cysts 1, hypertensive leukoencephalopathy, Metachromatic leukodystrophy, Krabbe disease, Canavan disease, X-linked adrenoleukodystrophy, Alexander disease, cerebrotendineous xanthomatosis, Pelizaeus-Merzbacher disease, Refsum disease.

In some embodiments, an active compound as described herein is used to treat Buerger's disease, also known as thromboangiitis obliterans.

In some embodiments, an active compound as described herein is used to treat giant cell arteritis.

In some embodiments, an active compound as described herein is used to treat Raynaud's disease.

In certain aspects, the disorder to be treated is a demyelinating disease of the peripheral nervous system, including, but not limited to, Guillain-Barré syndrome and its chronic counterpart, chronic inflammatory demyelinating polyneuropathy, anti-MAG peripheral neuropathy, Charcot-Marie-Tooth disease and its counterpart Hereditary neuropathy with liability to pressure palsy, Copper deficiency-associated conditions (peripheral neuropathy, myelopathy, and rarely optic neuropathy), and progressive inflammatory neuropathy.

In certain aspects, the disorder to be treated is a neurological inflammatory disorder. In embodiments, the disorder to be treated is cranial arteritis; giant cell arteritis; Holmes-Adie syndrome; inclusion body myositis (IBM); meningitis; neurologic paraneoplastic syndrome including, but not limited to, Lambert-Eaton myasthenic syndrome, stiff-person syndrome, encephalomyelitis (inflammation of the brain and spinal cord), myasthenia gravis, cerebellar degeneration, limbic and/or brainstem encephalitis, neuromyotonia, and opsoclonus (involving eye movement) and sensory neuropathy; polymyositis; transverse myelitis; vasculitis including temporal arteritis; arachnoiditis; Kinsbourne syndrome or opsoclonus myoclonus syndrome (OMS); or Saint Vitus Dance or sydenham chorea (SD) disease.

In some embodiments, an active compound or its salt or composition as described herein is used to treat transverse myelitis.

In certain aspects, the disorder to be treated is a peripheral neuropathy. In some embodiments, the peripheral neuropathy is a mononeuropathy. In some embodiments, the neuropathy is a polyneuropathy. In some embodiments, the polyneuropathy is distal axonopathy, diabetic neuropathy, a demyelinating polyneuropathy, small fiber peripheral neuropathy, mononeuritis multiplex, polyneuritis multiplex, autonomic neuropathy, or neuritis.

**In** some embodiments, an active compound or its salt or composition as described herein is used to treat an autoimmune vascular disease. **In** some embodiments, the autoimmune vascular disease is vasculitis. **In** some embodiments, the vasculitis includes, but is not limited to, autoimmune inflammatory vasculitis, Cutaneous small-vessel vasculitis, Granulomatosis with polyangiitis , Eosinophilic granulomatosis with polyangiitis, Behget's disease, Kawasaki disease, Buerger's disease, and "Limited" granulomatosis with polyangiitis vasculitis.

**In** some embodiments, an active compound or its salt or composition as described herein is used to treat an arteritis. **In** some embodiments, the arteritis includes, but is not limited to, giant cell arteritis, Takayasu arteritis, temporal arteritis, and polyarteritis nodosa.

**In** some embodiments, the complement-mediated disease or disorder comprises transplant rejection. **In** some embodiments, the complement-mediated disease or disorder is antibody-mediated transplant rejection.

**In** certain aspects, an active compound or its salt or composition as described herein is used to treat a proliferative disorder, including, but not limited to, cancer. Targeted cancers suitable for administration of an active compound or its salt described herein include, but are not limited to, estrogen-receptor positive cancer, HER2-negative advanced breast cancer, late-line metastatic breast cancer, liposarcoma, non-small cell lung cancer, liver cancer, ovarian cancer, glioblastoma, refractory solid tumors, retinoblastoma positive breast cancer as well as retinoblastoma positive endometrial, vaginal and ovarian cancers and lung and bronchial cancers, adenocarcinoma of the colon, adenocarcinoma of the rectum, central nervous system germ cell tumors, teratomas, estrogen receptor-negative breast cancer, estrogen receptor-positive breast cancer, familial testicular germ cell tumors, HER2-negative breast cancer, HER2-positive breast cancer, male breast cancer, ovarian immature teratomas, ovarian mature teratoma, ovarian monodermal and highly specialized teratomas, progesterone receptor-negative breast cancer, progesterone receptor-positive breast cancer, recurrent breast cancer, recurrent colon cancer, recurrent extragonadal germ cell tumors, recurrent extragonadal non-seminomatous germ cell tumor, recurrent extragonadal seminomas, recurrent malignant testicular germ cell tumors, recurrent melanomas, recurrent ovarian germ cell tumors, recurrent rectal cancer, stage **III** extragonadal non-seminomatous germ cell tumors, stage **III** extragonadal seminomas, stage **III** malignant testicular germ cell tumors, stage **III** ovarian germ cell tumors, stage IV breast cancers, stage IV colon cancers, stage IV extragonadal non-seminomatous germ cell tumors, stage IV extragonadal seminoma, stage IV melanomas, stage IV ovarian germ cell tumors, stage IV rectal cancers, testicular immature teratomas, testicular mature teratomas. In particular embodiments, the targeted cancers included estrogen-receptor positive, HER2-negative advanced breast cancer, late-line metastatic breast cancer, liposarcoma, non-small cell lung cancer, liver cancer, ovarian cancer, glioblastoma, refractory solid tumors, retinoblastoma positive breast cancer as well as retinoblastoma positive endometrial, vaginal and ovarian cancers and lung and bronchial cancers, metastatic colorectal cancer, metastatic melanoma with CDK4 mutation or amplification, or cisplatin-refractory, unresectable germ cell tumors, lung cancer, bone cancer, pancreatic cancer, skin cancer, cancer of the head or neck, cutaneous or intraocular melanoma, uterine cancer, ovarian cancer, rectal cancer, cancer of the anal region, stomach cancer, colon cancer, breast cancer, uterine cancer, carcinoma of the fallopian tubes, carcinoma of the endometrium, carcinoma of the cervix, carcinoma of the vagina, carcinoma of the vulva, cancer of the esophagus, cancer of the small intestine, cancer of the endocrine system, cancer of the thyroid gland, cancer of the parathyroid gland, cancer of the adrenal gland, sarcoma of soft tissue, cancer of the urethra, cancer of the penis, prostate cancer, cancer of the bladder, cancer of the kidney or ureter, renal cell carcinoma, carcinoma of the renal pelvis, neoplasms of the central nervous system (CNS), primary CNS lymphoma, spinal axis tumors, brain stem glioma, pituitary adenoma, fibrosarcoma, myxosarcoma, chondrosarcoma, osteosarcoma, chordoma, malignant fibrous histiocytoma, hemangiosarcoma, angiosarcoma, lymphangiosarcoma, Mesothelioma, leiomyosarcoma, rhabdomyosarcoma, squamous cell carcinoma; epidermoid carcinoma, malignant skin adnexal tumors, adenocarcinoma, hepatoma, hepatocellular carcinoma, renal cell carcinoma, hypernephroma, cholangiocarcinoma, transitional cell carcinoma, choriocarcinoma, seminoma, embryonal cell carcinoma, glioma anaplastic; glioblastoma multiforme, neuroblastoma, medulloblastoma, malignant meningioma, malignant schwannoma, neurofibrosarcoma, parathyroid carcinoma, medullary carcinoma of thyroid, bronchial carcinoid, pheochromocytoma, Islet cell carcinoma, malignant carcinoid, malignant paraganglioma, melanoma, Merkel cell neoplasm, cystosarcoma phylloide, salivary cancers, thymic carcinomas, bladder cancer, and Wilms tumor, a blood disorder or a hematologic malignancy, including, but not limited to, myeloid disorder, lymphoid disorder, leukemia, lymphoma, myelodysplastic syndrome (MDS), myeloproliferative disease (MPD), mast cell disorder, and myeloma (e.g., multiple myeloma), among others, T-cell or NK-cell lymphoma, for example, but not limited to: peripheral T-cell lymphoma; anaplastic large cell lymphoma, for example anaplastic lymphoma kinase (ALK) positive, ALK negative anaplastic large cell lymphoma, or primary cutaneous anaplastic large cell lymphoma; angioimmunoblastic lymphoma; cutaneous T-cell lymphoma, for example mycosis fungoides, Sézary syndrome, primary cutaneous anaplastic large cell lymphoma, primary cutaneous CD30+ T-cell lymphoproliferative disorder; primary cutaneous aggressive epidermotropic CD8+ cytotoxic T-cell lymphoma; primary cutaneous gamma-delta T-cell lymphoma; primary cutaneous small/medium CD4+ T-cell lymphoma, and lymphomatoid papulosis; Adult T-cell Leukemia/Lymphoma (ATLL); Blastic NK-cell Lymphoma; Enteropathy-type T-cell lymphoma; Hematosplenic gamma-delta T-cell Lymphoma; Lymphoblastic Lymphoma; Nasal NK/T-cell Lymphomas; Treatment-related T-cell lymphomas; for example lymphomas that appear after solid organ or bone marrow transplantation; T-cell prolymphocytic leukemia; T-cell large granular lymphocytic leukemia; Chronic lymphoproliferative disorder of NK-cells; Aggressive NK cell leukemia; Systemic EBV+ T-cell lymphoproliferative disease of childhood (associated with chronic active EBV infection); Hydroa vacciniforme-like lymphoma; Adult T-cell leukemia/ lymphoma; Enteropathy-associated T-cell lymphoma; Hepatosplenic T-cell lymphoma; or Subcutaneous panniculitis-like T-cell lymphoma. **In** some embodiments, the methods described herein can be used to treat a host, for example a human, with a lymphoma or lymphocytic or myelocytic proliferation disorder or abnormality. For example, the methods as described herein can be administered to a host with a Hodgkin Lymphoma or a Non-Hodgkin Lymphoma. For example, the host can have a Non-Hodgkin Lymphoma such as, but not limited to: an AIDS-Related Lymphoma; Anaplastic Large-Cell Lymphoma; Angioimmunoblastic Lymphoma; Blastic NK-Cell Lymphoma; Burkitt's Lymphoma; Burkitt-like Lymphoma (Small Non-Cleaved Cell Lymphoma); Chronic Lymphocytic Leukemia/Small Lymphocytic Lymphoma; Cutaneous T-Cell Lymphoma; Diffuse Large B-Cell Lymphoma; Enteropathy-Type T-Cell Lymphoma; Follicular Lymphoma; Hepatosplenic Gamma-Delta T-Cell Lymphoma; Lymphoblastic Lymphoma; Mantle Cell Lymphoma; Marginal Zone Lymphoma; Nasal T-Cell Lymphoma; Pediatric Lymphoma; Peripheral T-Cell Lymphomas; Primary Central Nervous System Lymphoma; T-Cell Leukemias; Transformed Lymphomas; Treatment-Related T-Cell Lymphomas; or Waldenstrom's Macroglobulinemia, a Hodgkin Lymphoma, such as, but not limited to: Nodular Sclerosis Classical Hodgkin's Lymphoma (CHL); Mixed Cellularity CHL; Lymphocyte-depletion CHL; Lymphocyte-rich CHL; Lymphocyte Predominant Hodgkin Lymphoma; or Nodular Lymphocyte Predominant HL, a specific B-cell lymphoma or proliferative disorder such as, but not limited to: multiple myeloma; Diffuse large B cell lymphoma; Follicular lymphoma; Mucosa-Associated Lymphatic Tissue lymphoma (MALT); Small cell lymphocytic lymphoma; Mediastinal large B cell lymphoma; Nodal marginal zone B cell lymphoma (NMZL); Splenic marginal zone lymphoma (SMZL); Intravascular large B-cell lymphoma; Primary effusion lymphoma; or Lymphomatoid granulomatosis; B-cell prolymphocytic leukemia; Hairy cell leukemia; Splenic lymphoma/leukemia, unclassifiable; Splenic diffuse red pulp small B-cell lymphoma; Hairy cell leukemia-variant; Lymphoplasmacytic lymphoma; Heavy chain diseases, for example, Alpha heavy chain disease, Gamma heavy chain disease, Mu heavy chain disease; Plasma cell myeloma; Solitary plasmacytoma of bone; Extraosseous plasmacytoma; Primary cutaneous follicle center lymphoma; T cell/histiocyte rich large B-cell lymphoma; DLBCL associated with chronic inflammation; Epstein-Barr virus (EBV)+ DLBCL of the elderly; Primary mediastinal (thymic) large B-cell lymphoma; Primary cutaneous DLBCL, leg type; ALK+ large B-cell lymphoma; Plasmablastic lymphoma; Large B-cell lymphoma arising in HHV8-associated multicentric; Castleman disease; B-cell lymphoma, unclassifiable, with features intermediate between diffuse large B-cell lymphoma; or B-cell lymphoma, unclassifiable, with features intermediate between diffuse large B-cell lymphoma and classical Hodgkin lymphoma, a leukemia, for example, an acute or chronic leukemia of a lymphocytic or myelogenous origin, such as, but not limited to: Acute lymphoblastic leukemia (ALL); Acute myelogenous leukemia (AML); Chronic lymphocytic leukemia (CLL); Chronic myelogenous leukemia (CML); juvenile myelomonocytic leukemia (JMML); hairy cell leukemia (HCL); acute promyelocytic leukemia (a subtype of AML); large granular lymphocytic leukemia; or Adult T-cell chronic leukemia. In some embodiments, the patient has an acute myelogenous leukemia, for example an undifferentiated AML (M0); myeloblastic leukemia (M1; with/without minimal cell maturation); myeloblastic leukemia (M2; with cell maturation); promyelocytic leukemia (M3 or M3 variant [M3V]); myelomonocytic leukemia (M4 or M4 variant with eosinophilia [M4E]); monocytic leukemia (M5); erythroleukemia (M6); or megakaryoblastic leukemia (M7), small cell lung cancer, retinoblastoma, HPV positive malignancies like cervical cancer and certain head and neck cancers, MYC amplified tumors such as Burkitts' Lymphoma, and triple negative breast cancer; certain classes of sarcoma, certain classes of non-small cell lung carcinoma, certain classes of melanoma, certain classes of pancreatic cancer, certain classes of leukemia, certain classes of lymphoma, certain classes of brain cancer, certain classes of colon cancer, certain classes of prostate cancer, certain classes of ovarian cancer, certain classes of uterine cancer, certain classes of thyroid and other endocrine tissue cancers, certain classes of salivary cancers, certain classes of thymic carcinomas, certain classes of kidney cancers, certain classes of bladder cancers, and certain classes of testicular cancers.

**In** certain aspects, an active compound or its salt as described herein can be used to preserve or prevent damage to an organ or blood product. For example, an active compound or its salt described herein can be used to prevent damage to an organ, tissue, cell product, or blood product, that has been harvested for transplantation. **In** some embodiments, the organ is the heart, kidney, pancreas, lung, liver, or intestine. **In** some embodiments, the tissue is derived from the cornea, bone, tendon, muscle, heart valve, nerve, artery or vein, or the skin. **In** some embodiments, the blood product is whole blood, plasma, red blood cells or reticulocytes.

**In** some embodiments, an active compound or its salt or composition as described herein prevents or delays the onset of at least one symptom of a complement-mediated disease or disorder in an individual. **In** some embodiment, an active compound or its salt or composition as described herein reduces or eliminates at least one symptom of a complement-mediated disease or disorder in an individual. Examples of symptoms include, but are not limited to, symptoms associated with autoimmune disease, cancer, hematological disease, infectious disease, inflammatory disease, ischemia-reperfusion injury, neurodegenerative disease, neurodegenerative disorder, renal disease, transplant rejection, ocular disease, vascular disease, or a vasculitis disorder. The symptom can be a neurological symptom, for example, impaired cognitive function, memory impairment, loss of motor function, etc. The symptom can also be the activity of factor D protein in a cell, tissue, or fluid of an individual. The symptom can also be the extent of complement activation in a cell, tissue, or fluid of an individual.

**In** some embodiments, administering an active compound or its salt or composition as described herein to an individual modulates complement activation in a cell, tissue, or fluid of an individual. **In** some embodiments, administration of an active compound or its salt or composition as described herein to an individual inhibits complement activation in a cell, tissue, or fluid of an individual. For example, in some embodiments, an active compound or its salt or composition as described herein, when administered in one or more doses as monotherapy or in combination therapy to an individual having a complement-mediated disease or disorder, inhibits complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the compounds described herein.

In some embodiments, an active compound or its salt or composition as described herein reduces C3 deposition onto red blood cells; for example, in some embodiments, an active compound or its salt or composition as described herein reduces deposition of C3b, iC3b, etc., onto RBCs. In some embodiments, an active compound or its salt or composition as described herein inhibits complement-mediated red blood cell lysis.

In some embodiments, an active compound or its salt or composition as described herein reduces C3 deposition onto platelets; for example, in some embodiments, an active compound or its salt or composition as described herein reduces deposition of C3b, iC3b, etc., onto platelets.

In some embodiments, administering an active compound or its salt or composition as described herein results in an outcome selected from the group consisting of: (a) a reduction in complement activation; (b) an improvement in cognitive function; (c) a reduction in neuron loss; (d) a reduction in phospho-Tau levels in neurons; (e) a reduction in glial cell activation; (f) a reduction in lymphocyte infiltration; (g) a reduction in macrophage infiltration; (h) a reduction in antibody deposition, (i) a reduction in glial cell loss; (j) a reduction in oligodendrocyte loss; (k) a reduction in dendritic cell infiltration; (l) a reduction in neutrophil infiltration; (m) a reduction in red blood cell lysis; (n) a reduction in red blood cell phagocytosis; (o) a reduction in platelet phagocytosis; (p) a reduction in platelet lysis; (q) an improvement in transplant graft survival; (r) a reduction in macrophage mediated phagocytosis; (s) an improvement in vision; (t) an improvement in motor control; (u) an improvement in thrombus formation; (v) an improvement in clotting; (w) an improvement in kidney function; (x) a reduction in antibody mediated complement activation; (y) a reduction in autoantibody mediated complement activation; (z) an improvement in anemia; (aa) reduction of demyelination; (ab) reduction of eosinophilia; (ac) a reduction of C3 deposition on red blood cells (e.g., a reduction of deposition of C3b, iC3b, etc., onto RBCs); and (ad) a reduction in C3 deposition on platelets (e.g., a reduction of deposition of C3b, iC3b, etc., onto platelets); and (ae) a reduction of anaphylatoxin toxin production; (af) a reduction in autoantibody mediated blister formation; (ag) a reduction in autoantibody induced pruritis; (ah) a reduction in autoantibody induced erythematosus; (ai) a reduction in autoantibody mediated skin erosion; (aj) a reduction in red blood cell destruction due to transfusion reactions; (ak) a reduction in red blood cell lysis due to alloantibodies; (al) a reduction in hemolysis due to transfusion reactions; (am) a reduction in allo-antibody mediated platelet lysis; (an) a reduction in platelet lysis due to transfusion reactions; (ao) a reduction in mast cell activation; (ap) a reduction in mast cell histamine release; (aq) a reduction in vascular permeability; (ar) a reduction in edema; (as) a reduction in complement deposition on transplant graft endothelium; (at) a reduction of anaphylatoxin generation in transplant graft endothelium; (au) a reduction in the separation of the dermal-epidermal junction; (av) a reduction in the generation of anaphylatoxins in the dermal-epidermal junction; (aw) a reduction in alloantibody mediated complement activation in transplant graft endothelium; (ax) a reduction in antibody mediated loss of the neuromuscular junction; (ay) a reduction in complement activation at the neuromuscular junction; (az) a reduction in anaphylatoxin generation at the neuromuscular junction; (ba) a reduction in complement deposition at the neuromuscular junction; (bb) a reduction in paralysis; (bc) a reduction in numbness; (bd) increased bladder control; (be) increased bowel control; (bf) a reduction in mortality associated with autoantibodies; and (bg) a reduction in morbidity associated with autoantibodies.

**In** some embodiments, an active compound or its salt or composition as described herein, when administered in one or more doses as monotherapy or in combination therapy to an individual having a complement-mediated disease or disorder, is effect to achieve a reduction of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: (a) complement activation; (b) decline in cognitive function; (c) neuron loss; (d) phospho-Tau levels in neurons; (e) glial cell activation; (f) lymphocyte infiltration; (g) macrophage infiltration; (h) antibody deposition, (i) glial cell loss; (j) oligodendrocyte loss; (k) dendritic cell infiltration; (l) neutrophil infiltration; (m) red blood cell lysis; (n) red blood cell phagocytosis; (o) platelet phagocytosis; (p) platelet lysis; (q) transplant graft rejection; (r) macrophage mediated phagocytosis; (s) vision loss; (t) antibody mediated complement activation; (u) autoantibody mediated complement activation; (v) demyelination; (w) eosinophilia; compared to the level or degree of the outcome in the individual before treatment with the active compound.

**In** some embodiments, an active compound or its salt or composition as described herein, when administered in one or more doses as monotherapy or in combination therapy to an individual having a complement-mediated disease or disorder, is effect to achieve an improvement of at least about 10%, at least about 15%, at least about 20%, at least about 25%, at least about 30%, at least about 40%, at least about 50%, at least about 60%, at least about 70%, at least about 80%, at least about 90%, or more than 90%, of one or more of the following outcomes: a) cognitive function; b) transplant graft survival; c) vision; d) motor control; e) thrombus formation; f) clotting; g) kidney function; and h) hematocrit (red blood cell count), compared to the level or degree of the outcome in the individual before treatment with the active compound.

**In** some embodiments, administering an active compound or its salt or composition as described herein to an individual reduces complement activation in the individual. For example, in some embodiments, an active compound or its salt or composition as described herein, when administered in one or more doses as monotherapy or in combination therapy to an individual having a complement-mediated disease or disorder, reduces complement activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to complement activation in the individual before treatment with the active compound or its salt.

**In** some embodiments, administering an active compound or its salt or composition as described herein improves cognitive function in the individual. For example, in some embodiments, an active compound described herein, when administered in one or more doses as monotherapy or in combination therapy to an individual having a complement-mediated disease or disorder, improves cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the cognitive function in the individual before treatment with the active compound.

**In** some embodiments, administering an active compound or its salt or composition as described herein reduces the rate of decline in cognitive function in the individual. For example, in some embodiments, an active compound or its salt, when administered in one or more doses as monotherapy or in combination therapy to an individual having a complement-mediated disease or disorder, reduces the rate of decline of cognitive function in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the rate of decline in cognitive function in the individual before treatment with the active compound or its salt.

**In** some embodiments, administering an active compound or its salt or composition as described herein to an individual reduces neuron loss in the individual. For example, in some embodiments, an active compound or its salt, when administered in one or more doses as monotherapy or in combination therapy to an individual having a complement-mediated disease or disorder, reduces neuron loss in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to neuron loss in the individual before treatment with the active compound.

**In** some embodiments, administering an active compound or its salt or composition as described herein to an individual reduces phospho-Tau levels in the individual. For example, in some embodiments, an active compound or its salt, when administered in one or more doses as monotherapy or in combination therapy to an individual having a complement-mediated disease or disorder, reduces phospho-Tau in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the phospho-Tau level in the individual before treatment with the active compound or its salt.

**In** some embodiments, administering an active compound or its salt or composition as described herein to an individual reduces glial cell activation in the individual. For example, in some embodiments, an active compound or its salt, when administered in one or more doses as monotherapy or in combination therapy to an individual having a complement-mediated disease or disorder, reduces glial activation in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to glial cell activation in the individual before treatment with the active compound or its salt. **In** some embodiments, the glial cells are astrocytes or microglia.

**In** some embodiments, administering an active compound or its salt or composition as described herein to an individual reduces lymphocyte infiltration in the individual. For example, in some embodiments, an active compound or its salt, when administered in one or more doses as monotherapy or in combination therapy to an individual having a complement-mediated disease or disorder, reduces lymphocyte infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to lymphocyte infiltration in the individual before treatment with the active compound or its salt.

**In** some embodiments, administering an active compound or its salt or composition as described herein to an individual reduces macrophage infiltration in the individual. For example, in some embodiments, an active compound or its salt, when administered in one or more doses as monotherapy or in combination therapy to an individual having a complement-mediated disease or disorder, reduces macrophage infiltration in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to macrophage infiltration in the individual before treatment with the active compound or its salt.

**In** some embodiments, administering an active compound or its salt or composition as described herein to an individual reduces antibody deposition in the individual. For example, in some embodiments, an active compound or its salt, when administered in one or more doses as monotherapy or in combination therapy to an individual having a complement-mediated disease or disorder, reduces antibody deposition in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to antibody deposition in the individual before treatment with the active compound or its salt.

**In** some embodiments, administering an active compound or its salt or composition as described herein to an individual reduces anaphylatoxin (e.g., C3a, C4a, C5a) production in an individual. For example, in some embodiments, an active compound or its salt, when administered in one or more doses as monotherapy or in combination therapy to an individual having a complement-mediated disease or disorder, reduces anaphylatoxin production in the individual by at least about 10%, at least about 15%, at least about 20%, at least about 25%, at least about 30%, at least about 40%, at least about 50%, at least about 60%, at least about 70%, at least about 80%, at least about 90%, or more than 90%, compared to the level of anaphylatoxin production in the individual before treatment with the active compound or its salt.

The present disclosure provides for use of an active compound or its salt of the present disclosure or a pharmaceutical composition comprising an active compound or its salt of the present disclosure and a pharmaceutically acceptable excipient to treat an individual having a complement-mediated disease or disorder. **In** some embodiments, the present disclosure provides for use of an active compound or its salt of the present disclosure to treat an individual having a complement-mediated disease or disorder. **In** some embodiments, the present disclosure provides for use of a pharmaceutical composition comprising an active compound or its salt of the present disclosure and a pharmaceutically acceptable excipient to treat an individual having a complement-mediated disease or disorder.

### COMBINATION THERAPY

**In** some embodiments an active compound or its salt or composition as described herein may be provided in combination or alternation with or preceded by, concomitant with or followed by, an effective amount of at least one additional therapeutic agent, for example, for treatment of a disorder listed herein. Non-limiting examples of second active agents for such combination therapy are provided below.

**In** some embodiments, an active compound or its salt or composition as described herein may be provided in combination or alternation with at least one additional inhibitor of the complement system or a second active compound with a different biological mechanism of action. **In** the description below and herein generally, whenever any of the terms referring to an active compound or its salt or composition as described herein are used, it should be understood that pharmaceutically acceptable salts or compositions are considered included, unless otherwise stated or inconsistent with the text.

**In** non-limiting embodiments, an active compound or its salt or composition as described herein may be provided together with a protease inhibitor, a soluble complement regulator, a therapeutic antibody (monoclonal or polyclonal), complement component inhibitor, receptor agonist, or siRNA.

In other embodiments, an active compound described herein is administered in combination or alternation with an antibody against tumor necrosis factor (TNF), including but not limited to infliximab (Remicade), adalimumab, certolizumab, golimumab, or a receptor fusion protein such as etanercept (Embrel).

In another embodiment, an active compound as described herein can be administered in combination or alternation with an anti-CD20 antibody, including but not limited to rituximab (Rituxan), adalimumab (Humira), ofatumumab (Arzerra), tositumomab (Bexxar), obinutuzumab (Gazyva), or ibritumomab (Zevalin).

In an alternative embodiment, an active compound as described herein can be administered in combination or alternation with an anti-IL6 antibody, including but not limited to tocilizumab (Actemra) and siltuximab (Sylvant).

In an alternative embodiment, an active compound as described herein can be administered in combination or alternation with an IL17 inhibitor, including but not limited to secukibumab (Cosentyx).

In an alternative embodiment, an active compound as described herein can be administered in combination or alternation with a p40 (IL12/IL23) inhibitor, including but not limited to ustekinumab (Stelara).

In an alternative embodiment, an active compound as described herein can be administered in combination or alteration with an IL23 inhibitor, including but not limited to risankizumab.

In an alternative embodiment, an active compound as described herein can be administered in combination or alteration with an anti-interferon α antibody, for example but not limited to sifalimumab.

In an alternative embodiment, an active compound as described herein can be administered in combination or alteration with a kinase inhibitor, for example but not limited to a JAK1/JAK3 inhibitor, for example but not limited to tofacitinib (Xelianz). In an alternative embodiment, an active compound as described herein can be administered in combination or alteration with a JAK1/JAK2 inhibitor, for example but not limited to baracitibib.

In an alternative embodiment, an active compound as described herein can be administered in combination or alteration with an anti-VEGF agent, for example but not limited to: aflibercept (Eylea^{®}; Regeneron Pharmaceuticals); ranibizumab (Lucentis^{®}: Genentech and Novartis); pegaptanib (Macugen^{®}; OSI Pharmaceuticals and Pfizer); bevacizumab (Avastin; Genentech/Roche); lapatinib (Tykerb); sunitinib (Sutent); axitinib (Inlyta); pazopanib; sorafenib (Nexavar); ponatinib (Inclusig); regorafenib (Stivarga); cabozantinib (Abometyx; Cometriq); vendetanib (Caprelsa); ramucirumab (Cyramza); lenvatinib (Lenvima); ziv-aflibercept (Zaltrap); cediranib (Recentin); anecortane acetate, squalamine lactate, and corticosteroids.

In another embodiment, an active compound as described herein can be administered in combination or alternation with an immune checkpoint inhibitor. Non-limiting examples of checkpoint inhibitors include anti-PD-1 or anti-PDL1 antibodies, for example, nivolumab (Opdivo), pembrolizumab (Keytruda), pidilizumab, AMP-224 (AstraZeneca and MedImmune), PF-06801591 (Pfizer), MEDI0680 (AstraZeneca), PDR001 (Novartis), REGN2810 (Regeneron), SHR-12-1 (Jiangsu Hengrui Medicine Company and Incyte Corporation), TSR-042 (Tesaro), and the PD-L1/VISTA inhibitor CA-170 (Curis Inc.), atezolizumab, durvalumab, and KN035, or anti-CTLA4 antibodies, for example Ipilimumab, Tremelimumab, AGEN1884 and AGEN2041 (Agenus).

Non-limiting examples of active agents that can be used in combination with active compounds described herein are:
Protease inhibitors: plasma-derived C1-INH concentrates, for example Cetor^{®} (Sanquin), Berinert-P^{®} (CSL Behring, Lev Pharma), and Cinryze^{®}; recombinant human C1-inhibitors, for example Rhucin^{®}; ritonavir (Norvir^{®}, Abbvie, Inc.);
Soluble complement regulators: Soluble complement receptor 1 (TP10) (Avant Immunotherapeutics); sCRI-sLe^{x}/TP-20 (Avant Immunotherapeutics); MLN-2222 /CAB-2 (Millenium Pharmaceuticals); Mirococept (Inflazyme Pharmaceuticals); Therapeutic antibodies: Eculizumab/Soliris and Ravulizumab/Ultomiris (Alexion Pharmaceuticals); Pexelizumab (Alexion Pharmaceuticals); ABP959 (Amgen); BOWo8o (Epirus Biopharmaceuticals); SB12 (Samsung Bioepis); Ofatumumab (Genmab A/S); TNX-234 (Tanox); TNX-558 (Tanox); TA106 (Taligen Therapeutics); Neutrazumab (G2 Therapies); Anti-properdin (Novelmed Therapeutics); HuMax-CD38 (Genmab A/S);
Complement component inhibitors: Compstatin/POT-4 (Potentia Pharmaceuticals); ARC1905 (Archemix); 4(1MEW)APL-1,APL-2 (Appelis); CP40/AMY-101,PEG-Cp40 (Amyndas);
PDGF inhibitors: Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; Anti-factor H or anti-factor B agents: Anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas);
Complement C3 or CAP C3 Convertase targeting molecules: TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH; Anti-CR3, anti-MASP2, anti C1s, and anti-C1n molecules: Cynryze (ViroPharma/Baxter); TNT003 (True North); OMS721 (Omeros); OMS906 (Omeros); and Imprime PGG (Biothera);
Receptor agonists: PMX-53 (Peptech Ltd.); JPE-137 (Jerini); JSM-7717 (Jerini);
Others: Recombinant human MBL (rhMBL; Enzon Pharmaceuticals); Imides and glutarimide derivatives such as thalidomide, lenalidomide, pomalidomide; Additional non-limiting examples that can be used in combination or alternation with an active compound or its salt or composition as described herein include the following.

| **Non-limiting examples for combination therapy** | | | |
|---|---|---|---|
| **Name** | **Target** | **Company** | **Class of Molecule** |
| LFG316 | C5 | Novartis/Morphosys | Monoclonal antibody |
| SB12 | C5 | Samsung Bioepsis | |
| IONIS-FB-LRx | CFB | Ionis | Antisense Inhibitor |
| 4(1MEW)APL-1,APL-2 | C3/C3b | Apellis | Compstatin Family |
| 4(1MeW)POT-4 | C3/C3b | Potentia | Compstatin Family |
| Anti-C5 siRNA | C5 | Alnylam | Si-RNA |
| Anti-FB siRNA | CFB | Alnylam | SiRNA |
| ARC1005 | C5 | Novo Nordisk | Aptamers |
| ATA | C5 | N.A. | Chemical |
| Coversin | C5 | Volution Immuno-Pharmaceuticals | Small animal protein |
| CP40/AMY-101,PEG-Cp40 | C3/C3b | Amyndas | Compstatin Family |
| CRIg/CFH | CAP C3 convertase | NA | CFH-based protein |
| Cynryze | C1n/C1s | ViroPharma/Baxter | Human purified protein |
| FCFD4514S | CFD | Genentech/Roche | Monoclonal antibody |
| H17 | C3 (C3b/iC3b) | EluSys Therapeutics | Monoclonal antibody |
| Mini-CFH | CAP C3 convertase | Amyndas | CFH-based protein |
| Mirococept (APT070) | CAP and CCP C3 | NA | CR1-based protein |
| Mubodine | C5 | Adienne | Monoclonal antibody |
| RA101348 | C5 | Rapharma | Small molecule |
| sCR1 (CDX-1135) | CAP and CP C3 | Celldex | CR1-based protein |
| SOBI002 | C5 | Swedish Orphan Biovitrum | Affibody |
| SOMAmers | C5 | SomaLogic | Aptamers |
| SOMAmers | CFB and CFD | SomaLogic | Aptamers (SELEX) |
| TA106 | CFB | Alexion Pharmaceuticals | Monoclonal antibody |
| TNT003 | C1s | True North | Monoclonal antibody |
| TT30 (CR2/CFH) | CAP C3 convertase | Alexion | CFH-based protein |
| TT32 (CR2/CR1) | CAP and CCP C3 | Alexion Pharmaceuticals | CR1-based protein |
| Nafamostat (FUT-175, Futhan) | Cls, CFD, other proteases | Torri Pharmaceuticals | Small molecule |
| OMS721 | MASP-2 | Omeros | Monoclonal antibody |
| OMS906 | MASP-2 | Omeros | Monoclonal antibody |
| Bikaciomab, NM9308 | CFB | Novelmed | Monoclonal antibody |
| NM9401 | Properdin | Novelmed | Monoclonal antibody |
| CVF, HC-1496 | C3 | InCode | Recombinant peptide |
| ALXN1102/ALXN1103 (TT30) | C3-conv, C3b | Alexion Pharmaceuticals | Regulator |
| rFH | C3-conv, C3b | Optherion | Regulator |
| 5C6, AMY-301 | CFH | Amyndas | Regulator |
| Erdigna | C5 | Adienne Pharma | Antibody |
| ARC1905 | C5 | Opthotech | Monoclonal Antibody |
| MEDI7814 | C5/C5a | MedImmune | Monoclonal Antibody |
| NOX-D19 | C5a | Noxxon | Aptamer (Spiegelmer) |
| IFX-1, CaCP29 | C5a | InflaRx | Monoclonal Antibody |
| PMX53, PMX205 | C5aR | Cephalon, Teva | Peptidomimetic |
| CCX168 | C5aR | ChemoCentryx | Small molecule |
| ADC-1004 | C5aR | Alligator Bioscience | Small molecule |
| Anti-C5aR-151, NN8209; Anti-C5aR-215, NN8210 | C5aR | Novo Nordisk | Monoclonal Antibody |
| Imprime PGG | CR3 | Biothera | Soluble beta-glucan |
| ANX005; ANX007 | Clq | Annexon | Monoclonal Antibody |
| Lampalizumab | fD | Roche | Monoclonal Antibody |
| avacincaptad pegol | C5 | Opthotech | Aptamer |
| regenemab | C6 | Regenesance | Monoclonal Antibody |
| BIVV020 | C1s | Bioverativ | Monoclonal Antibody |
| PRO-02 | C2 | Broteio/Argen-x | Monoclonal Antibody |
| 5C6, compsorbin | fH | Amyndas | Peptide |
| SOBI005 | C5 | Sobi | Protein |
| ISU305 | C5 | ISU ABXIS | Monoclonal Antibody |
| Mubodina | C5 | Adienne | Monoclonal Antibody |
| IFX-2, IFX-3 | C5a | InflaRx | Monoclonal Antibody |
| ALS-205 | C5aRl | Alsonex | Peptide |
| DF2593A | C5aRl | Dompé | Small Molecule |
| IPH5401 | C5aRl | Innate Pharma | Monoclonal Antibody |
| C6-LNA | C6 | Regenesance | Oligonucleotide |
| SKY59 | C5 | Roche | Monoclonal Antibody |
| REGN3918 | C5 | Regeneron | Monoclonal Antibody |
| Aptamers to Factor D | fD | Vitrisa Therapeutics | Aptamer |
| CLG561 | Properdin | Novartis | Monoclonal Antibody |
| Tesidolumab; LFG316 | C5 | Novartis and MorphoSys | Monoclonal Antibody |

In one embodiment the agent for combination therapy is a biosimilar of any agent named in the above table.

In some embodiments, an active compound or its salt or composition as described herein may be provided together with a compound that inhibits an enzyme that metabolizes an administered protease inhibitor. In some embodiments, a compound or salt may be provided together with ritonavir.

In some embodiments, an active compound or its salt or composition as described herein may be provided in combination with a complement C5 inhibitor or C5 convertase inhibitor. In another embodiment, an active compound or its salt or composition as described herein may be provided in combination with eculizumab, a monoclonal antibody directed to the complement factor C5 and manufactured and marketed by Alexion Pharmaceuticals under the tradename Soliris. Eculizumab has been approved by the U.S. FDA for the treatment of PNH, aHUS, and myasthenia gravis. In another embodiment, an active compound or its salt or composition as described herein may be provided in combination with ravulizumab, a monoclonal antibody directed to the complement factor C5 and manufactured and marketed by Alexion Pharmaceuticals under the tradename Ultomiris. In another embodiment, an active compound or its salt or composition as described herein may be provided in combination with ABP959, a monoclonal antibody directed to the complement factor C5 and manufactured and marketed by Amgen. In another embodiment, an active compound or its salt or composition as described herein may be provided in combination with BOWo8o, a monoclonal antibody directed to the complement factor C5 and manufactured and marketed by Epirus Biopharmaceuticals. In another embodiment, an active compound or its salt or composition as described herein may be provided in combination with SB12, a monoclonal antibody directed to the complement factor C5 and manufactured and marketed by Samsung Bioepis.

In some embodiments, an active compound or its salt or composition as described herein may be provided together with a compound that inhibits Complement Factor D. In some embodiments of the invention, an active compound or its salt or composition as described herein as described herein can be used in combination or alternation with a compound described in Biocryst Pharmaceuticals US Pat. No. 6,653,340 titled "Compounds useful in the complement, coagulate and kallikrein pathways and method for their preparation" describes fused bicyclic ring compounds that are potent inhibitors of Factor D; Novartis PCT patent publication WO2012/093101 titled "Indole compounds or analogs thereof useful for the treatment of age-related macular degeneration" describes certain Factor D inhibitors; Novartis PCT patent publications WO2013/164802, WO2013/192345, WO2014/002051, WO2014/002052, WO2014/002053, WO2014/002054, WO2014/002057, WO2014/002058, WO2014/002059, WO2014/005150, WO2014/009833, WO2014/143638, WO2015/009616, WO2015/009977, WO2015/066241, Bristol-Myers Squibb PCT patent publication WO2004/045518 titled "Open chain prolyl urea-related modulators of androgen receptor function"; Japan Tobacco Inc. PCT patent publication WO1999/048492 titled "Amide derivatives and nociceptin antagonists"; Ferring B.V. and Yamanouchi Pharmaceutical Co. LTD. PCT patent publication WO1993/020099 titled $CCK - "$ and/or gastrin receptor ligands"; Alexion Pharmaceuticals PCT patent publication WO1995/029697 titled "Methods and compositions for the treatment of glomerulonephritis and other inflammatory diseases"; or Achillion Pharmaceuticals filed PCT Patent Application No. PCT/US2015/017523 and U.S. Patent Application No. 14/631,090 titled "Alkyne Compounds for Treatment of Complement Mediated Disorders"; PCT Patent Application No. PCT/US2015/017538 and U.S. Patent Application No. 14/631,233 titled "Amide Compounds for Treatment of Complement Mediated Disorders"; PCT Patent Application No. PCT/US2015/017554 and U.S. Patent Application No. 14/631,312 titled "Amino Compounds for Treatment of Complement Mediated Disorders"; PCT Patent Application No. PCT/US2015/017583 and U.S. Patent Application No. 14/631,440 titled "Carbamate, Ester, and Ketone Compounds for Treatment of Complement Mediated Disorders"; PCT Patent Application No. PCT/US2015/017593 and U.S. Patent Application No. 14/631,625 titled "Aryl, Heteroaryl, and Heterocyclic Compounds for Treatment of Complement Mediated Disorders"; PCT Patent Application No. PCT/US2015/017597 and U.S. Patent Application No. 14/631,683 titled "Ether Compounds for Treatment of Complement Mediated Disorders"; PCT Patent Application No. PCT/US2015/017600 and U.S. Patent Application No. 14/631,785 titled "Phosphonate Compounds for Treatment of Complement Mediated Disorders"; and PCT Patent Application No. PCT/US2015/017609 and U.S. Patent Application No. 14/631,828 titled "Compounds for Treatment of Complement Mediated Disorders."

In some embodiments, an active compound or its salt or composition as described herein is administered in combination with an anti-inflammatory drug, antimicrobial agent, anti-angiogenesis agent, immunosuppressant, antibody, steroid, ocular antihypertensive drug or combinations thereof. Examples of such agents include amikacin, anecortane acetate, anthracenedione, anthracycline, an azole, amphotericin B, bevacizumab, camptothecin, cefuroxime, chloramphenicol, chlorhexidine, chlorhexidine digluconate, clortrimazole, a clotrimazole cephalosporin, corticosteroids, dexamethasone, desamethazone, econazole, eftazidime, epipodophyllotoxin, fluconazole, flucytosine, fluoropyrimidines, fluoroquinolines, gatifloxacin, glycopeptides, imidazoles, itraconazole, ivermectin, ketoconazole, levofloxacin, macrolides, miconazole, miconazole nitrate, moxifloxacin, natamycin, neomycin, nystatin, ofloxacin, polyhexamethylene biguanide, prednisolone, prednisolone acetate, pegaptanib, platinum analogs, polymicin B, propamidine isethionate, pyrimidine nucleoside, ranibizumab, squalamine lactate, sulfonamides, triamcinolone, triamcinolone acetonide, triazoles, vancomycin, anti-vascular endothelial growth factor (VEGF) agents, VEGF antibodies, VEGF antibody fragments, vinca alkaloid, timolol, betaxolol, travoprost, latanoprost, bimatoprost, brimonidine, dorzolamide, acetazolamide, pilocarpine, ciprofloxacin, azithromycin, gentamycin, tobramycin, cefazolin, voriconazole, gancyclovir, cidofovir, foscarnet, diclofenac, nepafenac, ketorolac, ibuprofen, indomethacin, fluoromethalone, rimexolone, anecortave, cyclosporine, methotrexate, tacrolimus, anti-PDGFR molecule, and combinations thereof.

In some embodiments of the present invention, an active compound or its salt or composition as described herein can be administered in combination or alternation with at least one immunosuppressive agent. The immunosuppressive agent as non-limiting examples, may be a calcineurin inhibitor, e.g. a cyclosporin or an ascomycin, e.g. Cyclosporin A (NEORAL^{®}), FK506 (tacrolimus), pimecrolimus, a mTOR inhibitor, e.g. rapamycin or a derivative thereof, e.g. Sirolimus (RAPAMUNE^{®}), Everolimus (Certican^{®}), temsirolimus, zotarolimus, biolimus-7, biolimus-9, a rapalog, e.g.ridaforolimus, azathioprine, campath 1H, a S1P receptor modulator, e.g. fingolimod or an analog thereof, an anti IL-8 antibody, mycophenolic acid or a salt thereof, e.g. sodium salt, or a prodrug thereof, e.g. Mycophenolate Mofetil (CELLCEPT^{®}), OKT3 (ORTHOCLONE OKT3^{®}), Prednisone, ATGAM^{®}, THYMOGLOBULIN^{®}, Brequinar Sodium, OKT4, T10B9.A-3A, 33B3.1, 15-deoxyspergualin, tresperimus, Leflunomide ARAVA^{®}, CTLAI-Ig, anti-CD25, anti-IL2R, Basiliximab (SIMULECT^{®}), Daclizumab (ZENAPAX^{®}), mizorbine, methotrexate, dexamethasone, ISAtx-247, SDZ ASM 981 (pimecrolimus, Elidel^{®}), CTLA4lg (Abatacept), belatacept, LFA3lg, etanercept (sold as Enbrel^{®} by Immunex), adalimumab (Humira^{®}), infliximab (Remicade^{®}), an anti-LFA-1 antibody, natalizumab (Antegren^{®}), Enlimomab, gavilimomab, antithymocyte immunoglobulin, siplizumab, Alefacept efalizumab, pentasa, mesalazine, asacol, codeine phosphate, benorylate, fenbufen, naprosyn, diclofenac, etodolac and indomethacin, tocilizumab (Actemra), siltuximab (Sylvant), secukibumab (Cosentyx), ustekinumab (Stelara), risankizumab, sifalimumab, aspirin and ibuprofen.

Examples of anti-inflammatory agents include methotrexate, dexamethasone, dexamethasone alcohol, dexamethasone sodium phosphate, fluromethalone acetate, fluromethalone alcohol, lotoprendol etabonate, medrysone, prednisolone acetate, prednisolone sodium phosphate, difluprednate, rimexolone, hydrocortisone, hydrocortisone acetate, lodoxamide tromethamine, aspirin, ibuprofen, suprofen, piroxicam, meloxicam, flubiprofen, naproxan, ketoprofen, tenoxicam, diclofenac sodium, ketotifen fumarate, diclofenac sodium, nepafenac, bromfenac, flurbiprofen sodium, suprofen, celecoxib, naproxen, rofecoxib, glucocorticoids, diclofenac, and any combination thereof. In some embodiments, an active compound or its salt or composition as described herein is combined with one or more non-steroidal anti-inflammatory drugs (NSAIDs) selected from naproxen sodium (Anaprox), celecoxib (Celebrex), sulindac (Clinoril), oxaprozin (Daypro), salsalate (Disalcid), diflunisal (Dolobid), piroxicam (Feldene), indomethacin (Indocin), etodolac (Lodine), meloxicam (Mobic), naproxen (Naprosyn), nabumetone (Relafen), ketorolac tromethamine (Toradol), naproxen/esomeprazole (Vimovo), and diclofenac (Voltaren), and combinations thereof.

In some embodiments, an active compound or its salt or composition as described herein is administered in combination or alteration with an omega-3 fatty acid or a peroxisome proliferator-activated receptor (PPARs) agonist. Omega-3 fatty acids are known to reduce serum triglycerides by inhibiting DGAT and by stimulating peroxisomal and mitochondrial beta oxidation. Two omega-3 fatty acids, eicosapentaenoic acid (EPA) and docosahexaenoic acid (DHA), have been found to have high affinity for both PPAR-alpha and PPAR-gamma. Marine oils, e.g., fish oils, are a good source of EPA and DHA, which have been found to regulate lipid metabolism. Omega-3 fatty acids have been found to have beneficial effects on the risk factors for cardiovascular diseases, especially mild hypertension, hypertriglyceridemia and on the coagulation factor VII phospholipid complex activity. Omega-3 fatty acids lower serum triglycerides, increase serum HDL- cholesterol, lower systolic and diastolic blood pressure and the pulse rate, and lower the activity of the blood coagulation factor VII-phospholipid complex. Further, omega-3 fatty acids seem to be well tolerated, without giving rise to any severe side effects. One such form of omega-3 fatty acid is a concentrate of omega-3, long chain, polyunsaturated fatty acids from fish oil containing DHA and EPA and is sold under the trademark Omacor^{®}. Such a form of omega-3 fatty acid is described, for example, in U.S. Patent Nos. 5,502,077, 5,656,667 and 5,698,594.

Peroxisome proliferator-activated receptors (PPARs) are members of the nuclear hormone receptor superfamily ligand-activated transcription factors that are related to retinoid, steroid and thyroid hormone receptors. There are three distinct PPAR subtypes that are the products of different genes and are commonly designated PPAR-alpha, PPAR-beta/delta (or merely, delta) and PPAR-gamma. General classes of pharmacological agents that stimulate peroxisomal activity are known as PPAR agonists, e.g., PPAR-alpha agonists, PPAR-gamma agonists and PPAR-delta agonists. Some pharmacological agents are combinations of PPAR agonists, such as alpha/gamma agonists, etc., and some other pharmacological agents have dual agonist/antagonist activity. Fibrates such as fenofibrate, bezafibrate, clofibrate and gemfibrozil, are PPAR-alpha agonists and are used in patients to decrease lipoproteins rich in triglycerides, to increase HDL and to decrease atherogenic-dense LDL. Fibrates are typically orally administered to such patients. Fenofibrate or 2-[4-(4-chlorobenzoyl)phenoxy]-2-methyl-propanoic acid, 1-methylethyl ester, has been known for many years as a medicinally active principle because of its efficacy in lowering blood triglyceride and cholesterol levels.

In some embodiments, the compound as described herein is provided for use in a method of treating or preventing age-related macular degeneration (AMD) by administering to a subject in need thereof an effective amount of an active compound or its salt or composition as described herein in combination with an anti-VEGF agent. Non-limiting examples of anti-VEGF agents include, but are not limited to, aflibercept (Eylea^{®}; Regeneron Pharmaceuticals); ranibizumab (Lucentis^{®}: Genentech and Novartis); pegaptanib (Macugen^{®}; OSI Pharmaceuticals and Pfizer); bevacizumab (Avastin; Genentech/Roche); lapatinib (Tykerb); sunitinib (Sutent); axitinib (Inlyta); pazopanib; sorafenib (Nexavar); ponatinib (Inclusig); regorafenib (Stivarga); Cabozantinib (Abometyx; Cometriq); vendetanib (Caprelsa); ramucirumab (Cyramza); lenvatinib (Lenvima); ziv-aflibercept (Zaltrap); cediranib (Recentin); anecortane acetate, squalamine lactate, and corticosteroids, including, but not limited to, triamcinolone acetonide.

In some embodiments, the compound as described herein is provided for use in a method of treating or preventing age-related macular degeneration (AMD) by administering to a subject in need thereof an effective amount of an active compound or its salt or composition as described herein in combination with a complement C5 inhibitor, for example, a complement C5 inhibitor described herein and in the table above titled Non-limiting examples of potential therapeutics for combination therapy, including, but not limited to, eculizumab; ravulizumab; ABP959 (Amgen); BOWo8o (Epirus Biopharmaceuticals); SB12 (Samsung Bioepis); LFG316 (Novartis/Morphosys); Anti-C5 siRNA (Alnylam); ARC1005 (Novo Nordisk); Coversin (Volution Immuno-Pharmaceuticals); Mubodine (Adienne Pharma); RA101348 (Ra Pharma); SOBI002 (Swedish Orphan Biovitrum); SOMAmers (SomaLogic); Erdigna (Adienne Pharma); ARC1905 (Opthotech); MEDI7814 (MedImmune); NOX-D19 (Noxxon); IFX-1, CaCP29 (InflaRx); PMX53, PMX205 (Cephalon, Teva); CCX168 (ChemoCentryx); ADC-1004 (Alligator Bioscience); and Anti-C5aR-151, NN8209; Anti-C5aR-215, NN8210 (Novo Nordisk).

In some embodiments, the present invention provides an active compound or its salt or composition as described herein for use in a method of treating or preventing age-related macular degeneration (AMD) by administering to a subject in need thereof an effective amount of the active compound or its salt or composition as described herein in combination with anti-properidin agent, for example, an anti-properidin agent as described above, including but not limited to NM9401 (Novelmed).

In some embodiments, the present invention provides an active compound or its salt or composition as described herein for use in a method of treating or preventing age-related macular degeneration (AMD) by administering to a subject in need thereof an effective amount of the active compound or its salt or composition as described herein in combination with a complement C3 inhibitor for example, a complement C3 inhibitor described above, including, but not limited to, a compstatin or compstatin analog, for example Compstatin/POT-4 (Potentia Pharmaceuticals); ARC1905 (Archemix); 4(1MEW)APL-1,APL-2 (Appelis); CP40/AMY-101,PEG-Cp40 (Amyndas) Complement C3 or CAP C3 Convertase targeting molecules: TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); and CRIg/CFH.

In some embodiments, the present invention provides an active compound or its salt or composition as described herein for use in a method of treating or preventing age-related macular degeneration (AMD) by administering to a subject in need thereof an effective amount of the active compound or its salt or composition as described herein in combination with an anti-factor H or anti-factor B agent selected from Anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas).

In some embodiments, the present invention provides an active compound or its salt or composition as described herein for use in a method of treating or preventing age-related macular degeneration (AMD) by administering to a subject in need thereof an effective amount of the active compound or its salt or composition as described herein in combination with an anti-MASP2, anti-C1s or anti-CR3 molecules, for example, but not limited to: Cynryze (ViroPharma/Baxter); TNT003 (True North); OMS721 (Omeros); OMS906 (Omeros); and Imprime PGG (Biothera).

In some embodiments, the present invention provides an active compound or its salt or composition as described herein for use in a method of treating or preventing age-related macular degeneration (AMD) by administering to a subject in need thereof an effective amount of the active compound or its salt or composition as described herein in combination with an PDGF inhibitor, for example as described herein including but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil.

In some embodiments, the present invention provides an active compound or its salt or composition as described herein for use in a method of treating or preventing paroxysmal nocturnal hemoglobinuria (PNH) by administering to a subject in need thereof an effective amount of the active compound or its salt or composition as described herein with an additional inhibitor of the complement system or another active compound with a different biological mechanism of action. In another embodiment, the present invention provides an active compound or its salt or composition as described herein for use in a method of treating or preventing paroxysmal nocturnal hemoglobinuria (PNH) by administering to a subject in need thereof an effective amount of the active compound or its salt or composition as described herein in combination or alternation with eculizumab, ravulizumab, ABP959 (Amgen), BOWo8o (Epirus Biopharmaceuticals), or SB12 (Samsung Bioepis). In another embodiment, the present invention provides an active compound or its salt or composition as described herein for use in a method of treating or preventing paroxysmal nocturnal hemoglobinuria (PNH) by administering to a subject in need thereof an effective amount of the active compound or its salt or composition as described herein in combination or alternation with CP40. In some embodiments, the additional agent is PEGylated-CP40. CP40 is a peptide inhibitor that shows a strong binding affinity for C3b and inhibits hemolysis of paroxysmal nocturnal hemoglobinuria (PNH) erythrocytes. In some embodiments, the additional agent is a complement component inhibitor, for example but not limited to Compstatin/POT-4 (Potentia Pharmaceuticals); ARC1905 (Archemix); 4(1MEW)APL-1,APL-2 (Appelis); CP40/AMY-1 01,PEG-Cp40 (Amyndas); a PDGF inhibitor, for example, but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; an anti-factor H or anti-factor B agent, for example anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas); a complement C3 or CAP C3 convertase targeting molecule, for example but not limited to TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH, an anti-CR3, anti-MASP2, anti C1s, or anti-C1n molecule, for example but not limited to Cynryze (ViroPharma/Baxter); TNT003 (True North); OMS721 (Omeros); OMS906 (Omeros); and Imprime PGG (Biothera)

In some embodiments, the present invention provides an active compound or its salt or composition as described herein for use in a method of treating or preventing rheumatoid arthritis by administering to a subject in need thereof an effective amount of a composition comprising the active compound or its salt or composition as described herein in combination or alternation with an additional inhibitor of the complement system, or an active agent that functions through a different mechanism of action. In another embodiment, the present invention provides an active compound or its salt or composition as described herein for use in a method of treating or preventing rheumatoid arthritis by administering to a subject in need thereof an effective amount of the active compound or its salt or composition as described herein in combination or alternation with methotrexate. In certain embodiments, an active compound or its salt or composition as described herein is administered in combination or alternation with at least one additional therapeutic agent selected from: salicylates including aspirin (Anacin, Ascriptin, Bayer Aspirin, Ecotrin) and salsalate (Mono-Gesic, Salgesic); nonsteroidal anti-inflammatory drugs (NSAIDs); nonselective inhibitors of the cyclo-oxygenase (COX-1 and COX-2) enzymes, including diclofenac (Cataflam, Voltaren), ibuprofen (Advil, Motrin), ketoprofen (Orudis), naproxen (Aleve, Naprosyn), piroxicam (Feldene), etodolac (Lodine), indomethacin, oxaprozin (Daypro), nabumetone (Relafen), and meloxicam (Mobic); selective cyclo-oxygenase-2 (COX-2) inhibitors including Celecoxib (Celebrex); disease-modifying antirheumatic drugs (DMARDs), including azathioprine (Imuran), cyclosporine (Sandimmune, Neoral), gold salts (Ridaura, Solganal, Aurolate, Myochrysine), hydroxychloroquine (Plaquenil), leflunomide (Arava), methotrexate (Rheumatrex), penicillamine (Cuprimine), and sulfasalazine (Azulfidine); biologic drugs including abatacept (Orencia), etanercept (Enbrel), infliximab (Remicade), adalimumab (Humira), and anakinra (Kineret); corticosteroids including betamethasone (Celestone Soluspan), cortisone (Cortone), dexamethasone (Decadron), methylprednisolone (SoluMedrol, DepoMedrol), prednisolone (Delta-Cortef), prednisone (Deltasone, Orasone), and triamcinolone (Aristocort); gold salts, including Auranofin (Ridaura); Aurothioglucose (Solganal); Aurolate; Myochrysine; or any combination thereof.

In some embodiments, the present invention provides an active compound or its salt or composition as described herein for use in a method of treating or preventing multiple sclerosis by administering to a subject in need thereof an effective amount of the active compound or its salt or composition as described herein in combination or alternation with an additional inhibitor of the complement system, or an active agent that functions through a different mechanism of action. In another embodiment, the present invention provides an active compound or its salt or composition as described herein for use in a method of treating or preventing multiple sclerosis by administering to a subject in need thereof an effective amount of the active compound or its salt or composition as described herein in combination or alternation with a corticosteroid. Examples of corticosteroids include, but are not limited to, prednisone, dexamethasone, solumedrol, and methylprednisolone. In some embodiments, an active compound or its salt or composition as described herein is combined with at least one anti-multiple sclerosis drug, for example, selected from: Aubagio (teriflunomide), Avonex (interferon beta-1a), Betaseron (interferon beta-1b), Copaxone (glatiramer acetate), Extavia (interferon beta-1b), Gilenya (fingolimod), Lemtrada (alemtuzumab), Novantrone (mitoxantrone), Plegridy (peginterferon beta-1a), Rebif (interferon beta-1a), Tecfidera (dimethyl fumarate), Tysabri (natalizumab), Solu-Medrol (methylprednisolone), High-dose oral Deltasone (prednisone), H.P. Acthar Gel (ACTH), or a combination thereof.

In some embodiments, an active compound or its salt or composition as described herein is useful in a combination with another pharmaceutical agent to ameliorate or reduce a side effect of the agent. For example, in some embodiments, an active compound or its salt or composition as described herein may be used in combination with adoptive cell transfer therapies to reduce an associated inflammatory response associated with such therapies, for example, a cytokine mediated response such as cytokine release syndrome. In some embodiments, the adoptive cell transfer therapy includes the use of a chimeric antigen receptor T-Cell (CAR T). In some embodiments, the adoptive cell transfer therapy includes the use of a chimeric antigen receptor T-Cell (CAR T) or a dendritic cell to treat a hematologic or solid tumor, for example, a B-cell related hematologic cancer. In some embodiments, the hematologic or solid tumor is acute lymphoblastic leukemia (ALL), acute myeloid leukemia (AML), non-Hodgkin's lymphoma, chronic lymphocytic leukemia (CLL), pancreatic cancer, glioblastoma, or a cancer that expresses CD19.

In an additional alternative embodiment, an active compound or its salt or composition as described herein may be provided in combination with eculizumab, ravulizumab, ABP959 (Amgen), BOWo8o (Epirus Biopharmaceuticals), or SB12 (Samsung Bioepis) for the treatment of PNH, aHUSs, STEC-HUS, ANCA-vasculitis, wet or dry AMD, CAD, diabetic retinopathy, irritable bowl disease(IBD), C3 glomerulopathy, for example DDD or C3GN, chronic hemolysis, neuromyelitis optica, myasthenia gravis, amyotrophic lateral sclerosis (ALS), primary progressive multiple sclerosis (PPMS), or transplantation rejection. In some embodiments, an active compound or its salt or composition as described herein may be provided in combination with compstatin or a compstatin derivative for the treatment of PNH, aHUSs, STEC-HUS, ANCA-vasculitis, AMD, CAD, C3 glomerulopathy, for example DDD or C3GN, chronic hemolysis, neuromyelitis optica, or transplantation rejection. In some embodiments, the additional agent is a complement component inhibitor, for example but not limited to Compstatin/POT-4 (Potentia Pharmaceuticals); ARC1905 (Archemix); 4(1MEW)APL-1,APL-2 (Appelis); CP40/AMY-101,PEG-Cp40 (Amyndas); a PDGF inhibitor, for example, but not limited to Sorafenib Tosylate; Imatinib Mesylate (STI571); Sunitinib Malate; Ponatinib (AP24534); Axitinib; Imatinib (STI571); Nintedanib (BIBF 1120); Pazopanib HCl (GW786034 HCl); Dovitinib (TKI-258, CHIR-258); Linifanib (ABT-869); Crenolanib (CP-868596); Masitinib (AB1010); Tivozanib (AV-951); Motesanib Diphosphate (AMG-706); Amuvatinib (MP-470); TSU-68 (SU6668, Orantinib); CP-673451; Ki8751; Telatinib; PP121; Pazopanib; KRN 633; Dovitinib (TKI-258) Dilactic Acid; MK-2461; Tyrphostin (AG 1296); Dovitinib (TKI258) Lactate; Sennoside B; Sunitinib; AZD2932; and Trapidil; an anti-factor H or anti-factor B agent, for example anti-FB siRNA (Alnylam); FCFD4514S (Genentech/Roche) SOMAmers for CFB and CFD (SomaLogic); TA106 (Alexion Pharmaceuticals); 5C6, and AMY-301 (Amyndas); a complement C3 or CAP C3 convertase targeting molecule, for example but not limited to TT30 (CR2/CFH) (Alexion); TT32 (CR2/CR1) (Alexion Pharmaceuticals); Nafamostat (FUT-175, Futhan) (Torri Pharmaceuticals); Bikaciomab, NM9308 (Novelmed); CVF, HC-1496 (InCode) ALXN1102/ALXN1103 (TT30) (Alexion Pharmaceuticals); rFH (Optherion); H17 C3 (C3b/iC3b) (EluSys Therapeutics); Mini-CFH (Amyndas) Mirococept (APT070); sCR1 (CDX-1135) (Celldex); CRIg/CFH, an anti-CR3, anti-MASP2, anti C1s, or anti-C1n molecule, for example but not limited to Cynryze (ViroPharma/Baxter); TNT003 (True North); OMS721 (Omeros); OMS906 (Omeros); and Imprime PGG (Biothera).

In some embodiments, an active compound or its salt or composition as described herein may be provided in combination with rituxan for the treatment of a complement mediated disorder. In some embodiments, the complement mediated disorder is, for example, rheumatoid arthritis, Granulomatosis with Polyangiitis (GPA) (Wegener's Granulomatosis), and Microscopic Polyangiitis (MPA). In some embodiments, the disorder is Lupus.

In some embodiments, an active compound or its salt or composition as described herein may be provided in combination with cyclophosphamide for the treatment of a complement mediated disorder. In some embodiments, the disorder is an autoimmune disease. In some embodiments, the complement mediated disorder is, for example, rheumatoid arthritis, Granulomatosis with Polyangiitis (GPA) (Wegener's Granulomatosis), and Microscopic Polyangiitis (MPA). In some embodiments, the disorder is Lupus.

In some embodiments, an active compound or its salt or composition as described herein is dosed in combination with a conventional DLE treatment for the treatment of lupus to a subject in need thereof.

Examples of conventional DLE treatments include topical corticosteroid ointments or creams, such as triamcinolone acetonide, fluocinolone, flurandrenolide, betamethasone valerate, or betamethasone dipropionate. Resistant plaques can be injected with an intradermal corticosteroid. Other potential DLE treatments include calcineurin inhibitors such as pimecrolimus cream or tacrolimus ointment. Particularly resistant cases can be treated with systemic antimalarial drugs, such as hydroxychloroquine (PLAQUENIL).

In some embodiments, an active compound or its salt or composition as described herein may be provided in combination with methotrexate for the treatment of Lupus.

In some embodiments, an active compound or its salt or composition as described herein may be provided in combination with azathioprine for the treatment of Lupus.

In some embodiments, an active compound or its salt or composition as described herein may be provided in combination with a non-steroidal anti-inflammatory drug for the treatment of Lupus.

In some embodiments, an active compound or its salt or composition as described herein may be provided in combination with a corticosteroid for the treatment of Lupus.

In some embodiments, an active compound or its salt or composition as described herein may be provided in combination with a belimumab (Benlysta) for the treatment of Lupus.

In some embodiments, an active compound or its salt or composition as described herein may be provided in combination with hydroxychloroquine (Plaquenil) for the treatment of Lupus.

In some embodiments, an active compound or its salt or composition as described herein may be provided in combination with sifalimumab for the treatment of Lupus.

In some embodiments, an active compound or its salt or composition as described herein may be provided in combination with OMS721 (Omeros) for the treatment of a complement mediated disorder. In some embodiments, an active compound or its salt or composition as described herein may be provided in combination with OMS906 (Omeros) for the treatment of a complement mediated disorder. In some embodiments, the complement mediated disorder is, for example, thrombotic thrombocytopenic purpura (TTP) or aHUS.

In some embodiments, an active compound or its salt or composition as described herein may be provided in combination with an anti-inflammatory agent, immunosuppressive agent, or anti-cytokine agent for the treatment or prevention of cytokine or inflammatory reactions in response to the administration of pharmaceuticals or biotherapeutics (e.g. adoptive T-cell therapy (ACT) such as CAR T-cell therapy, or monoclonal antibody therapy). In some embodiments, an active compound or its salt or composition as described herein may be provided in combination with a corticosteroid, for example prednisone, dexamethasone, solumedrol, and methylprednisolone, and/or anti-cytokine compounds targeting, e.g., IL-4, IL-10, IL-11, IL-13 and TGFβ. In some embodiments, an active compound or its salt or composition as described herein may be provided in combination with an anti-cytokine inhibitor including, but are not limited to, adalimumab, infliximab, etanercept, protopic, efalizumab, alefacept, anakinra, siltuximab, secukibumab, ustekinumab, golimumab, and tocilizumab, or a combination thereof. Additional anti-inflammatory agents that can be used in combination with an active compound or its salt or composition as described herein include, but are not limited to, non-steroidal anti-inflammatory drug(s) (NSAIDs); cytokine suppressive anti-inflammatory drug(s) (CSAIDs); CDP-571/BAY-10-3356 (humanized anti-TNFα antibody; Celltech/Bayer); cA2/infliximab (chimeric anti-TNFα antibody; Centocor); 75 kdTNFR-IgG/etanercept (75 kD TNF receptor-IgG fusion protein; Immunex); 55 kdTNF-IgG (55 kD TNF receptor-IgG fusion protein; Hoffmann-LaRoche); IDEC-CE9.1/SB 210396 (non-depleting primatized anti-CD4 antibody; IDEC/SmithKline); DAB 486-IL-2 and/or DAB 389-IL-2 (IL-2 fusion proteins; Seragen); Anti-Tac (humanized anti-IL-2Rα; Protein Design Labs/Roche); IL-4 (anti-inflammatory cytokine; DNAX/Schering); IL-10 (SCH 52000; recombinant IL-10, anti-inflammatory cytokine; DNAX/Schering); IL-4; IL-10 and/or IL-4 agonists (e.g., agonist antibodies); IL-1RA (IL-1 receptor antagonist; Synergen/Amgen); anakinra (Kineret^{®}/Amgen); TNF-bp/s-TNF (soluble TNF binding protein); R973401 (phosphodiesterase Type IV inhibitor); MK-966 (COX-2 Inhibitor); Iloprost, leflunomide (anti-inflammatory and cytokine inhibiton); tranexamic acid (inhibitor of plasminogen activation); T-614 (cytokine inhibitor); prostaglandin E1; Tenidap (non-steroidal anti-inflammatory drug); Naproxen (non-steroidal anti-inflammatory drug); Meloxicam (non-steroidal anti-inflammatory drug); Ibuprofen (non-steroidal anti-inflammatory drug); Piroxicam (non-steroidal anti-inflammatory drug); Diclofenac (non-steroidal anti-inflammatory drug); Indomethacin (non-steroidal anti-inflammatory drug); Sulfasalazine; Azathioprine; ICE inhibitor (inhibitor of the enzyme interleukin-1β converting enzyme); zap-70 and/or lck inhibitor (inhibitor of the tyrosine kinase zap-70 or lck); TNF-convertase inhibitors; anti-IL-12 antibodies; anti-IL-18 antibodies; interleukin-11; interleukin-13; interleukin-17 inhibitors; gold; penicillamine; chloroquine; chlorambucil; hydroxychloroquine; cyclosporine; cyclophosphamide; anti-thymocyte globulin; anti-CD4 antibodies; CD5-toxins; orally-administered peptides and collagen; lobenzarit disodium; Cytokine Regulating Agents (CRAB) HP228 and HP466 (Houghten Pharmaceuticals, Inc.); ICAM-1 antisense phosphorothioate oligo-deoxynucleotides (ISIS 2302; Isis Pharmaceuticals, Inc.); soluble complement receptor 1 (TP10; T Cell Sciences, Inc.); prednisone; orgotein; glycosaminoglycan polysulphate; minocycline; anti-IL2R antibodies; marine and botanical lipids (fish and plant seed fatty acids); auranofin; phenylbutazone; meclofenamic acid; flufenamic acid; intravenous immune globulin; zileuton; azaribine; mycophenolic acid (RS-61443); tacrolimus (FK-506); sirolimus (rapamycin); amiprilose (therafectin); cladribine (2-chlorodeoxyadenosine).

In a specific embodiment, an active compound or its salt or composition as described herein may be provided in combination with a corticosteroid for the treatment or prevention of cytokine or inflammatory reactions in response to the administration of pharmaceuticals or biotherapeutics. In another embodiment, an active compound or its salt or composition as described herein may be provided in combination with etarnercept for the treatment or prevention of cytokine or inflammatory reactions in response to the administration of pharmaceuticals or biotherapeutics. In another embodiment, an active compound or its salt or composition as described herein may be provided in combination with tocilizumab for the treatment or prevention of cytokine or inflammatory reactions in response to the administration of pharmaceuticals or biotherapeutics. In another embodiment, an active compound or its salt or composition as described herein may be provided in combination with etarnercept and tocilizumab for the treatment or prevention of cytokine or inflammatory reactions in response to the administration of pharmaceuticals or biotherapeutics. In another embodiment, an active compound or its salt or composition as described herein may be provided in combination with infliximab for the treatment or prevention of cytokine or inflammatory reactions in response to the administration of pharmaceuticals or biotherapeutics. In another embodiment, an active compound or its salt or composition as described herein may be provided in combination with golimumab for the treatment or prevention of cytokine or inflammatory reactions in response to the administration of pharmaceuticals or biotherapeutics.

### C5 Inhibitors

Provided herein are the compounds as described herein for use in methods for treating PNH in a subject, said methods comprising administering to the subject an effective amount of a C5 inhibitor in combination or alternation with an effective amount of a CFD inhibitor selected from the compounds of the invention.

C5 inhibitors are known in the art. In some embodiments, the C5 inhibitor is a monoclonal antibody targeting C5. In some embodiments, the C5 inhibitor is eculizumab (Soliris^{™} Alexion Pharmaceuticals, New Haven, CT, see, e.g., U.S. Patent No. 9,352,035). In some embodiments, the C5 inhibitor is ravulizumab (Ultomiris^{™} Alexion Pharmaceuticals, New Haven, CT, see, e.g., U.S. Patent No. 9,371,377; 9,079,949 and 9,663,574. In some embodiments, the C5 inhibitor is ABP959 (Amgen). In some embodiments, the C5 inhibitor is BOWo8o (Epirus Biopharmaceuticals). In some embodiments, the C5 inhibitor is SB12 (Samsung Bioepis).

In some embodiments, the C5 inhibitor may be, but is not limited to: a recombinant human minibody, for example Mubodina^{®} (monoclonal antibody, Adienne Pharma and Biotech, Bergamo, Italy; see U.S. Patent No. 7,999,081); coversin (small animal protein, Volution Immuno-pharmaceuticals, Geneva, Switzerland; see e.g. Penabad et al. Lupus, 2012, 23(12):1324-6); LFG316 (monoclonal antibody, Novartis, Basel, Switzerland, and Morphosys, Planegg, Germany; see U.S. Patent Nos. 8,241,628 and 8,883,158); ARC-1905 (pegylated RNA aptamer, Ophthotech, Princeton, NJ and New York, NY; see Keefe et al., Nature Reviews Drug Discovery, 9, 537-550); RA101348 and RA101495 (macrocyclic peptides, Ra Pharmaceuticals, Cambridge, MA); SOBI002 (affibody, Swedish Orphan Biovitrum, Stockholm, Sweden); ALN-CC5 (Si-RNA, Alnylam Pharmaceuticals, Cambridge, MA); ARC1005 (aptamers, Novo Nordisk, Bagsvaerd, Denmark); SOMAmers (aptamers, SomaLogic, Boulder, Co); SSL7 (bacterial protein toxin, see, e.g. Laursen et al. Proc. Natl. Acad. Sci. U.S.A., 107(8):3681-6); MEDI7814 (monoclonal antibody, MedImmune, Gaithersburg, MD); aurin tricarboxylic acid; aurin tricarboxylic acid derivatives (Aurin Biotech, Vancouver, BC, see U.S. Patent Appl. Pub. 2013/003592); RG6107 (anti-C5 recycling antibody, Roche Pharmaceuticals, Basel, Switzerland); ALXN1210 and ALXN5500 (monoclonal antibodies, Alexion Pharmaceuticals, New Haven, CT); TT30 (fusion protein, Alexion Pharmaceuticals, New Haven, CT); REGN3918 (monoclonal antibody, Regeneron, Tarrytown, NY); ABP959 (eculizumab biosimilar, Amgen, Thousand Oaks, CA); or combinations thereof.

In some embodiments, the C5 inhibitor is a recombinant human minibody, for example Mubodina^{®}. Mubodina^{®} is a fully human recombinant antibody C5 developed by Adienne Pharma and Biotech. Mubodina^{®} is described in U.S. Patent No. 7,999,081.

In some embodiments, the C5 inhibitor is coversin. Coversin is a recombinant protein derived from a protein discovered in the saliva of the *Ornithodoros moubata* tick currently developed as a recombinant protein by Akari Therapeutics. Coversin is described in Penabad et al. Lupus 2012, 23(12):1324-6.

In some embodiments, the C5 inhibitor is Tesidolumab/LFG316. Tesidolumab is a monoclonal antibody developed by Novartis and Morphosys. Tesidolumab is described in U.S. Patent Nos. 8,241,628 and 8,883,158.

In some embodiments, the C5 inhibitor is ARC-1905. ARC-1905 is a pegylated RNA aptamer developed by Ophthotech. ARC-1905 is described in Keefe et al. Nature Reviews Drug Discovery, 9:537-550.

In some embodiments, the C5 inhibitor is RA101348. RA101348 is a macrocyclic peptide developed by Ra Pharmaceuticals.

In some embodiments, the C5 inhibitor is RA101495. RA101495 is a macrocyclic peptide developed by Ra Pharmaceuticals.

In some embodiments, the C5 inhibitor is SOBI002. SOBI002 is an affibody developed by the Swedish Orphan Biovitrum.

In some embodiments, the C5 inhibitor is ARC1005. ARC1005 is an aptamer developed by Novo Nordisk.

In some embodiments, the C5 inhibitor is SOMAmers for C5. SOMAmers are aptamers developed by SomaLogic.

In some embodiments, the C5 inhibitor is SSL7. SSL7 is a bacterial protein toxin described in Laursen et al. Proc. Natl. Acad. Sci. U.S.A., 107(8):3681-6.

In some embodiments, the C5 inhibitor is MEDI7814. MEDI7814 is a monoclonal antibody developed by MedImmune.

In some embodiments, the C5 inhibitor is aurin tricarboxylic acid. In another embodiment, the C5 inhibitor is an aurin tricarboxylic acid derivative. These aurin derivatives were developed by Aurin Biotech and are further described in U.S. Patent Appl. Pub. No. 2013/003592).

In some embodiments, the C5 inhibitor is RG6107/SKY59. RG6107/SKY59 is an anti-C5 recycling antibody developed by Roche Pharmaceuticals.

In some embodiments, the C5 inhibtior is ALXN1210. In another embodiment, the C5 inhibitor is ALXN5500. ALXN1210 and ALXN5500 are monoclonal antibodies developed by Alexion Pharmaceuticals.

In some embodiments, the C5 inhibitor is TT30. TT30 is a fusion protein developed by Alexion Pharmaceuticals.

In some embodiments, the C5 inhibitor is ABP959. ABP959 is an eculizamab biosimilar monoclonal antibody developed by Amgen.

In some embodiments, the C5 inhibtor is Anti-C5 siRNA. Anti-C5 siRNA was developed by Alnylam Pharmaceuticals.

In some embodiments, the C5 inhibitor is Erdigna^{®}. Erdigna^{®} is an antibody developed by Adienne Pharma.

In some embodiments, the C5 inhibitor is avacincaptad pegol/Zimura^{®}. Avacincaptad pegol is in aptamer developed by Opthotech.

In some embodiments, the C5 inhibitor is SOBI005. SOBI005 is a protein in developed by the Swedish Orphan Biovitrum.

In some embodiments, the C5 inhibitor is ISU305. ISU305 is a monoclonal antibody developed by ISU ABXIS.

In some embodiments, the C5 inhibitor is REGN3918. REGN3918 is a monoclonal antibody developed by Regeneron.

### C3 Inhibitors

Provided herein are the compounds as described herein for use in methods for treating PNH in a subject comprising administering to the subject an effective amount of a C3 inhibitor in combination or alternation with an effective amount of a CFD inhibitor selected from the compounds of the invention.

C3 inhibitors are known in the art. In some embodiments, a compound of the present invention is administered in combination or alternation with compstatin and/or a compstatin analog. Compstatin and compastin analogs are known and are found to be useful inhibitors of C3, see U.S. Patent Nos. 9,056,076; 8,168,584; 9,421,240; 9,291,622; 8,580,735; 9371365; 9,169,307; 8,946,145; 7,989,589; 7,888,323; 6,319,897; and US Patent Appl. Pub. Nos. 2016/0060297; 2016/0015810; 2016/0215022; 2016/0215020; 2016/0194359; 2014/0371133; 2014/0323407; 2014/0050739; 2013/0324482; and 2015/0158915. In some embodiments, the compstatin analog having the amino acid sequence ICVVQDWGHHCRT (SEQ. ID. NO. 1). In another embodiment, the C3 inhibitor is a compstatin analog. In some embodiments, the compstatin analog is 4(1MeW)/APL-1 of the sequence Ac-ICV(1-mW)QDWGAHRCT(SEQ. ID. NO. 2), wherein Ac is acetyl and 1-mW is 1-methyltryptophan. In another embodiment, the compstatin analog is Cp40/AMY-101, which has an amino acid sequence yICV(1mW)QDW-Sar-AHRC-mI (SEQ. ID. NO. 3), wherein y is D-tyrosine, 1mW is 1-methyltryptophan, Sar is sarcosine, and mI is *N-*methylisoleucine. In yet another embodiment, the compstatin analog is PEG-Cp40, having the amino acid sequence PEG-yICV(1mW)QDW-Sar-AHRC-mI (SEQ. ID. NO. 4), wherein PEG is polyethyleneglycol (40 kDa), y is D-tyrosine, 1mW is 1-methyltryptophan, Sar is sarcosine, and mI is *N*-methylisoleucine. In yet another embodiment, the compstatin analog is 4(1MeW)POT-4. 4(1MeW)POT-4 was developed by Potentia. In yet another embodiment, the compstatin analog is AMY-201. AMY-201 was developed by Amyndas Pharmaceuticals.

In some embodiments, a compound of the present invention can be combined with C3 inhibitors that include, but are not limited to: H17 (monoclonal antibody, EluSys Therapeutics, Pine Brook, NJ); mirococept (CR1-based protein); sCR1 (CR1-based protein, Celldex, Hampton, NJ); TT32 (CR-1 based protein, Alexion Pharmaceuticals, New Haven, CT); HC-1496 (recombinant peptide); CB 2782 (enzyme, Catalyst Biosciences, South San Francisco, CA); APL-2 (pegylated synthetic cyclic peptide, Apellis Pharmaceuticals, Crestwood, KY); or combinations thereof.

In some embodiments, the C3 inhibitor is H17. H17 is a humanized monoclonal antibody in development by EluSys Therapeutics. H17 is described in Paixao-Cavalcante et al. J. Immunol. 2014, 192(10):4844-4851.

In some embodiments, the C3 inhibitor is mirococept. Mirococept is a CR1-based protein developed by Inflazyme Pharmaceuticals.

In some embodiments, the C3 inhibitor is sCR1. sCR1 is a soluble form of the CR1 protein developed by Celldex.

In some embodiments, the C3 inhibitor is TT32. TT32 is a CR-1 based protein developed by Alexion Pharmaceuticals.

In some embodiments, the C3 inhibitor is HC-1496. HC-1496 is a recombinant peptide developed by InCode.

In some embodiments, the C3 inhibitor is CB 2782. CB 2782 is novel protease derived from human membrane type serine protease 1 (MTSP-1) that was developed by Catalyst Biosciences.

In some embodiments, the C3 inhibitor is APL-2. APL-2 is a pegylated version of APL-1 developed by Apellis Pharmaceuticals.

### Complement Factor B (CFB) Inhibitors

Provided herein are compounds as described herein for use in methods for treating PNH comprising administering a CFB inhibitor in combination or alternation with a compound of the present invention. CFB inhibitors are known in the art. In some embodiments, a compound of the present invention can be combined with CFB inhibitors that include, but are not limited to: anti-FB SiRNA (Alnylam Pharmaceuticals, Cambridge, MA); TA106 (monoclonal antibody, Alexion Pharmaceuticals, New Haven, CT); LNP023 (small molecule, Novartis, Basel, Switzerland); SOMAmers (aptamers, SomaLogic, Boulder, CO); bikaciomab (Novelmed Therapeutics, Cleveland, OH); complin (see, Kadam et al., J. Immunol. 2010, DOI: 10.409/jimmunol. 10000200); Ionis-FB-L_{Rx} (ligand conjugated antisense drug, Ionis Pharmaceuticals, Carlsbad, CA); or a combination thereof. In another embodiment, CFB inhibitors that can be combined with a compound of the present invention include those disclosed in PCT/US17/39587. In another embodiment, CFB inhibitors that can be combined with a compound of the present invention as described herein include those disclosed in PCT/US17/014458. In another embodiment, CFB inhibitors that can be combined with a compound of the present invention as described herein include those disclosed in U.S. Patent Appl. Pub. No. 2016/0024079; . PCT Int. Appl. WO 2013/192345; PCT Int. Appl. WO 2013/164802; PCT Int. Appl. WO 2015/066241; PCT Int. Appl. WO 2015/009616 (assigned to Novartis AG).

In some embodiments, the CFB inhibitor is

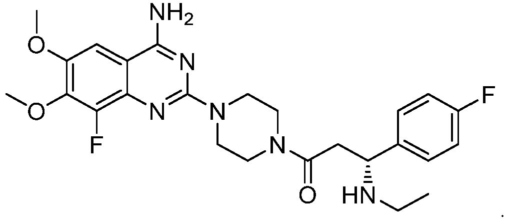

In another embodiment, the CFB inhibitor is

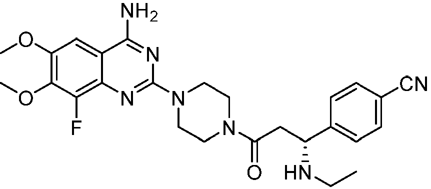

In another embodiment, the CFB inhibitor is

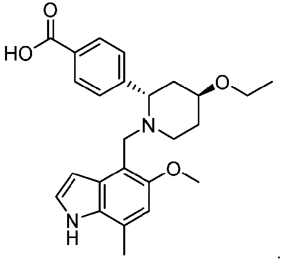

In some embodiments, the CFB inhibitor is anti-FB siRNA. Anti-FB siRNA was developed by Alnylam Pharmaceuticals.

In some embodiments, the CFB inhibitor is TA106. TA106 is a monoclonal antibody developed by Alexion Pharmaceuticals.

In some embodiments, the CFB inhibitor is LNP023. LNP023 is a small molecule inhibitor of CFB developed by Novartis.

In some embodiments, the CFB inhibitor is complin. Complin is a peptide inhibitor that is described in Kadam et al. J. Immunol. 2010 184(12):7116-24.

In some embodiments, the CFB inhibitor is Ionis-FB-L_{Rx}. Ionis-FB-L_{Rx} is a ligand conjugated antisense drug developed by Ionis Pharmaceuticals.

### Pan-inhibitors of Complement Components

Provided herein are the compounds as described herein for use in methods for treating PNH, said methods comprising administering a pan-inhibitor of complement components in combination or alternation with a compound of the present invention. Pan-inhibitors of complement components are known in the art. In some embodiments, the inhibitor is FUT-175.

### COMBINATIONS FOR PROPHYLACTIC OR CONCOMMITANT ANTI-BACTERIAL THERAPY

In one aspect of the present invention, an active compound or its salt or composition is provided for use in a method for treating a host in need thereof that comprises administering an effective amount of a prophylactic anti-bacterial vaccine prior to administration of the active compound or its salt or composition for any of the disorders described herein. In another aspect of the present invention, an active compound or its salt or composition is provided for use in a method for treating a host in need thereof that comprises administering an effective amount of a prophylactic anti-bacterial drug, such as a pharmaceutical drug, prior to administration of the active compound or its salt or composition for any of the disorders described herein. In one aspect of the present invention, an active compound or its salt or composition is provided for use in a method for treating a host in need thereof that comprises administering an effective amount of an anti-bacterial vaccine after administration of the active compound or its salt or composition for any of the disorders described herein. In another aspect of the present invention, an active compound or its salt or composition is provided for use in a method for treating a host in need thereof that comprises administering an effective amount of an anti-bacterial drug, such as a pharmaceutical drug, after administration of the active compound or its salt or composition for any of the disorders described herein. In one embodiment, the disorder is PNH, C3G, or aHUS. In one embodiment, the host has received an organ or other tissue or biological fluid transplant. In one embodiment, the host is also administered eculizumab.

In one aspect of the present invention, an active compound or its salt or composition as described herein is administered to a host concomitantly to a subject following the prophylactic administration of a vaccine against a bacterial infection. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, an active compound or its salt or composition as described herein is administered to a subject concomitantly with the prophylactic administration of a vaccine against a bacterial infection. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, an active compound or its salt or composition as described herein is administered to a subject and, during the administration period of the compound or salt, a vaccine against a bacterial infection is administered to the subject. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, the subject is administered an active compound or its salt or composition as described herein in combination with an antibiotic compound for the duration of Factor D inhibitor administration. In one embodiment, the complement mediated disorder is PNH, C3G, or aHUS. In one embodiment, the subject has received an organ or other tissue or biological fluid transplant. In one embodiment, the subject is also administered eculizumab.

In one aspect of the present invention, an active compound or its salt or composition as described herein is administered to a subject following the prophylactic administration of a vaccine against a bacterial infection, and in combination with an antibiotic compound for the duration of Factor D inhibitor administration. In one embodiment, the complement mediated disorder is PNH or aHUS. In one embodiment, the subject has received an organ or other tissue or biological fluid transplant. In one embodiment, the subject is also administered eculizumab. In one embodiment, the subject, prior to receiving an active compound or its salt or composition as described herein, is vaccinated against a bacterial infection caused by the bacterium Neisseria meningitidis. In one embodiment, the subject is vaccinated against a bacterial infection caused by the bacterium Haemophilus influenzae. In one embodiment, the Haemophilus influenzae is Haemophilus influenzae serotype B (Hib). In one embodiment, the subject is vaccinated against a bacterial infection caused by Streptococcus pneumoniae. In one embodiment, the subject is vaccinated against a bacterial infection caused by the bacterium Nisseria meningitidis, Haemophilus influenzae, or Streptococcus pneumoniae, or a combination of one or more of Nisseria meningitidis, Haemophilus influenzae, or Streptococcus pneumoniae. In one embodiment, the subject is vaccinated against a bacterial infection caused by the bacterium Nisseria meningitidis, Haemophilus influenzae, and Streptococcus pneumoniae.

In other embodiments, the subject is vaccinated against a bacterial infection caused by a bacterium selected from a Gram-negative bacterium. In one embodiment, the subject is vaccinated against a bacterial infection caused by a bacterium selected from a Gram-positive bacterium. In one embodiment, the subject is vaccinated against a bacterial infection caused by the bacterium Nisseria meningitidis, Haemophilus influenzae, or Streptococcus pneunemoniae, or a combination of one or more of Nisseria meningitidis, Haemophilus influenzae, or Streptococcus pneumoniae, and one or more of, but not limited to, Bacillus anthracis, Bordetella pertussis, Clostridium tetani, Corynebacterium diphtheria, Coxiella burnetii, Mycobacterium tuberculosis, Salmonella typhi, Vibrio cholerae, Anaplasma phagocytophilum, Ehrlichia ewingii, Ehrlichia chaffeensis, Ehrlichia canis, Neorickettsia sennetsu, Mycobacterium leprae, Borrelia burgdorferi, Borrelia mayonii, Borrelia afzelii, Borrelia garinii, Mycobacterium bovis, Staphylococcus aureus, Streptococcus pyogenes, Treponema pallidum, Francisella tularensis, Yersinia pestis,

In one embodiment, the subject is vaccinated with one or more vaccines selected from, but not limited to, typhoid vaccine, live (Vivotif Berna Vaccine, PaxVax), typhoid Vi polysaccharide vaccine (Typhim Vi, Sanofi), pneumococcal 23-polyvalent vaccine, PCV13 (Pneumovax 23, Merck), pneumococcal 7-valent vaccine, PCV7 (Prevnar, Pfizer), pneumococcal 13-valent vaccine, PCV13 (Prevnar 13, Pfizer), haemophilus b conjugate (prp-t) vaccine (ActHIB, Sanofi; Hibrix, GSK), haemophilus b conjugate (hboc) vaccine (HibTITER, Neuron Biotech), haemophilus b conjugate (prp-omp) vaccine (PedvaxHIB, Merck), haemophilus b conjugate (prp-t) vaccine/meningococcal conjugate vaccine (MenHibrix, GSK), haemophilus b conjugate (prp-t) vaccine/meningococcal conjugate vaccine/Hepatitis B vaccine (Comvax, Merck), meningococcal polysaccharide vaccine (Menomune A / C / Y / W-135, Sanofi), meningococcal conjugate vaccine/diphtheria CRM197 conjugate (Menveo, GSK; Menactra, Sanofi), meningococcal group B vaccine (Bexsero, GSK; Trumenba, Pfizer), anthrax vaccine adsorbed (Biothrax, Emergent Biosolutions), tetanus toxoid (Te Anatoxal Berna, Hendricks Regional Health), Bacillus Calmette and Guérin, live, intravesical (TheraCys, Sanofi; Tice BCG, Organon), cholera vaccine, live, oral (Vachora, Sanofi; Dukoral, SBL Vaccines; ShanChol, Shantha Biotec; Micromedex, Truven Health), tetanus toxoids and diphtheria absorbed (Tdap; Decavac, Sanofi; Tenivac, Sanofi; td, Massachusetts Biological Labs), diphtheria and tetanus toxois and pertussis (DTap; Daptacel, Sanofi; Infanrix, GSK; Tripedia, Sanofi), diphtheria and tetanus toxois and pertussis/polio (Kinrix, GSK; Quadracel, Sanofi), diphtheria and tetanus toxois and pertussis tetanus/hepatitis B/polio (Pediarix, GSK), diphtheria and tetanus toxois and pertussis/ polio, haemophilus influenza tybe b (Pentacel, Sanofi), and/or diphtheria, and pertussis (Tdap; Boostrix, GSK; Adacel, Sanofi), or a combination thereof.

As described above, a subject receiving a compound of the present invention to treat a disorder is prophylactically administered an antibiotic compound in addition to a Factor D inhibitor described herein. In one embodiment, the subject is administered an antibiotic compound for the duration of administration of the active compound to reduce the development of a bacterial infection. Antibiotic compounds for concomitant administration with a Factor D inhibitor described herein can be any antibiotic useful in preventing or reducing the effect of a bacterial infection. Antibiotics are well known in the art and include, but are not limited to, amikacin (Amikin), gentamicin (Garamycin), kanamycin (Kantrex), neomycin (Neo-Fradin), netilmicin (Netromycin), tobramycin (Nebcin), paromomycin (Humatin), streptomycin, spectinomycin (Trobicin), geldanamycin, herbimycin, rifaximin (Xifaxan), loracarbef (Lorabid), ertapenem (Invanz), doripenem (Doribax), imipenem/cilastatin (Primaxin), meropenem (Merrem), cefadroxil (Duricef), cefazolin (Ancef), cefalotin/cefalothin (Keflin), cephalexin (Keflex), cefaclor (Distaclor), cefamandole (Mandol), cefoxitin (Mefoxin), cefprozil (Cefzil), cefuroxime (Ceftin, Zinnat), cefixime (Cefspan), cefdinir (Omnicef, Cefdiel), cefditoren (Spectracef, Meiact), cefoperazone (Cefobid), cefotaxime (Claforan), cefpodoxime (Vantin) ceftazidime (Fortaz), ceftibuten (Cedax), ceftizoxime (Cefizox), ceftriaxone (Rocephin), cefepime (Maxipime), ceftaroline fosamil (Teflaro), ceftobiprole (Zeftera), teicoplanin (Targocid), vancomycin (Vancocin), telavancin (Vibativ), dalbavancin (Dalvance), oritavancin (Orbactiv), clindamycin (Cleocin), lincomycin (Lincocin), daptomycin (Cubicin), azithromycin (Zithromax, Sumamed, Xithrone), clarithromycin (Biaxin), dirithromycin (Dynabac), erythromycin (Erythocin, Erythroped), roxithromycin, troleandomycin (Tao), telithromycin (Ketek), spiramycin (Rovamycine), aztreonam (Azactam), furazolidone (Furoxone), nitrofurantoin (Macrodantin, Macrobid), linezolid (Zyvox), posizolid, radezolid, torezolid, amoxicillin (Novamox, Amoxil), ampicillin (Principen),azlocillin, carbenicillin (Geocillin), cloxacillin (Tegopen), dicloxacillin (Dynapen), flucloxacillin (Floxapen), mezlocillin (Mezlin), methicillin (Staphcillin), nafcillin (Unipen),oxacillin (Prostaphlin), penicillin G (Pentids),penicillin V (Veetids (Pen-Vee-K), piperacillin (Pipracil), penicillin G (Pfizerpen), temocillin (Negaban),ticarcillin (Ticar), amoxicillin/clavulanate (Augmentin), ampicillin/sulbactam (Unasyn), piperacillin/tazobactam (Zosyn), ticarcillin/clavulanate (Timentin),bacitracin, colistin (Coly-Mycin-S), polymyxin B, ciprofloxacin (Cipro, Ciproxin, Ciprobay), enoxacin (Penetrex), gatifloxacin (Tequin), gemifloxacin (Factive), levofloxacin (Levaquin), lomefloxacin (Maxaquin), moxifloxacin (Avelox), nalidixic acid (NegGram), norfloxacin (Noroxin), ofloxacin (Floxin, Ocuflox), trovafloxacin (Trovan), grepafloxacin (Raxar), sparfloxacin (Zagam), temafloxacin (Omniflox), mafenide (Sulfamylon), sulfacetamide (Sulamyd, Bleph-10), sulfadiazine (Micro-Sulfon), silver sulfadiazine (Silvadene), sulfadimethoxine (Di-Methox, Albon), sulfamethizole (Thiosulfil Forte), sulfamethoxazole (Gantanol), sulfanilamide, sulfasalazine (Azulfidine), sulfisoxazole (Gantrisin), trimethoprim-sulfamethoxazole (Co-trimoxazole) (TMP-SMX) (Bactrim, Septra), sulfonamidochrysoidine (Prontosil), demeclocycline (Declomycin), doxycycline (Vibramycin), minocycline (Minocin), oxytetracycline (Terramycin), tetracycline (Sumycin, Achromycin V, Steclin), clofazimine (Lamprene), dapsone (Avlosulfon), capreomycin (Capastat), cycloserine (Seromycin), ethambutol (Myambutol), ethionamide (Trecator), isoniazid (I.N.H.), pyrazinamide (Aldinamide), rifampicin (Rifadin, Rimactane), rifabutin (Mycobutin), rifapentine (Priftin), streptomycin, arsphenamine (Salvarsan), chloramphenicol (Chloromycetin), fosfomycin (Monurol, Monuril), fusidic acid (Fucidin), metronidazole (Flagyl), mupirocin (Bactroban), platensimycin, quinupristin/dalfopristin (Synercid), thiamphenicol, tigecycline (Tigacyl), tinidazole (Tindamax Fasigyn), trimethoprim (Proloprim, Trimpex), and/or teixobactin, or a combination thereof.

In one embodiment, the subject is administered a prophylactic antibiotic selected from cephalosporin, for example, ceftriaxone or cefotaxime, ampicillin-sulbactam, Penicillin G, ampicillin, chloramphenicol, fluoroquinolone, aztreonam, levofloxacin, moxifloxacin, gemifloxacin, vancomycin, clindamycin, cefazolin, azithromycin, meropenem, ceftaroline, tigecycline, clarithromycin, moxifloxacin, trimethoprim/sulfamethoxazole, cefuroxime, axetil, ciprofloxacin, rifampin, minocycline, spiramycin, and cefixime, or a combination of two or more thereof.

### PROCESS OF PREPARATION OF COMPOUNDS OF OF THE PRESENT INVENTION

### ABBREVIATIONS

- ACN: Acetonitrile
- Ac: Acetyl
- Ac₂O: Acetic anhydride
- AcOEt, EtOAc: ethyl acetate
- AcOH: Acetic acid
- Boc₂O: di-*tert*-butyl dicarbonate
- Bu: Butyl
- CAN: Ceric ammonium nitrate
- CBz: Carboxybenzyl
- CDI: Carbonyldiimidazole
- CH₃OH, MeOH: Methanol
- CsF: Cesium fluoride
- CuI: Cuprous iodide
- DCM, CH₂Cl₂: Dichloromethane
- DIEA, DIPEA: N,N-diisopropylethylamine
- DMA: N,N-dimethylacetamide
- DMAP: 4-Dimethylaminopyridine
- DMF: N,N-dimethylformamide
- DMS: Dimethyl sulfide
- DMSO: Dimethylsulfoxide
- DPPA: Diphenyl phosphoryl azide
- EDCI: 1-Ethyl-3-(3-dimethylaminopropyl)carbodiimide
- EEDQ: N-Ethoxycarbonyl-2-ethoxy-1,2-dihydroquinoline
- Et: Ethyl
- Et₃N, TEA: Triethylamine
- EtOAc: Ethylacetate
- EtOH: Ethanol
- HATU: 1-[Bis(dimethylamino)methylene]-1H-1,2,3-triazolo[4,5-b]pyridinium3-oxide hexafluorophosphate
- HCl: Hydrochloric acid
- HOBT: Hydroxybenzotriazole
- iBu, *i*-Bu, isoBu: Isobutyl
- iPr, *i*-Pr, isoPr: Isopropyl
- ⁱPr₂NEt: N,N-diisopropylethylamine
- K₂CO₃: Potassium carbonate
- K₂CO₃: Potassium carbonate
- LiOH: Lithium hydroxide
- Me: Methyl
- MeI: Methyl iodide
- Ms: Mesyl
- MsCl: Mesylchloride
- MTBE: Methyl 'butylether
- Na₂SO₄: Sodium sulfate
- NaCl: Sodium chloride
- NaH: Sodium hydride
- NaHCO₃: Sodium bicarbonate
- NBS: N-bromo succinimide
- NCS: N-chloro succinimide
- NEt₃: Trimethylamine
- NMP: N-Methyl-2-pyrrolidone
- PCC: Pyridinium chlorochromate
- Pd (OAc) ₂: Palladium acetate
- Pd(dppf)Cl₂: [1,1'-Bis(diphenylphosphino) ferrocene]dichloropalladium(II)
- Pd(PPh₃)₂Cl₂: Bis(triphenylphosphine)palladium(II) dichloride
- Pd(PPh₃)₄: Tetrakis(triphenylphosphine)palladium(0)
- Pd/C: Palladium on carbon
- Pd₂ (dba) ₃: Tris(dibenzylideneacetone)dipalladium(0)
- PMB: 4-Methoxybenzyl ether
- PPh₃: Triphenylphosphine
- Pr: Propyl
- Py, py: Pyridine
- RT: Room temperature
- T3P: Propane phosphonic acid anhydride
- TBAF: Tetra-n-butylammonium fluoride
- TBAT: Tetrabutylammonium difluorotriphenylsilicate
- tBu, *t*-Bu: *tert*butyl
- tBuOK: Potassium *tert*-butoxide
- TEA: Trimethylamine
- Tf₂O: Trifluoromethanesulfonic anhydride
- TFA: Trifluoroacetic acid
- THF: Tetrahydrofuran
- TMS: Trimethylsilane
- TMSBr: Bromotrimethylsilane
- *t*_{R}: Retention time
- Troc: 2,2,2-Trichlorethoxycarbonyl chloride
- Zn (CN)₂: Zinc cyanide

### GENERAL METHODS

All nonaqueous reactions were performed under an atmosphere of dry argon or nitrogen gas using anhydrous solvents. The progress of reactions and the purity of target compounds were determined using one of the two liquid chromatography (LC) methods listed below. The structure of starting materials, intermediates, and final products was confirmed by standard analytical techniques, including NMR spectroscopy and mass spectrometry.

### LC Method A

Instrument: Waters Acquity Ultra Performance LC
Column: ACQUITY UPLC BEH C18 2.1 × 50 mm, 1.7 µm
Column Temperature: 40 °C
Mobile Phase: Solvent A: H₂O + 0.05% FA; Solvent B: CH₃CN + 0.05% FA
Flow Rate: 0.8 mL/min
Gradient: 0.24 min @ 15% B, 3.26 min gradient (15-85% B), then 0.5 min @ 85% B.
Detection: UV (PDA), ELS, and MS (SQ in EI mode)

### LC Method B

Instrument: Shimadzu LC-2010A HT
Column: Athena, C18-WP, 50 × 4.6 mm, 5 µm
Column Temperature: 40 °C
Mobile Phase: Solvent A: H₂O/CH₃OH/FA= 90/10/0.1; Solvent B: H₂O/CH₃OH/FA= 10/90/0.1
Flow Rate: 3 mL/min
Gradient: 0.4 min @ 30% B, 3.4 min gradient (30-100% B), then 0.8 min @ 100% B
Detection: UV (220/254 nm)

### LC Method C

Instrument: Agilent 1100 / 1200 series LC system with DAD detector
Column: Atlantis dC18 (250 x 4.6) mm, 5 µm
Column Temperature: Ambient
Mobile Phase A: 0.1% TFA in water, Mobile Phase B: Acetonitrile
Flow Rate: 1.0 mL/min
Gradient:

| Time (min) | 0.0 | 15 | 20 | 23 | 30 |
|---|---|---|---|---|---|
| % B | 10 | 100 | 100 | 10 | 10 |

Detection: (210-400 nm)

### LC Method D

Instrument: Shimadzu LC 20AD system with PDA detector
Column: Phenomenex Gemini NX C18 (150 x 4.6) mm, 5 µm
Column Temperature: Ambient
Mobile Phase A: 10mM NH₄OAC in water, Mobile Phase B: Acetonitrile
Flow Rate: 1.0 mL/min
Gradient:

| Time (min) | 0.0 | 15 | 20 | 23 | 30 |
|---|---|---|---|---|---|
| % B | 10 | 100 | 100 | 10 | 10 |

Detection: (210-400 nm)

### EXAMPLE 1. NON-LIMITING SYNTHETIC EXAMPLES OF COMPOUNDS OF THE PRESENT INVENTION

The below schemes are non-limiting examples of methods to make compounds of the present invention. The skilled artisan will recognize that there are various modifications that can be performed to make analogs or prepare compounds in other ways. For example, wherever a Suzuki coupling is used there are other methods known in the art to conduct the coupling such as those described in the literature (Molander, G. A., Trice, S. L. J., & Kennedy, S. M. (2012). Scope of the two-step, one-pot palladium-catalyzed borylation/Suzuki cross-coupling reaction utilizing bis-boronic acid. Journal of Organic Chemistry, 77(19), 8678-8688.)

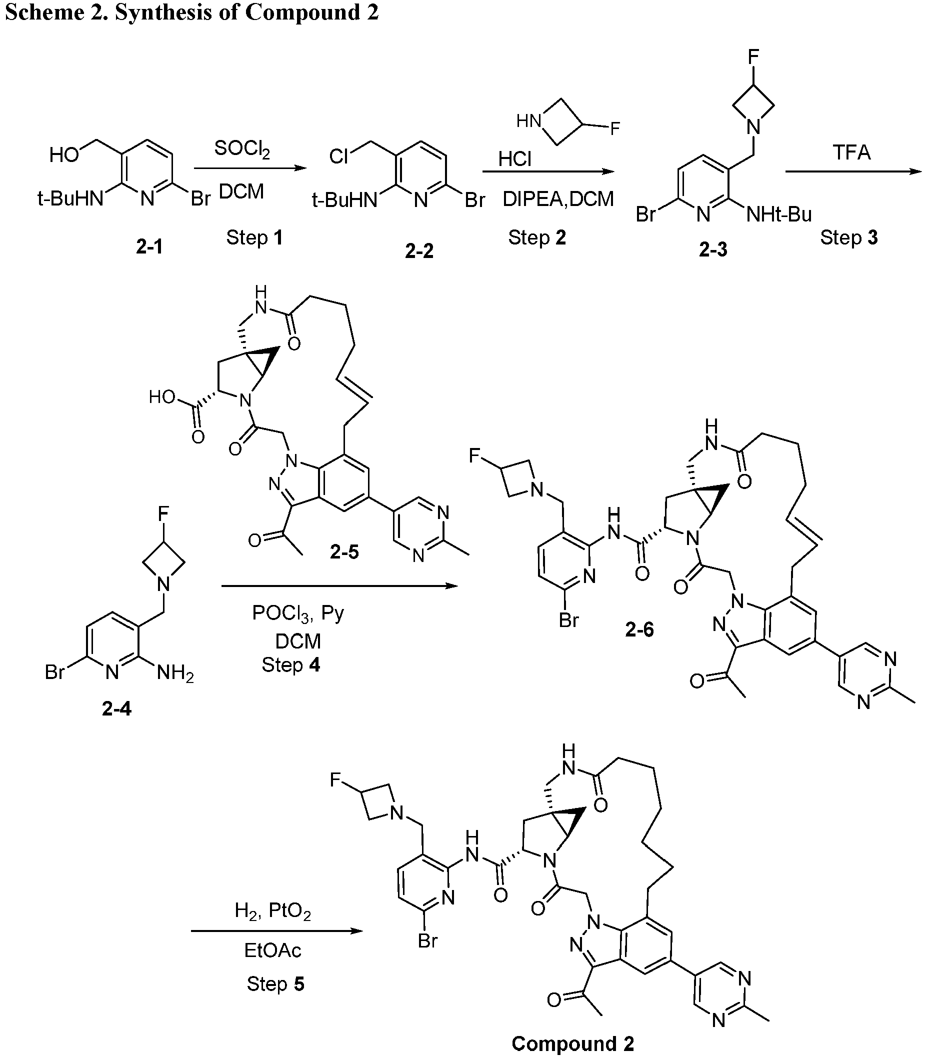

**Step 1: 6-Bromo-N-(tert-butyl)-3-(chloromethyl)pyridin-2-amine (2-2):** A solution of compound **2-1** (340 mg, 1.31 mmol) in DCM (6 mL) was added SOCl₂ (1 mL) dropwisely at 0 °C and the mixture was stirred at 0 °C to room temperature for 2 hours. The reaction mixture was concentrated to dryness, washed with diethyl ether and dried under vacuum to afford compound **2-2** (360 mg, yield 98.9%) as a yellow solid that was used directly in the next step. LC/MS (ESI) m/z: 277/279 (M+H)⁺.

**Step 2: 6-Bromo-N-(tert-butyl)-3-((3-fluoroazetidin-1-yl)methyl)pyridin-2-amine (2-3):** To a mixture of 3-fluoroazetidine hydrochloride (434 mg, 3.89 mmol) and compound **2-2** (360 mg, 1.30 mmol) in DCM (8 mL) was added DIPEA (838 mg, 6.48 mmol) at 0 °C and the mixture was stirred at room temperature for 1 hour. The mixture was diluted with DCM, washed with brine, dried over anhydrous Na₂SO₄, filtered and concentrated to dryness. The residue was purified by chromatography on silica gel (eluted with PE: EtOAc= 150: 1) to afford compound **2-3** (260 mg, yield 63.4%) as a light yellow oil. LC/MS (ESI) m/z: 316/318 (M+H)⁺.

**Step 3: 6-Bromo-3-((3-fluoroazetidin-1-yl)methyl)pyridin-2-amine (2-4):** A solution of compound **2-3** (260 mg, 0.82 mmol) in TFA (6 mL) was stirred at 70 °C for 1 hour. The reaction mixture was concentrated to dryness and the residue was neutralized with ice-cooled 5% aqueous NaHCO₃ solution. The mixture was extracted with DCM twice and the combined organic layers were washed with brine, dried over anhydrous Na₂SO₄, filtered and concentrated to dryness. The residue was purified by chromatography on silica gel (eluted with PE: EtOAc= 5: 1) to afford compound **2-4** (168 mg, yield 78.6%) as a white solid. LC/MS (ESI) m/z: 260/262 (M+H)⁺.

**Step 4: Compound 2-5:** To a mixture of compound **2-4** (12 mg, 0.05 mmol) and compound **5** (25 mg, 0.05 mmol) in DCM (3 mL) was added pyridine (18 mg, 0.23 mmol) followed by POCl₃ (8 mg, 0.05 mmol) at 0 °C and the mixture was stirred at room temperature under N₂ atmosphere for1 hour. The mixture was poured into ice-water and extracted with DCM twice. The combined organic layers were washed with brine, dried with anhydrous Na₂SO₄, filtered and concentrated to dryness. The residue was purified by chromatography on silica gel (eluted with DCM: MeOH= 100: 1 to 30: 1) to afford compound **2-5** (21 mg, yield 58.5%) as a white solid. LC/MS (ESI) m/z: 798/800 (M+H)⁺.

**Step 5: Compound 2:** To a solution of compound **2-5** (21 mg, 0.026 mmol) in EtOAc (5 mL) was added PtO₂ (6 mg) at 0 °C and the mixture was degassed under N₂ atmosphere for three times and stirred under a H₂ balloon at room temperature for 1 hour. The mixture was filtered and the filtrate was concentrated to dryness. The residue was purified by preparative HPLC to afford **Compound 2** (3 mg, yield 14.3%) as a white solid. ¹H-NMR (400 MHz, CD₃OD) δ 8.95 (s, 2H), 8.41 (d, J= 1.6 Hz, 1H), 7.58 (d, J= 8.0 Hz, 1H), 7.51 (d, J= 2.0 Hz, 1H), 7.37 (d, J= 8.0 Hz, 1H), 5.95 (d, J= 17.6 Hz, 1H), 5.66 (d, J= 17.6 Hz, 1H),4.54-4.44 (m,1 H) 3.62 (dd, J= 6.0, 6.0 Hz, 1H), 3.51 (d, J= 14.4 Hz, 1H), 3.35 (dd, J= 14.0, 14.0 Hz, 5H), 3.16-3.09 (m, 1H), 2.99 (s, 1H), 2.90-2.81 (m, 2H), 2.74 (s, 3H), 2.69 (s, 3H), 2.59 (d, J= 4.8 Hz, 2H), 2.42-2.28 (m, 2H), 1.88-1.51 (m, 8H), 1.42 (t, J= 6.0 Hz, 2H), 1.18 (dd, J= 6.0, 6.0 Hz, 1H). LC/MS (ESI) m/z: 800/802 (M+H)⁺.

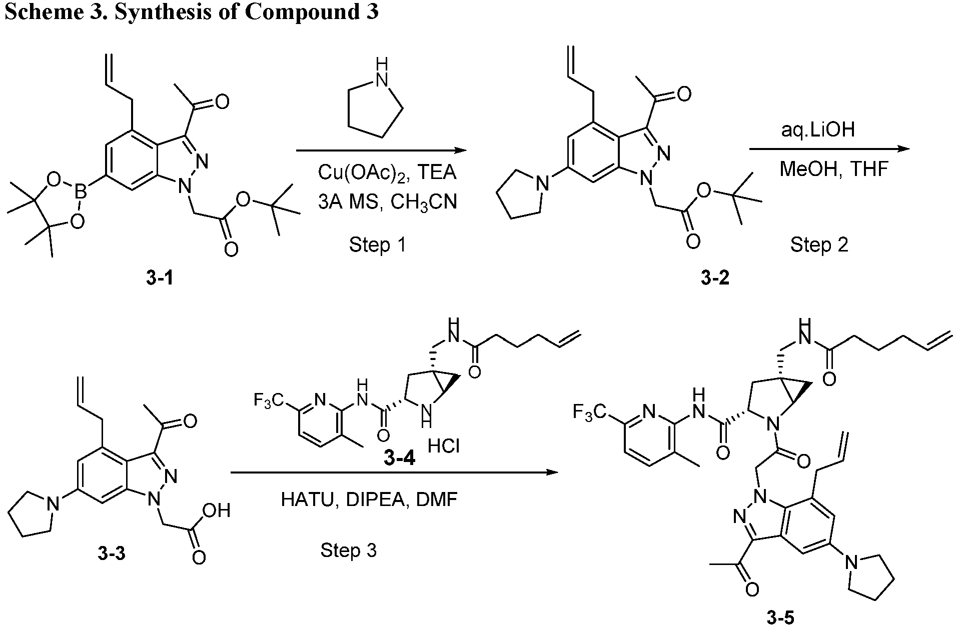

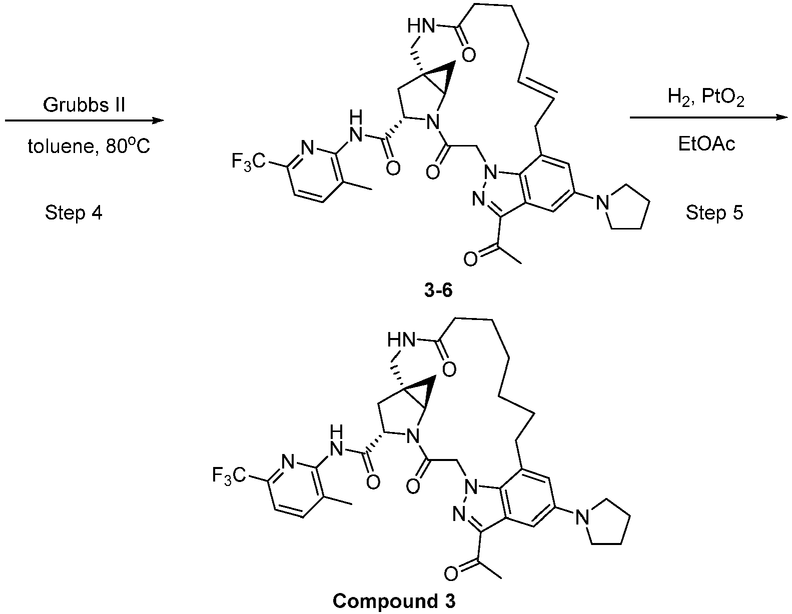

**Step 1: *tert*-Butyl 2-[3-acetyl-4-(prop-2-en-1-yl)-6-(pyrrolidin-1-yl)indazol-1-yl]acetate (3-2):** To a mixture of compound **3-1** (40 mg, 0.09 mmol) and pyrrolidine (10 mg, 0.18 mmol) in MeCN (1 mL) was added TEA (20 mg, 0.18 mmol), Cu(OAc)₂ (20 mg, 0.09 mmol) and 3A molecular sieve (10 mg) and the mixture was stirred under O₂ atmosphere at 80 °C for 24 hours. The reaction mixture was diluted with EtOAc and filtered and the filtrate was concentrated to dryness. The residue was purified by silica gel chromatography (eluted with PE: EtOAc= 3: 1) to afford compound **3-2** (30 mg, yield 74.6%) as a yellow oil. LC/MS (ESI) m/z: 384 (M+H)⁺.

**Step 2: [3-Acetyl-4-(prop-2-en-1-yl)-6-(pyrrolidin-1-yl)indazol-1-yl]acetic acid (3-3):** To a solution of compound 3-2 (26 mg, 0.07 mmol) in THF (2 mL) and MeOH (1 mL) was added a solution of LiOH (6 mg, 0.14 mmol) in water (1 mL) and the mixture was stirred at room temperature for 2 hours. The mixture was concentrated to dryness and the residue was diluted with water and washed with ether twice. The aqueous layer was acidified with 1N aqueous HCl to a pH of approximately 3 and the mixture was extracted with DCM twice. The combined organic layers were washed with brine, dried over anhydrous Na₂SO₄, filtered and concentrated to dryness to afford compound 3-3 (20 mg, yield 90.1%) as a colorless oil. LC/MS (ESI) m/z: 328 (M+H)⁺.

**Step 3: (1*R*,3*S*,5*R*)-2-{2-[3-Acetyl-7-(prop-2-en-1-yl)-5-(pyrrolidin-1-yl)indazol-1-yl]acetyl}-5-(hex-5-enamidomethyl)-*N*-[3-methyl-6-(trifluoromethyl)pyridin-2-yl]-2-azabicyclo[3.1.0]hexane-3-carboxamide (3-5):** To a mixture of compound **3-3** (44 mg, 0.13 mmol) and (1*R*,3*S*,5*R*)-5-(hex-5-enamidomethyl)-*N*-(3-methyl-6-(trifluoromethyl)pyridin-2-yl)-2-azabicyclo[3.1.0]hexane-3-carboxamide hydrochloride (58 mg, 0.13 mmol) in DMF (2 mL) was added HATU (102 mg, 0.27 mmol) and DIPEA (52 mg, 0.40 mmol) at 0 °C and the resulting mixture was stirred at room temperature for 4 hours. The mixture was diluted with EtOAc and washed with saturated aqueous NH₄Cl solution and brine, dried over anhydrous Na₂SO₄, filtered and concentrated to dryness. The residue was purified by silica gel chromatography (eluted with PE: EtOAc= 10: 1 to 3: 1) to afford compound **3-5** (36 mg, yield 37.2%) as a white solid. LC/MS (ESI) m/z: 720 (M+H)⁺.

**Step 4: Compound 3-6:** To a solution of compound **3-5** (36 mg, 0.05 mmol) in degassed toluene (25 mL) was added Grubbs 2^{nd} catalyst (8 mg, 0.01 mmol) and the reaction mixture was stirred at 80 °C under N₂ for 16 hours. The mixture was concentrated to dryness and the residue was purified by silica gel column chromatography (eluted with PE: EtOAc= 5: 1 to 2: 1) to afford compound **3-6** (32 mg, yield 92.5%) as a brown solid. LC/MS (ESI) m/z: 691 (M+H)⁺.

**Step 5: Compound 3:** To a solution of compound **3-6** (32 mg, 0.05 mmol) in EtOAc (3 mL) was added PtO₂ (53mg, 0.03 mmol) and the reaction mixture was stirred under a H₂ balloon at room temperature for 0.5 hour. The mixture was filtered and the filtrate was concentrated to dryness. The residue was purified by preparative HPLC to afford **Compound 3** (2 mg, yield 6.2%) as a white solid. ¹H-NMR (400 MHz, CD₃OD) δ 7.82 (d, J= 7.8 Hz, 1H), 7.56 (d, J= 7.8 Hz, 1H), 7.14 (d, J= 2.0 Hz, 1H), 6.69 (s, 1H), 5.82 (d, J= 17.7 Hz, 1H), 5.52 (d, J= 17.9 Hz, 1H), 4.53 (d, J= 7.0 Hz, 1H), 3.60 (m, 1H), 3.47 (m, 1H), 3.35 (m, 1H), 3.05-2.97 (m, 1H), 2.71 (m, 1H), 2.62 (s, 3H), 2.59 (m, 2H), 2.39-2.15 (m, 4H), 2.15 (s, 3H), 2.03 (m, 4H), 1.66 (m, 8H), 1.42-1.37 (m, 2H), 1.15 (m, 1H), 0.89 (m, 1H). LC/MS (ESI) m/z: 694 (M+H)⁺.

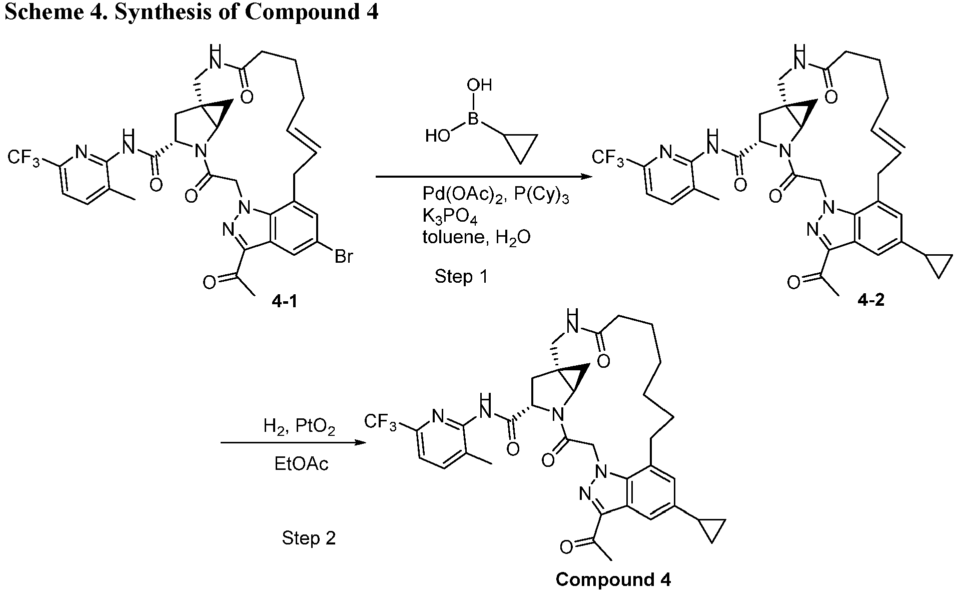

**Step 1: Compound 4-2:** To a mixture of cyclopropylboronic acid (4 mg, 0.051 mmol) and compound **4-1** (30 mg, 0.043 mmol) in toluene (4 mL) and water (1 mL) was added K₃PO₄ (23 mg, 0.11 mmol), tricyclohexyl phosphine (2 mg, 0.009 mmol) and Pd(OAc)₂ (4 mg, 0.004 mmol). The mixture was degassed under N₂ atmosphere three times and stirred at 90 °C under N₂ atmosphere for 16 hours. The mixture was diluted with EtOAc and washed with water and brine, dried over anhydrous Na₂SO₄, filtered and concentrated to dryness. The residue was purified by silica gel chromatography (eluted with DCM: MeOH= 30: 1) to afford compound **4-2** (28 mg, yield 98.8%) as a yellow solid. LC/MS (ESI) m/z: 663 (M+H)⁺.

**Step 2: Compound 4:** To a solution of compound **4-2** (28 mg, 0.042 mmol) in EtOAc (5 mL) was added PtO₂ (5 mg) and the mixture was degassed under N₂ atmosphere three times and stirred under a H₂ balloon at room temperature for 1 hour. The mixture was filtered and the filtrate was concentrated to dryness. The residue product was purified by preparative HPLC to afford **Compound 4** (7 mg, yield 24.3%) as a white solid. ¹H-NMR (400 MHz, DMSO-*d₆*) δ 10.45 (s, 1H), 8.18 (dd, J= 8.3, 3.5 Hz, 1H), 7.90 (d, J= 7.7 Hz, 1H), 7.72 (s, 1H), 7.65 (d, J= 7.7 Hz, 1H), 6.92 (s, 1H), 5.81 (d, J= 17.8 Hz, 1H), 5.41 (d, J= 17.8 Hz, 1H), 4.32 (m, 1H), 3.54 (m, 1H), 3.28 (d, J= 8.9 Hz, 1H), 3.17 (m, 1H), 3.05-2.96 (m, 1H), 2.72 (m, 1H), 2.57 (s, 3H), 2.47-2.40 (m, 2H), 2.20-2.11 (m, 2H), 2.09 (s, 3H), 2.06-2.00 (m, 1H), 1.77-1.21 (m, 10H), 1.18 (m, 1H), 1.07 (m, 1H), 0.98-0.91 (m, 2H), 0.69-0.63 (m, 2H). LC/MS (ESI) m/z: 665 (M+H)⁺.

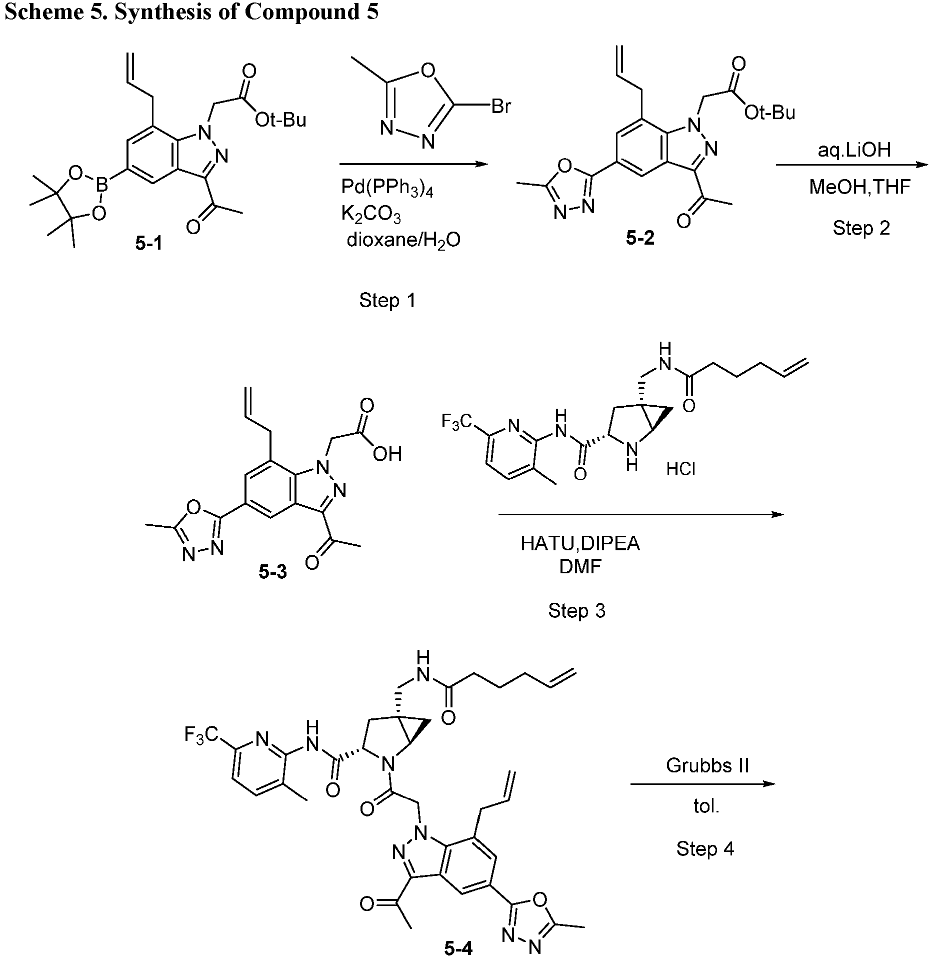

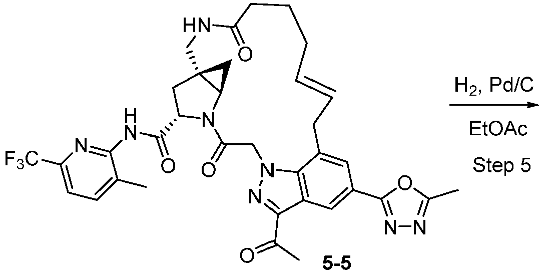

**Step 1: *tert*-Butyl 2-(3-acetyl-7-allyl-5-(5-methyl-1,3,4-oxadiazol-2-yl)-1*H*-indazol-1-yl)acetate (5-2):** To a solution of 2-bromo-5-methyl-1,3,4-oxadiazole (70 mg, 0.43 mmol) and compound **5-1** (227 mg, 0.52 mmol) in 1,4-dioxane (5 mL) and water (1 mL) was added K₂CO₃ (148 mg, 1.07 mmol) and Pd(PPh₃)₄ (50 mg, 0.043 mmol). The mixture was degassed under N₂ three times and stirred at 90 °C under N₂ atmosphere for 16 hours. The mixture was diluted with EtOAc and washed with water and brine, dried over anhydrous Na₂SO₄, filtered and concentrated to dryness. The residue was purified by silica gel chromatography (eluted with PE: EtOAc= 20: 1) to afford compound **5-2** (75 mg, yield 44.1%) as a yellow solid. LC/MS (ESI) m/z: 397 (M+H)⁺.

**Step 2: 2-(3-Acetyl-7-allyl-5-(5-methyl-1,3,4-oxadiazol-2-yl)-1*H*-indazol-1-yl)acetic acid (5-3):** To a solution of compound **5-2** (75 mg, 0.19 mmol) in THF (1 mL), MeOH (1 mL) and water (0.5 mL) was added LiOH (11 mg, 0.47 mmol) and the mixture was stirred at room temperature for 2 hours. The mixture was concentrated to dryness and diluted with water. The mixture was washed with EtOAc and acidified with 1 N aqueous HCl to a pH of approximately 3 at 0 °C. The mixture was extracted with DCM twice and the combined organic layers were washed with brine, dried over anhydrous Na₂SO₄, filtered and concentrated to dryness to afford compound **5-3** (65 mg, yield 101%) as a light yellow solid that was directly used to the next reaction without purification. LC/MS (ESI) m/z: 341 (M+H)⁺.

**Step 3: (1*R*,3*S*,5*R*)-2-(2-(3-Acetyl-7-allyl-5-(5-methyl-1,3,4-oxadiazol-2-yl)-1*H-*indazol-1-yl)acetyl)-5-(hex-5-enamidomethyl)-*N*-(3-methyl-6-(trifluoromethyl)pyridin-2-yl)-2-azabicyclo[3.1.0]hexane-3-carboxamide (5-4):** To a mixture of compound **5-3** (65 mg, 0.19 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (70 mg, 0.159 mmol) in DMF (3 mL) was added HATU (73 mg, 0.19 mmol) and DIPEA (51 mg, 0.40 mmol) at 0 °C and the reaction mixture was stirred at room temperature for 2 hours. The mixture was diluted with EtOAc, washed with water and brine, dried over Na₂SO₄, filtered and concentrated to dryness. The residue was purified by column chromatography via silica gel (eluted with DCM: MeOH= 30: 1) to afford compound **5-4** (50 mg, yield 42.9%) as a white solid. LC/MS (ESI) m/z: 733 (M+H)⁺.

**Step 4: Compound 5-5:** To a solution of compound **5-4** (50 mg, 0.068 mmol) in degassed toluene (50 mL) was added Grubbs 2^{nd} catalyst (14 mg) and the mixture was degassed under N₂ atmosphere three times and stirred at 80°C under N₂ atmosphere for 16 hours. The mixture was concentrated to dryness and the residue was purified by silica gel chromatography (eluted with DCM: MeOH= 30: 1) to afford compound **5-5** (40 mg, yield 83.2%) as a light yellow solid. LC/MS (ESI) m/z: 705 (M+H)⁺.

**Step 5: Compound 5:** To a solution of compound **5-5** (40 mg, 0.057 mmol) in EtOAc (5 mL) was added PtO₂ (14 mg, 0.064 mmol) and the mixture was degassed under N₂ three times. The mixture was stirred under a H₂ balloon at room temperature for 1 hour. The mixture was filtered and the filtrate was concentrated to dryness. The residue product was purified by preparative HPLC to afford **Compound 5** (5 mg, yield 11.1%) as a white solid. ¹H-NMR (400 MHz, CD₃OD) δ 8.78 (d, *J=* 1.3 Hz, 1H), 7.87 (s, 1H), 7.80 (d, *J=* 7.8 Hz, 1H), 7.54 (d, *J=* 7.8 Hz, 1H), 5.99 (d, *J=* 17.9 Hz, 1H), 5.65 (d, *J=* 17.9 Hz, 1H), 4.49 (d, *J=* 6.7 Hz, 1H), 3.67 (dd, *J=* 5.8, 2.7 Hz, 1H), 3.48 (d, *J=* 14.5 Hz, 1H), 3.34 (s, 1H), 3.20-3.10 (m, 1H), 2.98-2.87 (m, 1H), 2.68 (s, 3H), 2.64 (s, 3H), 2.59 (t, *J=* 6.6 Hz, 2H), 2.31 (dd, *J=* 13.0, 6.8 Hz, 2H), 2.11 (s, 3H), 1.89-1.45 (m, 8H), 1.39 (t, *J*= 5.8 Hz, 1H), 1.15 (dd, *J=* 5.7, 2.7 Hz, 1H). LC/MS (ESI) m/z: 707 (M+H)⁺.

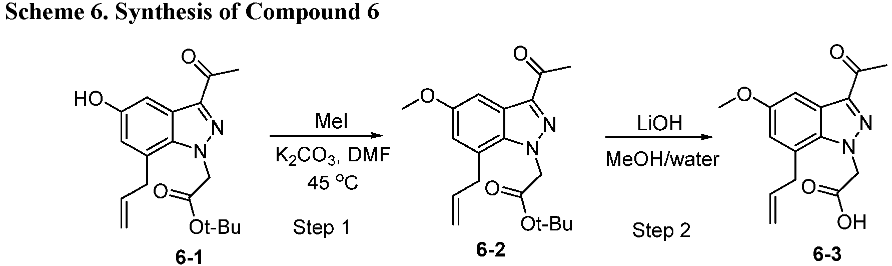

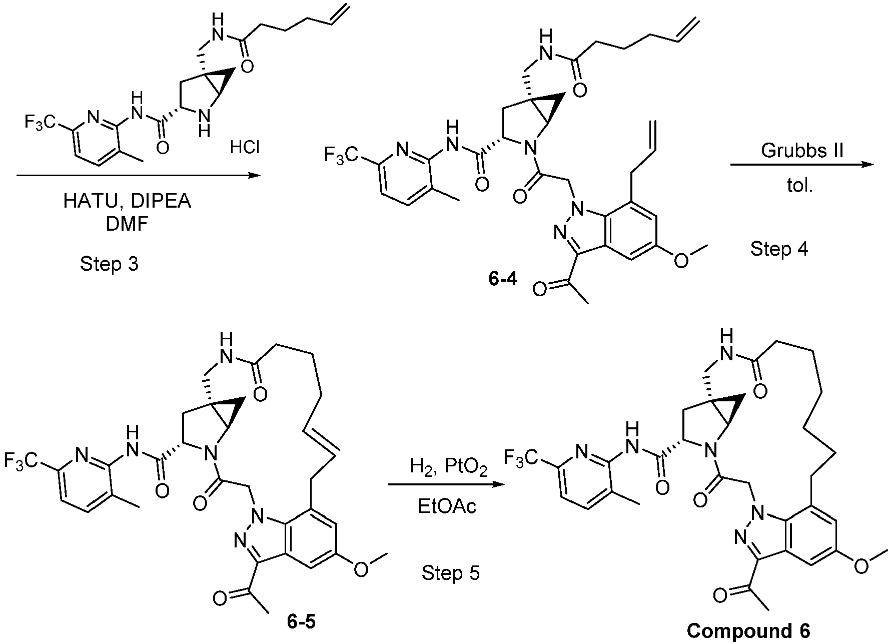

**Step 1: *tert-Butyl* 2-[3-acetyl-5-methoxy-7-(prop-2-en-1-yl)indazol-1-yl]acetate (6-2):** To a solution of tert-butyl 2-[3-acetyl-5-hydroxy-7-(prop-2-en-1-yl)indazol-1-yl]acetate (60 mg, 0.18 mmol) in DMF (1 mL) was added methyl iodide (129 mg, 0.91 mmol) followed by potassium carbonate (50 mg, 0.36 mmol) at 0 °C and the mixture was stirred in a sealed tube at 50 °C for 16 hours. The mixture was diluted with EtOAc and washed with water and brine, dried over anhydrous Na₂SO₄, filtered and concentrated to dryness. The residue was purified by column chromatography on silica gel (eluted with PE: EtOAc= 4: 1) to afford compound **6-2** (50 mg, yield 79.9%) as a white solid. LC/MS (ESI) m/z: 345 (M+H)⁺.

**Step 2: [3-Acetyl-5-methoxy-7-(prop-2-en-1-yl)indazol-1-yl]acetic acid (6-3):** To a solution of compound 6-2 (50 mg, 0.145 mmol) in MeOH (3 mL) was added a solution of lithiumol (17 mg, 0.73 mmol) in water (1 mL) and the mixture was stirred at room temperature for 2 hours. The mixture was diluted with water and washed with ether. The aqueous layer was acidified with 1N aqueous HCl solution to a pH of approximately 3 and extracted with DCM. The organic layer was washed with brine, dried over anhydrous Na₂SO₄, filtered and concentrated to dryness to afford compound **6-3** (40 mg, yield 95.6%) as a white solid. LC/MS (ESI) m/z: 289 (M+H)⁺.

**Step 3: (1*R*,3*S*,5*R*)-2-{2-[3-Acetyl-5-methoxy-7-(prop-2-en-1-yl)indazol-1-yl]acetyl}-5-(hex-5-enamidomethyl)-*N*-[3-methyl-6-(trifluoromethyl)pyridin-2-yl]-2-azabicyclo[3.1.0]hexane-3-carboxamide (6-4):** To a mixture of compound **6-3** (40 mg, 0.14 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (63 mg, 0.14 mmol) in DMF (3 mL) was added DIPEA (45 mg, 0.35 mmol) followed by HATU (63 mg, 0.17 mmol) at 0 °C and the mixture was stirred at room temperature for 1 hour. The mixture was diluted with water and extracted with EtOAc twice. The combined organic layers were washed with saturated aqueous NH₄Cl solution and brine, dried over anhydrous Na₂SO₄, filtered and concentrated to dryness. The residue was purified by chromatography on silica gel (eluted with PE: EtOAc= 1: 3) to afford compound **6-4** (43 mg, yield 45.5%) as a colorless oil. LC/MS (ESI) m/z: 681 (M+H)⁺.

**Step 4: Compound 6-5:** To a solution of compound **6-4** (39 mg, 0.057 mmol) in degassed toluene (40 mL) was added Grubbs 2^{nd} catalyst (10 mg, 0.011 mmol) at 0 °C under N₂ atmosphere and the mixture was stirred at 80 °C under N₂ atmosphere overnight. The mixture was concentrated to dryness and the residue was purified by chromatography on silica gel (eluted with DCM: MeOH= 100: 1 to 50: 1) to afford compound **6-5** (25 mg, yield 69.1%) as a brown solid. LC/MS (ESI) m/z: 653 (M+H)⁺.

**Step 5: Compound 6:** To a solution of compound **6-5** (30 mg, 0.046 mmol) in EtOAc (15 mL) was added platinum dioxide (10 mg) at 0 °C and the mixture was degassed under N₂ atmosphere three times and stirred under a H₂ balloon at room temperature for 1 hour. The mixture was filtered and the filtrate was concentrated to dryness. The residue was purified by preparative HPLC to afford **Compound 6** (5 mg, yield 16.6%) as a white solid. ¹H-NMR (400 MHz, CD₃OD) δ 7.81 (d, J=8.0 Hz, 1H), 7.55 (d, J= 7.6 Hz, 1H), 7.53 (d, J= 2.4 Hz, 1H), 6.83 (d, J=2.4 Hz, 1H), 5.88 (d, J= 18.0 Hz, 1H), 5.54 (d, J= 18.0 Hz, 1H), 4.50 (m, 1H), 3.83 (s, 3H), 3.62 (m, 1H), 3.50-3.42 (m, 1H), 3.36-3.33 (m, 1H), 3.09-2.95 (m, 1H), 2.81-2.69 (m, 1H) , 2.64 (s, 3H), 2.61-2.54 (m, 2H), 2.40-2.21 (m, 2H), 2.13 (s, 3H), 1.84-1.45 (m, 7H), 1.43-1.33 (m, 2H), 1.16-1.10 (m, 1H). LC/MS (ESI) m/z: 655 (M+H)⁺.

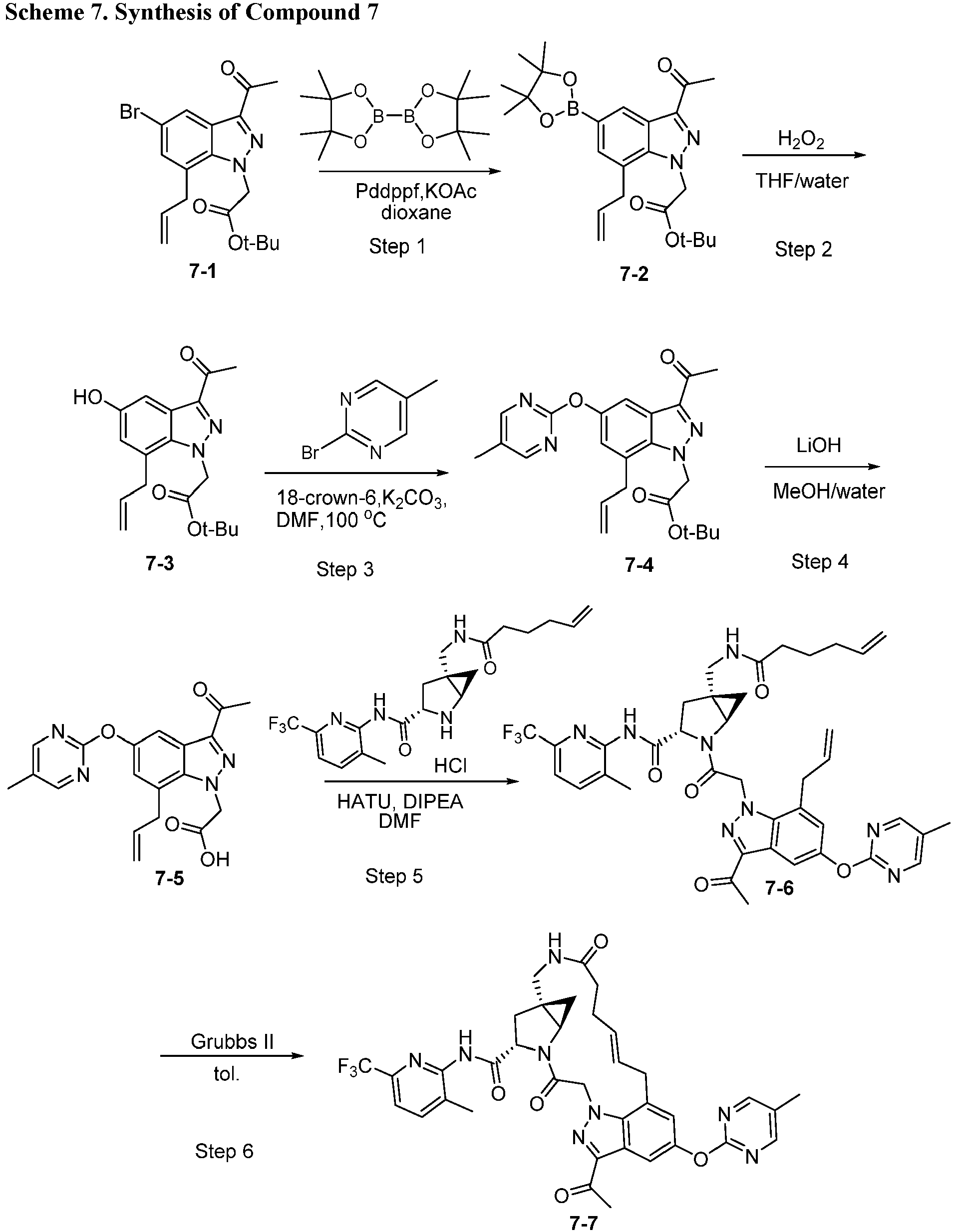

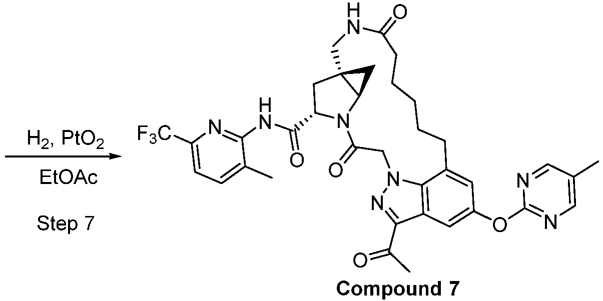

**Step 1: *tert*-Butyl 2-(3-acetyl-7-allyl-5-(4,4,5,5-tetramethyl-1,3,2-dioxaborolan-2-yl)-1*H*-indazol-1-yl)acetate (7-2):** To a mixture of *tert*-butyl 2-[3-acetyl-5-bromo-7-(prop-2-en-1-yl)indazol-1-yl]acetate (500 mg, 1.27 mmol) and potassium acetate (374 mg, 3.8 mmol) in 1,4-dioxane (6 mL) was added bis(pinacolato)diboron (355 mg, 1.4 mmol) followed by 1,1'-bis(diphenylphosphino)ferrocene palladium(II)dichloride (93 mg, 0.13 mmol) under N₂ atmosphere. The mixture was stirred at 100°C under N₂ atmosphere for 30 hours. The mixture was filtered and the filtrate was concentrated to dryness. The residue was purified by chromatography on silica gel (eluted with PE: EtOAc= 50: 1 to 20: 1) to afford compound **7-2** (290 mg, yield 51.8%) as a yellow solid. LC/MS (ESI) m/z: 441 (M+H)⁺.

**Step 2: *tert*-Butyl 2-(3-acetyl-7-allyl-5-hydroxy-1*H*-indazol-1-yl)acetate (7-3):** To a solution of compound **7-2** (150 mg, 0.34 mmol) in THF (3 mL) was added hydrogen peroxide (193 mg, 1.70 mmol, 30% wt) and the mixture was stirred at room temperature for 1 hour. The mixture was diluted with EtOAc and washed with water and brine, dried over anhydrous Na₂SO₄, filtered and concentrated to dryness to afford compound **7-3** (105 mg, yield 93.3%) as a white solid. LC/MS (ESI) m/z: 331 (M+H)⁺.

**Step 3: *tert*-Butyl 2-(3-acetyl-7-allyl-5-((5-methylpyrimidin-2-yl)oxy)-1*H*-indazol-1-yl)acetate (7-4):** To a mixture of compound 7-3 (80 mg, 0.24 mmol) and 2-bromo-5-methylpyrimidine (46 mg, 0.27 mmol) in DMF (2 mL) was added potassium carbonate (67 mg, 0.48 mmol) and 18-crown-6 (64 mg, 0.24 mmol) and the mixture was stirred at 100 °C for 16 hours. The mixture was diluted with EtOAc and washed with saturated aqueous NH₄Cl solution and brine, dried over anhydrous Na₂SO₄, filtered and concentrated to dryness. The residue was purified by chromatography on silica gel (eluted with PE: EtOAc= 2: 1) to afford compound **7-4** (47 mg, yield 45.9 %) as a yellow solid. LC/MS (ESI) m/z: 423 (M+H)⁺.

**Step 4: 2-(3-Acetyl-7-allyl-5-((5-methylpyrimidin-2-yl)oxy)-1*H*-indazol-1-yl)acetic acid (7-5):** To a solution of compound **7-4** (47 mg, 0.11 mmol) in MeOH (3 mL) was added a solution of lithiumol (14 mg, 0.57 mmol) in water (1 mL) at 0 °C, and the mixture was stirred at room temperature for 2 hours. The mixture was diluted with water and washed with diethyl ether. The aqueous layer was acidified with 1N aqueous HCl solution to a pH of approximately 3 and extracted with DCM. The organic layer was washed with brine, dried over anhydrous Na₂SO₄, filtered and concentrated to dryness to afford compound **7-5** (40 mg, yield 96.1 %) as a white solid. LC/MS (ESI) m/z: 367 (M+H)⁺.

**Step 5: (1*R*,3*S*,5*R*)-2-(2-(3-Acetyl-7-allyl-5-((5-methylpyrimidin-2-yl)oxy)-1*H-*indazol-1-yl)acetyl)-5-(hex-5-enamidomethyl)-N-(3-methyl-6-(trifluoromethyl)pyridin-2-yl)-2-azabicyclo[3.1.0]hexane-3-carboxamide (7-6):** To a mixture of compound **7-5** (45 mg, 0.11 mmol) and (1*R*,3*S*,5*R*)-5-(hex-5-enamidomethyl)-*N*-(3-methyl-6-(trifluoromethyl)pyridin-2-yl)-2-azabicyclo[3.1.0]hexane-3-carboxamide hydrochloride (49 mg, 0.11 mmol) in DMF (3 mL) was added DIPEA (53 mg, 0.4 mmol) followed by HATU (50 mg, 0.13 mmol) at 0 °C and the mixture was stirred at room temperature for 1 hour. The mixture was diluted with water and extracted with EtOAc twice. The combined organic layers were washed with saturated aqueous NH₄Cl solution and brine, dried over anhydrous Na₂SO₄, filtered and concentrated to dryness. The residue was purified by chromatography on silica gel (eluted with DCM: MeOH= 30: 1) to afford compound **7-6** (43 mg, yield 51.9%) as a light yellow solid. LC/MS (ESI) m/z: 759 (M+H)⁺.

**Step 6: Compound 7-7:** To a solution of compound **7-6** (43 mg, 0.06 mmol) in degassed toluene (40 mL) was added Grubbs 2^{nd} Catalyst (10 mg, 0.01 mmol) at 0 °C under N₂ atmosphere and the mixture was stirred at 80 °C under N₂ atmosphere overnight. The mixture was concentrated to dryness and the residue was purified by chromatography on silica gel (eluted with DCM: MeOH= 30: 1) to afford compound **7-7** (25 mg, yield 61.5%) as a brown solid. LC/MS (ESI) m/z: 717 (M+H)⁺.

**Step 7: Compound 7:** To a solution of compound **7-7** (25 mg, 0.035 mmol) in EtOAc (15 mL) was added platinum dioxide (10 mg) at 0 °C, and the mixture was degassed under N₂ atmosphere three times and stirred under a H₂ balloon at room temperature for 1 hour. The mixture was filtered and the filtrate was concentrated to dryness. The residue was purified by preparative HPLC to afford **Compound 7** (1 mg, yield 3.98%) as a white solid. ¹H-NMR (400 MHz, CD₃OD) δ 8.41 (s, 2H), 7.84 (d, J=2.0 Hz, 1H), 7.81 (d, J=8.0 Hz, 1H), 7.55 (d, J=7.6 Hz, 1H), 7.02 (d, J=2.4 Hz, 1H), 5.72 (d, J= 17.6 Hz, 1H), 5.90 (d, J= 17.6 Hz, 1H), 4.32 (m, 1H), 3.75 (m, 1H), 3.55 (d, J= 14.8 Hz, 1H), 3.38-3.31 (m, 1H), 3.08-2.97 (m, 1H), 2.94-2.82 (m, 1H), 2.70-2.60 (m, 1H) , 2.65 (s, 3H), 2.54-2.46 (m, 1H), 2.28 (s, 3H), 2.23-2.21 (m, 2H), 2.18 (s, 3H), 1.90-1.51 (m, 6H), 1.37-1.24 (m, 1H), 1.18-1.11 (m, 1H). LC/MS (ESI) m/z: 719 (M+H)⁺.

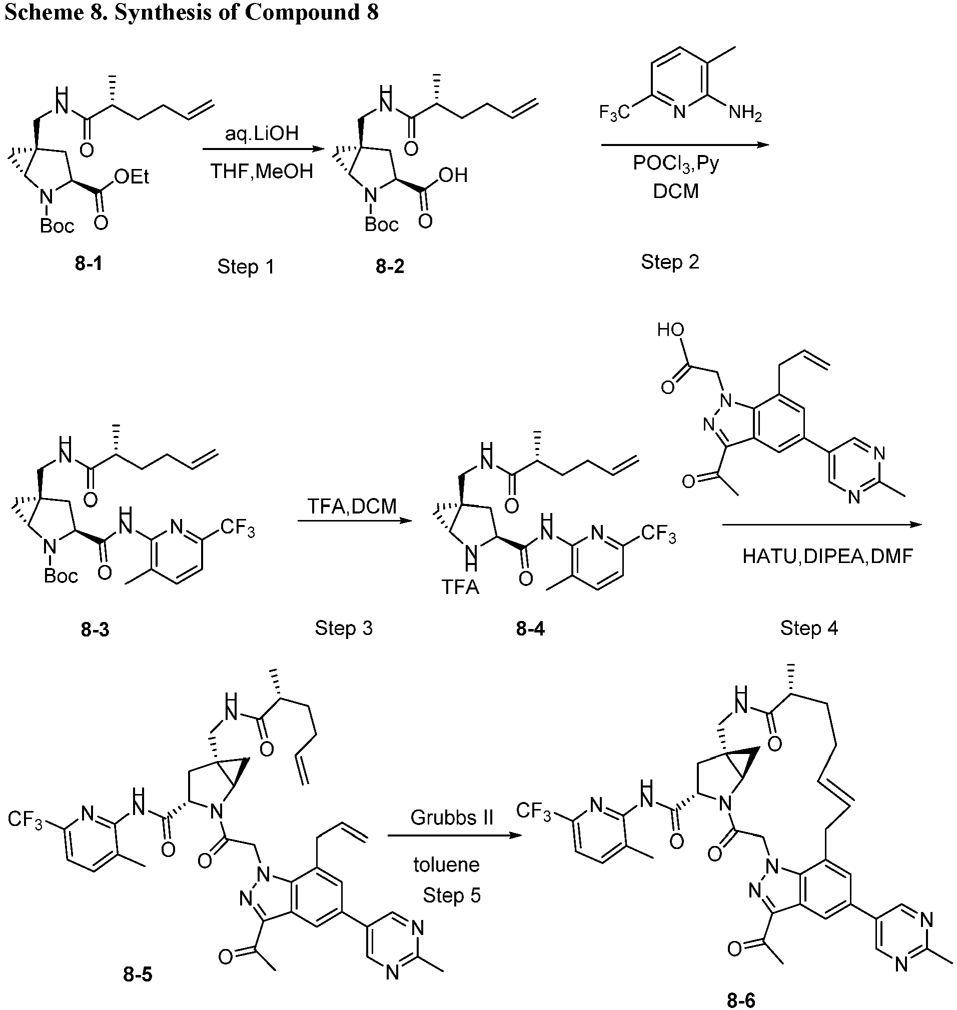

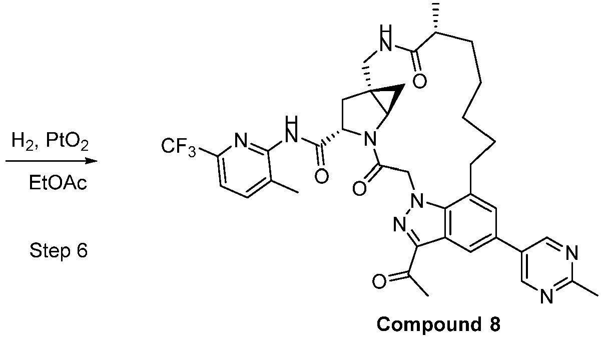

**Step 1: (1*R*,3*S*,5*R*)-2-(*tert*-Butoxycarbonyl)-5-(((R)-2-methylhex-5-enamido)methyl)-2-azabicyclo[3.1.0]hexane-3-carboxylic acid (8-2):** To a solution of compound **8-1** (290 mg ,0.74 mmol) in MeOH (2 mL),THF(2 mL) and water (2 mL) was added LiOH (88 mg, 3.68 mmol) and the mixture was stirred at room temperature for 2 hours. The mixture was concentrated to dryness and the residue was dissolved in water and washed with diethyl ether twice. The aqueous layer was acidified with 1N aqueous HCl to a pH of approximately 4 and extracted with EtOAc twice. The combined organic layers were washed with brine, dried over Na₂SO₄ and concentrated under reduced pressure to afford compound **8-2** (230 mg, yield 85.4%) as a white solid. LC/MS (ESI) m/z: 365 (M+H)⁻.

**Step 2: (1*R*,3*S*,5*R*)-*tert*-Butyl 3-((3-methyl-6-(trifluoromethyl)pyridin-2-yl)carbamoyl)-5-(((*R*)-2-methylhex-5-enamido)methyl)-2-azabicyclo[3.1.0]hexane-2-carboxylate (8-3):** To a mixture of compound 2 (290 mg, 0.79 mmol) and 3-methyl-6-(trifluoromethyl)pyridin-2-amine (139 mg, 0.79 mmol) in DCM (6 mL) was added pyridine (313 mg, 3.96 mmol) followed by the drop-wise addition of POCl₃ (121 mg, 0.79 mmol) at 0 °C and the mixture was stirred at room temperature for 1 hour. The mixture was diluted with DCM and washed with 1N aqueous HCl solution and brine, dried over Na₂SO₄, filtered and concentrated to dryness. The residue was purified by silica gel chromatography (eluted with PE: EtOAc= 10: 1 to 5: 1) to afford compound **8-3** (230 mg, yield 55.4%) as a white solid. LC/MS (ESI) m/z: 525 (M+H)⁺.

**Step 3: (1*R*,3*S*,5*R*)-*N*-(3-Methyl-6-(trifluoromethyl)pyridin-2-yl)-5-(((*R*)-2-methylhex-5-enamido)methyl)-2-azabicyclo[3.1.0]hexane-3-carboxamide TFA salt (8-4):** To a solution of compound **8-3** (112 mg, 0.21 mmol) in DCM (2 mL) was added TFA (1 mL) at 0 °C and the mixture was stirred at room temperature for 1 hour. Then the mixture was concentrated to dryness to afford compound **8-4** (110 mg, yield 99.3%) as a brown solid that was directly used in the next reaction without further purification. LC/MS (ESI) m/z: 425 (M+H)⁺.

**Step 4: (1*R*,3*s*,5*R*)-2-(2-(3-Acetyl-7-allyl-5-(2-methylpyrimidin-5-yl)-1*H*-indazol-1-yl)acetyl)-*N*-(3-methyl-6-(trifluoromethyl)pyridin-2-yl)-5-(((*R*)-2-methylhex-5-enamido)methyl)-2-azabicyclo[3.1.0]hexane-3-carboxamide (8-5):** To a mixture of 2-(3-acetyl-7-allyl-5-(2-methylpyrimidin-5-yl)-1H-indazol-1-yl)acetic acid (74 mg, 0.21 mmol) and compound **4** (110 mg, 0.21 mmol) in DMF (5 mL) was added DIPEA (82 mg, 0.64 mmol) followed by HATU (121 mg, 0.32 mmol) at 0 °C and the mixture was stirred at 25 °C for 1 hour. The mixture was diluted with EtOAc, washed with saturated aqueous NH₄Cl solution and brine, dried and concentrated to dryness. The residue was purified by silica gel chromatography (eluted with PE: EtOAc=20: 1 to 1: 1) to afford compound **8-5** (130 mg, yield 81.0%) as a white solid. LC/MS (ESI) m/z: 757 (M+H)⁺.

**Step 5: Compound 8-6:** To a solution of compound 5 (130 mg, 0.172 mmol) in degassed toluene (100 mL) was added Grubbs 2^{nd} catalyst (36 mg, 0.04 mmol) and the mixture was stirred at 80 °C under N₂ atmosphere for 16 hours. The mixture was concentrated to dryness and the residue was purified by silica gel chromatography (eluted with PE: EtOAc= 20: 1 to 1: 3) to afford compound **8-6** (60 mg, yield 47.9%) as a brown solid. LC/MS (ESI) m/z: 729 (M+H)⁺.

**Step 6: Compound 8:** To a mixture of compound 8-6 (60 mg, 0.082 mmol) in EtOAc (2 mL) was added PtO₂ (12 mg) and the mixture was degassed under N₂ atmosphere three times and stirred under a H₂ balloon at room temperature for 1 hour. The mixture was filtered and the filtrate was concentrated to dryness. The residue was purified by preparative HPLC to afford **Compound 8** (10 mg, yield 16.6%) as a white solid. ¹H-NMR (400 MHz, DMSO-*d₆*) δ 10.52-10.29 (m, 1H), 9.01 (s, 2H), 8.30 (d, *J=* 1.4 Hz, 1H), 7.99 (t, *J=* 6.2 Hz, 1H), 7.89 (d, *J=* 7.8 Hz, 1H), 7.65 (d, *J*= 7.8 Hz, 1H), 7.55 (s, 1H), 5.93 (d, *J=* 17.7 Hz, 1H), 5.52 (d, *J=* 17.7 Hz, 1H), 4.27 (t, *J*= 8.2 Hz, 1H), 3.63 (m, 1H), 3.56 (dd, *J=* 14.2, 5.7 Hz, 1H), 3.15-2.94 (m, 3H), 2.68 (s, 2H), 2.64 (s, 2H), 2.49-2.44 (m, 2H), 2.33-2.24 (m, 1H), 2.13 (s, 3H), 1.90-1.75 (m, 1H), 1.73-1.61 (m, 2H), 1.60-1.54 (m, 2H), 1.54-1.46 (m, 1H), 1.43-1.32 (m, 2H), 1.20-1.13 (m, 1H), 1.11-1.03 (m, 4H). LC/MS (ESI) m/z: 731 (M+H)⁺.

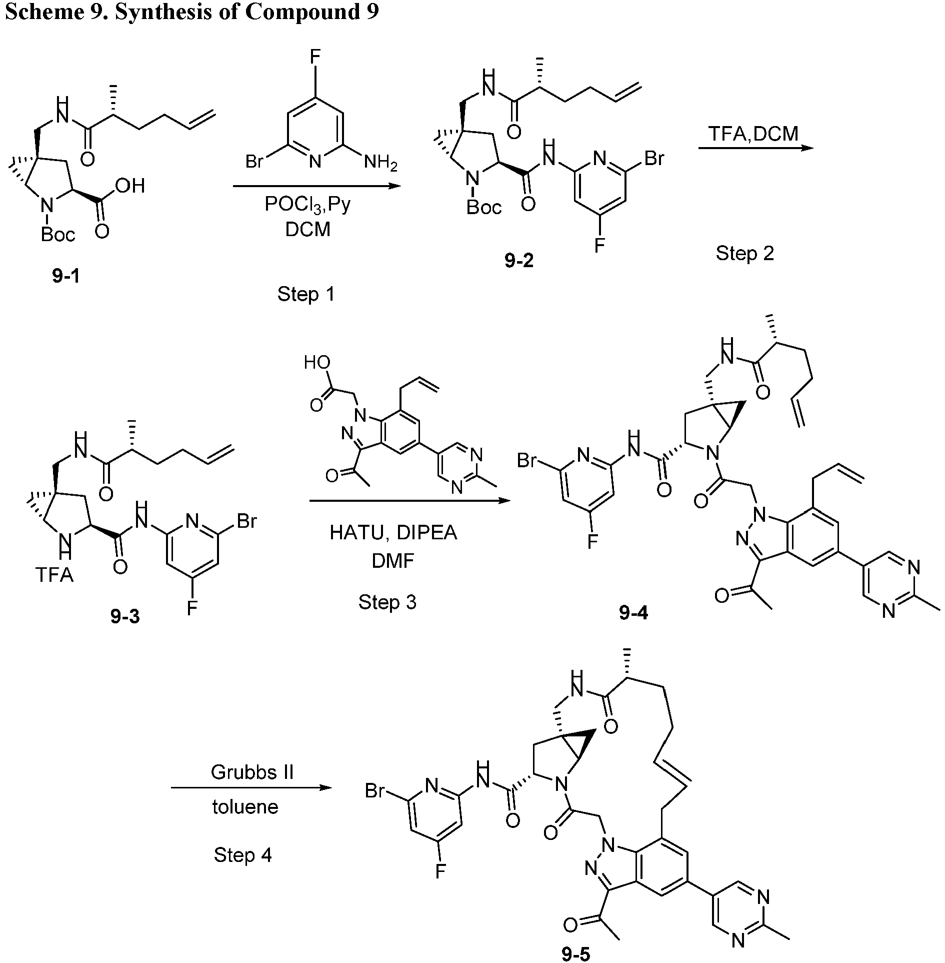

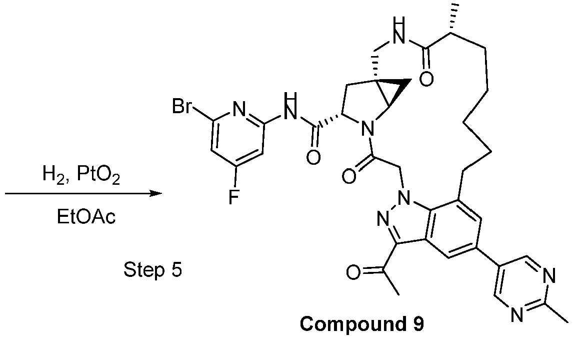

**Step 1: (1*R*,3*S*,5*R*)-*tert*-Butyl 3-((6-bromo-4-fluoropyridin-2-yl)carbamoyl)-5-(((*R*)-2-methylhex-5-enamido)methyl)-2-azabicyclo[3.1.0]hexane-2-carboxylate (9-2):** To a mixture of compound **9-1** (50 mg, 0.14 mmol) and 6-bromo-4-fluoropyridin-2-amine (26 mg, 0.14 mmol) in DCM (2 mL) was added pyridine (54 mg, 0.68 mmol) followed by POCl₃ (42 mg, 0.28 mmol) at 0 °C and the mixture was stirred at room temperature for 1 hour. The mixture was diluted with DCM and washed with 1N aqueous HCl solution and brine, dried over Na₂SO₄, filtered and concentrated to dryness. The residue was purified by silica gel chromatography (eluted with PE: EtOAc= 10: 1) to afford compound **9-2** (37 mg, yield 50.3%) as a white solid. LC/MS (ESI) m/z: 539/541 (M+H)⁺.

**Step 2: (1*R*,3*S*,5*R*)-*N*-(6-Bromo-4-fluoropyridin-2-yl)-5-(((*R*)-2-methylhex-5-enamido)methyl)-2-azabicyclo[3.1.0]hexane-3-carboxamide TFA salt (9-3):** To a solution of compound **9-2** (37 mg, 0.069 mmol) in DCM (2 mL) was added TFA (1 mL) at 0 °C and the resulting mixture was stirred at room temperature for 1 hour. Then the mixture was concentrated to dryness to afford compound **9-3** (35 mg, yield 99.6%) as a brown solid that was directly used in the next reaction without further purification. LC/MS (ESI) m/z: 439/441 (M+H)⁺.

**Step 3: (1*R*,3*s*,5*R*)-2-(2-(3-Acetyl-7-allyl-5-(2-methylpyrimidin-5-yl)-1*H*-indazol-1-yl)acetyl)-*N*-(6-bromo-4-fluoropyridin-2-yl)-5-(((*R*)-2-methylhex-5-enamido)methyl)-2-azabicyclo[3.1.0]hexane-3-carboxamide (9-4):** To a mixture of 2-(3-acetyl-7-allyl-5-(2-methylpyrimidin-5-yl)-1*H*-indazol-1-yl)acetic acid (24 mg, 0.068 mmol) and compound **9-3** (35 mg, 0.068 mmol) in DMF (3 mL) was added HATU (39 mg, 0.10 mmol) and DIPEA (26 mg, 0.20 mmol) at 0 °C and the mixture was stirred at 25 °C for 1 hour. The mixture was diluted with EtOAc, washed with saturated aqueous NH₄Cl solution and brine, dried and concentrated to dryness. The residue was purified by silica gel chromatography (eluted with PE: EtOAc= 20: 1 to 1: 2) to afford compound **9-4** (50 mg, yield 94.9%) as a white solid. LC/MS (ESI) m/z: 771/773 (M+H)⁺.

**Step 4: Compound 9-5:** To a solution of compound **9-4** (50 mg, 0.065 mmol) in degassed toluene (40 mL) was added Grubbs 2^{nd} catalyst (14 mg, 0.016 mmol) and the mixture was stirred at 80 °C under N₂ atmosphere for 16 hours. The mixture was concentrated to dryness and the residue was purified by silica gel chromatography (eluted with PE: EtOAc= 20: 1 to 1: 2) to afford compound **9-5** (35 mg, yield 72.6%) as a brown solid. LC/MS (ESI) m/z: 743/745 (M+H)⁺.

**Step 6: Compound 9:** To a mixture of compound 9-5 (35 mg, 0.047 mmol) in EtOAc (2 mL) was added PtO₂ (7 mg) and the mixture was degassed under N₂ atmosphere three times and stirred under a H₂ balloon at room temperature for 1 hour. The mixture was filtered and the filtrate was concentrated to dryness. The residue was purified by preparative HPLC to afford **Compound 9** (4 mg, yield 11.4%) as white solid. ¹H-NMR (400 MHz, CD₃OD) δ 8.97 (s, 2H), 8.40 (d, *J*= 1.5 Hz, 1H), 7.89 (dd, *J=* 10.9, 1.6 Hz, 1H), 7.54 (s, 1H), 7.09 (dd, *J=* 7.5, 2.0 Hz, 1H), 6.01 (d, *J=* 17.9 Hz, 1H), 5.61 (d, *J=* 17.9 Hz, 1H), 4.58 (m, 1H), 4.30 (t, *J=* 7.9 Hz, 1H), 3.74-3.68 (m, 1H), 3.59 (d, *J=* 14.5 Hz, 1H), 3.28-3.22 (m, 1H), 3.20-3.11 (m, 1H), 3.08-3.00 (m, 1H), 2.73 (s, 3H), 2.67 (s, 3H), 2.61-2.51 (m, 2H), 2.47-2.39 (m, 1H), 2.00-1.90 (m, 1H), 1.84-1.76 (m, 2H), 1.76-1.71 (m, 2H), 1.62-1.53 (m, 1H), 1.51-1.42 (m, 1H), 1.31-1.28 (m, 1H), 1.23-1.17 (m, 3H), 1.16-1.12 (m, 1H). LC/MS (ESI) m/z: 745/747 (M+H)⁺.

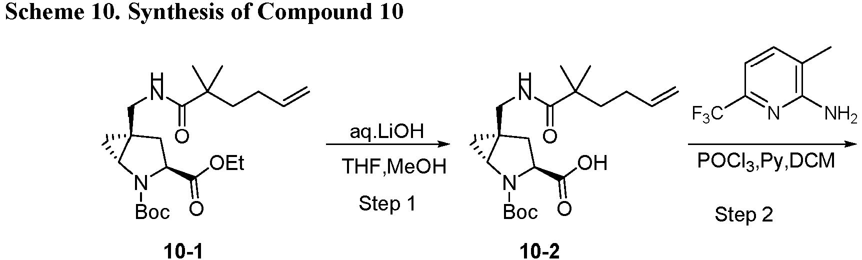

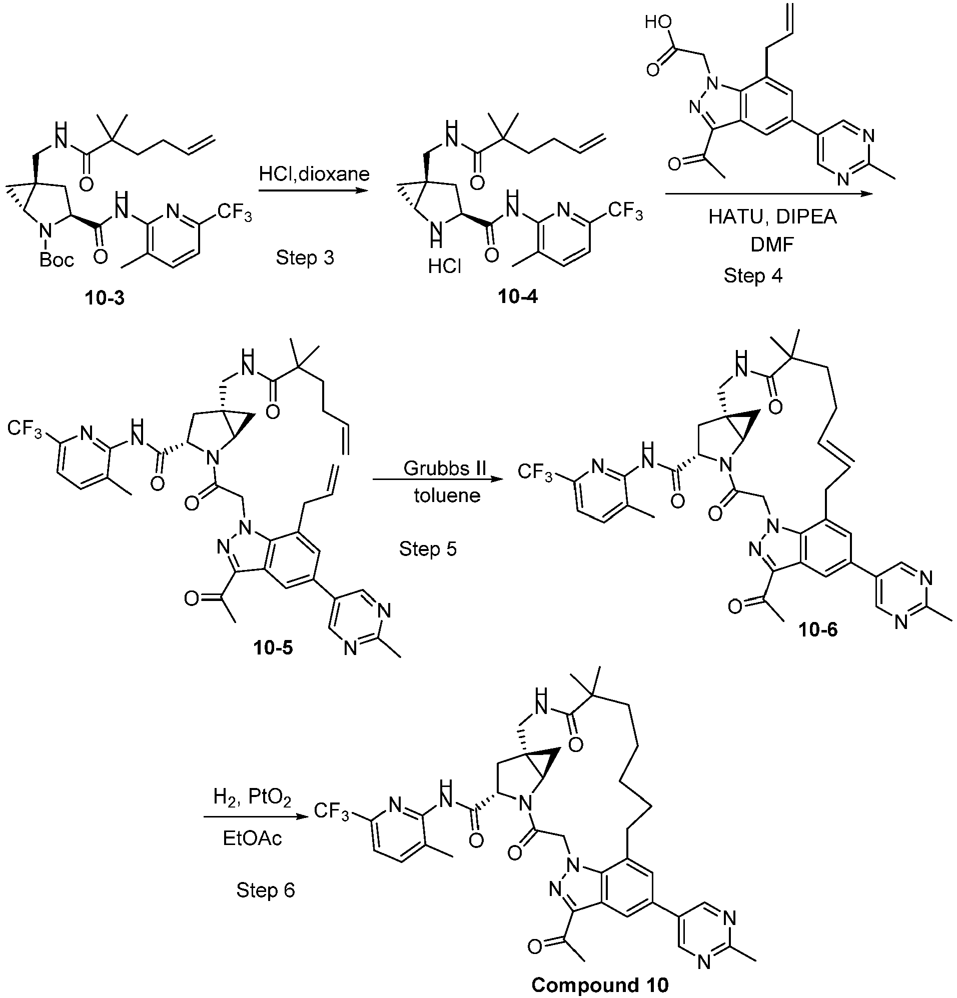

**Step 1: (1*R*,3*S*,5*R*)-2-(*tert*-Butoxycarbonyl)-5-((2,2-dimethylhex-5-enamido)methyl)-2-azabicyclo[3.1.0]hexane-3-carboxylic acid (10-2):** To a solution of compound **10-1** (0.13 g, 0.32 mmol) in THF (2 mL), methanol (2 mL), and water (2 mL) was added lithium hydroxide hydrate (67 mg, 1.59 mmol) and the mixture was stirred at 25 °C for 1 hour. The mixture was diluted with water and washed with diethyl ether twice. The aqueous layer was acidified with 1N aqueous HCl to pH of approximately 3 and extracted with DCM twice. The combined organic layers were washed with brine, dried over anhydrous Na₂SO₄, filtered and concentrated to dryness to afford compound **10-2** (0.12 mg, yield 99.1%) as a light yellow solid. LC/MS (ESI) m/z: 381 (M+H)⁺.

**Step 2: (1*R*,3*S*,5*R*)-*tert*-Butyl-5-((2,2-dimethylhex-5-enamido)methyl)-3-((3-methyl-6-(trifluoromethyl)pyridin-2-yl)carbamoyl)-2-azabicyclo[3.1.0]hexane-2-carboxylate (10-3):** To a mixture of (1*R*,3*S*,5*R*)-2-(*tert*-butoxycarbonyl)-5-[(2,2-dimethylhex-5-enamido)methyl]-2-azabicyclo[3.1.0]hexane-3-carboxylic acid (120 mg, 0.315 mmol) and 3-methyl-6-(trifluoromethyl)pyridin-2-amine (56 mg, 0.315 mmol) in DCM (8 mL) was added pyridine (125 mg, 1.577 mmol) followed by the dropwise addition of phosphoryl chloride (73 mg, 0.473 mmol) at 0 °C and the mixture was stirred at 25 °C for 12 hours. The mixture was diluted with DCM and washed with 1N aqueous HCl solution and brine, dried over Na₂SO₄, filtered and concentrated to afford crude product that was purified by silica gel chromatography (eluted with PE: EtOAc= 2: 1) to afford compound **10-3** (95 mg, yield 55.9%) as a yellow solid. LC/MS (ESI) m/z: 539 (M+H)⁺.

**Step 3: (1*R*,3*S*,5*R*)-5-((2,2-Dimethylhex-5-enamido)methyl)-*N*-(3-methyl-6-(trifluoromethyl)pyridin-2-yl)-2-azabicyclo[3.1.0]hexane-3-carboxamide hydrochloride (10-4):** A solution of tert-butyl (1R,3S,5R)-5-[(2,2-dimethylhex-5-enamido)methyl]-3-{[3-methyl-6-(trifluoromethyl)pyridin-2-yl]carbamoyl}-2-azabicyclo[3.1.0]hexane-2-carboxylate (95 mg, 0.176 mmol) in HCl/1,4-dioxane solution (2 mL) was stirred at room temperature for 2 hours. The mixture was concentrated to dryness and the residue was washed with diethyl ether, dried under vacuum to afford compound **10-4** (75 mg, yield 89.7%) as a yellow solid. LC/MS (ESI) m/z: 439 (M+H)⁺.

### Step 4: (1R,3S,5R)-2-(2-(3-Acetyl-7-allyl-5-(2-methylpyrimidin-5-yl)-1H-indazol-1-yl)acetyl)-5-((2,2-dimethylhex-5-enamido)methyl)-N-(3-methyl-6-(trifluoromethyl)pyridin-2-yl)-2-azabicyclo[3.1.0]hexane-3-carboxamide (10-5)

To a mixture of (1*R*,3*S*,5*R*)-5-[(2,2-dimethylhex-5-enamido)methyl]-*N*-[3-methyl-6-(trifluoromethyl)pyridin-2-yl]-2-azabicyclo[3.1.0]hexane-3-carboxamide hydrochloride (75 mg, 0.16 mmol) and [3-acetyl-5-(2-methylpyrimidin-5-yl)-7-(prop-2-en-1-yl)indazol-1-yl]acetic acid (56 mg, 0.16 mmol) in DMF (5 mL) was added DIPEA (0.11 g, 0.8 mmol) followed by HATU (109 mg, 0.29 mmol) at 0 °C and the mixture was stirred at room temperature for 2 hours. The mixture was diluted with EtOAc and washed with saturated aqueous NH₄Cl solution and brine, dried over Na₂SO₄, filtered and concentrated to afford crude product that was purified by silica gel chromatography (eluted with PE: EtOAc= 2: 1) to afford compound **10-5** (80 mg, yield 65.0%) as a yellow solid. LC/MS (ESI) m/z: 771 (M+H)⁺.

**Step 5: Compound 10-6:** To a solution of compound **10-5** (80 mg, 0.104 mmol) in degassed toluene (65 mL) was added Grubbs 2^{nd} catalyst (22 mg, 0.026 mmol) under N₂ atmosphere and the mixture was stirred under N₂ atmosphere at 80 °C for 16 hours. The mixture was concentrated to dryness and the residue was purified by silica gel chromatography (eluted with DCM: MeOH=30: 1) to afford compound **10-6** (55 mg, yield 71.347%) as a yellow solid. LC/MS (ESI) m/z: 743 (M+H)⁺.

**Step 6: Compound 10:** To a solution of compound **10-6** (50 mg, 0.067 mmol) in EtOAc (5 mL) and THF (3 mL) was added PtO₂ (15 mg, 0.067 mmol) and the mixture was degassed under N₂ atmosphere three times and stirred under a H₂ balloon at 25 °C for 1 hour. The mixture was filtered and the filtrate was concentrated to dryness. The residue was purified by preparative HPLC to afford **Compound 10** (5.1 mg, yield 10.2%) as a white solid. ¹H-NMR (400 MHz, CD₃OD) δ 9.01 (s, 2H), 8.44 (s, 1H), 7.58 (s, 1H), 7.38-7.32 (t, J= 16 Hz,1H), 7.25 (t, J= 12 Hz, 1H), 7.11 (t, J= 16 Hz, 1H), 5.91 (d, J= 20 Hz, 1H), 5.61 (d, J= 16 Hz, 1H), 5.17 (m, 1H), 4.27 (m, 1H), 3.55 (dd, J= 8 Hz, 1H), 3.38 (m, 2H), 3.18-3.09 (m, 1H), 2.93 (m, 1H), 2.75 (s, 3H), 2.69 (s, 3H), 2.43 (m, 1H), 2.27 (m, 3H), 1.77 (m, 8H), 1.34 (m, 4H), 1.15 (m, 1H). LC/MS (ESI) m/z: 745 (M+H)⁺.

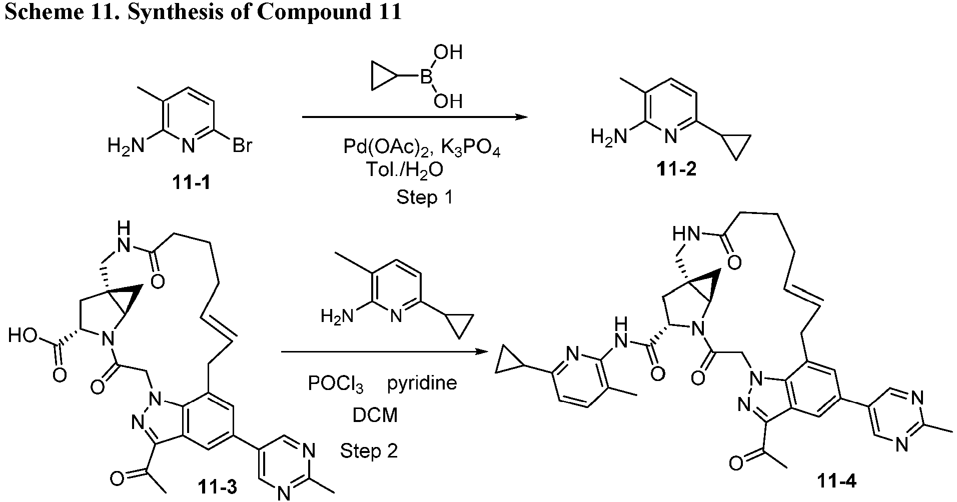

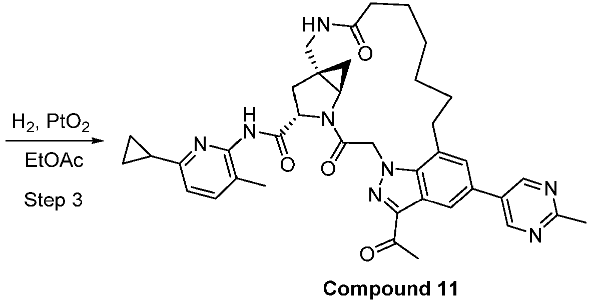

**Step 1: 6-Cyclopropyl-5-methylpyridin-2-amine (11-2):** To a solution of 6-bromo-5-methylpyridin-2-amine (0.15 g, 0.81 mmol) and cyclopropylboronic acid (69 mg, 0.81 mmol) in 1,4-dioxane (4 mL) and water (1 mL) was added K₃PO₄ (0.51 g, 2.41 mmol), tricyclohexylphosphine (22 mg, 0.08 mmol) and Pd(OAc)₂ (9 mg, 0.04 mmol) and the mixture was degassed under N₂ atmosphere three times and stirred under N₂ atmosphere at 100 °C for 24 hours. The mixture was diluted with EtOAc, washed with brine, dried over anhydrous Na₂SO₄, filtered and concentrated to dryness. The residue was purified by column chromatography on silica gel (eluted with DCM: MeOH= 100: 1) to afford compound **11-2** (42 mg, yield 35.3%) as a yellow solid. LC/MS (ESI) m/z: 149 (M+H)⁺.

**Step 2: Compound 11-4:** To a mixture of compound **11-3** (25 mg, 0.045 mmol) and 6-cyclopropyl-3-methylpyridin-2-amine (7 mg, 0.045 mmol) in DCM (3 mL) was added pyridine (28 mg, 0.35 mmol) and phosphorus oxychloride (14 mg, 0.09 mmol) at 0 °C and the mixture was stirred at room temperature for 30 minutes. The mixture was diluted with DCM and washed with 1N aqueous HCl and brine, dried over anhydrous Na₂SO₄, filtered and concentrated to dryness. The residue was purified by column chromatography on silica gel (eluted with DCM : MeOH=40: 1) to afford compound **11-4** (16 mg, yield 51.8%) as a light yellow solid. LC/MS (ESI) m/z: 687 (M+H)⁺.

**Step 3: Compound 11:** To a solution of compound **11-4** (16 mg, 0.023 mmol) in EtOAc (3 mL) was added PtO₂ (5 mg) and the mixture was degassed under N₂ atmosphere three times and stirred under a H₂ balloon at room temperature for 2 hours. The mixture was filtered and the filtrate was concentrated to dryness. The residue was purified by preparative HPLC to afford **Compound 11** (3 mg, yield 18.7%) as a white solid. ¹H-NMR (400 MHz, CD₃OD) δ 8.98 (s, 2H), 8.42 (d, *J=* 1.6 Hz, 1H), 7.51 (s, 1H), 7.43 (d, *J=* 7.8 Hz, 1H), 6.95 (d, *J=* 7.8 Hz, 1H), 5.92 (d, *J=* 17.8 Hz, 1H), 5.64 (d, *J=* 17.8 Hz, 1H), 4.49-4.58 (m, 4H), 3.63-3.60 (m, 1H), 3.51-3.46 (m, 1H), 3.35 (d, *J=* 14.4 Hz, 1H), 3.19-3.11 (m, 1H), 2.91-2.83 (m, 1H), 2.74 (s, 3H), 2.68 (s, 3H), 2.59 (d, *J=* 7.4 Hz, 2H), 2.39-2.26 (m, 2H), 2.02-1.98 (m, 4H), 1.88-1.50 (m, 8H), 1.46-1.38 (m, 2H), 1.18-1.15 (m, 1H), 0.94-0.86 (m, 4H). LC/MS (ESI) m/z: 689 (M+H)⁺.

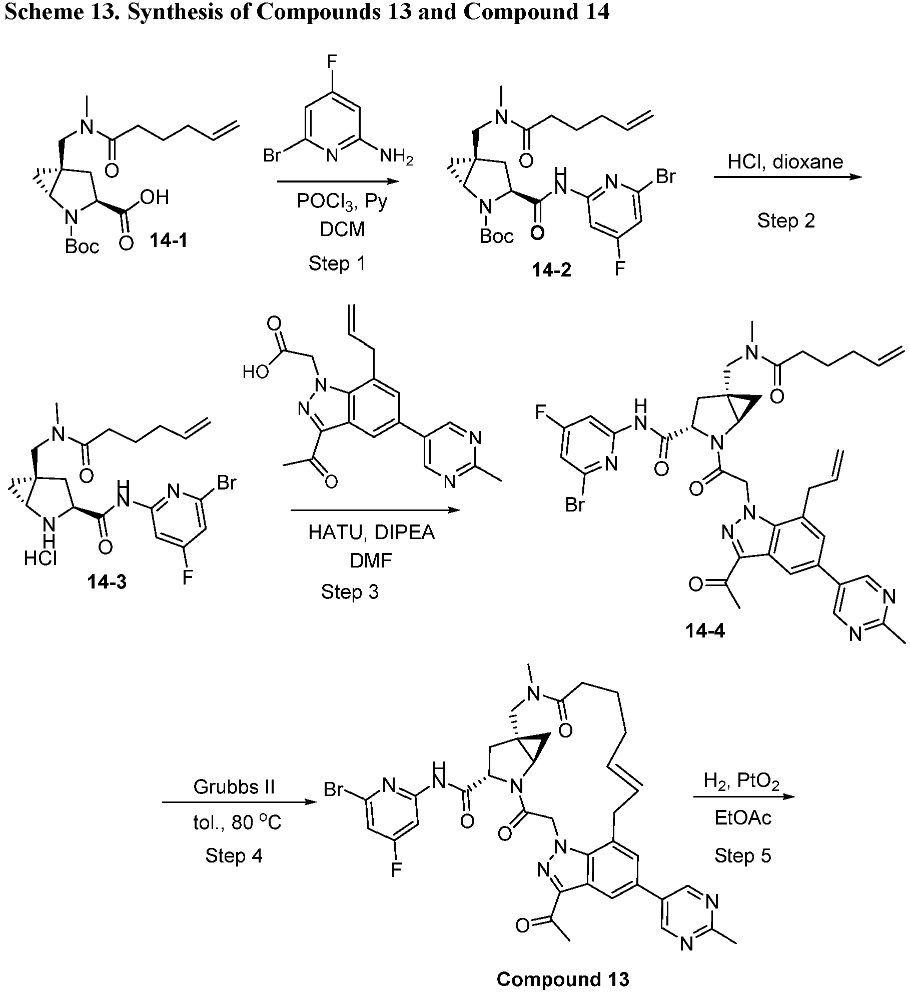

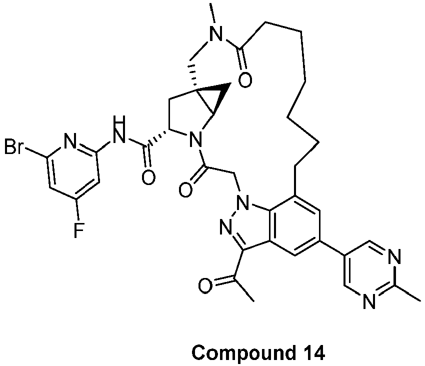

**Step 1: (1*R*,3*S*,5*R*)-*tert*-Butyl 3-((6-bromo-4-fluoropyridin-2-yl)carbamoyl)-5-((*N-*methylhex-5-enamido)methyl)-2-azabicyclo[3.1.0]hexane-2-carboxylate (14-2):** To a mixture of compound **14-1** (120 mg, 0.33 mmol) and 6-bromo-4-fluoropyridin-2-amine (63 mg, 0.33 mmol) in DCM (5 mL) was added pyridine (130 mg, 1.64 mmol) followed by the drop-wise addition of POCl₃ (55 mg, 0.36 mmol) at 0 °C and the mixture was stirred at room temperature under N₂ atmosphere for 1 hour. The mixture was poured into ice-water and extracted with DCM twice. The combined organic layers were washed with brine, dried with anhydrous Na₂SO₄, filtered and concentrated to dryness. The residue was purified by chromatography on silica gel (eluted with PE: EtOAc= 10: 1 to 2:1) to afford compound **14-2** (41 mg, yield 23.2%) as a yellow solid. LC/MS (ESI) m/z: 539/541 (M+H)⁺.

**Step 2: (1*R*,3*S*,5*R*)-*N*-(6-Bromo-4-fluoropyridin-2-yl)-5-((*N*-methylhex-5-enamido)methyl)-2-azabicyclo[3.1.0]hexane-3-carboxamide hydrochloride (14-3):** A solution of compound **14-2** (41 mg, 0.076 mmol) in HCl/1,4-dioxane solution (1 mL, 4M) was stirred at 0 °C to room temperature for 1 hour. The reaction mixture was concentrated to dryness, washed with diethyl ether and dried under vacuum to afford compound **14-3** (35 mg, yield 96.8%) as a light yellow solid that was used directly in the next step. LC/MS (ESI) m/z: 439/441 (M+H)⁺.

**Step 3: (1*R*,3*S*,5*R*)-2-(2-(3-Acetyl-7-allyl-5-(2-methylpyrimidin-5-yl)-1*H*-indazol-1-yl)acetyl)-N-(6-bromo-4-fluoropyridin-2-yl)-5-((N-methylhex-5-enamido)methyl)-2-azabicyclo[3.1.0]hexane-3-carboxamide (14-4):** To a mixture of compound **14-3** (35 mg, 0.075 mmol) and 2-(3-acetyl-7-allyl-5-(2-methylpyrimidin-5-yl)-1*H*-indazol-1-yl)acetic acid (26 mg, 0.075 mmol) in DMF (1 mL) was added DIPEA (49 mg, 0.38 mmol) followed by HATU (43 mg, 0.11 mmol) at 0 °C and the mixture was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by chromatography on silica gel (eluted with DCM: MeOH= 100: 1) to afford compound **14-4** (45 mg, yield 77.6%) as a white solid. LC/MS (ESI) m/z: 771/773(M+H)⁺.

**Step 4: Compound 13:** To a solution of compound **14-4** (45 mg, 0.058 mmol) in degassed toluene (45 mL) was added Grubbs 2^{nd} catalyst (12 mg, 0.015 mmol) under N₂ atmosphere and the mixture was stirred at 80 °C under N₂ atmosphere for 16 hours. The mixture was concentrated to dryness and the residue was purified by chromatography on silica gel (eluted with DCM: MeOH= 200: 1 to 100: 1) to afford **Compound 13** (41 mg, yield 94.5%) as a brown solid. ¹H-NMR (400 MHz, CD₃OD) δ 8.99 (s, 2H), 8.46 (d, *J=* 1.6 Hz, 1H), 7.98 (d, *J=* 10.8 Hz, 1H), 7.60-7.55 (m, 1H), 7.16 (dd, *J=* 7.6, 7.6 Hz, 1H), 6.34-6.38 (m, 1H), 6.04-6.08 (d, *J=* 17.6 Hz, 1H), 5.78-5.80 (m, 1H), 5.63-5.68 (d, *J=* 17.8 Hz, 1H), 5.56-5.60 (m, 1H), 4.65-4.67 (m, 1H), 4.20 (d, *J*= 15.6 Hz, 1H), 3.96-3.81 (m, 1H), 3.69 (d, *J=* 17.6 Hz, 1H), 3.51 (d, *J=* 15.6 Hz, 1H), 3.38 (d, *J* = 6.0 Hz, 1H), 3.11-2.98 (m, 1H), 2.93 (s, 3H), 2.74 (s, 3H), 2.67 (s, 3H), 2.59-2.62 (m, 1H), 2.35-2.18 (m, 4H), 1.96-1.90 (m, 1H), 1.83-1.76 (m, 1H), 1.16-1.9 (m, 1H), 1.06-1.11 (m, 1H). LC/MS (ESI) m/z: 743/745 (M+H)⁺.

**Step 5: Compound 14:** To a solution of **Compound 13** (41 mg, 0.055 mmol) in EtOAc (4 mL) was added PtO₂ (10 mg) at 0 °C and the mixture was degassed under N₂ atmosphere three times and stirred under a H₂ balloon at room temperature for 20 minutes. The mixture was filtered and the filtrate was concentrated to dryness. The residue was purified by preparative HPLC to afford **Compound 14** (4.6 mg, yield 11.2%) as white solid. ¹H-NMR (400 MHz, CD₃OD) δ 8.98 (d, *J=* 2.0 Hz, 2H), 8.41 (dd, *J=* 8.4, 1.7 Hz, 1H), 7.92 (d, *J=* 10.6 Hz, 1H), 7.58-7.51 (m, 1H), 7.11-7.16 (m, 1H), 5.97-6.01 (d, *J=* 17.9 Hz, 1H), 5.77-5.61 (m, 1H), 4.45-4.48 (m, 1H), 3.91 (d, *J* = 2.5 Hz, 1H), 3.59-3.46 (m, 1H), 3.24 (s, 3H), 3.20-3.08 (m, 1H), 2.92-2.94 (m, 1H), 2.73 (s, 3H), 2.67 (s, 3H), 2.62-2.50 (m, 2H), 2.35-2.28 (m, 1H), 2.07-1.49 (m, 10H), 1.35-1.38 (m, 1H), 1.06 (dd, *J=* 5.8, 2.8 Hz, 1H). LC/MS (ESI) m/z: 745/747 (M+H)⁺.

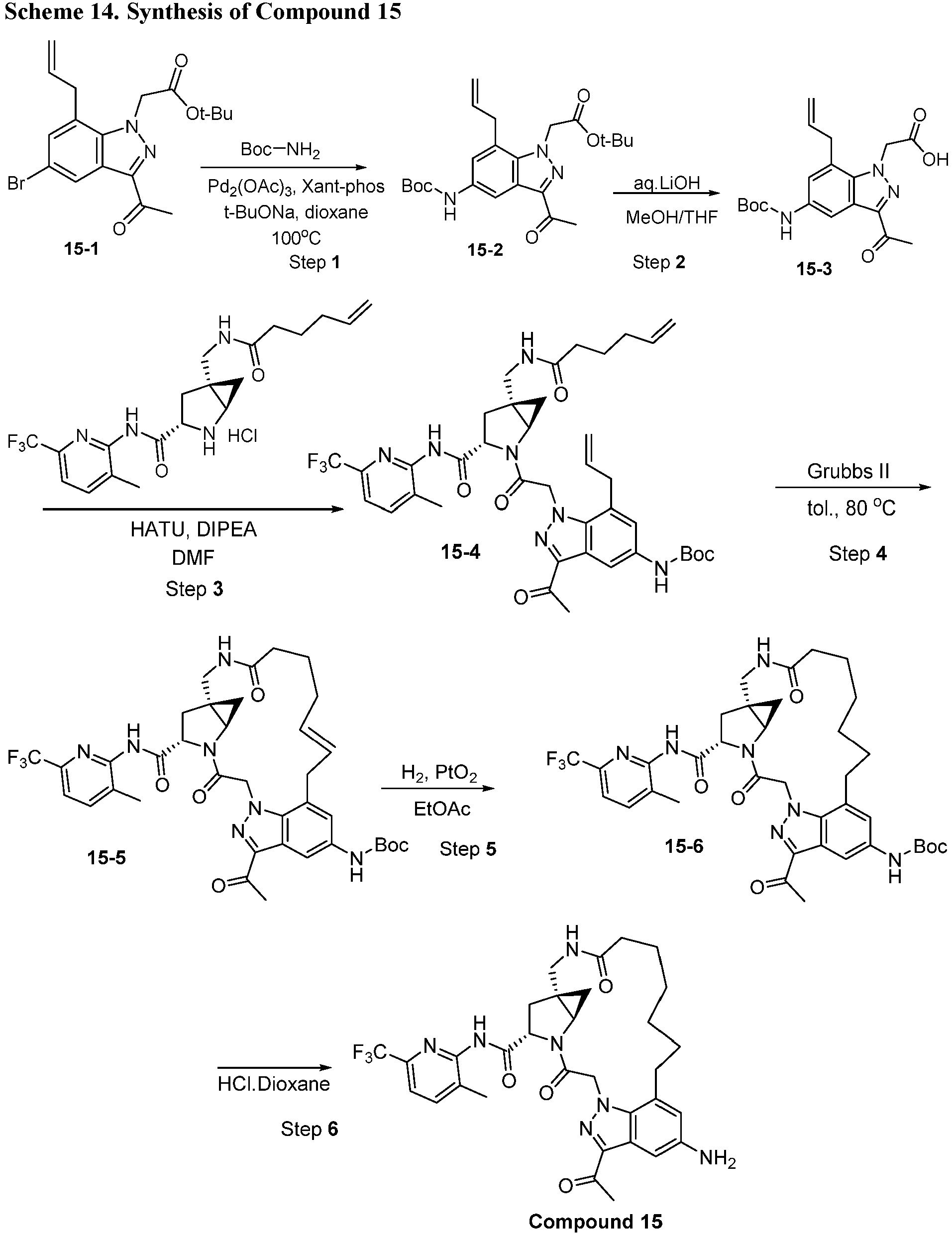

**Step 1: *tert*-Butyl 2-(3-acetyl-7-allyl-5-((tert-butoxycarbonyl)amino)-1*H*-indazol-1-yl)acetate (15-2):** To a mixture of compound **15-1** (250 mg, 0.64 mmol) and tert-butyl carbamate (223 mg, 1.91 mmol) in 1,4-dioxane were added sodium tert-butoxide (122 mg, 1.27 mmol), Xant-phos (147 mg, 0.25 mmol) and palladium(II) acetate (29 mg, 0.13 mmol) at 0 °C and the mixture was stirred at 100 °C under N₂ atmosphere for 4 hours. The mixture was diluted with EtOAc, washed with water and brine, dried over anhydrous Na₂SO₄, filtered and concentrated to dryness. The residue was purified by chromatography on silica gel (eluted with PE: EtOAc= 20: 1) to afford compound **15-2** (115 mg, yield 42.1%) as a white solid. LC/MS (ESI) m/z: 430 (M+H)⁺.

**Step 2: 2-(3-Acetyl-7-allyl-5-((*tert*-butoxycarbonyl)amino)-1*H*-indazol-1-yl)acetic acid (15-3):** To a solution of compound **15-2** (50 mg, 0.12 mmol) in methanol (2 mL) and THF (1 mL) was added a solution of LiOH (15 mg, 0.35 mmol) in water (1 mL) at 0 °C and the mixture was stirred at room temperature for 16 hours. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound **15-3** (40 mg, yield 92.0%) as a white solid. LC/MS (ESI) m/z: 374 (M+H)⁺.

**Step 3: *tert*-Butyl (3-acetyl-7-allyl-1-(2-((1*R*,3*S*,5*R*)-5-(hex-5-enamidomethyl)-3-((3-methyl-6-(trifluoromethyl)pyridin-2-yl)carbamoyl)-2-azabicyclo[3.1.0]hexan-2-yl)-2-oxoethyl)-1*H*-indazol-5-yl)carbamate (15-4):** To a mixture of compound **15-3** (40 mg, 0.11 mmol) and (1*R*,3*S*,5*R*)-5-(hex-5-enamidomethyl)-N-(3-methyl-6-(trifluoromethyl)pyridin-2-yl)-2-azabicyclo[3.1.0]hexane-3-carboxamide hydrochloride (49 mg, 0.11 mmol) in DMF (3 mL) was added DIPEA (69 mg, 0.54 mmol) followed by HATU (61 mg, 0.16 mmol) at 0 °C and the mixture was stirred at room temperature for 1 hour. The mixture was diluted with EtOAc, washed with saturated aqueous NH₄Cl solution and brine, dried over anhydrous Na₂SO₄, filtered and concentrated to dryness. The residue was purified by chromatography on silica gel (eluted with DCM: MeOH= 80: 1) to afford compound **15-4** (50 mg, yield 60.9%) as a yellow solid. LC/MS (ESI) m/z: 766 (M+H)⁺.

Step **4: Compound 15-5:** To a solution of compound **15-4** (50 mg, 0.065 mmol) in degassed toluene (50 mL) was added Grubbs 2^{nd} catalyst (14 mg, 0.016 mmol) under N₂ atmosphere and the mixture was stirred at 80 °C under N₂ atmosphere for 16 hours. The mixture was concentrated to dryness and the residue was purified by chromatography on silica gel (eluted with DCM: MeOH= 200: 1 to 80: 1) to afford compound **15-5** (38 mg, yield 78.9%) as a brown solid. LC/MS (ESI) m/z: 738 (M+H)⁺.

**Step 5: Compound 15-6:** To a solution of compound **15-5** (38 mg, 0.052 mmol) in EtOAc (8 mL) was added PtO₂ (10 mg) at 0 °C and the mixture was degassed under N₂ atmosphere three times and stirred under a H₂ balloon at room temperature for 15 minutes. The mixture was filtered and the filtrate was concentrated to dryness to afford compound **15-6** (38 mg, yield 99.7%) as a brown solid that was used directly in the next step. LC/MS (ESI) m/z: 740 (M+H)⁺.

**Step 6: Compound 15:** A solution of compound **15-6** (38 mg, 0.051 mmol) in HCl/1,4-dioxane solution (1 mL, 4M) was stirred at 0 °C to room temperature for 1 hour. The reaction mixture was concentrated to dryness and the residue was purified by preparative HPLC to afford **Compound 6** (2 mg, yield 6.1%) as a yellow solid. ¹H-NMR (400 MHz, DMSO-*d*₆) δ 10.46 (s, 1H), 8.16 (dd, *J=* 8.4, 8.4 Hz, 1H), 7.90 (d, *J=* 8.0 Hz, 1H), 7.65 (d, *J=* 7.6 Hz, 1H), 7.14 (d, *J=* 2.0 Hz, 1H), 6.59 (d, *J=* 2.0 Hz, 1H), 5.74 (d, *J=* 17.6 Hz, 1H), 5.31 (d, *J=* 17.6 Hz, 1H), 5.07 (s, 2H), 4.32 (t, *J=* 7.6 Hz, 1H), 3.55-3.50 (m, 1H), 3.30-3.26 (m, 2H), 3.16 (dd, *J=* 14.4, 14.4 Hz, 1H), 2.98-2.90 (m, 1H), 2.46-2.41 (m, 2H), 2.18-2.06 (m, 5H), 1.65-1.32 (m, 8H), 1.17 (t, *J*= 5.6 Hz, 1H), 1.06 (dd, *J=* 5.2, 5.2 Hz, 1H). LC/MS (ESI) m/z: 640 (M+H)⁺.

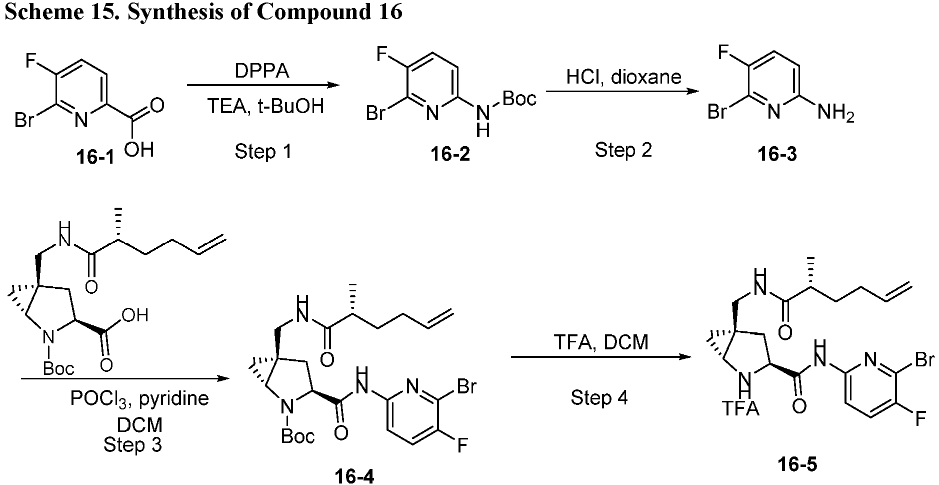

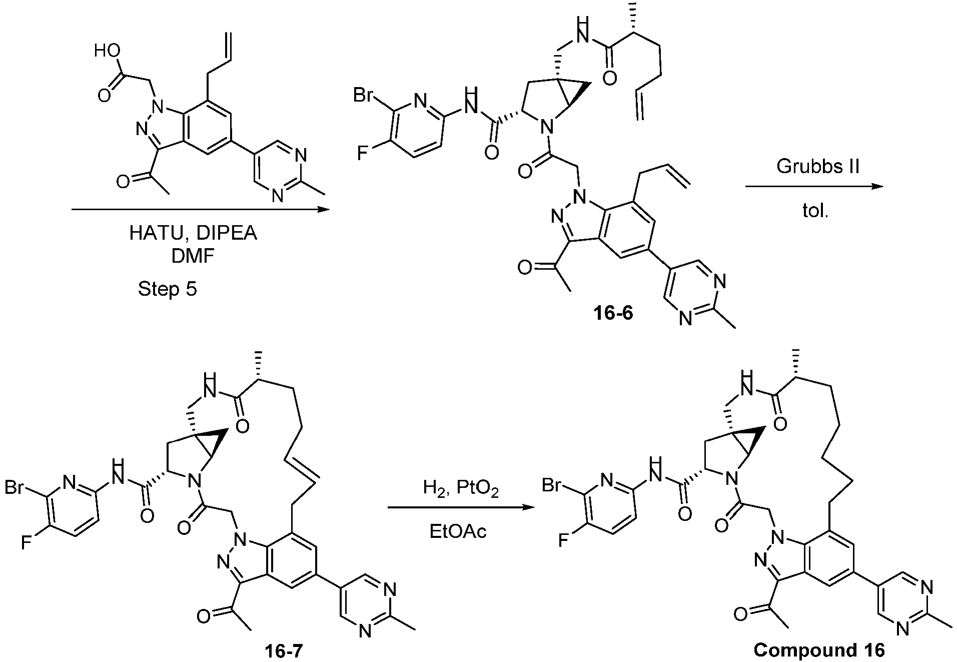

**Step 1: *tert*-Butyl (6-bromo-5-fluoropyridin-2-yl)carbamate (16-2):** To a solution of compound **16-1** (0.50 g, 2.27 mmol) in t-BuOH (10 mL) was added TEA (0.23 g, 2.27 mmol) and DPPA (0.63 g, 2.27 mmol) and the reaction was stirred at 85 °C under N₂ atmosphere for 2 hours. The mixture was diluted with EtOAc and washed with water and brine, dried with anhydrous Na₂SO₄, filtered and concentrated to dryness. The residue was purified by column chromatography on silica gel (eluted with PE: EtOAc= 50: 1) to afford compound **16-2** (0.24 g, yield 36.71 %) as a yellow oil. LC/MS (ESI) m/z: 291 (M+H)⁺.

**Step 2: 6-Bromo-5-fluoropyridin-2-amine (16-3):** A solution of compound **16-2** (0.24 g, 0.83 mmol) in HCl/1,4-dioxane solution (3 mL, 4M) was stirred at room temperature for 1 hour. The mixture was concentrated to dryness and the residue was neutralized with saturated aqueous NaHCO₃ solution and extracted with EtOAc. The mixture was washed with brine, dried over anhydrous Na₂SO₄, filtered and concentrated to dryness to afford compound **16-3** (150 mg, yield 94.6%) as a yellow solid. LC/MS (ESI) m/z: 191 (M+H)⁺.

**Step 3: (1*R*,3*S*,5*R*)-*tert*-Butyl 3-((6-bromo-5-fluoropyridin-2-yl)carbamoyl)-5-(((R)-2-methylhex-5-enamido)methyl)-2-azabicyclo[3.1.0]hexane-2-carboxylate (16-4):** To a mixture of compound **16-3** (30 mg, 0.16 mmol) and (1R,3S,5R)-2-(tert-butoxycarbonyl)-5-(((R)-2-methylhex-5-enamido)methyl)-2-azabicyclo[3.1.0]hexane-3-carboxylic acid (55 mg, 0.16 mmol) in DCM (2 mL) was added pyridine (62 mg, 0.78 mmol) followed by phosphoryl chloride (27 mg, 0.17 mmol) at 0 °C under N₂ atmosphere. Then the reaction mixture was stirred at room temperature for 1 hour. The mixture was poured into ice water and extracted with DCM twice. The combined organic layers were washed with brine, dried with Na₂SO₄ and concentrated. The residue was purified by chromatography on silica gel (eluted with DCM: MeOH = 70: 1) to afford compound **16-4** (60 mg, yield 70.63%) as a white solid. LC/MS (ESI) m/z: 539 (M+H)⁺.

**Step 4: (1*R*,3*S*,5*R*)-*N*-(6-Bromo-5-fluoropyridin-2-yl)-5-(((*R*)-2-methylhex-5-enamido)methyl)-2-azabicyclo[3.1.0]hexane-3-carboxamide (16-5):** To a solution of compound **16-4** (60 mg, 0.11 mmol) in DCM (1 mL) was added TFA (1 mL) at 0 °C and the reaction was stirred at room temperature for 1 hour. The mixture was concentrated to dryness to afford compound **16-5** (58 mg, yield 100%) as a yellow solid that was directly used in the next reaction without purification. LC/MS (ESI) m/z: 439(M+H)⁺.

**Step 5: (1*R*,3*s*,5*R*)-2-(2-(3-Acetyl-7-allyl-5-(2-methylpyrimidin-5-yl)-1*H*-indazol-1-yl)acetyl)-N-(6-bromo-5-fluoropyridin-2-yl)-5-(((*R*)-2-methylhex-5-enamido)methyl)-2-azabicyclo[3.1.0]hexane-3-carboxamide (16-6):** To a mixture of compound 5 (58 mg, 0.11 mmol) and 2-(3-acetyl-7-allyl-5-(2-methylpyrimidin-5-yl)-1H-indazol-1-yl)acetic acid (39 mg, 0.11 mmol) in DMF (2 mL) was added DIPEA (71 mg, 0.55 mmol) followed by HATU (84 mg, 0.22 mmol) at 0 °C and the reaction was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by column chromatography on silica gel (eluted with DCM: MeOH= 40: 1) to afford compound **16-6** (76 mg, yield 90.1%) as a yellow solid. LC/MS (ESI) m/z: 771 (M+H)⁺.

**Step 6: Compound 7:** To a solution of compound **16-6** (76 mg, 0.10 mmol) in degassed toluene (60 mL) was added Grubbs 2^{nd} catalyst (21 mg, 0.03 mmol) at 0 °C under N₂ atmosphere and the mixture was stirred at 80 °C under N₂ atmosphere overnight. The mixture was concentrated to dryness and the residue was purified by chromatography on silica gel (eluted with DCM: MeOH= 50: 1) to afford compound **16-7** (46 mg, yield 62.8%) as a brown solid. LC/MS (ESI) m/z: 743 (M+H)⁺.

**Step 7: Compound 16:** To a solution of compound **16-7** (46 mg, 0.06 mmol) in EtOAc (4 mL) was added PtO₂ (12 mg) at 0 °C and the mixture was degassed under N₂ atmosphere three times and stirred under a H₂ balloon at room temperature for 1.5 hours. The mixture was filtered and the filtrate was concentrated to dryness. The residue was purified by preparative HPLC to afford **Compound 16** (3.8 mg, yield 8.50%) as a white solid. ¹H-NMR (400 MHz, DMSO-*d₆*) δ 10.85 (d, 1H), 9.02 (s, 2H), 8.31 (d, J= 5.4 Hz, 1H), 8.06-7.96 (m, 2H), 7.86 (t, 1H), 7.59 (s, 1H), 5.95 (d, J= 17.9 Hz, 1H), 5.54 (d, J= 17.7 Hz, 1H), 4.24 (t, J= 8.3 Hz, 1H), 3.63 (m, 1H), 3.51 (dd, J= 14.2, 5.5 Hz, 1H), 3.21-2.96 (m, 4H), 2.68 (s, 3H), 2.63 (s, 3H), 2.40 (d, J= 8.3 Hz, 2H), 1.87-1.61 (m, 6H), 1.45 (m, 2H), 1.16 (m, 1H), 1.09 (d, J= 6.9 Hz, 3H), 1.06-1.04 (m, 1H). LC/MS (ESI) m/z: 745/747 (M+H)⁺.

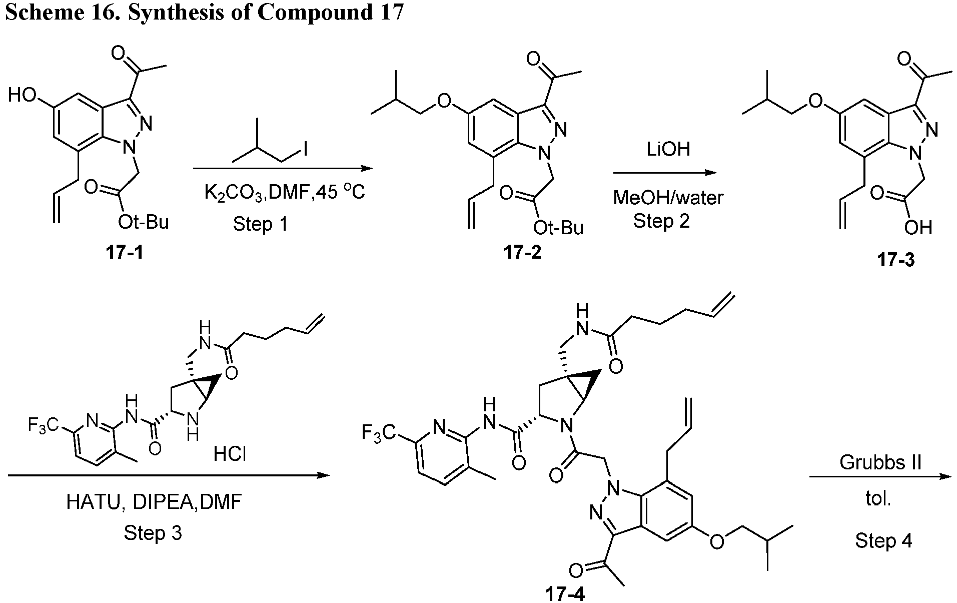

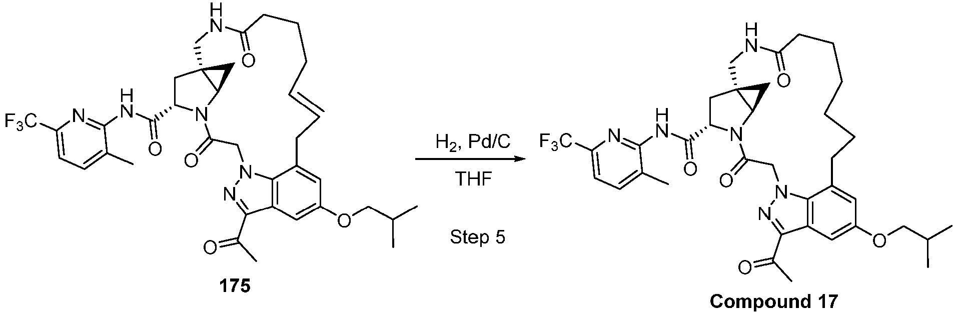

**Step 1: *tert*-Butyl 2-(3-acetyl-7-allyl-5-isobutoxy-1*H*-indazol-1-yl)acetate (17-2):** To a mixture of compound **17-1** (50mg, 0.15 mmol) and 1-iodo-2-methylpropane (28 mg, 0.15 mmol) in DMF (2 mL) was added K₂CO₃ (52 mg, 0.38 mmol) and the mixture was stirred at 75 °C for 12 hours. The mixture was diluted with EtOAc, washed with water and brine, dried over anhydrous Na₂SO₄, filtered and concentrated to dryness. The residue was purified by column chromatography via silica gel (eluted with PE: EtOAc= 100: 1 to 10: 1) to afford compound **17-2** (40 mg, yield 68.4%) as a white solid. LC/MS (ESI) m/z: 387 (M+H)⁺.

**Step 2: 2-(3-Acetyl-7-allyl-5-isobutoxy-1*H*-indazol-1-yl)acetic acid (17-3):** To a solution of compound **17-2** (40 mg, 0.10 mmol) in MeOH (3 mL) was added a solution of lithiumol (6 mg, 0.25 mmol) in water (0.5 mL) and the mixture was stirred at 25 °C for 4 hours. The mixture was diluted with water and washed with diethyl ether twice. The aqueous layer was acidified with 1N aqueous HCl and extracted with DCM twice. The combined organic layers were washed with brine, dried over anhydrous Na₂SO₄, filtered and concentrated to dryness to afford compound **17-3** (32 mg, yield 93.6%) as a white solid. LC/MS (ESI) m/z: 331 (M+H)⁺.

**Step 3: (1*R*,3*S*,5*R*)-2-(2-(3-Acetyl-7-allyl-5-isobutoxy-1*H*-indazol-1-yl)acetyl)-5-(hex-5-enamidomethyl)-*N*-(3-methyl-6-(trifluoromethyl)pyridin-2-yl)-2-azabicyclo[3.1.0]hexane-3-carboxamide (17-4):** To a mixture of compound **17-3** (32 mg, 0.097 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (43 mg, 0.097 mmol) in DMF (2 mL) was added DIPEA (38 mg, 0.29 mmol) followed by added HATU (74 mg, 0.19 mmol) and the mixture was stirred under N₂ atmosphere at 25 °C for 12 hours. The mixture was diluted with EtOAc, washed with water, dried over anhydrous Na₂SO₄, filtered and concentrated to dryness. The residue was purified by column chromatography via silica gel (eluted with PE: EtOAc= 20: 1 to 1: 3) to afford compound **17-4** (52 mg, yield 74.3%) as a yellow oil. LC/MS (ESI) m/z: 723 (M+H)⁺.

**Step 4: Compound 17-5:** To a solution of compound **17-4** (52 mg, 0.072 mmol) in toluene (42 mL) was added Grubbs 2^{nd} catalyst (13 mg, 0.016 mmol) and the mixture was degassed under N₂ atmosphere three times. The mixture was stirred under N₂ atmosphere at 80 °C for 16 hours. The mixture was concentrated to dryness and the residue was purified by column chromatography via silica gel (eluted with DCM: MeOH= 200: 1 to 50: 1) to afford compound **17-5** (46 mg, yield 92.0%) as a yellow solid. LC/MS (ESI) m/z: 695 (M+H)⁺.

**Step 5: Compound 17:** To a solution of compound **17-5** (46 mg, 0.066 mmol) in EtOAc (10 mL) was added PtO₂ (9 mg, 0.04 mmol), the mixture was stirred at 25 °C under a H₂ balloon for 2 hours. The mixture was filtered and the filtrate was concentrated to dryness. The residue was purified by preparative HPLC to afford **Compound 17** (1.2 mg, yield 2.6%) as a white solid. ¹H-NMR (400 MHz, DMSO-d₆) δ 10.47 (s, 1H), 8.19 (dd, *J=* 4.7,3.9 Hz, 1H), 7.90 (d, *J*= 7.8 Hz, 1H), 7.66 (d, *J=* 7.8 Hz, 1H), 7.41 (d, *J=* 2.2 Hz, 1H), 6.83 (d, *J=* 2.1 Hz, 1H), 5.84 (d, *J=* 17.9 Hz, 1H), 5.40 (d, *J=* 17.8 Hz, 1H), 4.32 (t, *J=* 7.8 Hz, 1H), 3.77 (d, *J=* 6.4 Hz, 2H), 3.56 (dd, *J=* 5.6,2.4 Hz, 1H), 3.28-3.25 (m, 1H), 3.19-3.15 (m, 1H), 3.06-2.99 (m, 1H), 2.78-2.69 (m, 1H), 2.58 (s, 3H), 2.47-2.39 (m, 2H), 2.24-2.12 (m, 2H), 2.09 (s, 3H), 2.06-2.00 (m, 1H), 1.72-1.64 (m, 3H), 1.63-1.60 (m, 1H), 1.60-1.54 (m, 1H), 1.51-1.41 (m, 2H), 1.40-1.33 (m, 1H), 1.24 (s, 1H), 1.18 (t, *J*= 5.5 Hz, 1H), 1.09-1.05 (m, 1H), 1.02 (s, 3H), 1.00 (s, 3H). LC/MS (ESI) m/z: 697 (M+H)⁺.

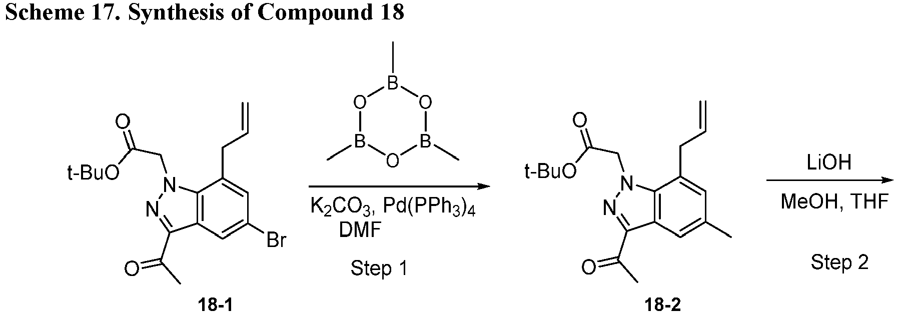

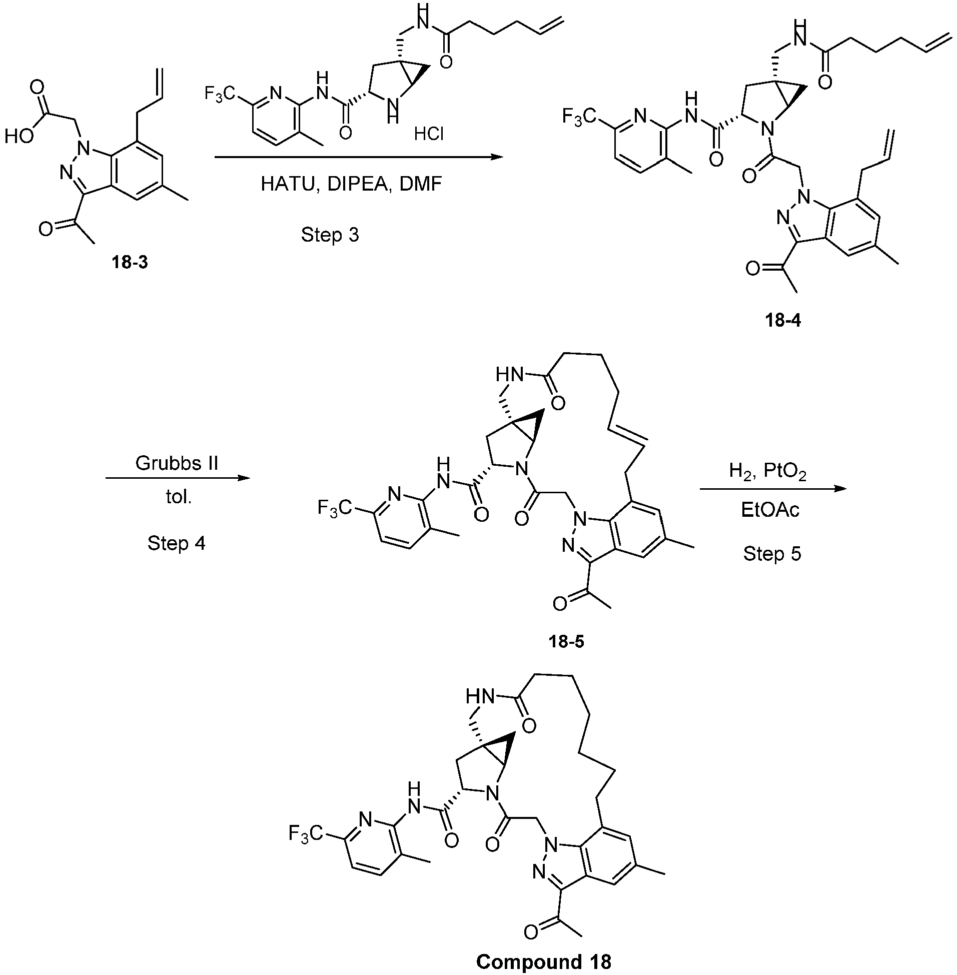

**Step 1: *tert*-Butyl 2-(3-acetyl-7-allyl-5-methyl-1*H*-indazol-1-yl)acetate (18-2):** To a mixture of compound **18-1** (50 mg, 0.13 mmol) and trimethyl-1,3,5,2,4,6-trioxatriborinane (16 mg, 0.13 mmol) in DMF (2mL) was added K₂CO₃ (35 mg, 0.26 mmol) and Pd(PPh₃)₄ (24 mg, 0.02 mmol) and the mixture was stirred at 100 °C under N₂ atmosphere for 16 hours. The mixture was diluted with EtOAc, washed with water and brine, dried over anhydrous Na₂SO₄, filtered and concentrated to dryness. The residue was purified by column chromatography via silica gel (eluted with PE: EtOAc= 50: 1 to 5: 1) to afford compound **18-2** (35 mg, yield 82%) as a white solid. LC/MS (ESI) m/z: 329 (M+H)⁺.

**Step 2: 2-(3-Acetyl-7-allyl-5-methyl-1*H*-indazol-1-yl)acetic acid (18-3):** To a solution of compound **18-2** (35 mg, 0.11 mmol) in MeOH (2 mL) was added lithiumol (6 mg, 0.26 mmol) in water (0.5 mL) and the mixture was stirred at 25 °C for 16 hours. The mixture was acidified with 1N aqueous HCl to a pH of approximately 3 and extracted with DCM twice. The combined organic layers were washed with brine, dried over anhydrous Na₂SO₄, filtered and concentrated to dryness to afford compound **18-3** (30 mg, yield 98.6%) as a yellow solid. LC/MS (ESI) m/z: 273 (M+H)⁺.

**Step 3: (1*R*,3*S*,5*R*)-2-(2-(3-Acetyl-7-allyl-5-methyl-1*H*-indazol-1-yl)acetyl)-5-(hex-5-enamidomethyl)*-*N*-*(3-methyl-6-(trifluoromethyl)pyridin-2-yl)-2-azabicyclo[3.1.0]hexane-3-carboxamide (18-4):** To a mixture of compound **18-3** (30 mg, 0.11 mmol) and (1R,3S,5R)-5-(hex-5-enamidomethyl)-N-[3-methyl-6-(trifluoromethyl)pyridin-2-yl]-2-azabicyclo[3.1.0]hexane-3-carboxamide hydrochloride (49 mg, 0.11 mmol) in DMF (2 mL) was added DIPEA (43 mg, 0.33 mmol), followed by added HATU (84 mg, 0.22 mmol) and the mixture was stirred at 25 °C for 2 hours. The mixture was diluted with EtOAc, washed with water and brine, dried over anhydrous Na₂SO₄, filtered and concentrated to dryness. The residue was purified by column chromatography via silica gel (eluted with DCM: MeOH= 200: 1 to 75: 1) to afford compound **18-4** (40 mg, yield 54.6%) as a yellow oil. LC/MS (ESI) m/z: 665 (M+H)⁺.

**Step 4: Compound 18-5:** To a solution of compound **18-4** (40 mg, 0.06 mmol) in degassed toluene (32 mL) was added Grubbs 2^{nd} catalyst (11 mg, 0.013 mmol) under N₂ atmosphere and the mixture was stirred at 80 °C under N₂ atmosphere for 12 hours. The mixture was concentrated to dryness and the residue was purified by column chromatography via silica gel (eluted with DCM: MeOH= 200: 1 to 70: 1) to afford compound **18-5** (24 mg, yield 62.6%) as a yellow solid. LC/MS (ESI) m/z: 637 (M+H)⁺.

**Step 5: Compound 18:** To a solution of compound **18-5** (24 mg, 0.038 mmol) in EtOAc (5 mL) was added PtO₂ (5 mg, 0.023 mmol) and the mixture was stirred at 25 °C under a H₂ balloon for 2 hours. The mixture was filtered and the filtrate was concentrated to dryness. The residue was purified by preparative HPLC to afford **Compound 18** (3.4 mg, yield 14.1%) as a white solid. ¹H-NMR (400 MHz, DMSO-*d₆*) δ 10.46 (s, 1H), 8.19 (q, *J=* 4.0 Hz, 1H), 7.90 (d, J= 7.9 Hz, 1H), 7.83 (s, 1H), 7.66 (d, *J=* 7.8 Hz, 1H), 7.03 (s, 1H), 5.83 (d, *J=* 17.7 Hz, 1H), 5.42 (d, *J=* 17.9 Hz, 1H), 4.33 (t, *J=* 7.8 Hz, 1H), 3.56 (dd, *J=* 5.2, 2.0 Hz, 1H), 3.29-3.26 (m, 1H), 3.20-3.15 (m, 1H), 3.07-2.99 (m, 1H), 2.77-2.68 (m, 1H), 2.58 (s, 3H), 2.47-2.43 (m, 1H), 2.39 (s, 3H), 2.21-2.12 (m, 2H), 2.09 (s, 3H), 1.72-1.64 (m, 3H), 1.63-1.54 (m, 2H), 1.52-1.43 (m, 2H), 1.42-1.35 (m, 1H), 1.24 (m, 1H), 1.19 (m, 1H), 1.10-1.06 (m, 1H). LC/MS (ESI) m/z: 639 (M+H)⁺.

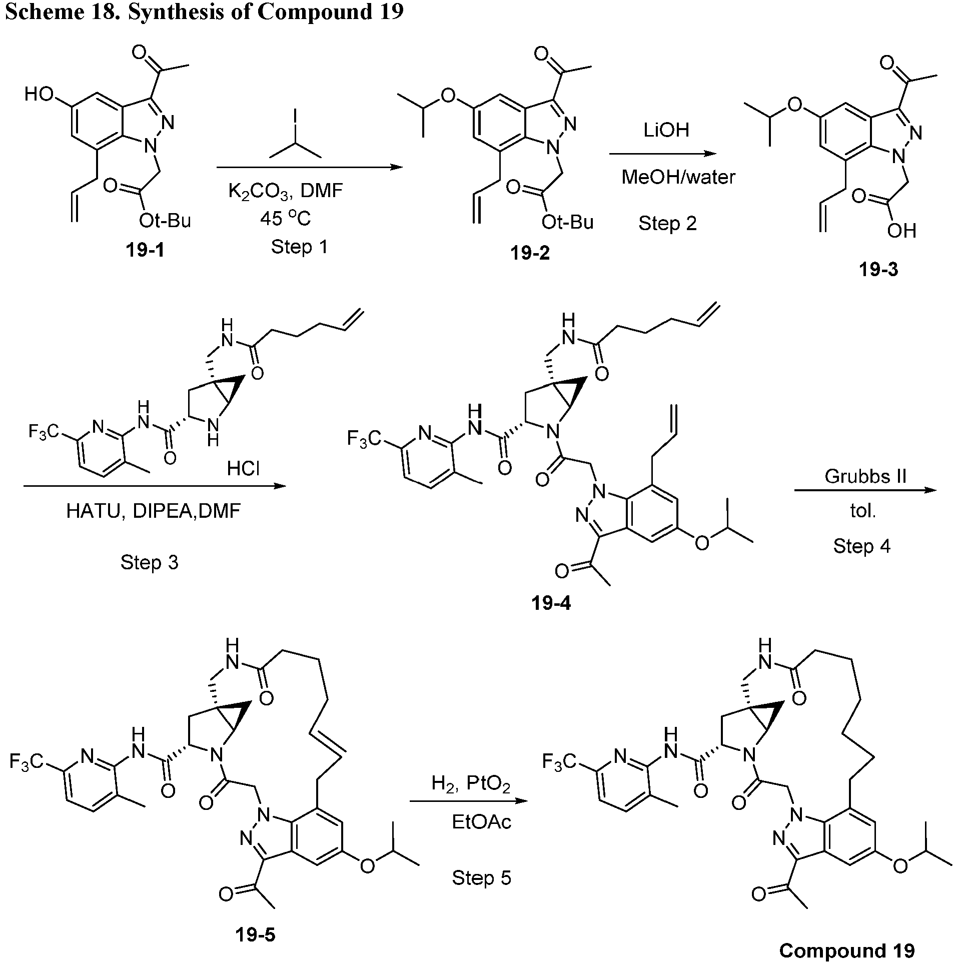

**Step 1: *tert*-Butyl 2-(3-acetyl-7-allyl-5-methyl-1*H*-indazol-1-yl)acetate** (19-2): To a mixture of compound 19-1 (50 mg, 0.15 mmol) and 2-iodopropane (26 mg, 0.15 mmol) in DMF (2 mL) was added K₂CO₃ (52 mg, 0.38 mmol) and the mixture was stirred at 75 °C for 12 hours. The mixture was diluted with EtOAc, washed with water, dried over anhydrous Na₂SO₄, filtered and concentrated to dryness. The residue was purified by column chromatography via silica gel (eluted with PE: EtOAc= 100: 1 to 10: 1) to afford compound **19-2** (45 mg, yield 79.8%) as a white solid. LC/MS (ESI) m/z: 373 (M+H)⁺.

**Step 2: 2-(3-Acetyl-7-allyl-5-methyl-1*H*-indazol-1-yl)acetic acid (19-3):** To a solution of compound **19-2** (43 mg, 0.12 mmol) in MeOH (2 mL) was added a solution of lithiumol (7 mg, 0.29 mmol) in water (0.5 mL) and the mixture was stirred at 25 °C for 4 hours. The mixture was diluted with water and washed with diethyl ether twice. The aqueous layer was acidified with 1N aqueous HCl and extracted with DCM twice. The combined organic layers were washed with brine, dried over anhydrous Na₂SO₄, filtered and concentrated to dryness to afford compound **19-3** (37 mg, yield 98.5%) as a yellow solid. LC/MS (ESI) m/z: 317 (M+H)⁺.

**Step 3: (1*R*,3*S*,5*R*)-2-(2-(3-Acetyl-7-allyl-5-methyl-1*H*-indazol-1-yl)acetyl)-5-(hex-5-enamidomethyl)-N-(3-methyl-6-(trifluoromethyl)pyridin-2-yl)-2-azabicyclo[3.1.0]hexane-3-carboxamide (19-4):** To a mixture of compound **19-3** (37 mg, 0.12 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (51 mg, 0.12 mmol) in DMF (2 mL) was added DIPEA (45 mg, 0.35 mmol), followed by added HATU (90 mg, 0.23 mmol) at 0 °C and the mixture was stirred at 25 °C under N₂ atmosphere for 2 hours. The mixture was diluted with EtOAc, washed with saturated aqueous NH₄Cl solution and brine, dried over anhydrous Na₂SO₄, filtered and concentrated to dryness. The residue was purified by column chromatography via silica gel (eluted with DCM: MeOH= 200: 1 to 75: 1) to afford compound **19-4** (60 mg, yield 72.4%) as a yellow oil. LC/MS (ESI) m/z: 709 (M+H)⁺.

**Step 4: (4¹R,4³S,4⁵R,E)-1³-Acetyl-1⁵-methyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphan-11-ene-4³-carboxamide (19-5):** To a solution of compound **19-4** (60 mg, 0.085 mmol) in toluene (48 mL) was added Grubbs 2^{nd} catalyst (16 mg, 0.019 mmol) and the mixture was degassed under N₂ atmosphere three times. The mixture was stirred under N₂ atmosphere at 80 °C for 16 hours. The mixture was concentrated to dryness and the residue was purified by column chromatography via silica gel (eluted with DCM: MeOH= 200: 1 to 75: 1) to afford compound **19-5** (50 mg, yield 86.7%) as a yellow solid. LC/MS (ESI) m/z: 681 (M+H)⁺.

**Step 5: Compound 19:** To a solution of compound **19-5** (50 mg, 0.073 mmol) in EtOAc (10 mL) was added PtO₂ (10 mg, 0.044 mmol) and the mixture was stirred at 25 °C under a H₂ balloon for 2 hours. The mixture was filtered and the filtrate was concentrated to dryness. The residue was purified by preparative HPLC to afford **Compound 19** (3 mg, yield 6.0%) as a white solid. ¹H-NMR (400 MHz, DMSO-d₆) δ 10.47 (s, 1H), 8.19 (dd, *J=* 8.3, 3.7 Hz, 1H), 7.90 (d, *J=* 7.9 Hz, 1H), 7.66 (d, *J*= 7.8 Hz, 1H), 7.43 (d, *J*= 2.2 Hz, 1H), 6.78 (d, *J=* 2.0 Hz, 1H), 5.84 (d, *J=* 17.9 Hz, 1H), 5.39 (d, *J=* 17.8 Hz, 1H), 4.59 (m, 1H), 4.33 (t, *J=* 7.9 Hz, 1H), 3.56 (dd, *J=* 5.5, 2.5 Hz, 1H), 3.31-3.28 (m, 1H), 3.26-3.14 (m, 2H), 3.05-2.97 (m, 1H), 2.77-2.68 (m, 1H), 2.57 (s, 3H), 2.46-2.35 (m, 2H), 2.26 -2.12 (m, 2H), 2.09 (s, 3H), 1.72-1.63 (m, 3H), 1.62-1.53 (m, 2H), 1.52-1.42 (m, 2H), 1.40-1.34 (m, 1H), 1.30 (s, 3H), 1.28 (s, 3H), 1.18 (m, 1H), 1.09-1.05 (m, 1H). LC/MS (ESI) m/z: 683 (M+H)⁺.

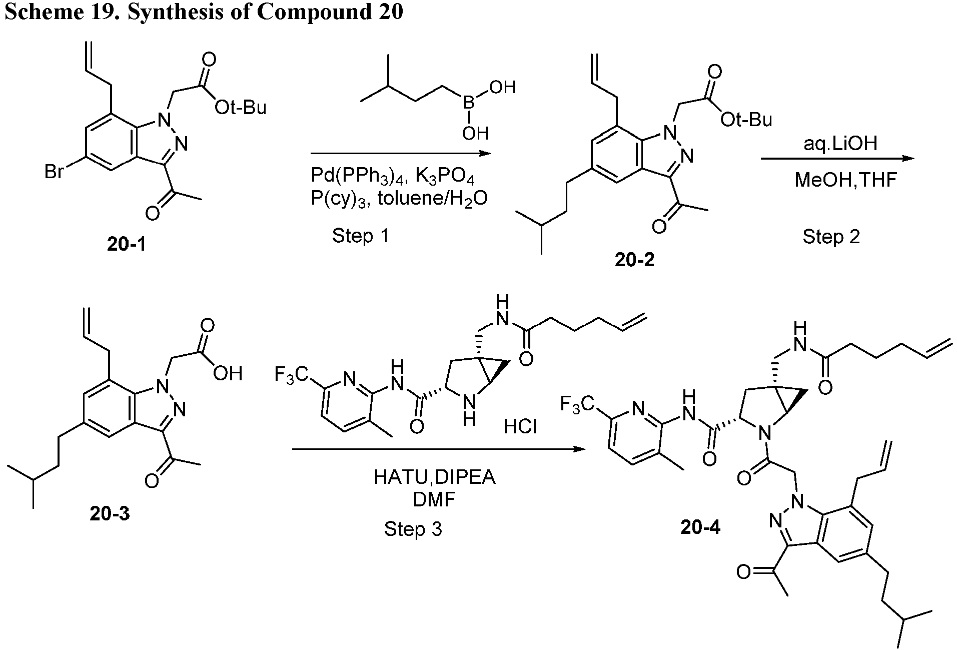

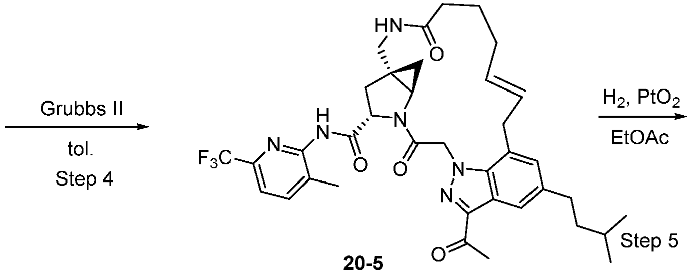

**Step 1: *tert*-Butyl 2-(3-acetyl-7-allyl-5-isopentyl-1*H*-indazol-1-yl)acetate (20-2):** To a solution of compound **20-1** (60 mg, 0.15 mmol) and 3-methylbutylboronic acid (21 mg, 0.183 mmol) in toluene (3 mL) and water (0.5 mL) was added K₃PO₄ (81 mg, 0.38 mmol), tricyclohexyl phosphine (9 mg, 0.031 mmol) and Pd(PPh₃)₄ (18 mg, 0.015 mmol). The mixture was degassed under N₂ three times and stirred at 90 °C under N₂ atmosphere for 10 hours. The mixture was diluted with EtOAc and washed with water and brine, dried over anhydrous Na₂SO₄, filtered and concentrated to dryness. The residue was purified by silica gel chromatography (eluted with PE: EtOAc= 20: 1) to afford compound **20-2** (50 mg, yield 85.2%) as a yellow oil. LC/MS (ESI) m/z: 385 (M+H)⁺.

**Step 2: 2-(3-Acetyl-7-allyl-5-isopentyl-1*H*-indazol-1-yl)acetic acid (20-3):** To a solution of compound **20-2** (50 mg, 0.13 mmol) in MeOH (1 mL), THF (1 mL) and water (0.5 mL) was added lithium hydroxide (9 mg, 0.39 mmol) and the mixture was stirred at room temperature for 2 hours. The mixture was concentrated to dryness and diluted with water. The mixture was washed with EtOAc and acidified with 1 N aqueous HCl to a pH of approximately 3 at 0 °C. The mixture was extracted with DCM twice and the combined organic layers were washed with brine, dried over anhydrous Na₂SO₄, filtered and concentrated to dryness to afford compound **20-3** (45 mg, yield 105.4%) as a light yellow solid that was directly used to the next reaction without purification. LC/MS (ESI) m/z: 329(M+H)⁺.

**Step 3: (1*R*,3*S*,5*R*)-2-(2-(3-Acetyl-7-allyl-5-isopentyl-1*H*-indazol-1-yl)acetyl)-5-(hex-5-enamidomethyl)-*N*-(3-methyl-6-(trifluoromethyl)pyridin-2-yl)-2-azabicyclo[3.1.0]hexane-3-carboxamide (20-4):** To a mixture of compound **20-3** (45 mg, 0.13 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (71 mg, 0.16 mmol) in DMF (4 mL) was added HATU (63 mg, 0.16 mmol) followed by DIPEA (44 mg, 0.34 mmol) at 0 °C and the mixture was stirred at room temperature for 2 hours. The mixture was diluted with EtOAc, washed with saturated aqueous NH₄Cl solution and brine, dried over anhydrous Na₂SO₄, filtered and concentrated to dryness. The residue was purified by column chromatography via silica gel (eluted with DCM: MeOH= 30: 1) to afford compound **20-4** (45 mg, yield 45.6%) as a yellow solid. LC/MS (ESI) m/z: 721 (M+H)⁺.

**Step 4: Compound 20-5:** To a solution of compound **20-4** (45 mg, 0.062 mmol) in degassed toluene (45 mL) was added Grubbs 2^{nd} catalyst (13 mg, 0.016 mmol) and the resulting mixture was degassed again and stirred at 80°C under N₂ atmosphere for 16 hours. The mixture was concentrated to dryness and the residue was purified by silica gel chromatography (eluted with DCM: MeOH=30: 1) to afford compound **20-5** (30 mg, yield 27.7%) as a light yellow solid. LC/MS (ESI) m/z: 693 (M+H)⁺.

**Step 5: Compound 20:** To a solution of compound **20-5** (30 mg, 0.043 mmol) in EtOAc (3 mL) was added PtO₂ (10 mg, 0.043 mmol) and the mixture was degassed under N₂ three times. The mixture was stirred under a H₂ balloon at room temperature for 1 hour. The mixture was filtered and the filtrate was concentrated to dryness. The residue product was purified by preparative HPLC to afford **Compound 20** (5 mg, yield 16.6%) as a white solid. ¹H-NMR (400 MHz, CD₃OD) δ 7.90 (d, *J=* 10.8 Hz, 1H), 7.81 (d, *J=* 7.6 Hz, 1H), 7.56 (d, *J=* 7.8 Hz, 1H), 7.03 (d, *J=* 11.5 Hz, 1H), 5.87 (d, *J=* 17.8 Hz, 1H), 5.57 (d, *J=* 17.7 Hz, 1H), 4.52 (t, *J=* 6.7 Hz, 1H), 3.62 (d, *J=* 3.0 Hz, 1H), 3.47 (d, *J=* 14.5 Hz, 1H), 3.35 (s, 1H), 3.04 (m, 1H), 2.77 (dd, *J=* 16.9, 5.6 Hz, 1H), 2.73-2.67 (m, 2H), 2.64 (s, 3H), 2.59 (d, *J=* 7.1 Hz, 2H), 2.37-2.24 (m, 2H), 2.13 (s, 3H), 1.86-1.58 (m, 6H), 1.53 (dd, *J=* 14.2, 6.4 Hz, 4H), 1.40 (t, *J=* 5.9 Hz, 2H), 1.15 (m, 1H), 0.95 (d, *J=* 6.3 Hz, 6H). LC/MS (ESI) m/z: 695 (M+H)⁺.

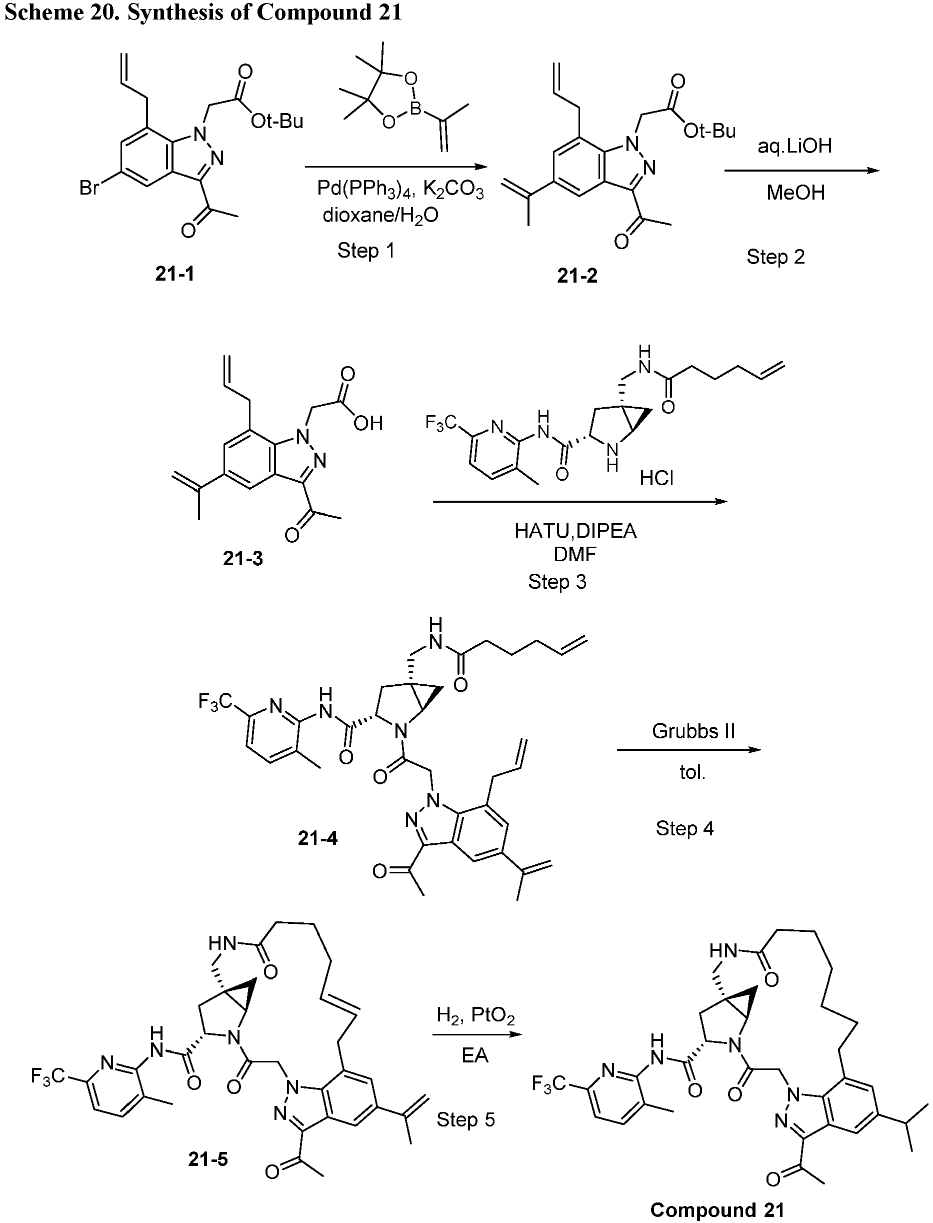

**Step 1: *tert*-Butyl 2-(3-acetyl-7-allyl-5-(prop-1-en-2-yl)-1*H*-indazol-1-yl)acetate (21-2):** To a solution of compound **21-1** (50 mg, 0.13 mmol) and 4,4,5,5-tetramethyl-2-(prop-1-en-2-yl)-1,3,2-dioxaborolane (18 mg, 0.11 mmol) in 1,4-dioxane (1.5 mL) and water (1 mL) was added K₂CO₃ (59 mg, 0.42 mmol) and Pd(dppf)Cl₂ (13 mg, 0.013 mmol) and the mixture was stirred at 90 °C under N₂ atmosphere for 16 hours. The mixture was filtered and the filter cake was washed with ethyl acetate. The combined filtrate was washed with brine, dried over anhydrous Na₂SO₄, filtered and concentrated to dryness. The residue was purified by silica gel chromatography (eluted with PE: EtOAc= 30: 1) to afford compound **21-2** (30 mg, yield 64.1%) as a yellow solid. LC/MS (ESI) m/z: 369 (M+H)⁺.

**Step 2: 2-(3-acetyl-7-allyl-5-(prop-1-en-2-yl)-1*H*-indazol-1-yl)acetic acid (21-3):** To a solution of compound **21-2** (30 mg, 0.085 mmol) in THF (1 mL), MeOH (1 mL) and water (0.5 mL) was added LiOH (5 mg, 0.21 mmol) and the mixture was stirred at room temperature for 2 hours. The mixture was concentrated to dryness and diluted with water. The mixture was washed with EtOAc twice and acidified with 1 N aqueous HCl to a pH of approximately 3 at 0 °C. The mixture was extracted with DCM twice and the combined organic layers were washed with brine, dried over anhydrous Na₂SO₄, filtered and concentrated to dryness to afford compound **21-3** (25 mg, yield 99.0%) as a light yellow solid that was directly used to the next reaction without purification. LC/MS (ESI) m/z: 299 (M+H)⁺.

**Step 3: (1*R*,3*S*,5*R*)-2-(2-(3-Acetyl-7-allyl-5-(prop-1-en-2-yl)-1*H*-indazol-1-yl)acetyl)-5-(hex-5-enamidomethyl)-*N*-(3-methyl-6-(trifluoromethyl)pyridin-2-yl)-2-azabicyclo[3.1.0]hexane-3-carboxamide (21-4):** To a mixture of compound **21-3** (25 mg, 0.084 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (41 mg, 0.10 mmol) in DMF (3 mL) was added HATU (0.038 g, 0.10 mmol) followed by DIPEA (27 mg, 0.21 mmol) at 0 °C and the mixture was stirred at room temperature for 2 hours. The mixture was diluted with EtOAc, washed with saturated aqueous NH₄Cl solution and brine, dried over anhydrous Na₂SO₄, filtered and concentrated to dryness. The residue was purified by column chromatography via silica gel (DCM: MeOH= 30: 1) to afford compound **21-4** (30 mg, yield 51.8%) as a yellow solid.LC/MS (ESI) m/z: 691 (M+H)⁺

**Step 4: (4¹R,4³S,4⁵R,E)-1³-Acetyl-N-(3-methyl-6-(trifluoromethyl)pyridin-2-yl)-3,7-dioxo-1⁵-(prop-1-en-2-yl)-1¹H-4²,6-diaza-1(1,7)-indazola-4(2,5)-bicyclo[3.1.0]hexanacyclotridecaphan-11-ene-4³-carboxamide (21-5):** To a solution of compound **21-4** (30 mg, 0.043 mmol) in degassed toluene (30 mL) was added Grubbs 2^{nd} catalyst (15 mg) and the resulting mixture was degassed again and stirred at 80°C under N₂ atmosphere for 16 hours. The mixture was concentrated to dryness and the residue was purified by silica gel chromatography (eluted with DCM: MeOH= 30: 1) to afford compound **21-5** (25 mg, yield 21.7%) as a light yellow solid. LC/MS (ESI) m/z: 663 (M+H)⁺.

**Step 5: (4¹R,4³S,4⁵R)-1³-Acetyl-1⁵-isopropyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide (Compound 21):** To a solution of compound 21-5 (25 mg, 0.038 mmol) in EtOAc (5 mL) was added PtO₂ (8 mg) and the mixture was degassed under N₂ three times and stirred under a H₂ balloon at room temperature for 1 hour. The mixture was filtered and the filtrate was concentrated to dryness. The residue product was purified by preparative HPLC to afford **Compound 21** (4 mg, yield 15.9%) as a white solid. ¹H-NMR (400 MHz, CD₃OD) δ 7.96 (s, 1H), 7.81 (d, *J=* 7.8 Hz, 1H), 7.56 (d, *J=* 7.8 Hz, 1H), 7.10 (s, 1H), 5.87 (d, *J=* 17.8 Hz, 1H), 5.57 (d, *J*= 17.8 Hz, 1H), 4.52 (t, *J=* 6.9 Hz, 1H), 3.62 (dd, *J*= 5.8, 2.8 Hz, 1H), 3.47 (d, *J=* 14.5 Hz, 1H), 3.35 (s, 1H), 3.11-3.02 (m, 1H), 2.99 (dd, *J*= 13.7, 6.8 Hz, 1H), 2.77 (dd, *J=* 22.0, 10.7 Hz, 1H), 2.65 (s, 3H), 2.59 (d, *J=* 7.3 Hz, 2H), 2.37-2.25 (m, 2H), 2.13 (s, 3H), 1.64 (m,8H), 1.40 (t, *J=* 5.8 Hz, 2H), 1.28 (dd, *J=* 6.9, 1.4 Hz, 6H), 1.15 (m, 1H). LC/MS (ESI) m/z: 667 (M+H)⁺.

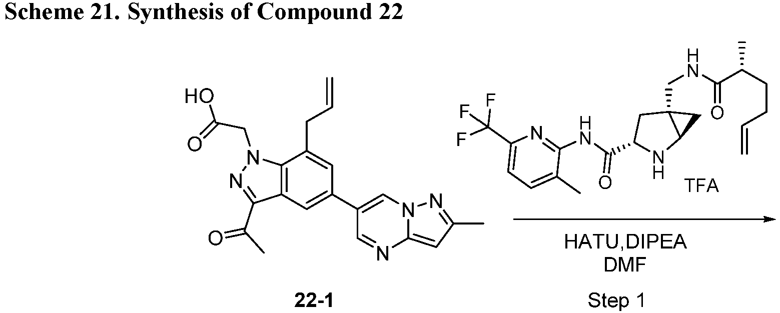

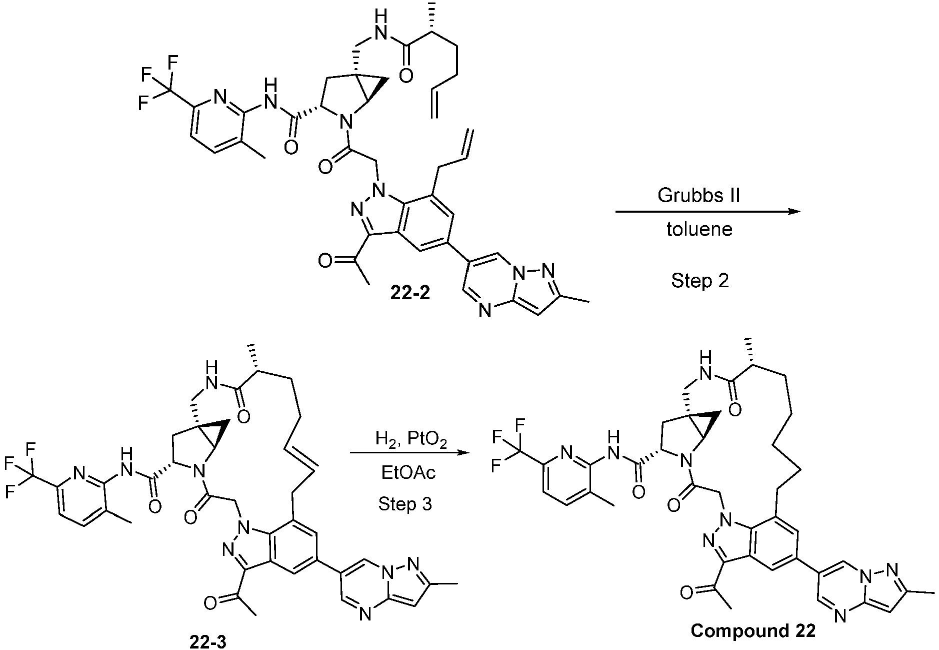

**Step 1: (1*R*,3*S*,5*R*)-2-(2-(3-Acetyl-7-allyl-5-(2-methylpyrazolo[1,5-a]pyrimidin-6-yl)-1H-indazol-1-yl)acetyl)-*N*-(3-methyl-6-(trifluoromethyl)pyridin-2-yl)-5-(((*R*)-2-methylhex-5-enamido)methyl)-2-azabicyclo[3.1.0]hexane-3-carboxamide (22-2):** To a mixture of compound **22-1** (54 mg, 0.14 mmol) and (1*R*,3*S*,5*R*)-*N*-(3-methyl-6-(trifluoromethyl)pyridin-2-yl)-5-(((R)-2-methylhex-5-enamido)methyl)-2-azabicyclo[3.1.0]hexane-3-carboxamide TFA salt (73 mg, 0.14 mmol) in DMF (2 mL) was added DIPEA (90 mg, 0.69 mmol) followed by HATU (106 mg, 0.28 mmol) at 0 °C and the reaction was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by column chromatography on silica gel (eluted with DCM: MeOH = 80: 1) to afford compound **22-2** (84 mg, yield 76.1%) as a yellow solid. LC/MS (ESI) m/z: 796 (M+H)⁺.

**Step 2: Compound 22-3:** To a solution of compound **22-2** (84 mg, 0.11 mmol) in degassed toluene (67 mL) was added Grubbs 2^{nd} catalyst (23 mg, 0.03 mmol) at 0 °C under N₂ atmosphere and the mixture was stirred at 80 °C under N₂ atmosphere overnight. The mixture was concentrated to dryness and the residue was purified by chromatography on silica gel (eluted with DCM: MeOH= 50: 1) to afford compound **22-3** (54 mg, yield 66.7%) as a brown solid. LC/MS (ESI) m/z: 768 (M+H)⁺.

**Step 3: Compound 22:** To a solution of compound **22-4** (54 mg, 0.07 mmol) in EtOAc (5 mL) was added PtO₂ (14 mg) at 0 °C and the mixture was degassed under N₂ atmosphere three times and stirred under a H₂ balloon at room temperature for 1.5 hours. The mixture was filtered and the filtrate was concentrated to dryness. The residue was purified by preparative HPLC to afford **Compound 22** (5.6 mg, yield 10.4%) as a white solid. ¹H-NMR (400 MHz, DMSO-*d₆*) δ 10.42 (s, 1H), 9.32 (d, J= 1.5 Hz, 1H), 8.82 (d, J= 2.3 Hz, 1H), 8.33 (d, J= 1.6 Hz, 1H), 7.98 (t, J= 6.2 Hz, 1H), 7.89 (d, J= 7.8 Hz, 1H), 7.69-7.51 (m, 2H), 6.56 (s, 1H), 5.93 (d, J= 17.8 Hz, 1H), 5.52 (d, J= 17.6 Hz, 1H), 4.27 (t, J= 8.2 Hz, 1H), 3.62-3.64 (m, 1H), 3.52-3.57 (dd, J= 14.2, 5.7 Hz, 1H), 3.31-3.26 (m, 1H), 3.15-2.93 (m, 3H), 2.64 (s, 3H), 2.47-2.51 (m, 1H), 2.46 (s, 3H), 2.27-2.29 (m, 1H), 2.10 (s, 3H), 1.91-1.50 (m, 6H), 1.38-1.39 (m, 2H), 1.15-1.16 (m, 1H), 1.06-1.08 (d, J=7.2 Hz, 3H), 1.05-1.07 (m, 1H). LC/MS (ESI) m/z: 770 (M+H)⁺.

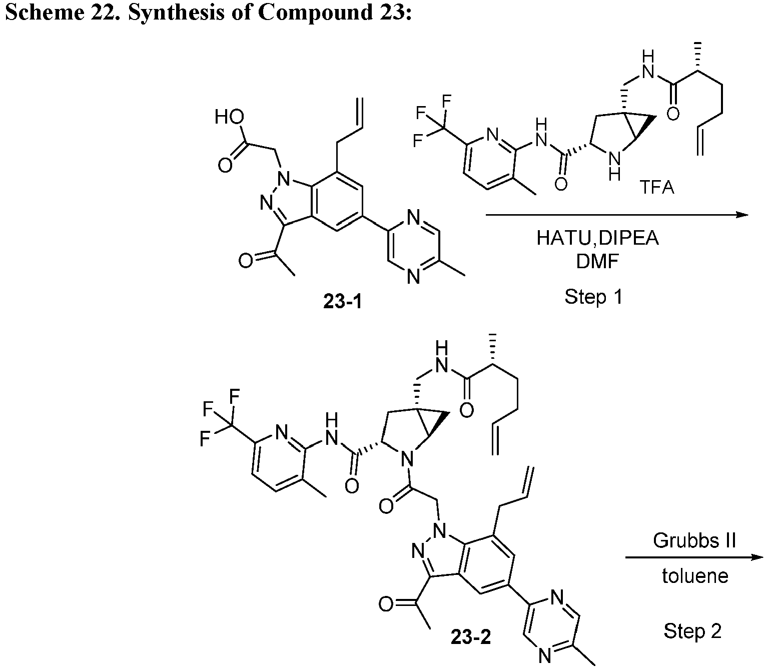

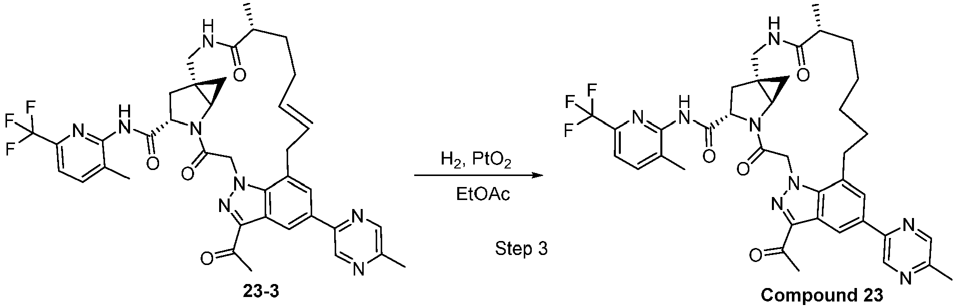

**Step** 1: **(1*R,*3*S*,5*R*)-2-(2-(3-Acetyl-7-allyl-5-(5-methylpyrazin-2-yl)-1*H*-indazol-1-yl) acetyl)- *N*-(3-methyl-6-(trifluoromethyl) pyridin-2-yl)-5-(((*R*)-2-methylhex-5-enamido) methyl)-2- azabicyclo [3.1.0] hexane-3-carboxamide (23-2):** To a mixture of compound **23-1** (40 mg, 0.11 mmol) and (1*R*,3*S*,5*R*)-*N*-(3-methyl-6-(trifluoromethyl)pyridin-2-yl)-5-(((*R*)-2-methylhex-5-enamido)methyl)-2-azabicyclo[3.1.0]hexane-3-carboxamide TFA salt (57 mg, 0.11 mmol) in DMF (3 mL) was added DIPEA (74 mg, 0.57 mmol) followed by HATU (65 mg, 0.17 mmol) at 0 °C and the mixture was stirred at room temperature for 2 hours. The mixture was diluted with EtOAc and washed with saturated aqueous NH₄Cl solution and brine, dried over anhydrous Na₂SO₄, filtered and concentrated to dryness. The residue was purified by chromatography on silica gel (eluted with PE: EtOAc=2: 1 to 1: 2) to afford compound **23-2** (60 mg, yield 69.4%) as a yellow oil. LC/MS (ESI) m/z: 757 (M+H)⁺.

**Step 2: Compound 23-3:** To a solution of compound **23-2** (60 mg, 0.079 mmol) in degassed toluene (60 mL) was added Grubbs 2^{nd} catalyst (13 mg, 0.016 mmol) at 0 °C under N₂ atmosphere and the mixture was stirred at 80 °C overnight under N₂ atmosphere. The mixture was concentrated to dryness and the residue was purified by chromatography on silica gel (eluted with PE: EtOAc= 2: 1 to 1: 2) to afford compound **23-3** (36 mg, yield 62.1%) as a brown solid. LC/MS (ESI) m/z: 729(M+H)⁺.

**Step 3: Compound 23:** To a solution of compound **23-3** (35 mg, 0.048 mmol) in EtOAc (5 mL) was added PtO₂ (15 mg) at 0 °C and the mixture was degassed under N₂ atmosphere three times and stirred under a H₂ balloon at room temperature for 0.5 hour. The mixture was filtered and the filtrate was concentrated to dryness. The residue was purified by preparative HPLC to afford **Compound 23** (2.3 mg, yield 6.55%) as a white solid. ¹H-NMR (400 MHz, DMSO-*d₆*) δ 10.43 (s, 1H), 9.11 (d, J= 1.6 Hz, 1H), 8.75 (d, J= 1.6 Hz, 1H), 8.62 (s, 1H), 7.98 (t, J= 6.4 Hz, 1H), 7.94 (s, 1H), 7.88 (d, J= 7.6 Hz, 1H), 7.64 (d, J= 7.8 Hz, 1H), 5.93 (d, J= 17.6 Hz, 1H), 5.51 (d, J= 18.0 Hz, 1H), 4.27 (t, J= 8.0 Hz, 1H), 3.63 (dd, J= 5.6, 2.4 Hz, 1H), 3.54 (dd, J= 14.4, 5.6 Hz, 1H), 3.31-3.26 (m, 1H), 3.14-2.95 (m, 3H), 2.64 (s, 3H), 2.54 (s, 3H), 2.47-2.45 (m, 1H), 2.31-2.22 (m, 1H), 2.11 (s, 3H), 1.89-1.78 (m, 1H), 1.73-1.62 (m, 2H), 1.61-1.49 (m, 3H), 1.44-1.33 (m, 2H), 1.16 (t, J= 6.0 Hz, 1H), 1.10-1.04 (m, 4H). LC/MS (ESI) m/z: 731 (M+H)⁺.

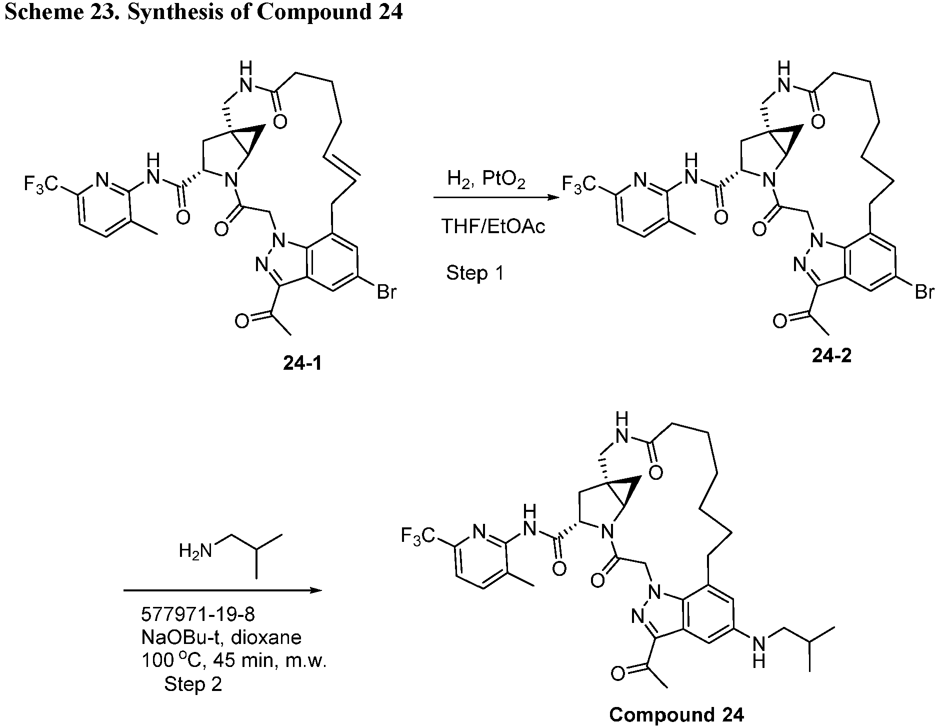

**Step** 1: Compound **24-2:** To a solution of compound 24-1 (100 mg, 0.14mmol) in EtOAc (5 mL) and THF (5 mL) was added PtO₂ (20 mg) and the mixture was degassed and stirred under a H₂ balloon at room temperature for 1 hour. The mixture was filtered and the filtrate was concentrated to dryness. The residue was purified by column chromatography on silica gel (eluted with DCM: MeOH =60: 1) to afford compound **24-2** (90 mg, yield 90%) as a yellow solid. LC/MS (ESI) m/z: 703 (M+H)⁺.

**Step 2: Compound 24:** To a mixture of compound **24-2** (30 mg, 0.042mmol) and 2-methylpropan-1-amine (16 mg, 0.21mmol) in 1,4-dioxane (1 mL) was added NaOBu-t (12 mg, 0.13 mmol) and 2-(2'-Di-tert-butylphosphine)biphenylpalladium(II) acetate (4 mg 0.008 mmol). The mixture was degassed under N₂ three times and stirred at 100 °C in CEM microwave reactor for 45 minutes. The reaction mixture was diluted with EtOAc, washed with water and brine, dried over anhydrous Na₂SO₄, filtered and concentrated to dryness. The residue was purified by preparative HPLC to afford **Compound 24** (4 mg, yield 13.5%) as a white solid. ¹H-NMR (400 MHz, CD₃OD) δ 7.80-7.82 (d, *J=* 7.6Hz, 1H), 7.54-7.56 (d, *J=* 7.6Hz, 1H), 7.15 (s, 1H), 7.69 (s, 1H), 5.79-5.84 (d, *J=* 17.6Hz, 1H), 5.46-5.50 (d, *J=* 17.6Hz, 1H), 4.51 (s, 1H), 3.5-3.61 (m, 1H), 3.44-3.48 (m, 2H), 2.91-2.92 (m, 3H), 2.59-2.61 (m, 5H), 2.31-2.34 (m, 2H), 2.14 (s, 3H), 1.74-1.96 (m, 9H), 1.52-1.63 (m, 3H), 0.96-1.09 (m, 6H). LC/MS (ESI) m/z: 696 (M+H)⁺.

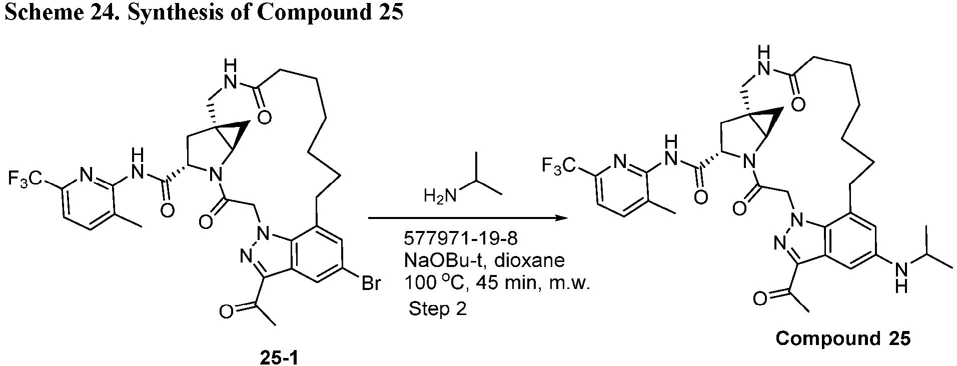

To a mixture of compound 25-1 (30 mg, 0.042 mmol) and 2-methylpropan-1-amine (13 mg, 0.21 mmol) in 1,4-dioxane (1 mL) was added NaOBu-t (12 mg, 0.13 mmol) and 2-(2'-di-tert-butylphosphine)biphenylpalladium(II) acetate (4 mg 0.008 mmol). The mixture was degassed under N₂ three times and stirred at 100 °C in CEM microwave reactor for 45 minutes. The reaction mixture was diluted with EtOAc, washed with water and brine, dried over anhydrous Na₂SO₄, filtered and concentrated to dryness. The residue was purified by preparative HPLC to afford **Compound 25** (3.5 mg, yield 12.0%) as a white solid. ¹H-NMR (400 MHz, CD₃OD): δ 7.80-7.82 (d, *J=* 7.6Hz, 1H), 7.54-7.56 (d, *J=* 7.6Hz, 1H), 7.23 (s, 1H), 7.67(s, 1H), 5.80-5.84(d, J= 17.6Hz, 1H), 5.47-5.51 (d, *J=* 17.6Hz, 1H), 4.49-4.79 (m, 2H), 3.59-3.64 (m, 2H), 3.44-3.48 (m, 1H), 2.95-2.97 (m, 1H), 2.57-2.70 (m, 5H), 2.24-2.34 (m, 2H), 2.21 (s, 3H), 1.62-1.78 (m, 9H), 1.15-1.26 (m, 6H), 1.09-1.13 (m, 1H). LC/MS (ESI) m/z: 682 (M+H)⁺.

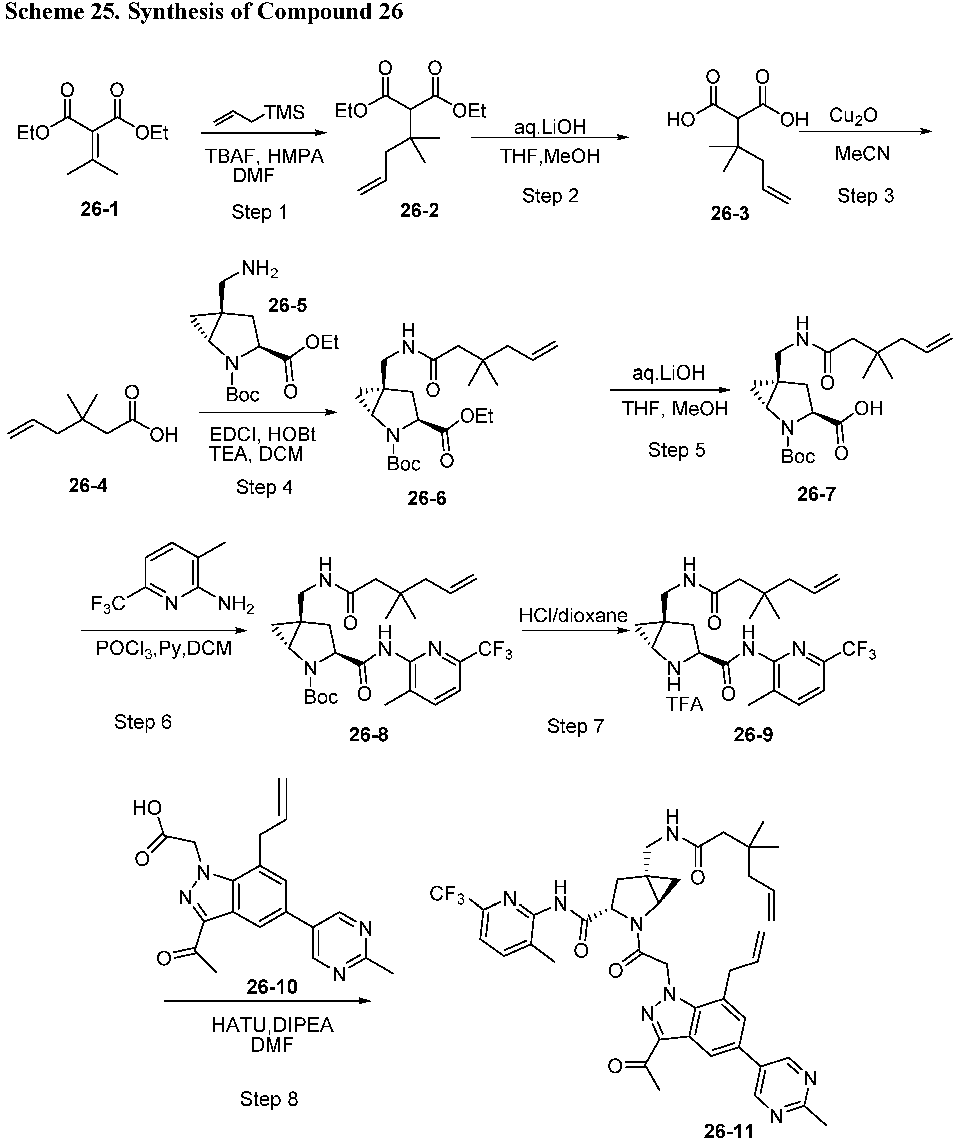

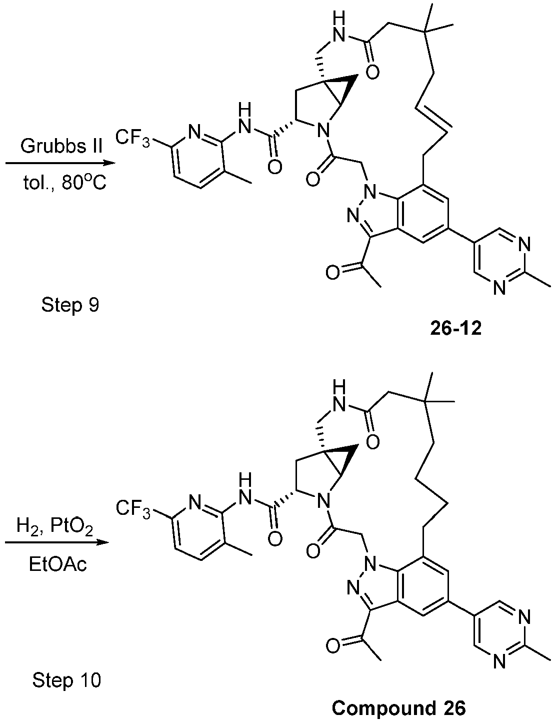

**Step 1: Diethyl 2-(2-methylpent-4-en-2-yl)malonate (26-2):** To a solution of **compound 26-1** (1 g, 5 mmol) in DMF (10 mL) was added 4A molecular sieves (1 g) and TBAF (0.6 mL, 1 M in THF) at 0 °C followed by HMPA (2.69 g, 15 mmol) and allyltrimethylsilane (1.7 g, 15 mmol). After addition, the mixture was stirred at room temperature for 2 hours. The mixture was diluted with EtOAc and washed with saturated aqueous NH₄Cl solution and brine, dried with anhydrous Na₂SO₄, filtered and concentrated to dryness. The residue was purified by chromatography on silica gel (eluted with PE: EtOAc= 60: 1) to afford compound **26-2** (700 mg, yield 57.9%) as a yellow oil.

**Step 2: 2-(2-Methylpent-4-en-2-yl)malonic acid (26-3):** To a solution of compound **26-2** (700 mg, 2.89 mmol) in MeOH (5 mL) and THF (5 mL) was added a solution of LiOH (364 mg, 8.67 mmol) in water (5 mL) at 0 °C. After addition, the mixture was stirred at room temperature for 1 hour. The reaction mixture was concentrated to dryness and the residue was dissolved in water. The mixture was washed with tert-butyl methyl ether and acidified by adding 1 N HCl to a pH of approximately 3. The mixture was extracted with DCM twice and the combined organic layers were washed with brine, dried over Na₂SO₄, filtered and concentrated to dryness to afford compound **26-3** (500 mg, yield 93.0%) as a yellow oil. LC/MS (ESI) m/z: 185 (M-H)-.

**Step 3: 3,3-Dimethylhex-5-enoic acid (26-4):** To a solution of compound **26-3** (200 mg, 1.08 mmol) in CH3CN (5 mL) was added CuO (8.35 mg, 0.11 mmol) and the mixture was stirred at 90°C for 40 hours. The mixture was acidified by adding 3 N aqueous HCl to a pH of approximately 2 and extracted with DCM twice. The combined organic layers were washed with brine, dried over Na₂SO₄, filtered and concentrated to dryness to afford compound **26-4** (150 mg, yield 98.0%) as a yellow oil. LC/MS (ESI) m/z: 141 (M-H)-.

**Step 4: (1*R*,3*S*,5*R*)-2-*tert*-Butyl 3-ethyl 5-((3,3-dimethylhex-5-enamido)methyl)-2-azabicyclo[3.1.0]hexane-2,3-dicarboxylate (26-6):** To a mixture of compound **26-4** (150 mg, 1.06 mmol) and (1*R*,3*S*,5*R*)-2-*tert*-butyl *3*-ethyl 5-(aminomethyl)-2-azabicyclo[3.1.0]hexane-2,3-dicarboxylate (200 mg, 0.71 mmol) in DCM (5 mL) was added HOBt (172 mg, 1.27 mmol) and EDCI (302 mg, 1.59 mmol) followed by triethylamine (428 mg, 4.24 mmol) at 0 oC. After addition, the mixture was stirred at 30 °C for 16 hours. The mixture was diluted with EtOAc and washed with saturated aqueous NH₄Cl solution and brine, dried over Na₂SO₄, filtered and concentrated to dryness. The residue was purified by silica gel chromatography (eluted with PE: EtOAc = 4: 1 to 2: 1) to afford compound **26-6** (100 mg, yield 23.1%) as a colorless oil. LC/MS (ESI) m/z: 409 (M+H)+.

**Step 5: (1*R*,3*S*,5*R*)-2-(*tert*-Butoxycarbonyl)-5-((3,3-dimethylhex-5-enamido)methyl)-2-azabicyclo[3.1.0]hexane-3-carboxylic acid (26-7):** To a solution of compound **26-6** (100 mg, 0.24 mmol) in MeOH (2 mL) and THF (2 mL) was added a solution of LiOH (30.8 mg, 0.73 mmol) in water (2 mL) at 0 °C. After addition, the mixture was stirred at room temperature for 1 hour. The reaction mixture was concentrated to dryness and the residue was dissolved in water. The mixture was washed with tert-butyl methyl ether and acidified by adding 1 N HCl to a pH of approximately 3. The mixture was extracted with DCM twice and the combined organic layers were washed with brine, dried over Na₂SO₄, filtered and concentrated to dryness to afford compound **26-7** (80 mg, yield 87.5%) as a yellow oil. LC/MS (ESI) m/z: 381 (M+H)+.

**Step 6: (1*R*,3*S*,5*R*)-*tert*-Butyl 5-((3,3-dimethylhex-5-enamido)methyl)-3-((3-methyl-6-(trifluoromethyl)pyridin-2-yl)carbamoyl)-2-azabicyclo[3.1.0]hexane-2-carboxylate (26-8):** To a mixture of compound 7 (80 mg, 0.21 mmol) and 3-methyl-6-(trifluoromethyl)pyridin-2-amine (37 mg, 0.21 mmol) in DCM (3 mL) was added pyridine (83 mg, 1.05 mmol) followed by POCl3 (48 mg, 0.32 mmol) at 0 °C. After addition, the mixture was stirred at room temperature for 1 hour. The mixture was diluted with DCM and washed with 0.5 N HCl and brine successively, dried and concentrated to dryness. The residue was purified by column chromatography on silica gel (eluted with PE: EtOAc= 2: 1) to afford compound **26-8** (70 mg, yield 62.0%) as a light yellow oil. LC/MS (ESI) m/z: 539 (M+H)+.

**Step 7: (1*R*,3*S*,5*R*)-5-((3,3-Dimethylhex-5-enamido)methyl)-*N*-(3-methyl-6-(trifluoromethyl)pyridin-2-yl)-2-azabicyclo[3.1.0]hexane-3-carboxamide hydrochloride (26-9):** A solution of compound **26-8** (70 mg, 0.13 mmol) in HCl/1,4-dioxane solution (3 mL, 4M) was stirred at room temperature for 1 hour. The mixture was concentrated to dryness to afford compound **26-9** (65 mg, yield 100%) as a white solid that was directly used to the next reaction without purification. LC/MS (ESI) m/z: 439 (M+H)+.

**Step 8: (1*R*,3*S*,5*R*)-2-(2-(3-Acetyl-7-allyl-5-(2-methylpyrimidin-5-yl)-1*H*-indazol-1-yl)acetyl)-5-((3,3-dimethylhex-5-enamido)methyl)-*N*-(3-methyl-6-(trifluoromethyl)pyridin-2-yl)-2-azabicyclo[3.1.0]hexane-3-carboxamide (26-11):** To a mixture of compound **26-9** (65 mg, 0.13 mmol) and 2-(3-acetyl-7-allyl-5-(2-methylpyrimidin-5-yl)-1*H*-indazol-1-yl)acetic acid (46 mg, 0.13 mmol) in DMF (3 mL) was added DIPEA (67 mg, 0.52 mmol) followed by HATU (89 mg, 0.23 mmol) at 0 °C. After addition, the mixture was stirred at room temperature for 1 hour. The mixture was diluted with water and extracted with EtOAc twice. The combined organic layers were washed with saturated aqueous NH4Cl solution and brine, dried with anhydrous Na2SO4, filtered and concentrated to dryness. The residue was purified by chromatography on silica gel (DCM: MeOH= 60: 1) to afford compound **26-11** (50 mg, yield 50.0%) as a yellow solid. LC/MS (ESI) m/z: 771 (M+H)+.

**Step 9: Compound 26-12:** To a solution of compound **26-11** (50 mg, 0.06 mmol) in toluene (50 mL) was added Grubbs 2nd catalyst (14 mg, 0.017 mmol) under N₂ atmosphere. The mixture was degassed under N₂ three times and stirred at 80 oC under N₂ atmosphere overnight. The mixture was concentrated to dryness and the residue was purified by column chromatography on silica gel (eluted with DCM: MeOH= 40: 1) to afford compound **26-12** (30 mg, yield 67.4%) as a brown solid. LC/MS (ESI) m/z: 743 (M+H)+.

**Step 10: Compound 26:** To a degassed solution of compound **26-12** (30 mg, 0.04 mmol) in THF (4 mL) and EtOAc (4 mL) was added PtO2 (9.6 mg, 0.04 mmol) and the mixture was degassed under N2 atmosphere three times and stirred under a H2 balloon at 25 °C for 16 hours. The mixture was concentrated to dryness and the residue was purified by preparative HPLC to afford **Compound 26** (2 mg, yield 6.7%) as a white solid. 1H-NMR (400 MHz, CH3OD) δ 9.00 (d, J= 8.9 Hz, 2H), 8.43 (d, J= 1.6 Hz, 1H), 7.82 (d, J= 7.9 Hz, 1H), 7.55 (d, J= 7.3 Hz, 2H), 5.85 (d, J= 17.8 Hz, 1H), 5.65 (d, J= 17.7 Hz, 1H), 4.47 (t, J= 7.8 Hz, 1H), 3.73-3.76 (m, 1H), 3.35 (d, J= 4.2 Hz, 2H), 3.10-3.16 (m, 1H), 2.83-2.93 (m, 1H), 2.74 (s, 3H), 2.69 (s, 3H), 2.61-2.69 (m, 1H), 2.51-2.56 (m, 1H), 2.29 (t, J= 14.0 Hz, 1H), 2.18 (s, 3H), 2.10 (d, J= 14.7 Hz, 1H), 1.82-1.94 (m, 2H), 1.59-1.75 (m, 3H), 1.33 -1.38 (m, 2H), 1.15 (d, J= 3.0 Hz, 1H), 1.13 (s, 3H), 0.98 (s, 3H), 0.88-0.92 (m, 1H). LC/MS (ESI) m/z: 745 (M+H)+.

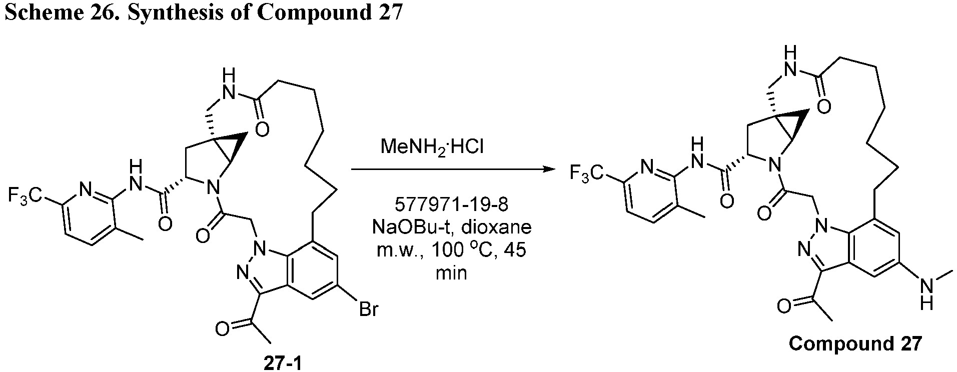

To a mixture of compound **27-1** (30 mg, 0.042 mmol) and methyl-amine hydrochloride (13 mg, 0.21 mmol) in 1,4-dioxane (1 mL) was added NaOBu-t (28 mg,3 mmol) and 2-(2'-Di-tert-butylphosphine)biphenylpalladium(II) acetate (4 mg 0.008 mmol). The mixture was degassed under N₂ three times and stirred at 100 °C in CEM microwave reactor for 45 minutes. The reaction mixture was diluted with EtOAc, washed with water and brine, dried over anhydrous Na₂SO₄, filtered and concentrated to dryness. The residue was purified by preparative HPLC to afford **Compound 27** (3.3 mg, yield 11.8%) as a white solid. ¹H-NMR (400 MHz, CH₃OD) δ 7.82 (d, J= 7.9 Hz, 1H), 7.56 (d, J= 7.8 Hz, 1H), 7.16 (d, J= 2.1 Hz, 1H), 6.67 (d, J= 2.2 Hz, 1H), 5.83 (d, J= 17.7 Hz, 1H), 5.50 (d, J= 17.8 Hz, 1H), 4.53 (d, J= 7.3 Hz, 1H), 3.58-3.63 (m, 1H), 3.46 (d, J= 14.7 Hz, 1H), 2.93-3.05 (m, 1H), 2.80 (s, 3H), 2.69 (d, J= 10.4 Hz, 1H), 2.62 (s, 3H), 2.59 (d, J= 7.3 Hz, 2H), 2.24-2.37 (m, 2H), 2.14 (s, 3H), 1.48-1.84 (m, 8H), 1.39 (t, J= 5.9 Hz, 2H), 1.28 (m, 2H), 1.01-1.14 (m, 1H). LC/MS (ESI) m/z: 654 (M+H)⁺.

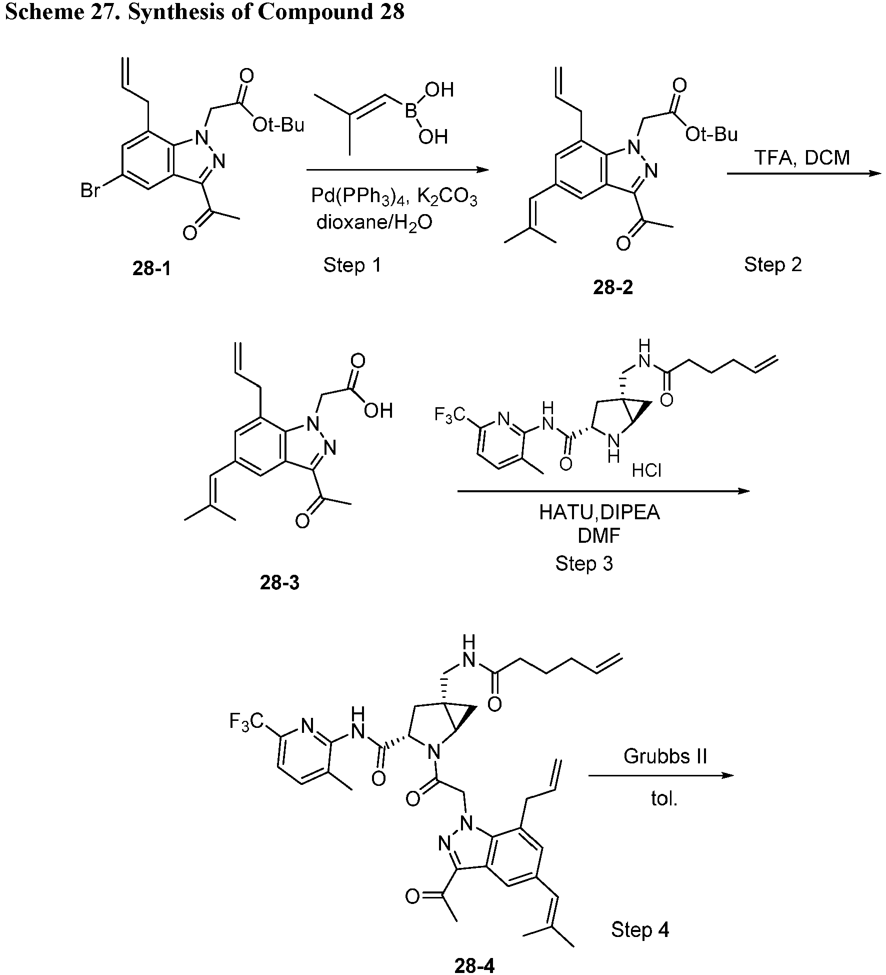

**Step 1: *tert*-Butyl 2-(3-acetyl-7-allyl-5-(2-methylprop-1-en-1-yl)-1*H*-indazol-1-yl)acetate (28-2):** To a solution of compound **28-1** (50 mg, 0.13 mmol) and (2-methylprop-1-en-1-yl)boronic acid (15 mg, 0.15 mmol) in 1,4-dioxane (1.5 mL) and water (1 mL) was added K₂CO₃ (44 mg, 0.32 mmol) and Pd(dppf)Cl₂ (10 mg, 0.013 mmol) and the mixture was stirred under N₂ atmosphere at 90 °C for 16 hours. The mixture was filtered and the filter cake was washed with EtOAc. The combined filtrate was washed with brine, dried over anhydrous Na₂SO₄, filtered and concentrated to dryness. The residue was purified by silica gel chromatography (eluted with PE: EtOAc= 30: 1) to afford compound **28-2** (30 mg, yield 79.9%) as a yellow oil. LC/MS (ESI) m/z: 369 (M+H)⁺.

**Step 2: 2-(3-Acetyl-7-allyl-5-(2-methylprop-1-en-1-yl)-1*H*-indazol-1-yl)acetic acid (28-3):** A solution of compound **28-2** (30 mg, 0.081 mmol) in TFA (1 mL) and DCM (3 mL) was stirred at room temperature for 2 hours. The mixture was concentrated to dryness to afford compound **28-3** (25 mg, yield 98.3%) as a yellow solid that was directly used to the next reaction without purification. LC/MS (ESI) m/z: 313(M+H)⁺.

**Step 3: (1*R*,3*S*,5*R*)-2-(2-(3-Acetyl-7-allyl-5-(2-methylprop-1-en-1-yl)-1*H*-indazol-1-yl)acetyl)-5-(hex-5-enamidomethyl)-*N*-(3-methyl-6-(trifluoromethyl)pyridin-2-yl)-2-azabicyclo[3.1.0]hexane-3-carboxamide (28-4):** To a mixture of compound **28-3** (25 mg, 0.08 mmol) and (1*R*,3*S*,5*R*)-5-(hex-5-enamidomethyl)-*N*-[3-methyl-6-(trifluoromethyl)pyridin-2-yl]-2-azabicyclo[3.1.0]hexane-3-carboxamide hydrochloride (29 mg, 0.067 mmol) in DMF (3 mL) was added HATU (30 mg, 0.08 mmol) followed by DIPEA (0.022 g, 0.167 mmol) at 0 °C and the mixture was stirred at room temperature for 2 hours. The mixture was diluted with EtOAc, washed with saturated aqueous NH₄Cl solution and brine, dried over anhydrous Na₂SO₄, filtered and concentrated to dryness. The residue was purified by column chromatography via silica gel (eluted with DCM: MeOH= 30: 1) to afford compound **28-4** (35 mg, yield 62.1%) as a yellow solid. LC/MS (ESI) m/z: 705 (M+H)⁺.

**Step 4: Compound 28-5:** To a solution of compound **28-4** (30 mg, 0.05 mmol) in degassed toluene (30 mL) was added Grubbs 2^{nd} catalyst (9 mg, 0.011 mmol) and the mixture was degassed again and stirred at 80°C under N₂ atmosphere for 16 hours. The mixture was concentrated to dryness and the residue was purified by silica gel chromatography (eluted with DCM: MeOH= 30: 1) to afford compound **28-5** (20 mg, yield 69.4%) as a light yellow solid. LC/MS (ESI) m/z: 677 (M+H)⁺.

**Step 5: Compound 28:** To a solution of compound **28-5** (20 mg, 0.03 mmol) in EtOAc (2 mL) was added PtO₂ (5 mg) and the mixture was degassed under N₂ three times and stirred under a H₂ balloon at room temperature for 1 hour. The mixture was filtered and the filtrate was concentrated to dryness. The residue product was purified by preparative HPLC to afford **Compound 28** (0.5 mg, yield 2.5%) as a white solid. ¹H-NMR (400 MHz, CD₃OD) δ 7.89 (s, 1H), 7.81 (d, *J=* 7.8 Hz, 1H), 7.56 (d, *J=* 7.7 Hz, 1H), 7.02 (s, 1H), 5.88 (d, *J=* 17.9 Hz, 1H), 5.57 (d, *J=* 17.7 Hz, 1H), 4.52 (t, J= 6.7 Hz, 1H), 3.62 (d, *J=* 3.6 Hz, 1H), 3.47 (d, J= 9.2 Hz, 1H), 3.35 (s, 1H), 3.13 (s, 1H), 3.02 (d, *J=* 11.9 Hz, 1H), 2.85-2.78 (m, 1H), 2.65 (s, 3H), 2.60-2.53 (m, 4H), 2.21-2.17 (m, 2H), 2.12 (s, 3H), 1.91-1.86 (m, 1H), 1.72 (s, 4H), 1.60 (s, 6H), 1.15 (dd, *J*= 5.4, 2.8 Hz, 2H). LC/MS (ESI) m/z: 681 (M+H)⁺.

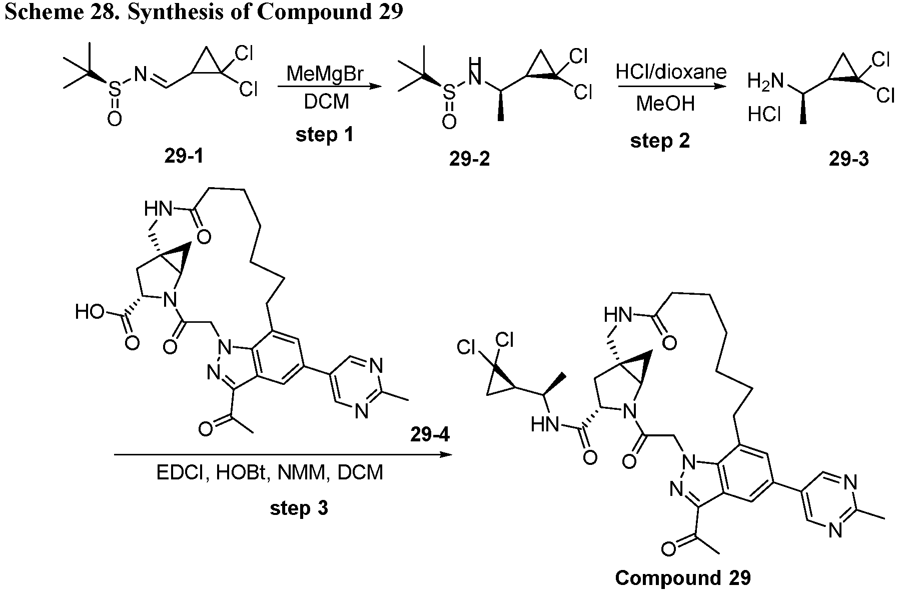

**Step 1: (*S*)-*N*-((*R*)-1-((*R*)-2, 2-Dichlorocyclopropyl)ethyl)-2-methylpropane-2-sulfinamide (29-2):** To a solution of compound **29-1** (230 mg, 0.95 mmol) prepared in the same manner as Scheme 29, Compound 30) in DCM (8 mL) was added methylmagnesium bromide (0.95 mL, 1.91 mmol, 2 M/L in THF) at -78 °C under N₂ atmosphere and the resulting mixture was stirred at this temperature for 3.5 hours and at room temperature for 16 hours. The mixture was quenched with saturated aqueous NH₄Cl solution and extracted with DCM twice. The combined organic layers were washed with brine, dried with anhydrous Na₂SO₄, filtered and concentrated to dryness. The residue was purified by chromatography on silica gel (eluted with PE: EtOAc= 20: 1 to 3: 1) to afford compound **29-2** (50 mg, yield 20.4%) as a light yellow oil. LC/MS (ESI) m/z: 258 (M+H) ⁺.

**Step 2: (*R*)-1-((*R*)-2, 2-Dichlorocyclopropyl)ethanamine hydrochloride (29-3):** To a solution of compound **29-2** (30 mg, 0.12 mmol) in MeOH (1 mL) was added HCl/1,4-dioxane solution (0.5 mL) at 0 °C and the mixture was stirred at room temperature overnight. The mixture was concentrated to dryness to afford compound **29-3** (22 mg, yield 100%) as a yellow solid that was used directly in the next step. LC/MS (ESI) m/z: 154 (M+H) ⁺.

**Step 3: Compound 29:** To a mixture of compound **29-3** (11 mg, 0.072 mmol) and compound 4 (8 mg, 0.048 mmol) in DCM (2 mL) was added NMM (24mg, 0.240 mmol), EDCI (14 mg,0.072 mmol) and HOBt (8 mg, 0.072 mmol) at 0 °C and the mixture was stirred at room temperature overnight. The mixture was diluted with DCM, washed with water and brine, dried over anhydrous Na₂SO₄, filtered and concentrated to dryness. The residue was purified by preparative HPLC to afford **Compound 29** (3 mg, yield 9.1%) as a white solid. ¹H-NMR (400 MHz, CD₃OD) δ 8.98 (s, 2H), 8.42 (d, J= 1.6 Hz, 1H), 7.53 (s, 1H), 5.90 (d, J= 18.0 Hz, 1H), 5.68-5.52 (m, 1H), 4.29 (dd, J= 9.2, 4.8 Hz, 1H), 3.73-3.59 (m, 1H), 3.53 (dd, J= 5.6, 2.8 Hz, 1H), 3.40 (d, J= 6.8 Hz, 1H), 3.22-3.06 (m, 2H), 2.92-2.83 (m, 1H), 2.74 (s, 3H), 2.69 (s, 3H), 2.52-2.40 (m, 2H), 2.39-2.28 (m, 2H), 1.93-1.72 (m, 6H), 1.68-1.58 (m, 2H), 1.56-1.51 (m, 1H), 1.50-1.42 (m, 1H), 1.39 (t, J= 6.0 Hz, 1H), 1.36-1.29 (m, 1H), 1.29-1.25 (m, 3H), 1.16 (dd, J= 6.0, 3.2 Hz, 1H). LC/MS (ESI) m/z: 694 (M+H)⁺.

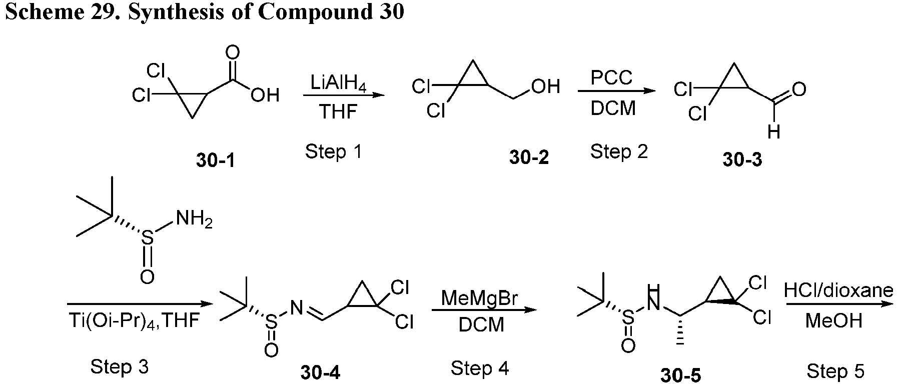

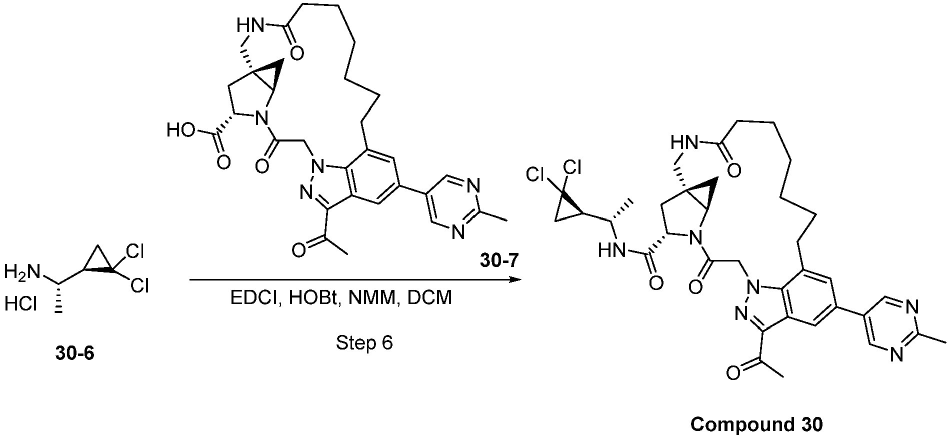

**Step 1: (2, 2-Dichlorocyclopropyl) methanol (30-2):** To a solution of LiAlH₄ (191 mg, 5.03 mmol) in diethyl ether (4 mL) was added a solution of compound 1 (600 mg, 3.87 mmol) in diethyl ether (6 mL) at 0 °C under N₂ atmosphere and the resulting mixture was stirred at room temperature for 6 hours. The mixture was quenched with water at 0 °C and extracted with diethyl ether twice. The combined organic layers were washed with brine, dried with anhydrous Na₂SO₄, filtered and concentrated to dryness. The residue was purified by chromatography on silica gel (eluted with PE: EtOAc= 10: 1 to 3: 1) to afford compound **30-2** (340 mg, yield 62.7%) as a light yellow oil. LC/MS (ESI) m/z: 139(M-H)⁻.

**Step 2: 2, 2-Dichlorocyclopropanecarbaldehyde (30-3):** To a solution of compound **30-2** (300 mg, 2.14 mmol) in DCM (6 mL) was added PCC (693 mg, 3.21 mmol) and the resulting mixture was stirred at room temperature for 6 hours. The mixture was diluted with DCM and washed with water and brine, dried over anhydrous Na₂SO₄, filtered and concentrated to afford compound **30-3** (220 mg, yield 74.4%) as a light yellow oil.

**Step 3: (*R*,*E*)-*N*-((2, 2-Dichlorocyclopropyl) methylene)-2-methylpropane-2-sulfinamide (30-4):** To a mixture of compound **30-3** (220 mg, 1.59 mmol) and (R)-2-methylpropane-2-sulfinamide (212 mg, 1.75 mmol) in THF (6 mL) was added tetraisopropoxytitanium (906 mg, 3.19 mmol) and the resulting mixture was stirred at 60 °C overnight. The mixture was quenched with ice-water and extracted with EtOAc twice. The combined organic layers were washed with brine, dried with anhydrous Na₂SO₄, filtered and concentrated to dryness. The residue was purified by chromatography on silica gel (eluted with PE: EtOAc= 9: 1 to 5: 1) to afford compound **30-4** (260 mg, yield 67.7%) as a light yellow oil. LC/MS (ESI) m/z: 242(M-H) ⁻.

**Step 4: (*R*)-*N*-((*S*)-1-((*R*)-2, 2-Dichlorocyclopropyl) ethyl)-2-methylpropane-2-sulfinamide (30-5):** To a solution of compound **30-4** (200 mg, 0.83 mmol) in DCM (8 mL) was added methylmagnesium bromide (0.83 mL, 1.66 mmol, 2 M/L in THF) at -78 °C under N₂ atmosphere and the resulting mixture was stirred at this temperature for 3.5 hours. The reaction was stirred at room temperature overnight under N₂ atmosphere. The mixture was quenched with saturated aqueous NH₄Cl solution and extracted with DCM twice. The combined organic layers were washed with brine, dried with anhydrous Na₂SO₄, filtered and concentrated to dryness. The residue was purified by chromatography on silica gel (eluted with PE: EtOAc= 20: 1 to 3: 1) to afford compound **30-5** (98 mg, yield 46.0%) as a light yellow oil. LC/MS (ESI) m/z: 258 (M+H)⁺.

**Step 5: (*S*)-1-((*R*)-2, 2-Dichlorocyclopropyl) ethanamine hydrochloride (30-6):** To a solution of compound **30-5** (45 mg, 0.175 mmol) in MeOH (2 mL) was added HCl/1,4-dioxane (1 mL) at 0 °C and the mixture was stirred at room temperature overnight. The mixture was concentrated to dryness to afford compound **30-6** (33 mg, yield 100%) as a yellow solid that was used directly in the next step. LC/MS (ESI) m/z: 154 (M+H)⁺.

**Step 6 : Synthesis of Compound 30:** To a mixture of compound **30-6** (10 mg, 0.067 mmol)and compound 7 (25 mg, 0.045 mmol) in DCM (2 mL) was added NMM (23mg, 0.224 mmol), EDCI (13 mg,0.067 mmol) and HOBt (9 mg, 0.067 mmol) at 0 °C and the mixture was stirred at room temperature overnight. The mixture was diluted with DCM and washed with water and brine, dried over anhydrous Na₂SO₄, filtered and concentrated to dryness. The residue was purified by preparative HPLC to afford **Compound 30** (4 mg, yield 12.9%) as a white solid. ¹H-NMR (400 MHz, CD₃OD) δ 9.00 (s, 2H), 8.43 (d, J= 1.6 Hz, 1H), 7.56 (s, 1H), 5.90 (d, J= 18.0 Hz, 1H), 5.59 (d, J= 18.0 Hz, 1H), 4.19 (dd, J= 8.8, 5.6 Hz, 1H), 3.71-3.59 (m, 1H), 3.55 (dd, J= 6.0, 2.8 Hz, 1H), 3.39 (s, 2H), 3.16-3.07 (m, 1H), 2.95-2.84 (m, 1H), 2.74 (s, 3H), 2.68 (s, 1H), 2.48-2.27 (m, 4H), 1.93-1.73 (m, 4H), 1.71-1.55 (m, 5H), 1.53-1.27 (m, 4H), 1.25 (d, J= 6.4 Hz, 1H), 1.14 (dd, J= 6.0, 3.2 Hz, 1H). LC/MS (ESI) m/z: 694 (M+H)⁺.

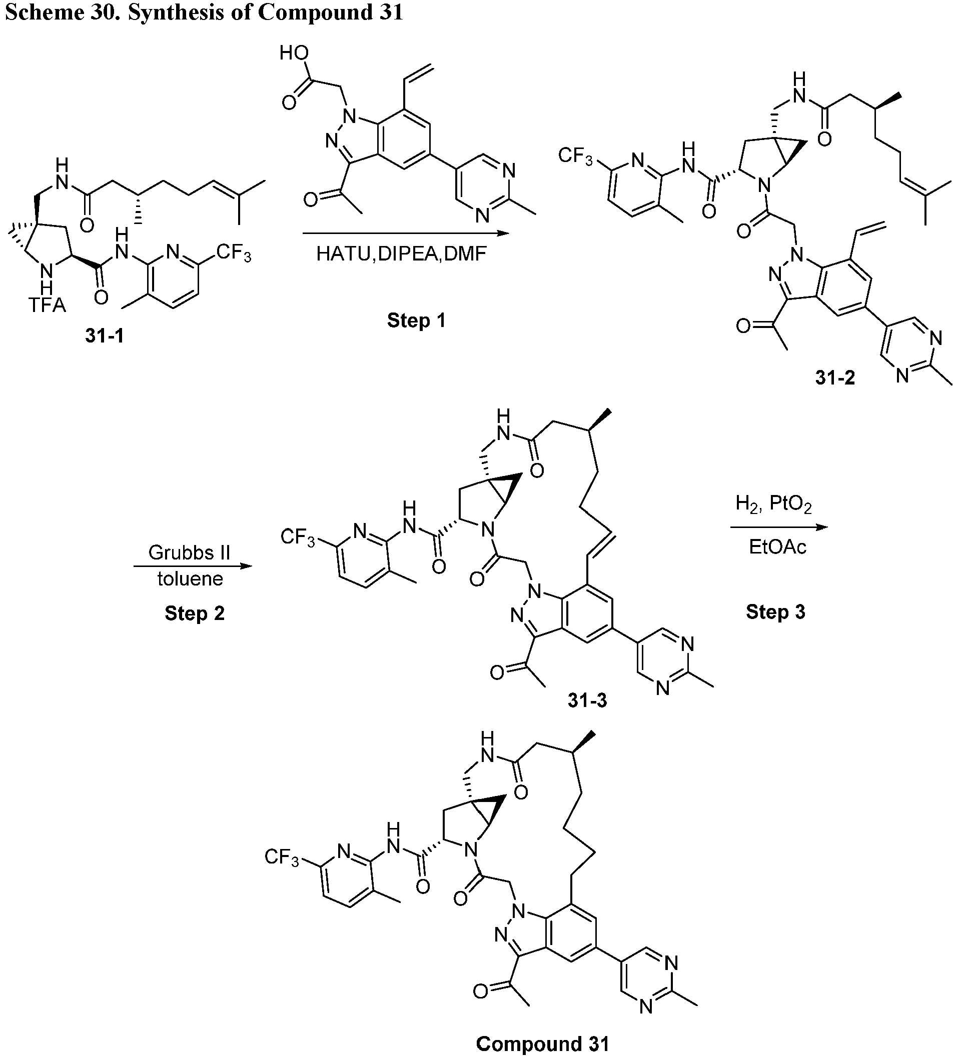

**Step 1: (1*R*,3*S*,5*R*)-2-(2-(3-Acetyl-5-(2-methylpyrimidin-5-yl)-7-vinyl-1*H*-indazol-1-yl)acetyl)-5-(((S)-3,7-dimethyloct-6-enamido)methyl)-*N*-(3-methyl-6-(trifluoromethyl)pyridin-2-yl)-2-azabicyclo[3.1.0]hexane-3-carboxamide (31-2):** To a mixture of 2-(3-acetyl-5-(2-methylpyrimidin-5-yl)-7-vinyl-1*H*-indazol-1-yl)acetic acid (63 mg, 0.19 mmol) and compound **31-1** (107 mg, 0.19 mmol, prepared in the same manner as Scheme 33 (Compound 34)) in DMF (3 mL) was added DIPEA (123 mg, 0.95 mmol) followed by HATU (108 mg, 0.29 mmol) at 0 °C and the mixture was stirred at room temperature for 1 hour. The mixture was diluted with EtOAc and washed with saturated aqueous NH₄Cl solution and brine, dried over anhydrous Na₂SO₄, filtered and concentrated to dryness. The residue was purified by chromatography on silica gel (eluted with DCM: MeOH= 100: 1 to 20: 1) to afford compound **31-2** (110 mg, yield 73.8%) as a white solid. LC/MS (ESI) m/z: 785 (M+H)⁺.

**Step 2: Compound 31-3:** To a solution of compound **31-2** (40 mg, 0.05 mmol) in degassed DCE (40 mL) was added Grubbs 2^{nd} catalyst (11 mg, 0.013 mmol) at 0 °C under N₂ atmosphere and the mixture was stirred at 50 °C under N₂ atmosphere overnight. The mixture was concentrated to dryness and the residue was purified by chromatography on silica gel (eluted with DCM: MeOH= 20: 1) to afford compound **31-3** (20 mg, yield 55.6%) as a brown solid. LC/MS (ESI) m/z: 729 (M+H)⁺.

**Step 3: Compound 31:** To a solution of compound **31-3** (20 mg, 0.027 mmol) in EtOAc (3 mL) was added Pd/C (6 mg, 10% wt) at 0 °C and the mixture was degassed under N₂ atmosphere three times and stirred under a H₂ balloon at room temperature for 20 minutes. The mixture was filtered and the filtrate was concentrated to dryness. The residue was purified by preparative HPLC to afford **Compound 31** (0.3 mg, yield 1.6%) as a white solid. ¹H-NMR (400 MHz, CD₃OD) δ 8.99 (s, 2H), 8.42 (s, 1H), 7.80 (d, J= 7.6 Hz, 1H), 7.54 (d, J= 7.6 Hz, 2H), 6.04 (d, J= 18.0 Hz, 1H), 5.60 (d, J= 18.0 Hz, 1H), 4.58 (s, 2H), 4.41 (t, J= 7.8 Hz, 1H), 3.79 (dd, J= 5.6, 5.6 Hz, 1H), 3.50-3.45 (m, 1H), 3.21-3.16 (m, 1H), 3.05-2.99 (m, 1H), 2.75 (s, 3H), 2.69 (s, 3H), 2.63 (dd, J= 13.2, 13.2 Hz, 1H), 2.55-2.46 (m, 2H), 2.18 (s, 3H), 1.97-1.88 (m, 3H), 1.80-1.64 (m, 4H), 1.24-1.17 (m, 1H), 1.12 (m, 1H), 1.02 (d, J= 6.0 Hz, 3H). LC/MS (ESI) m/z: 731 (M+H)⁺.

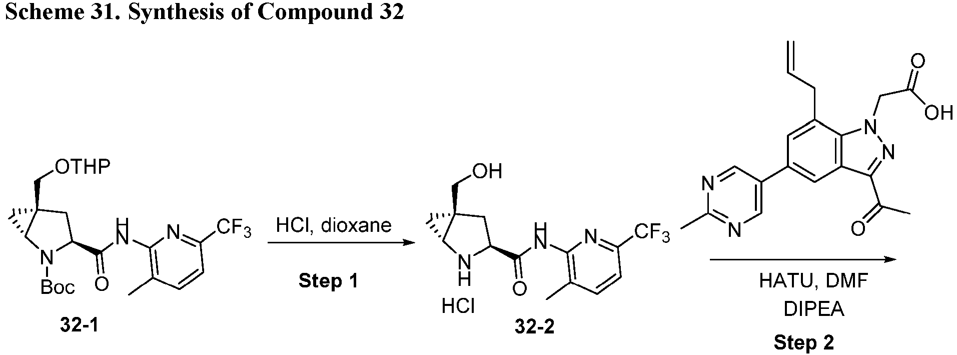

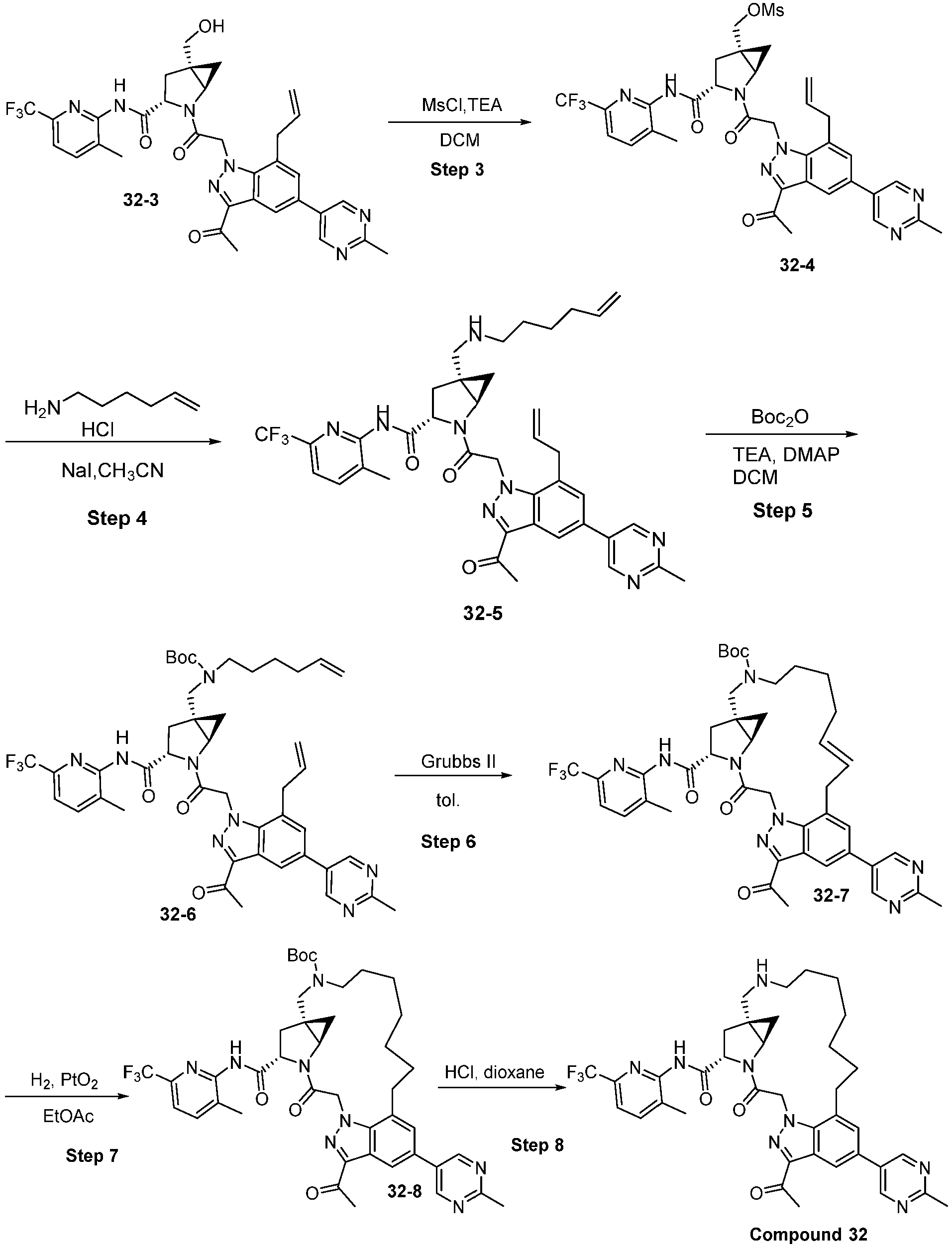

**Step 1: (1*S*,3*S*,5*R*)-5-(Hydroxymethyl)-*N*-(3-methyl-6-(trifluoromethyl)pyridin-2-yl)-2-azabicyclo[3.1.0]hexane-3-carboxamide hydrochloride (32-2):** A solution of compound **32-1** (280 mg, 0.56 mmol) in HCl/1,4-dioxane solution (4 mL, 4M) was stirred at 0 °C to room temperature for 1 hour. The reaction mixture was concentrated to dryness, washed with ether, and dried under vacuum to afford compound 32-**2** (176 mg, yield 89.3%) as a yellow solid. LC/MS (ESI) m/z: 316 (M+H)⁺.

**Step 2: (1*R*,3*S*,5*S*)-2-(2-(3-Acetyl-7-allyl-5-(2-methylpyrimidin-5-yl)-1*H*-indazol-1-yl)acetyl)-5-(hydroxymethyl)-*N*-(3-methyl-6-(trifluoromethyl)pyridin-2-yl)-2-azabicyclo[3.1.0]hexane-3-carboxamide (32-3):** To a mixture of compound **32-2** (176 mg, 0.50 mmol) and 2-(3-acetyl-7-allyl-5-(2-methylpyrimidin-5-yl)-1*H*-indazol-1-yl)acetic acid (196 mg, 0.50 mmol) in DMF (5 mL) was added DIPEA (322 mg, 2.5 mmol) followed by HATU (285 mg, 0.75 mmol) at 0 °C and the mixture was stirred at room temperature for 1 hour. The mixture was diluted with EtOAc, washed with saturated aqueous NH₄Cl solution and brine, dried over anhydrous Na₂SO₄, filtered and concentrated to dryness. The residue was purified by chromatography on silica gel (eluted with DCM: MeOH= 60: 1) to afford compound **32-3** (204 mg, yield 65.9%) as a white solid. LC/MS (ESI) m/z: 648 (M+H)⁺.

**Step 3: ((1*R*,3*S*,5*S*)-2-(2-(3-Acetyl-7-allyl-5-(2-methylpyrimidin-5-yl)-1*H*-indazol-1-yl)acetyl)-3-((3-methyl-6-(trifluoromethyl)pyridin-2-yl)carbamoyl)-2-azabicyclo[3.1.0]hexan-5-yl)methyl methanesulfonate (32-4):** To a solution of compound **32-3** (200 mg, 0.28 mmol) in DCM (10 mL) was added TEA (85 mg, 0.84 mmol), followed by the drop-wise addition of MsCl (48 mg, 0.42 mmol) at 0 °C. The reaction was stirred at room temperature for 1 hour. The mixture was poured into ice-water and extracted with DCM twice. The combined organic layers were washed with brine, dried over anhydrous Na₂SO₄, filtered and concentrated under reduced pressure to afford compound **32-4** (220 mg, crude) as a yellow solid that was directly used in the next step without further purification. LC/MS (ESI) m/z: 726 (M+H)⁺.

**Step 4: (1*R*,3*S*,5*R*)-2-(2-(3-Acetyl-7-allyl-5-(2-methylpyrimidin-5-yl)-1*H*-indazol-1-yl)acetyl)-5-((hex-5-en-1-ylamino)methyl)-*N*-(3-methyl-6-(trifluoromethyl)pyridin-2-yl)-2-azabicyclo[3.1.0]hexane-3-carboxamide (32-5):** To a solution of compound **32-4** (220mg, 0.42 mmol) in CH₃CN (5 mL) was added DIPEA (271 mg, 2.10 mmol), NaI (63 mg, 0.42 mmol) and hex-5-en-1-amine TFA salt (171 mg, 1.26 mmol) and the reaction mixture was stirred at 45 °C for 16 hours. The mixture was diluted with EtOAc, washed with water and brine, dried over Na₂SO₄, filtered and concentrated to dryness. The residue was purified by chromatography on silica gel (eluted with DCM: MeOH= 30: 1 to 15: 1) to afford compound **32-5** (268 mg, yield 87.6%) as a yellow solid. LC/MS (ESI) m/z: 729 (M+H)⁺.

**Step 5: *tert*-Butyl (((1*R*,3*S*,5*R*)-2-(2-(3-acetyl-7-allyl-5-(2-methylpyrimidin-5-yl)-1*H-*indazol-1-yl)acetyl)-3-((3-methyl-6-(trifluoromethyl)pyridin-2-yl)carbamoyl)-2-azabicyclo[3.1.0]hexan-5-yl)methyl)(hex-5-en-1-yl)carbamate (32-6):** To a solution of compound **32-5** (268 mg, 0.37 mmol) in DCM (5 mL) was added di-tert-butyl dicarbonate (121 mg, 0.56 mmol), DMAP (9 mg, 0.07 mmol) and triethylamine (112 mg, 1.11 mmol) and the mixture was stirred at room temperature for 2 hours. The mixture was diluted with DCM and washed with water and brine, dried and concentrated to dryness. The residue was purified by column chromatography on silica gel (eluted with DCM: MeOH = 30: 1 to 20: 1) to afford compound **32-6** (100 mg, yield 32.6%) as a yellow solid. LC/MS (ESI) m/z: 829 (M+H)⁺.

**Step 6: Compound 32-7:** To a solution of compound **32-6** (55 mg, 0.066 mmol) in degassed toluene (55 mL) was added Grubbs II catalyst (11 mg, 0.013 mmol) at 0 °C under N₂ atmosphere and the mixture was stirred at 80 °C overnight under N₂ atmosphere. The mixture was concentrated to dryness and the residue was purified by chromatography on silica gel (eluted with PE: EtOAc=3: 1 to 1: 2) to afford compound **32-7** (32 mg, yield 60.38%) as a brown solid. LC/MS (ESI) m/z: 801 (M+H)⁺.

**Step 7: Compound 32-8:** To a solution of compound **32-7** (32 mg, 0.04 mmol) in EtOAc (5 mL) was added 10% PtO₂ (15 mg) at 0 °C and the mixture was degassed under N₂ atmosphere three times and stirred under a H₂ balloon at room temperature for 2 hours. The mixture was filtered and the filtrate was concentrated to dryness. The residue was purified by chromatography on silica gel (eluted with PE: EtOAc=2: 1 to 1: 2) to afford compound **32-8** (30 mg, yield 93.5%) as a yellow solid. LC/MS (ESI) m/z: 803 (M+ H)⁺.

**Step 8: Synthesis of Compound 32:** To a bottom flask charged with compound **32-8** (30 mg, 0.037 mmol) was added HCl/1,4-dioxane (2 mL) at 0 °C and the mixture was stirred at room temperature for 1 hour. The mixture was concentrated to dryness. The residue was purified by preparative HPLC to afford **Compound 32** (4 mg, yield 15.4%) as a white solid. ¹H-NMR (400 MHz, CD₃OD) δ 9.00 (s, 2H), 8.44 (d, J= 1.6 Hz, 1H), 7.83 (d, J= 7.6 Hz, 1H), 7.64-7.47 (m, 2H), 5.90 (d, J= 17.6 Hz, 1H), 5.73 (d, J= 17.6 Hz, 1H), 4.58 (s, 1H), 3.97-3.88 (m, 1H), 3.57-3.46 (m, 1H), 3.27-3.17 (m, 2H), 3.06 -2.95 (m, 1H), 2.87-2.77 (m, 1H), 2.75 (s, 3H), 2.69 (s, 2H), 2.68-2.61 (m, 1H), 2.60-2.51 (m, 2H), 2.18 (s, 3H), 2.14-2.03 (m, 1H), 1.92 (m, 1H), 1.76-1.55 (m, 6H), 1.43 (m, 2H), 1.15 (t, J= 5.6 Hz, 1H), 1.12-1.06 (m, 1H). LC/MS (ESI) m/z: 703 (M+H)⁺.

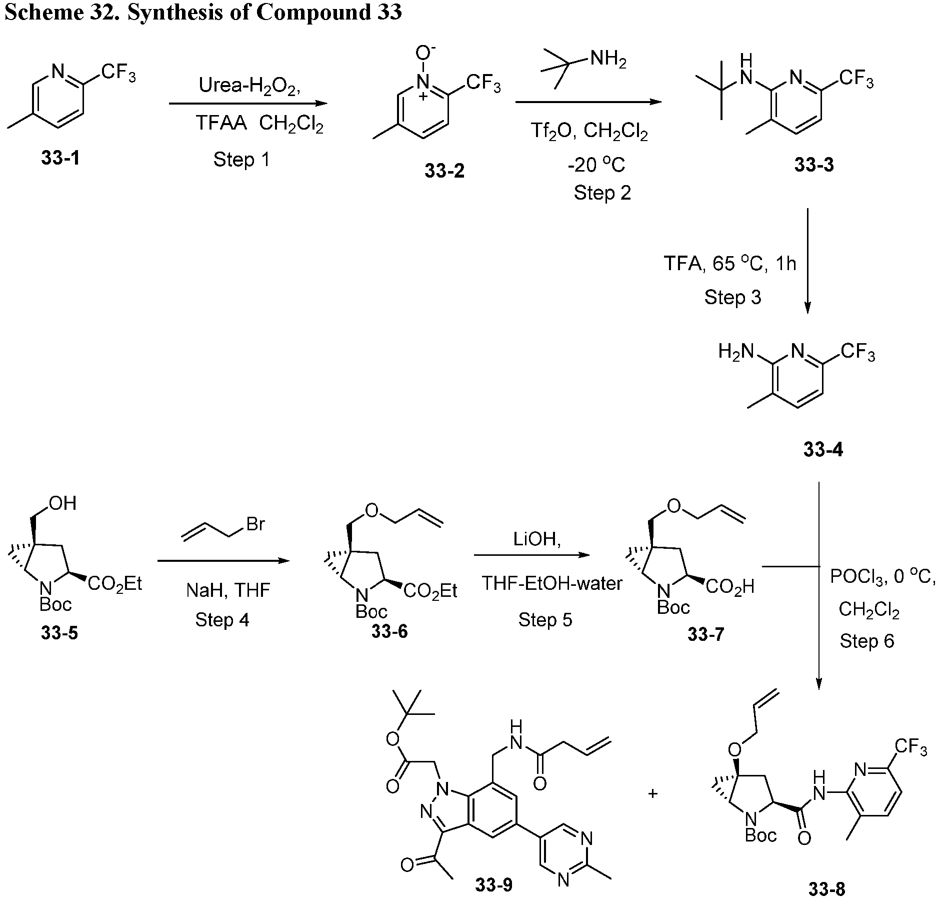

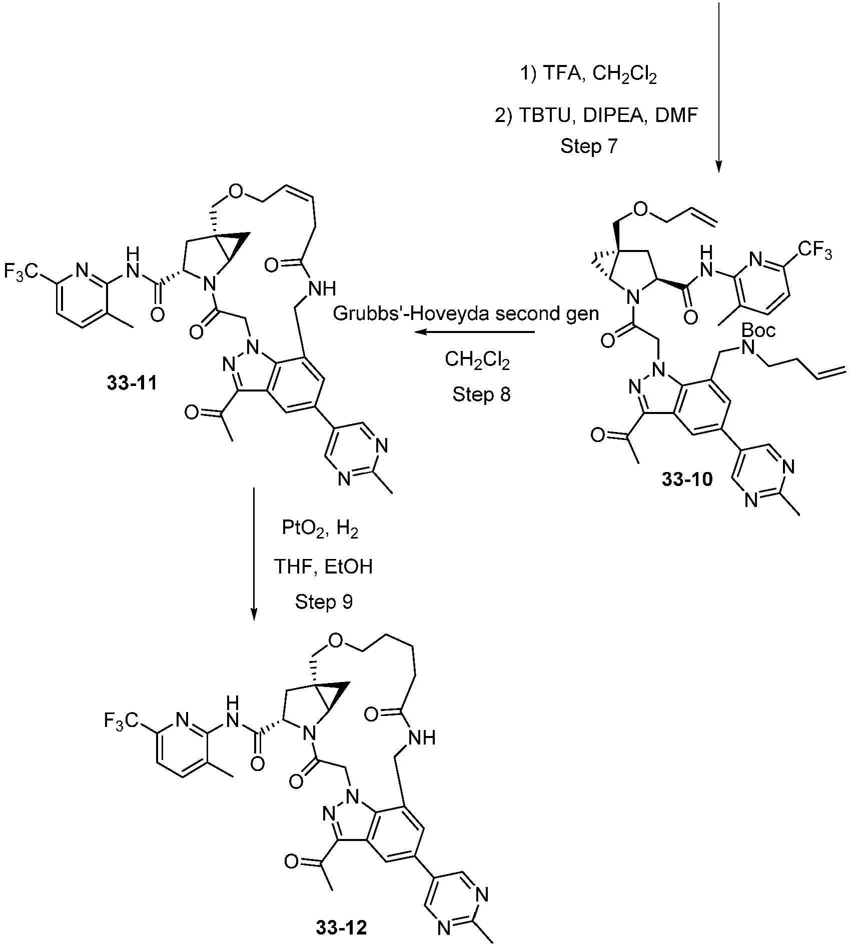

**Step 1: 5-Methyl-2-(trifluoromethyl)pyridine-*N*-oxide (33-2):** 5-Methyl-2-(trifluoromethyl)pyridine **(33-1,** 1 g, 6.206 mmol, 1 equiv.) was taken up in dichloromethane and the solution was cooled in an ice bath. Powdered urea hydrogen peroxide complex (0.899 g, 9.557 mmol, 1.54 equiv.) was added followed by the dropwise addition of trifluoroacetic anhydride (3.26 g, 2.158 mL, 15.522 mmol, 2.501 equiv.). The cooling bath was removed and the reaction was stirred overnight at room temperature. The reaction was quenched by the careful addition of saturated sodium metabisulfite solution and the reaction was stirred at room temperature for 15 minutes. The solution was diluted with dichloromethane and made basic by the careful addition of saturated aqueous sodium bicarbonate solution. The organic layers was separated and the aqueous layer was extracted with dichloromethane. The combined organic layers were dried and concentrated to afford an orange red oil that was used directly in the next step.

**Step 2: *N*-(tert-Butyl)-3-methyl-6-(trifluoromethyl)pyridin-2-amine (33-3):** To a stirred solution of 5-methyl-2-(trifluoromethyl)pyridine-*N*-oxide (**33-2,** 1.05 g, 5.928 mmol, 1 equiv.) in dichloromethane (25 mL) at -20 ° C was added *tert*-butylamine (3.21 mL). Triflic anhydride (3 mL) was added dropwise and the reaction was stirred at -20 ° C for 1 hour and quenched by the addition of water. The reaction was stirred at room temperature for 15 minutes. The layers were then separated and the aqueous layer was extracted once with dichloromethane. The combined organic layers was dried and concentrated. The residue was purified by silica gel column chromatography (eluent: hexane) to afford a colorless liquid.

**Step 3: 3-Methyl-6-(trifluoromethyl)pyridin-2-amine (33-4):** A solution of *N-tert-*butyl-3-methyl-6-(trifluoromethyl)pyridin-2-amine **(33-3,** 0.72 g, 3.1 mmol, 1 equiv.) in TFA was stirred at 65 °C for 1 hour. The reaction was cooled to room temperature and the volatiles were removed under reduced pressure. The residue was taken up in dichloromethane and made basic with saturated aqueous NaHCO₃ solution. The organic layers were separated, dried and concentrated to afford 0.49 g of a white solid that was used as such for the next step.

**Step 4. 2-(*tert*-Butyl) 3-ethyl (1R,3S,5S)-5-((allyloxy)methyl)-2-azabicyclo[3.1.0]hexane-2,3-dicarboxylate (33-6):** To an ice-cold solution of 2-tert-butyl 3-ethyl (1R,3S,5S)-5-(hydroxymethyl)-2-azabicyclo[3.1.0]hexane-2,3-dicarboxylate (**33-5**, 1.25 g, 4.381 mmol, 1 equiv.) in DMF (15 mL) was added sodium hydride portion-wise. The reaction was stirred at 0 °C for 15 minutes and allyl bromide (1.59 g, 1.137 mL, 13.142 mmol, 3 equiv.) was added dropwise. After the addition was complete, the reaction was stirred overnight at room temperature. The reaction was cooled in an ice-bath and quenched by the careful addition of water. The reaction was then extracted repeatedly with EtOAc. The combined organic layers were dried and concentrated. The residue was purified by silica gel flash column chromatography (eluent: 0-15 % EtOAc in hexanes) to afford 0.8 g of a colorless oil.

**Step 5. (1*R*,3*S*,5*S*)-5-((Allyloxy)methyl)-2-(*tert*-butoxycarbonyl)-2-azabicyclo[3.1.0]hexane-3-carboxylic acid (33-7):** 2-*tert*-Butyl 3-ethyl (1R,3S,5S)-5-[(prop-2-en-1-yloxy)methyl]-2-azabicyclo[3.1.0]hexane-2,3-dicarboxylate (**33-6**, 0.8 g, 2.458 mmol, 1 equiv.) was taken up in THF (4 mL) and EtOH (2 mL). The solution was cooled in an ice bath. LiOH (0.124 g) was dissolved in water (2 mL) and added dropwise to the ice cooled solution. The cooling bath was removed and the reaction was stirred at room temperature for 2 hours. The organic solvents were removed under reduced pressure and 5 mL of water was added. The reaction was once extracted with ether and the organic layers were discarded. The aqueous layer was cooled in an ice bath and acidified with cold 1*N* aqueous HCl. The acidified aqueous layer was extracted with dichloromethane-MeOH (20:1). The organic layer was dried and concentrated to afford 0.7 g of acid as a white solid.

**Step 6. *tert*-Butyl (1*R*,3*S*,5*S*)-5-(allyloxy)-3-((3-methyl-6-(trifluoromethyl)pyridin-2-yl)carbamoyl)-2-azabicyclo[3.1.0]hexane-2-carboxylate (33-8):** To an ice cold solution of 3-methyl-6-(trifluoromethyl)pyridin-2-amine (0.436 g) and (1*R*,3*S*,5*S*)-5-(allyloxy)-2-(*tert-*butoxycarbonyl)-2-azabicyclo[3.1.0]hexane-3-carboxylic acid (**33-7**, 0.7 g) in dichloromethane (15 mL), pyridine (0.9 mL) was added followed by the dropwise addition of POCl₃ (0.21 mL). The stirring was continued at 0 ° C for 1 hour before the reaction was quenched by the addition of saturated aqueous NaHCO₃ solution. The cooling bath was removed and the reaction was stirred at room temperature for 15 minutes. The layers were separated and the organic layer was washed with cold 1*N* aqueous HCl and water. The organic layer was dried and concentrated to afford 0.85 g of a white solid that was used as such for the next step.

**Step 7. *tert*-Butyl ((3-acetyl-1-(2-((1*R*,3*S*,5*S*)-5-((allyloxy)methyl)-3-((3-methyl-6-(trifluoromethyl)pyridin-2-yl)carbamoyl)-2-azabicyclo[3.1.0]hexan-2-yl)-2-oxoethyl)-5-(2-methylpyrimidin-5-yl)-1*H*-indazol-7-yl)methyl)(but-3-en-1-yl)carbamate (33-10):** *tert*-Butyl 2-[3-acetyl-7-(but-3-enamidomethyl)-5-(2-methylpyrimidin-5-yl)indazol-1-yl]acetate (**33-9**, 0.03 g, 0.065 mmol, 1 equiv.) was stirred with TFA (1 mL) and dichloromethane (0.5 mL) in a vial for 3 hours at room temperature. In another vial, *tert*-butyl (1*R*,3*S*,5*S*)-3-{[3-methyl-6-(trifluoromethyl)pyridin-2-yl]carbamoyl}-5-[(prop-2-en-1-yloxy)methyl]-2-azabicyclo[3.1.0]hexane-2-carboxylate (**33-8**, 0.032 g, 0.071 mmol, 1.1 equiv.) was stirred at room temperature with TFA (0.5 mL) and dichloromethane (0.5 mL) for 15 minutes. The contents from both the vials were combined and the volatiles were removed. The residue was taken up in DMF (1 mL) and the vial was cooled in an ice-bath. The solution was made basic with DIPEA (56 µL) and TBTU (0.023 g) was added. The cooling bath was removed and the reaction was stirred at room temperature for 1 hour. DMF was removed under reduced pressure and the residue was dissolved in dichloromethane. The organic layer was washed with saturated aqueous NaHCO₃ solution, dried (Na₂SO₄) and concentrated. The crude product was purified by silica gel column chromatography (eluent: 0-3.5 % MeOH in dichloromethane) to afford 30 mg of a cream colored solid.

**Step 8. Compound 33-11:** To a solution of (1*R*,3*S*,5*S*)-2-{2-[3-acetyl-7-(but-3-enamidomethyl)-5-(2-methylpyrimidin-5-yl)indazol-1-yl]acetyl}-*N*-[3-methyl-6-(trifluoromethyl)pyridin-2-yl]-5-[(prop-2-en-1-yloxy)methyl]-2-azabicyclo[3.1.0]hexane-3-carboxamide (**33-10**, 0.03 g, 0.04 mmol, 1 equiv.) in dichloromethane (5 mL) was added solid Hoveyda-Grubbs' 2nd gen catalyst (0.003 g, 0.004 mmol, 0.099 equiv.) and the solution was degassed with argon. The reaction was stirred at room temperature overnight. Additional catalyst (2.5 mg) was added and the reaction was stirred for 2 hours. The solvent was removed under reduced pressure and the residue was purified by silica gel column chromatography (eluent: 0-3 % MeOH in dichloromethane) to afford 33-11 mg of a white solid as a mixture of *cis* and *trans* isomers.

**Step 9. Compound 33:** Compound **33-11** (0.011 g, 0.015 mmol, 1 equiv.) was taken up in THF (1 mL)-EtOH (1 mL) and stirred under a hydrogen atmosphere in presence of PtO₂ for 90 minutes. The solvent was removed under reduced pressure and the residue was purified by silica gel column chromatography (eluent: 0-3.5 % MeOH in dichloromethane) to afford **Compound 33** as a white solid. ¹H NMR (400 MHz, Chloroform-*d*) δ 1.01 (dd, *J* = 2.9, 5.8 Hz, 1H), 1.25-1.28 (m, 2H), 1.48-1.61 (m, 1H), 1.63-1.74 (m, 1H), 1.92-2.03 (m, 1H), 2.05-2.14 (m, 1H), 2.18-2.29 (m, 1H), 2.22 (s, 3H), 2.43 - 2.52 (m, 1H), 2.73 (s, 3H), 2.80 (s, 3H), 3.02 (d, *J* = 11.3 Hz, 1H), 3.17 (d, *J =* 13.6 Hz, 1H), 3.34 - 3.45 (m, 2H), 3.67-3.73 (m, 1H), 4.07 (d, *J =* 11.3 Hz, 1H), 4.48 (d, *J =* 14.8 Hz, 1H), 4.83 (d, *J* = 8.7 Hz, 1H), 5.32-5.42 (m, 1H), 5.53 (d, *J =* 17.5 Hz, 1H), 5.86 (d, *J =* 17.5 Hz, 1H), 7.46 - 7.54 (m, 2H), 7.67 (brs, 1H), 7.71 (d, J = 7.6 Hz, 1H), 8.59 (d, *J =* 1.6 Hz, 1H), 8.87 (s, 2H), 9.61 (s, 1H). ¹⁹F: δ -66.9.

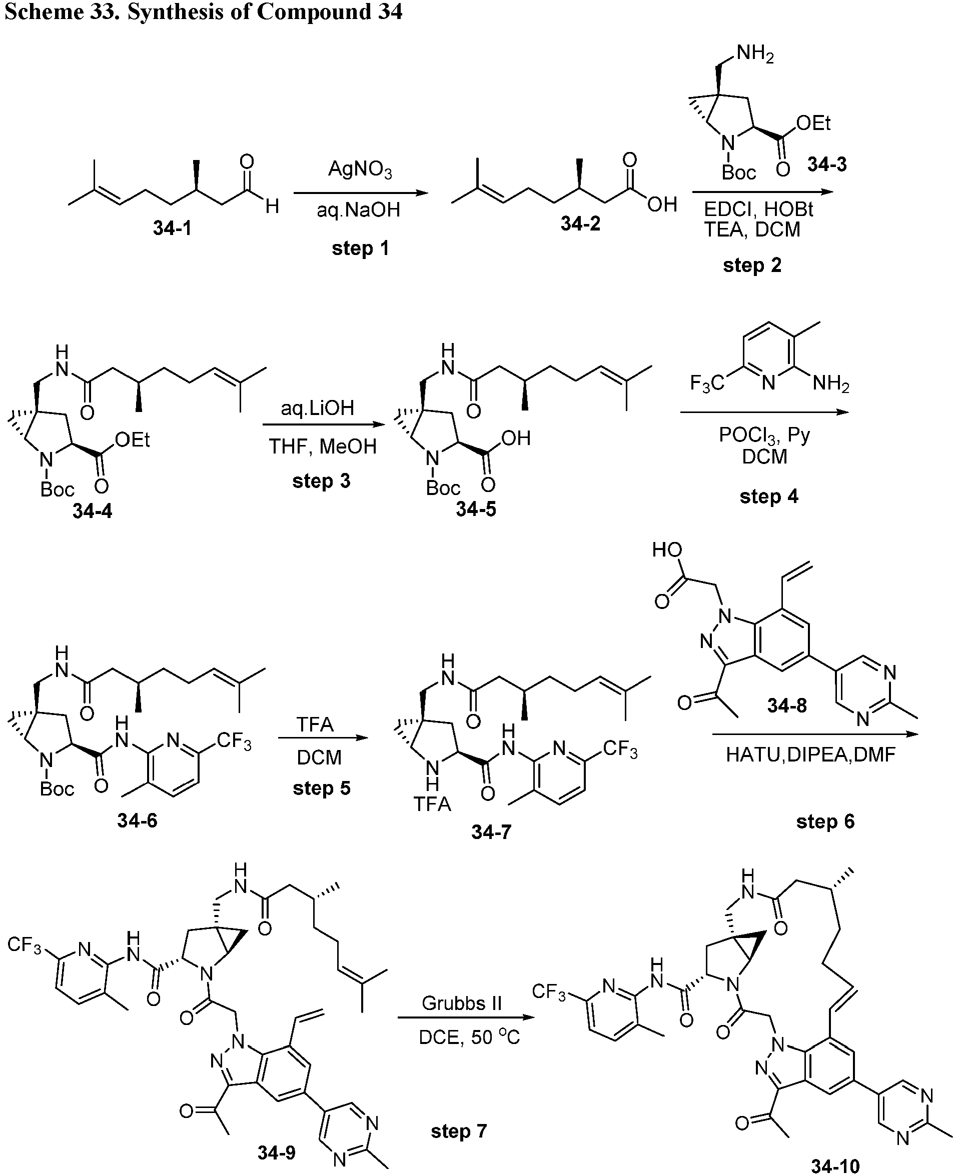

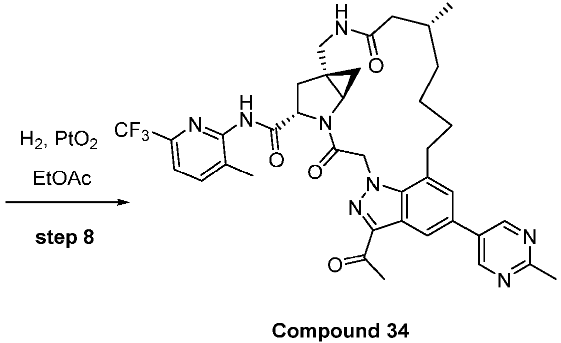

**Step 1: (*R*)-3, 7-Dimethyloct-6-enoic acid (34-2):** To a solution of NaOH (1.272 g, 31.82 mmol) in water (26 mL) was added a drop-wise addition of a solution of AgNO₃ (2.539 g, 14.93 mmol) in water (26 mL) at 0 °C and the mixture was stirred at room temperature for 30 minutes. Compound **34-1** (1.0 g, 6.49 mmol) was added drop-wise and the mixture was stirred at room temperature overnight. The mixture was filtered and the filtrate was acidified with 4 N aqueous HCl solution and extracted with EtOAc twice. The combined organic layers were washed with brine, dried with anhydrous Na₂SO₄, filtered and concentrated to dryness. The residue was purified by chromatography on silica gel (eluted with PE: EtOAc=10: 1 to 8: 1) to afford compound **34-2** (880 mg, yield 80.01%) as a yellow oil. LC/MS (ESI) m/z: 171 (M+H)⁺.

**Step 2: (1*R*, 3*S*, 5*R*)-2-*tert*-Butyl 3-ethyl 5-(((R)-3, 7-dimethyloct-6-enamido) methyl)-2- azabicyclo [3.1.0] hexane-2, 3-dicarboxylate (34-4):** To a mixture of compound **34-2** (300 mg, 1.76 mmol) and compound **34-3** (501 mg, 1.76 mmol) in DCM (6 mL) was added TEA ( 891 mg, 8.82 mmol), EDCI (507 mg, 2.65 mmol) and HOBt (357 mg, 2.65 mmol) at 0 °C and the mixture was stirred at room temperature overnight. The mixture was diluted with water and extracted with EtOAc twice. The combined organic layers were washed with brine, dried with anhydrous Na₂SO₄, filtered and concentrated to dryness. The residue was purified by chromatography on silica gel (eluted with PE: EtOAc=10: 1 to 8: 1) to afford compound **34-4** (300 mg, yield 39.01%) as a yellow oil. LC/MS (ESI) m/z: 437 (M+H)⁺.

**Step 3: (1*R*,3*S*,5*R*)-2-(*tert*-Butoxycarbonyl)-5-(((*R*)-3, 7-dimethyloct-6-enamido) methyl)-2- azabicyclo [3.1.0] hexane-3-carboxylic acid (34-5):** To a solution of compound **34-4** (300 mg, 0.69 mmol) in methanol (1 mL) and THF (1 mL) was added a solution of LiOH (87 mg, 2.06 mmol) in water (1 mL) at 0 °C and the mixture was stirred at room temperature overnight. The mixture was washed with Et₂O twice and the aqueous layer was acidified with 0.5 M aqueous HCl solution and extracted with EtOAc twice. The combined organic layers were washed with brine, dried with anhydrous Na₂SO₄, filtered and concentrated to dryness to afford compound **34-5** (250 mg, yield 89.06%) as a white solid. LC/MS (ESI) m/z: 409 (M+H)⁺.

**Step 4: (1*R*,3*S*,5*R*)-*tert*-Butyl 5-(((R)-3, 7-dimethyloct-6-enamido) methyl)-3-(3-methyl-6-(trifluoromethyl) pyridin-2-ylcarbamoyl)-2-azabicyclo [3.1.0] hexane-2-carboxylate (34-6):** To a mixture of compound **34-5** (232 mg, 0.57 mmol) and 3-methyl-6-(trifluoromethyl)pyridin-2-amine (100 mg, 0.57 mmol) in DCM (6 mL) was added pyridine (224 mg, 2.84 mmol) followed by phosphoryl chloride (96 mg, 0.63 mmol) at 0 °C and the mixture was stirred at room temperature under N₂ atmosphere for 1 hour. The mixture was poured into ice-water and extracted with DCM twice. The combined organic layers were washed with brine, dried with anhydrous Na₂SO₄, filtered and concentrated to dryness. The residue was purified by chromatography on silica gel (eluted with PE: EtOAc= 10: 1 to 2: 1) to afford compound **34-6** (170 mg, yield 52.8%) as a white solid. LC/MS (ESI) m/z: 567 (M+H)⁺.

**Step 5: (1*R*,3*S*,5*R*)-5-(((*R*)-3,7-Dimethyloct-6-enamido) methyl)-N-(3-methyl-6-(trifluoromethyl) pyridin-2-yl)-2-azabicyclo [3.1.0] hexane-3-carboxamide (34-7):** To a solution of compound **34-6** (0.17 g, 0.30 mmol) in DCM (3 mL) was added TFA (1.5 mL) at 0 °C and the mixture was stirred at room temperature for 2 hours. The reaction mixture was concentrated to dryness, washed with ether and dried under vacuum to afford compound **34-7** (0.13 g, yield 92.8%) as a yellow oil that was used directly in the next step. LC/MS (ESI) m/z: 467 (M+H)⁺.

**Step 6: (1*R*,3*S*,5*R*)-2-(2-(3-Acetyl-5-(2-methylpyrimidin-5-yl)-7-vinyl-1*H*-indazol-1-yl) acetyl)-5-(((*R*)-3,7-dimethyloct-6-enamido) methyl)-N-(3-methyl-6-(trifluoromethyl) pyridin-2-yl)-2-azabicyclo [3.1.0] hexane-3-carboxamide (34-9):** To a mixture of compound **34-7** (130 mg, 0.28 mmol) and compound 8 (94 mg, 0.28 mmol) in DMF (2 mL) was added DIPEA (180 mg, 1.40 mmol), followed by HATU (159 mg, 0.42 mmol) at 0 °C and the mixture was stirred at room temperature for 1 hour. The mixture was diluted with water and extracted with EtOAc twice. The combined organic layers were washed with 10% aqueous LiCl solution and brine, dried with anhydrous Na₂SO₄, filtered and concentrated to dryness. The residue was purified by chromatography on silica gel (eluted with DCM: MeOH= 80: 1 to 30: 1) to afford compound **34-9** (68 mg, yield 31.1%) as a yellow solid. LC/MS (ESI) m/z: 785 (M+H)⁺.

**Step 7: Compound 34-10:** To a solution of compound **34-9** (50 mg, 0.064 mmol) in degassed DCE (50 mL) was added Grubbs 2^{nd} catalyst (13 mg, 0.016 mmol) at 0 °C under N₂ atmosphere and the mixture was stirred at 70 °C under N₂ atmosphere overnight. The mixture was concentrated to dryness and the residue was purified by chromatography on silica gel (eluted with DCM: MeOH= 100: 1 to 30: 1) to afford compound **34-10** (10 mg, yield 21.5%) as a brown solid. LC/MS (ESI) m/z: 729 (M+H)⁺.

**Step 8: Compound 34:** To a solution of compound **34-10** (10 mg, 0.014 mmol) in EtOAc (3 mL) was added 10% Pd/C (5 mg) at 0 °C and the mixture was degassed under N₂ atmosphere three times and stirred under a H₂ balloon at room temperature for 0.5 hour. The mixture was filtered and the filtrate was concentrated to dryness. The residue was purified by preparative HPLC to afford **Compound 34** (2 mg, yield 19.98%) as a white solid. ¹H-NMR (400 MHz, DMSO-d₆) δ 10.43 (s, 1H), 9.01 (s, 2H), 8.29 (s, 1H), 8.22-8.14 (m, 1H), 7.89 (d, J= 7.6 Hz, 1H), 7.64 (d, J= 7.6 Hz, 1H), 7.54 (s, 1H), 5.89 (d, J= 17.6 Hz, 1H), 5.50 (d, J= 17.6 Hz, 1H), 4.54-4.40 (m, 1H), 4.40-4.30 (m, 1H), 3.64-3.53 (m, 1H), 3.14-3.08 (m, 2H), 2.93-2.86 (m, 1H), 2.68 (s, 3H), 2.65-2.61 (m, 3H), 2.28 -2.14 (m, 2H), 2.10 (s, 3H), 2.01-1.91 (m, 2H), 1.87-1.80 (m, 1H), 1.74-1.63 (m, 2H), 1.61-1.47 (m, 2H), 1.43-1.34 (m, 2H), 1.22-1.18 (m, 1H), 1.10-1.07 (m, 1H), 0.91 (d, J= 6.0 Hz, 3H). LC/MS (ESI) m/z: 731 (M+H)⁺.

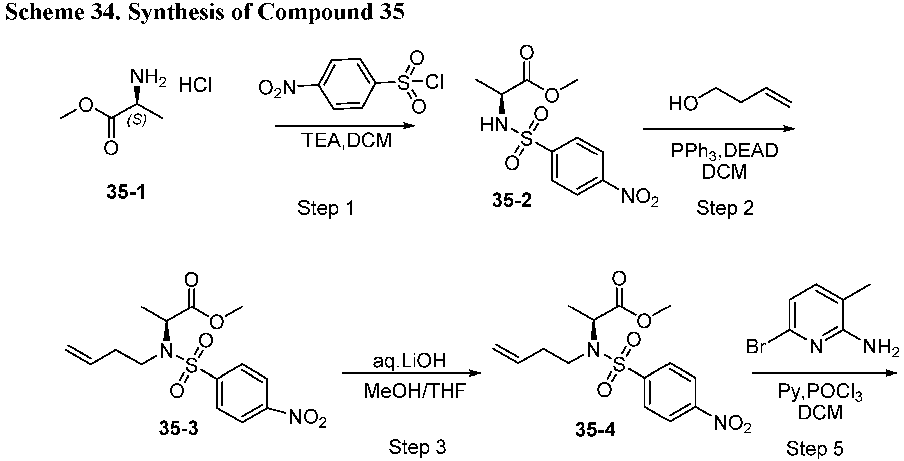

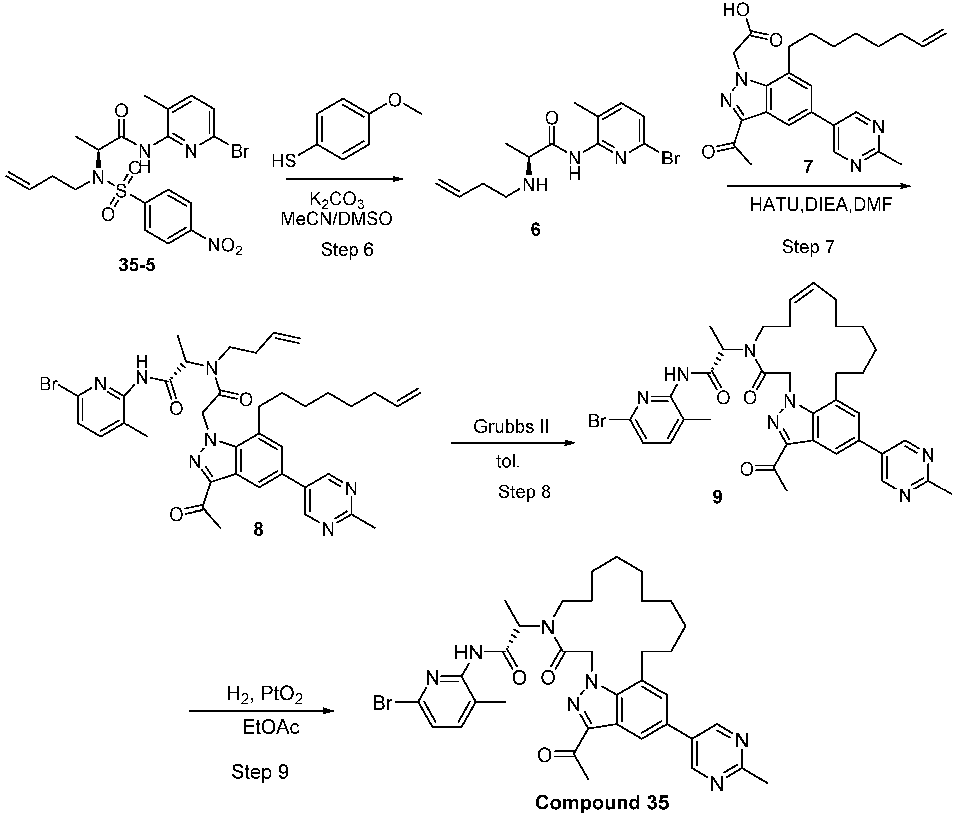

**Step 1: (*S*)-Methyl 2-(4-nitrophenylsulfonamido)propanoate (35-2):** To a solution of compound **35-1** (580 mg, 4.16 mmol) in DCM (10 mL) was added TEA (1.26 g, 12.48 mmol) followed by 4-nitrobenzene-1-sulfonyl chloride (1.01 g, 4.57 mmol) at 0 °C and the mixture was stirred at room temperature for 16 hours. The mixture was diluted with DCM, washed with brine, dried over anhydrous Na₂SO₄, filtered and concentrated to dryness. The residue was purified by flash chromatography (eluted with PE: EtOAc= 100: 1 to 1: 1) to afford compound 35-2 (720 mg, yield 60.1%) as a yellow solid. LC/MS (ESI) m/z: 289 (M+H)⁺.

**Step 2: (*S*)-Methyl 2-(*N*-(but-3-en-1-yl)-4-nitrophenylsulfonamido)propanoate (35-3):** To a solution of compound 35-2 (680 mg, 2.36 mmol) in DCM (10 mL) was added PPh₃ (805 mg, 3.07 mmol) and but-3-en-1-ol (224 mg, 3.07 mmol) followed by the drop-wise addition of DEAD (534 mg, 3.07 mmol) at 0 °C. The mixture was stirred at room temperature for 16 hours. The mixture was diluted with DCM and washed with water and brine, dried over anhydrous Na₂SO₄, filtered and concentrated to dryness. The residue was purified by flash chromatography (eluted with PE: EtOAc= 100: 1 to 3: 1) to afford compound **35-3** (670 mg, yield 83.0%) as a light oil. LC/MS (ESI) m/z: 343 (M+H)⁺.

**Step 3: (*S*)-2-(*N*-(But-3-en-1-yl)-4-nitrophenylsulfonamido)propanoic acid (35-4):** To a solution of compound **35-3** (670 mg, 1.96 mmol) in methanol (4 mL) and THF (2 mL) was added a solution of LiOH (247 mg, 5.87 mmol) in water (2 mL) at 0 °C and the mixture was stirred at room temperature for 1 hour. The mixture was diluted with water and washed with ether. The aqueous layer was acidified with 1N aqueous HCl solution to a pH of approximately 3 and extracted with DCM twice. The combined organic layers were washed with brine, dried with anhydrous Na₂SO₄, filtered and concentrated to dryness to afford compound **35-4** (508 mg, yield 79.0%) as a white solid. LC/MS (ESI) m/z: 329 (M+H)⁺.

**Step 4: (*S*)-*N*-(6-Bromo-3-methylpyridin-2-yl)-2-(*N*-(but-3-en-1-yl)-4-nitrophenylsulfonamido)propanamide (35-5):** To a mixture of compound **35-4** (508 mg, 1.55 mmol) and 6-bromo-3-methylpyridin-2-amine (288 mg, 1.55 mmol) in DCM (8 mL) was added pyridine (612 mg, 7.75 mmol) followed by the drop-wise addition of POCl₃ (261 mg, 1.71 mmol) at 0 °C and the mixture was stirred at room temperature under N₂ atmosphere for 2 hours. The mixture was poured into ice-water and extracted with DCM twice. The combined organic layers were washed with brine, dried with anhydrous Na₂SO₄, filtered and concentrated to dryness. The residue was purified by flash chromatography (eluted with PE: EtOAc= 50: 1 to 1: 1) to afford compound **35-5** (560 mg, yield 72.8%) as a white solid. LC/MS (ESI) m/z: 497/499 (M+H)⁺.

**Step 5: (*S*)-*N*-(6-Bromo-3-methylpyridin-2-yl)-2-(but-3-en-1-ylamino)propanamide (35-6):** To a solution of compound 35-5 (560 mg, 1.13 mmol) in MeCN/DMSO (10 mL, v/v=49/1) was added K₂CO₃(624 mg, 4.52 mmol) followed by 4-methoxybenzenethiol (475 mg, 3.39 mmol) at 0 °C and the mixture was stirred at room temperature for 4 hours. The mixture was diluted with DCM and washed with brine, dried over anhydrous Na₂SO₄, filtered and concentrated to dryness. The residue was purified by flash chromatography (eluted with PE: EtOAc= 10: 1 to 1: 4) to afford compound **35-6** (284 mg, yield 80.9%) as a light yellow oil. LC/MS (ESI) m/z: 312/314 (M+H)⁺.

**Step 6: (*S*)-2-(2-(3-Acetyl-5-(2-methylpyrimidin-5-yl)-7-(oct-7-en-1-yl)-1*H*-indazol-1-yl)-N-(but-3-en-1-yl)acetamido)-N-(6-bromo-3-methylpyridin-2-yl)propanamide (35-8):** To a mixture of compound **35-6** (67 mg, 0.21 mmol) and compound 7 (90 mg, 0.21 mmol) in DMF (5 mL) was added DIPEA (135 mg, 1.05 mmol) followed by HATU (122 mg, 0.32 mmol) at 0 °C and the mixture was stirred at room temperature for 2 hours. The mixture was diluted with EtOAc and washed with saturated aqueous NH₄Cl solution and brine, dried over anhydrous Na₂SO₄, filtered and concentrated to dryness. The residue was purified by flash chromatography (eluted with DCM: MeOH= 100: 1 to 20: 1) to afford compound **35-8** (30 mg, yield 20.1%) as a white solid. LC/MS (ESI) m/z: 714/716 (M+H)⁺.

**Step 7: Compound 35-9:** To a solution of compound **35-8** (30 mg, 0.04 mmol) in degassed toluene (30 mL) was added Grubbs 2^{nd} catalyst (9 mg, 0.01 mmol) under N₂ atmosphere and the mixture was stirred at 80 °C under N₂ atmosphere for 16 hours. The mixture was concentrated to dryness and the residue was purified by flash chromatography (eluted with DCM: MeOH= 100: 1 to 20: 1) to afford compound **35-9** (24 mg, yield 88.9%) as a brown solid. LC/MS (ESI) m/z: 686/688 (M+H)⁺.

**Step 8: Compound 35:** To a solution of compound **35-9** (24 mg, 0.035 mmol) in EtOAc (5 mL) was added PtO₂ (7 mg) at 0 °C and the mixture was degassed under N₂ atmosphere three times and stirred under a H₂ balloon at room temperature for 15 minutes. The mixture was filtered and the filtrate was concentrated to dryness. The residue was purified by preparative HPLC to afford **Compound 35** (1.9 mg, yield 7.92%) as a white solid. ¹H-NMR (400 MHz, CD₃OD) δ 9.00 (s, 2H), 8.45 (d, J= 1.6 Hz, 1H), 7.58 (d, J= 2.0 Hz, 1H), 7.55 (d, J= 8.0 Hz, 1H), 7.38 (d, J= 7.6 Hz, 1H), 5.81 (d, J= 17.2 Hz, 1H), 5.70 (d, J= 17.2 Hz, 1H), 4.74 (d, J= 7.2 Hz, 1H), 3.63 (tt, J= 7.6, 4.4 Hz, 2H), 2.95 (q, J= 6.6 Hz, 2H), 2.75 (s, 3H), 2.65 (s, 3H), 2.08 (s, 3H), 1.83-1.70 (m, 4H), 1.63 (d, J= 7.2 Hz, 3H), 1.61-1.39 (m, 12H). LC/MS (ESI) m/z: 688/690 (M+H)⁺.

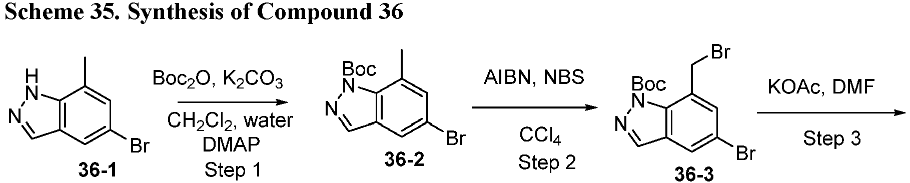

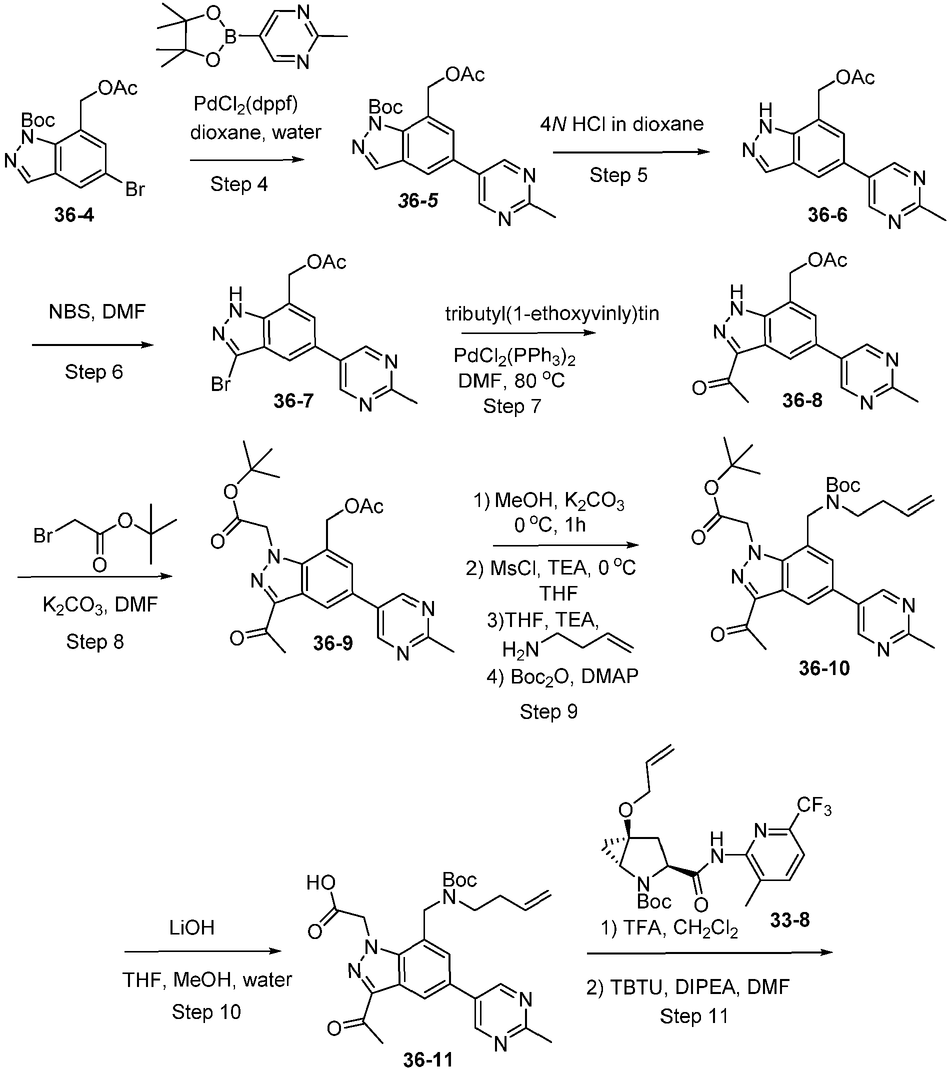

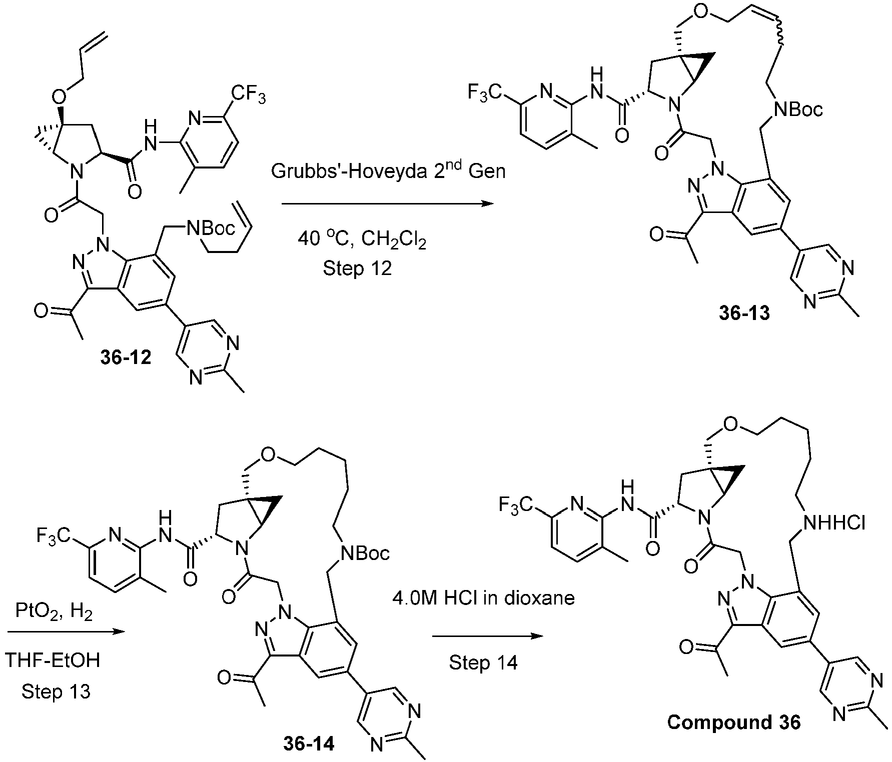

**Step 1: *tert*-Butyl 5-bromo-7-methyl-1*H*-indazole-1-carboxylate (36-2):** A solution of 5-bromo-7-methyl-1*H*-indazole (**36-1**, 45 g), Boc₂O (55.8 g), potassium carbonate (5.9 g) and DMAP (0.26 g) in dichloromethane (900 mL) and water (90 mL) was stirred overnight at room temperature. The layers were separated and the organic layer was dried and concentrated. The residue was purified by silica gel column chromatography to afford the title compound.

**Step 2: *tert*-Butyl 5-bromo-7-(bromomethyl)-1*H*-indazole-1-carboxylate (36-3):** A heterogeneous mixture of tert-butyl 5-bromo-7-methylindazole-1-carboxylate (**36-2**, 0.2 g, 0.643 mmol, 1 equiv.), NBS (0.114 g, 0.641 mmol, 0.997 equiv.) and AIBN (0.017 g, 0.104 mmol, 0.161 equiv.) in carbon tetrachloride was refluxed for 4 hours. 15% of S2 was remaining and therefore an additional 0.1 equiv of NBS and a crystal of AIBN was added. The reaction was refluxed for 1 hour. The reaction was cooled and diluted with water. Extracted with EtOAc. The organic layer was dried and concentrated. The residue was purified by silica gel column chromatography (eluent:0-5 % EtOAc in hexanes) to afford the product as a white solid. ¹H-NMR (CDCl₃): δ 1.72 (s, 9H), 4.88 (s, 2H), 7.48 (s, 1H), 7.77 (d, J = 1.2 Hz, 1H), 8.56 (s, 1H).

**Step 3: *tert*-Butyl 7-(acetoxymethyl)-5-bromo-1*H*-indazole-1-carboxylate (36-4):** To a solution of tert-butyl 5-bromo-7-(bromomethyl)indazole-1-carboxylate (**36-3**, 41.43 g, 106.21 mmol, 1 equiv.) in DMF (410 mL) at room temperature was added potassium acetate (31.27 g) and the reaction was stirred at room temperature for 15 minutes. The reaction mixture was placed in a 40 °C bath and stirred for 1.5 hours. The reaction was cooled to room temperature, diluted with EtOAc and washed with water. The organic layer was dried and concentrated. The residue was purified by silica gel column chromatography to afford the title compound (eluent:0-1 % EtOAc in dichloromethane) compound as a yellow solid.

**Step 4: *tert*-Butyl 7-(acetoxymethyl)-5-(2-methylpyrimidin-5-yl)-1*H*-indazole-1-carboxylate (36-5):** A heterogeneous mixture of 5-bromo-2-methylpyrimidine (23.91 g, 138.198 mmol, 1.501 equiv.), bis(pinacolato)diboron (38.47 g, 152.031 mmol, 1.651 equiv.), potassium acetate (27.13 g, 276.414 mmol, 3.002 equiv.), and dichloro 1,1'-bis(diphenylphosphino) palladium (II) dichloromethane adduct (5.64 g, 6.906 mmol, 0.075 equiv.) in dioxane was degassed with argon and heated at 100 °C overnight. The reaction was cooled to room temperature and charged with tert-butyl 7-[(acetyloxy)methyl]-5-bromoindazole-1-carboxylate (**36-4**, 34 g, 92.087 mmol, 1 equiv.), potassium carbonate (38.2 g, 276.391 mmol, 3.001 equiv.) and water. The reaction was again degassed and heated at 100 ° C for 1.5 hours. The solvent was removed under reduced pressure and the residue was partitioned between dichloromethane and water. The organic layer was separated and washed with water. The combined aqueous layers were extracted with dichloromethane. The combined organic layers were dried and concentrated. The residue was purified by silica gel column chromatography (eluent: 0-5 % MeOH in dichloromethane) to afford the title compound as a cream colored solid.

**Step 5: (5-(2-Methylpyrimidin-5-yl)-1*H*-indazol-7-yl)methyl acetate (36-6):** Finely powdered *tert*-butyl 7-[(acetyloxy)methyl]-5-(2-methylpyrimidin-5-yl)indazole-1-carboxylate (**36-5**, 20.8 g, 54.39 mmol, 1 equiv.) was suspended in 4N HCl in dioxane (210 mL, 54.39 mmol, 1 equiv.) and stirred at room temperature for 1 hour with frequent sonication. After the reaction was complete, the solid was isolated by filtration. The solid was partitioned between chloroform (1 L) and saturated aqueous sodium bicarbonate. The organic layer was separated, dried and concentrated to afford the title compound as cream colored solid.

**Step 6: (3-Bromo-5-(2-methylpyrimidin-5-yl)-1*H*-indazol-7-yl)methyl acetate (36-7):** [5-(2-Methylpyrimidin-5-yl)-1*H*-indazol-7-yl]methyl acetate (**36-6**, 20 g, 70.846 mmol, 1 equiv.) was suspended in DMF (200 mL) and the mixture was warmed until the solution was homogeneous. The solution was allowed to warm to room temperature with stirring. Once the solution was cooled to room temperature, NBS (13.88 g) was added portion-wise at room temperature. The reaction was stirred at room temperature for 30 minutes and then poured into 2L of water with stirring. The solid was isolated by filtration, washed with 100 mL of saturated aqueous sodium bicarbonate solution, water and dried to afford the title compound as a cream colored solid. ¹H-NMR (DMSO-d₆): δ 2.10 (s, 3H), 2.68 (s, 3H), 5.38 (s, 2H), 7.89 (s, 1H), 7.95 (s, 1H), 9.09 (s, 2H), 13.75 (s, 1H).

**Step 7: (3-Acetyl-5-(2-methylpyrimidin-5-yl)-1*H*-indazol-7-yl)methyl acetate (36-8):** A solution of [3-bromo-5-(2-methylpyrimidin-5-yl)-1H-indazol-7-yl]methyl acetate (**36-7**, 10 g, 27.686 mmol, 1 equiv.), and *trans*-dchlorobis(triphenylphosphine)palladium (II) (1.94 g, 2.764 mmol, 0.1 equiv.) in DMF (200 mL) was purged with argon and tributyl(1-ethoxyvinyl)tin (14.99 g, 14.03 mL, 41.506 mmol, 1.499 equiv.) was added while the reaction was continually purged with argon for 5 additional minutes. Then the reaction was heated at 80 °C overnight. The solvent was removed under reduced pressure. Chloroform (200 mL) and cold 2N aqueous HCl (50 mL) was added and the reaction was stirred for 10 minutes in an ice bath. The reaction was made basic by the careful addition of saturated aqueous NaHCO₃ solution. The organic layer was separated and the aqueous layer was extracted with chloroform. The combined organic layers were dried and the residue was used as such for the next step.

**Step 8: *tert*-Butyl 2-(7-(acetoxymethyl)-3-acetyl-5-(2-methylpyrimidin-5-yl)-1*H-*indazol-1-yl)acetate (36-9):** [3-Acetyl-5-(2-methylpyrimidin-5-yl)-1H-indazol-7-yl]methyl acetate (**36-8**) was taken up in DMF (150 mL) and potassium carbonate (7.65 g, 55.351 mmol, 2 equiv.) was added followed by *tert*-butyl bromoacetate (6.5 g, 4.921 mL, 33.325 mmol, 1.204 equiv.). The reaction was stirred at room temperature for 1 hour. The reaction was filtered through a fritted funnel and the filtrate was concentrated under reduced pressure. The residue was purified by silica gel column chromatography (0-5 % MeOH in dichloromethane) to afford the title compound as a cream colored solid.

**Step 9: *tert*-Butyl 2-(3-acetyl-7-((but-3-en-1-yl(*tert*-butoxycarbonyl)amino)methyl)-5-(2-methylpyrimidin-5-yl)-1*H*-indazol-1-yl)acetate (36-10):** An ice-cold mixture of {3-acetyl-1-[2-(tert-butoxy)-2-oxoethyl]-5-(2-methylpyrimidin-5-yl)indazol-7-yl}methyl acetate (**36-9**, 0.2 g, 0.456 mmol, 1 equiv.) and potassium carbonate (0.032 g, 0.228 mmol, 0.5 equiv.) in MeOH (5 mL) was stirred for 1 hour. The solvent was removed under reduced pressure and the residue was taken up in dichloromethane and washed with water. The organic layer was dried and concentrated. The crude product was used as such for the next step.

To an ice-cold solution of the hydroxy compound from above in THF (3 mL), TEA (0.092 g, 0.127 mL, 0.912 mmol, 2 equiv.) was added followed by the dropwise addition of MsCl (0.063 g, 0.042 mL, 0.547 mmol, 1.2 equiv.). The reaction was stirred for at 0° C for 3 hours. The solvent was removed under reduced pressure. The residue was dissolved in dichloromethane and washed with water. The organic layer was dried and concentrated. The residue was used as such for the next step.

To an ice-cold solution of mesylate in THF (3 mL), was added TEA (0.127 mL), followed by 3-buten-1-amine (0.039 g, 0.05 mL, 0.547 mmol, 1.2 equiv.). The reaction was stirred overnight at room temperature. The volatiles were removed under reduced pressure. The residue was dissolved in dichloromethane (5 mL). To this solution, Boc₂O (0.199 g, 0.912 mmol, 2 equiv.) and a crystal of DMAP were added. The reaction was stirred at room temperature for 24 hours. The solvent was removed under reduced pressure and the residue was purified by silica gel column chromatography (eluent: 0-2 % MeOH in dichloromethane) to afford 0.15 g of the product as a cream colored solid.

**Step 10: 2-(3-Acetyl-7-((but-3-en-1-yl(*tert*-butoxycarbonyl)amino)methyl)-5-(2-methylpyrimidin-5-yl)-1*H*-indazol-1-yl)acetic acid (36-11):** To a solution of *tert*-butyl 2-(3-acetyl-7-{[but-3-en-1-yl(tert-butoxycarbonyl)amino]methyl}-5-(2-methylpyrimidin-5-yl)indazol-1-yl)acetate (**36-10**, 0.15 g, 0.273 mmol, 1 equiv.) in THF (1 mL) was added LiOH·H₂O (0.023 g, 0.546 mmol, 2 equiv.) in water (1 mL). Methanol (0.5 mL) was added and the reaction was stirred at room temperature for 2 hours. The reaction was diluted with water (3 mL) and the volatiles were removed under reduced pressure. The aqueous solution was extracted once with ether and the organic layer was discarded. The aqueous layer was cooled in an ice-bath and acidified with cold 1*N* aqueous HCl. The resulting heterogeneous mixture was extracted with chloroform. The chloroform layer was dried and concentrated to afford 130 mg of a cream colored solid that was used as such for the next step.

**Step 11: *tert*-Butyl ((3-acetyl-1-(2-((1*R*,3*S*,5*S*)-5-(allyloxy)-3-((3-methyl-6-(trifluoromethyl)pyridin-2-yl)carbamoyl)-2-azabicyclo[3.1.0]hexan-2-yl)-2-oxoethyl)-5-(2-methylpyrimidin-5-yl)-1*H*-indazol-7-yl)methyl)(but-3-en-1-yl)carbamate (36-12):** (R2) *tert-*Butyl (1*R*,3*S*,5*S*)-3-{[3-methyl-6-(trifluoromethyl)pyridin-2-yl]carbamoyl}-5-[(prop-2-en-1-yloxy)methyl]-2-azabicyclo[3.1.0]hexane-2-carboxylate (0.130 g) was stirred at room temperature for 30 minutes in TFA (1 mL)-dichloromethane (1mL). The volatiles were removed under reduced pressure. The residue was dissolved in DMF (2 mL) and DIPEA (0.23 mL) was added at 0 ° C. (3-Acetyl-7-{[but-3-en-1-yl(tert-butoxycarbonyl)amino]methyl}-5-(2-methylpyrimidin-5-yl)indazol-1-yl)acetic acid (**33-8 from Example 33**) was added to the reaction at 0 °C. TBTU (0.102 g) was added and the cooling bath was removed. The reaction was stirred at room temperature for 30 minutes. The DMF was removed under reduced pressure. The residue was dissolved in chloroform and washed with saturated sodium bicarbonate solution. The organic layer was dried (Na₂SO₄) and concentrated. Crude product was purified by silica gel column chromatography (eluent: 0-2 % MeOH in dichloromethane) to afford 170 mg of a colorless solid. ¹H NMR (400 MHz, Chloroform-*d*) δ 1.08 (dd, *J* = 2.7, 5.6 Hz, 1H), 1.41 (s, 9H), 1.42-1.45 (m, 1H), 1.92-2.02 (m, 1H), 2.03-2.13 (m, 1H), 2.27 (s, 3H), 2.51 (dd, *J* = 9.4, 13.9 Hz, 1H), 2.64 - 2.73 (m, 1H), 2.72 (s, 3H), 2.81 (s, 3H), 3.03 - 3.14 (m, 1H), 3.16-3.26 (m, 1H), 3.52 (d, *J* = 9.9 Hz, 1H), 3.61 (dd, *J* = 2.6, 5.7 Hz, 1H), 3.68 (d, *J=* 9.9 Hz, 1H), 4.03-4.05 (m, 2H), 4.21 (d, *J* = 15.3 Hz, 1H), 4.74 - 4.86 (m, 3H), 5.17 (d, J = 10.4 Hz, 1H), 5.26 (dd, J = 17.2, 1.2 Hz, 1H), 5.40-5.52 (m, 2H), 5.66 (d, *J =* 17.4 Hz, 1H), 5.75 (d, *J =* 17.5 Hz, 1H), 5.89 (ddt, *J* = 5.5, 10.7, 16.1 Hz, 1H), 7.38 (d, *J* = 1.8 Hz, 1H), 7.45 (d, *J* = 7.8 Hz, 1H), 7.68 (d, *J* = 7.8 Hz, 1H), 8.62 (d, *J* = 1.6 Hz, 1H), 8.89 (s, 2H), 9.70 (s, 1H). ¹⁹ F (376 MHz): δ -67.4.

**Step 12: Compound 36-13:** To a solution of *tert*-butyl *N*-(3-acetyl-1-{2-[(1R,3S,5S)-3-{[3-methyl-6-(trifluoromethyl)pyridin-2-yl]carbamoyl}-5-[(prop-2-en-1-yloxy)methyl]-2-azabicyclo[3.1.0]hexan-2-yl]-2-oxoethyl}-5-(2-methylpyrimidin-5-yl)indazol-7-yl)-N-(pent-4-en-1-yl)carbamate (**36-12**, 0.1 g, 0.12 mmol, 1 equiv.) in dichloromethane (10 mL) under argon was added the Grubbs'-Hoveyda 2^{nd} generation (8 mg) catalyst and the reaction was stirred overnight at 40 °C. Additional catalyst (8 mg) was added and the reaction was stirred at 40 °C for an additional 24 hours. The reaction was adsorbed on silica and purified by silica gel column chromatography (eluent: 0-3 % MeOH in dichloromethane) to afford 42 mg of a light brown solid as a *cis -trans* mixture (10 mg of *trans* **36-13** was separated).

**Step 13: Compound 36-14:** A solution of *tert*-butyl (1S,22R,25S)-16-acetyl-25-{[3-methyl-6-(trifluoromethyl)pyridin-2-yl]carbamoyl}-13-(2-methylpyrimidin-5-yl)-20-oxo-3-oxa-9,17,18,21-tetraazapentacyclo[19.2.2.1¹¹,¹⁵.0¹,²².0¹⁸,²⁶]hexacosa-5,11,13,15(26),16-pentaene-9-carboxylate (**36-12**, 0.04 g, 0.05 mmol, 1 equiv.) and platinum oxide in THF (1 mL) -EtOH (1 mL) was stirred under hydrogen for 2 hours at room temperature. The reaction was then adsorbed on silica and purified by silica gel column chromatography (eluent: 0-3 % MeOH in dichloromethane) to afford 23 mg of a white solid.

**Step 14: Compound 36:** A solution of *tert*-butyl (1S,22R,25S)-16-acetyl-25-{[3-methyl-6-(trifluoromethyl)pyridin-2-yl]carbamoyl}-13-(2-methylpyrimidin-5-yl)-20-oxo-3-oxa-9,17,18,21-tetraazapentacyclo[19.2.2.1¹¹,¹⁵.0¹,²².0¹⁸,²⁶]hexacosa-11,13,15(26),16-tetraene-9-carboxylate (**36-14**, 0.02 g, 0.025 mmol, 1 equiv.) in 4.0 M HCl in dioxane was stirred at room temperature for 1 hour. The volatiles were then removed under reduced pressure. The residue was washed with 1:1 mixture of heptane-ether (1 mL). The solid was dried under high vacuum to afford a cream colored solid. ¹H NMR (400 MHz, DMSO-*d*₆) δ 0.86 (t, J = 6.8 Hz, 1H), 1.24-1.28 (m, 1H), 1.51-1.64 (m, 4 H), 2.13-2.24 (m, 1H), 2.18 (s, 3H), 2.37 - 2.49 (m, 2H), 2.65 (s, 3H), 2.69 (s, 3H), 2.97 (d, *J* = 12.2 Hz, 1H), 3.14-3.20 (m, 1H), 3.39 - 3.56 (m, 2H), 3.63 - 3.77 (m, 2H), 4.00 (d, *J* = 12.2 Hz, 1H), 4.15 (t, *J* = 4.3 Hz, 1H), 4.40 - 4.53 (m, 2H), 5.02 (t, *J* = 12.7 Hz, 1H), 5.61 (d, *J =* 18.0 Hz, 1H), 6.65 (d, *J* = 18.1 Hz, 1H), 7.67 (d, *J =* 7.8 Hz, 1H), 7.89 - 7.96 (m, 2H), 8.51 (d, *J =* 1.4 Hz, 1H), 9.12 (s, 2H), 9.57 (s, 1H), 10.24 (s, 1H), 10.68 (s, 1H). ¹⁹F: δ -66.1. 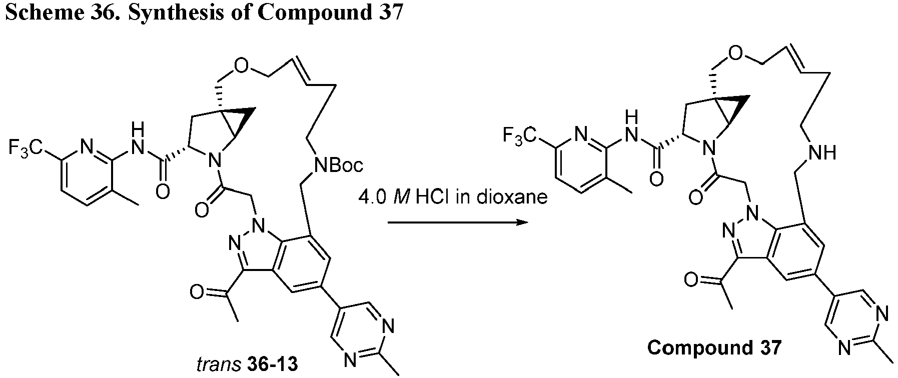 **Compound 37:** A solution of ***trans*-36-13** (0.01 g, 0.012 mmol, 1 equiv.) in 4.0 M HCl in dioxane (1mL) was stirred at room temperature for 2 hours. The solvent was removed under reduced pressure. The residue was dissolved in dichloromethane and 5 drops of TEA and purified by silica gel column chromatography (eluent: 0-4 % MeOH in dichloromethane) to afford a white solid. ¹H NMR (400 MHz, methanol-*d*₄) δ 0.35 (t, *J* = 6.0 Hz, 1H), 0.46-0.47 (m, 1H), 2.27-2.32 (m, 1H), 2.36 (s, 3H), 2.42-2.54 (m, 3H), 2.68 (s, 3H), 2.74 (s, 3H), 2.94-2.99 (m, 2H), 3.04 - 3.12 (m, 1H), 3.78 - 4.01 (m, 4H), 4.19 - 4.34 (m, 2H), 4.50 - 4.62 (m, 1H), 5.62-5.69 (m, 1H), 5.91-5.98 (m, 1H), 6.04 (d, *J* = 15.3 Hz, 1H), 6.15 (d, *J =* 15.3 Hz, 1H), 7.58 (d, J = 7.8 Hz, 1H), 7.64 (d, J = 1.2 Hz, 1H), 7.88 (d, *J* = 7.8 Hz, 1H), 8.52 (d, *J* = 1.6 Hz, 1H), 9.00 (s, 2H). ¹⁹F: δ -69.1.

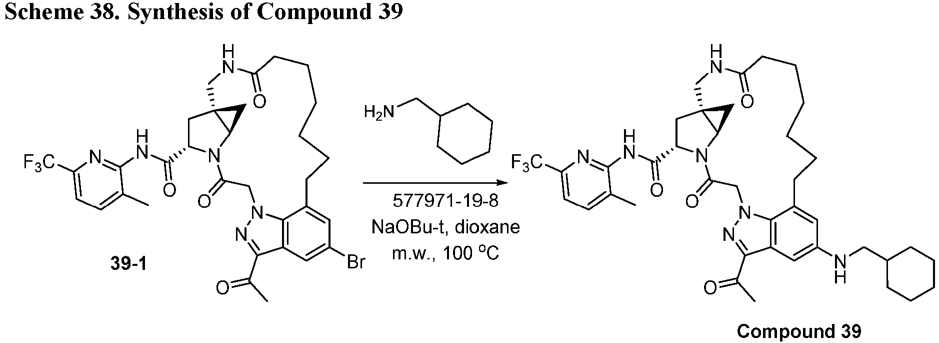

To a mixture of compound **39-1** (30 mg, 0.042 mmol) and cyclohexylmethanamine (22 mg, 0.2 mmol) in 1,4-dioxane (1 mL) were added NaOBu-t (12 mg, 0.12 mmol) and 2-(2'-di-tert-butylphosphine)biphenylpalladium(II) acetate (9 mg 0.02 mmol, CAS: 577971-19-8 ). The mixture was degassed under N₂ three times and stirred at 100 °C in a CEM microwave reactor for 45 minutes. The reaction mixture was diluted with EtOAc, washed with water and brine, dried over anhydrous Na₂SO₄, filtered and concentrated to dryness. The residue was purified by preparative HPLC to afford **Compound 39** (3.7 mg, yield 12.6 %) as a yellow solid. ¹H-NMR (400 MHz, DMSO-*d*₆) δ 10.45 (s, 1H), 8.17 (dd, *J=* 8.4, 8.4 Hz, 1H), 7.89 (d, *J=* 7.6 Hz, 1H), 7.65 (d, *J=* 7.6 Hz, 1H), 6.94 (d, *J=* 2.0 Hz, 1H), 6.67 (d, *J=* 1.6 Hz, 1H), 5.76 (d, *J=* 18.0 Hz, 1H), 5.63 (t, *J=* 5.6 Hz, 1H), 5.31 (d, *J=* 18.0 Hz, 1H), 4.32 (t, *J=* 7.8 Hz, 1H), 3.53 (dd, *J=* 6.0, 6.0 Hz, 1H), 3.30-3.26 (m, 1H), 3.16 (dd, *J=* 14.4, 14.4 Hz, 1H), 2.96-2.90 (m, 1H), 2.84 (t, *J*= 6.0 Hz, 2H), 2.68-2.59 (m, 1H), 2.52 (s, 3H), 2.43-2.37 (m, 2H), 2.12-2.00 (m, 5H), 1.81 (d, *J=* 12.4 Hz, 2H), 1.71-1.57 (m, 8H), 1.47-0.95 (m, 11H). LC/MS (ESI) m/z: 736 (M+H)⁺.

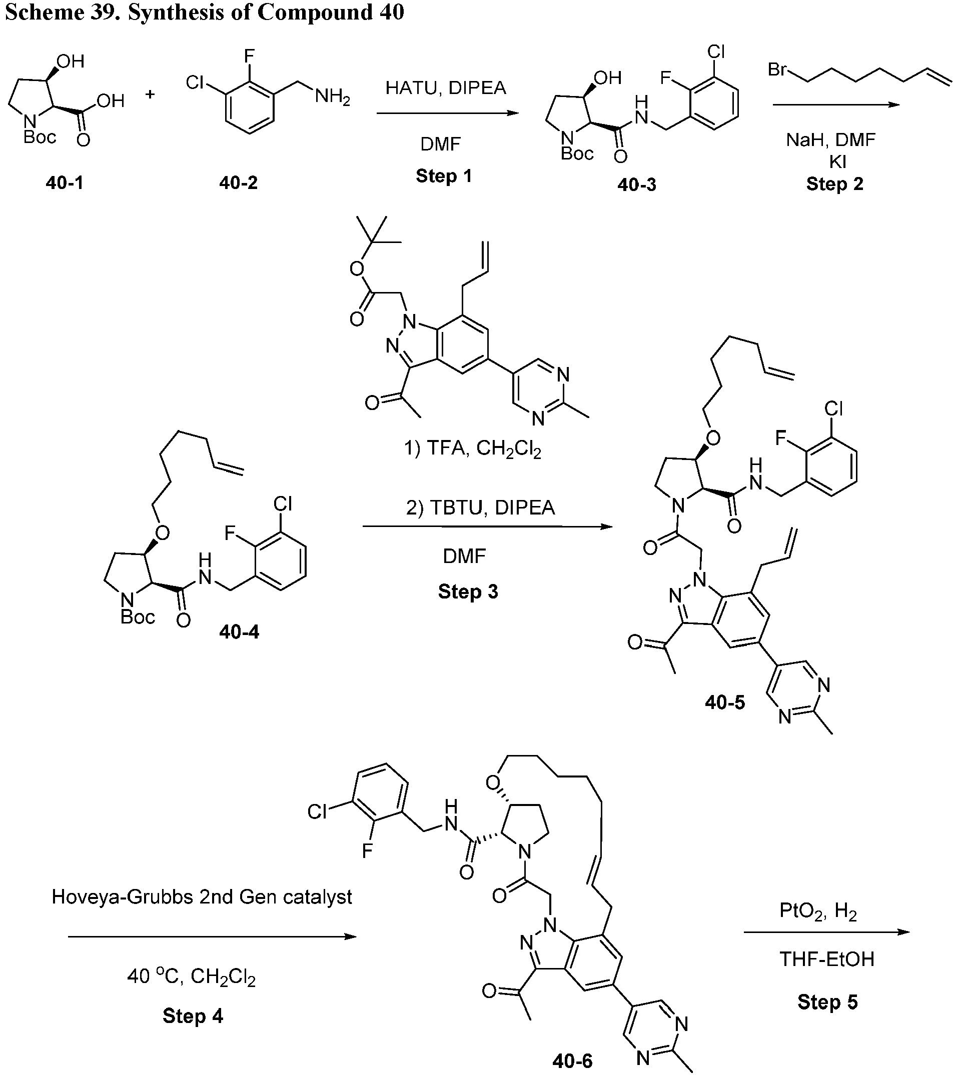

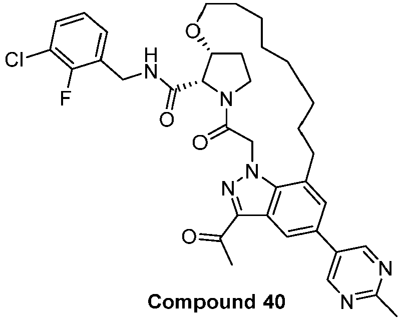

**Step 1. *tert-Butyl* (2S,3R)-2-((3-chloro-2-fluorobenzyl)carbamoyl)-3-hydroxypyrrolidine-1-carboxylate (40-3):** To an ice-cold solution of (2*S*,3*R*)-1-(*tert-*butoxycarbonyl)-3-hydroxypyrrolidine-2-carboxylic acid (40-1, 1 g) in DMF (10 mL) were added HATU (1.97 g) and DIPEA (2.3 mL) followed by (3-chloro-2-fluorophenyl)methanamine (40-2, 0.76 g). The cooling bath was removed and the reaction was stirred overnight at room temperature. The solvent was removed under reduced pressure and the residue was dissolved in chloroform. The resulting solution was washed successively with 1.0 M aqueous citric acid, water, and saturated aqueous NaHCO₃ solution. The organic layer was dried with Na₂SO₄ and concentrated. The crude product was purified by silica gel column chromatography (eluent: 0-2.5 % MeOH in dichloromethane) to afford white solid.

**Step 2. *tert*-Butyl (2*S*,3*R*)-2-((3-chloro-2-fluorobenzyl)carbamoyl)-3-(hept-6-en-1-yloxy)pyrrolidine-1-carboxylate (40-4):** To an ice-cold solution of *tert*-butyl (2*S*,3*R*)-2-{[(3-chloro-2-fluorophenyl)methyl]carbamoyl}-3-hydroxypyrrolidine-1-carboxylate (1 g) in DMF (10 mL) was added sodium hydride (0.160 g, 60 % in mineral oil) portion-wise and the solution was stirred for 30 minutes at 0 °C. 7-Bromo-1-heptene (1.43 g) was added dropwise followed by the addition of a crystal of KI. The reaction was stirred at 0 °C for 2 hours and then at room temperature overnight. The solvent was removed under reduced pressure and the residue was diluted with dichloromethane-water. The organic layer was separated and the aqueous layer was extracted once with dichloromethane. The combined organic layers were dried with Na₂SO₄ and concentrated. The crude product was purified by silica gel column chromatography (eluent: 0-40 % EtOAc in hexanes) to afford 0.38 g of a colorless resin.

**Step 3. (2*S*,3*R*)-1-(2-(3-Acetyl-7-allyl-5-(2-methylpyrimidin-5-yl)-1*H*-indazol-1-yl)acetyl)-*N*-(3-chloro-2-fluorobenzyl)-3-(hept-6-en-1-yloxy)pyrrolidine-2-carboxamide (40-5):** *tert*-Butyl 2-[3-acetyl-5-(2-methylpyrimidin-5-yl)-7-(prop-2-en-1-yl)indazol-1-yl]acetate (0.33 g, 0.812 mmol, 1 equiv.) was stirred in a vial with 3 mL of TFA and 1 mL of CH₂Cl₂ for 3 hours. In another vial, *tert*-butyl (2S,3R)-2-{[(3-chloro-2-fluorophenyl)methyl]carbamoyl}-3-(hept-6-en-1-yloxy)pyrrolidine-1-carboxylate (0.38 g) was stirred with 2 mL of TFA and 2 mL of CH₂Cl₂ for 30 minutes. The contents of the two vials were combined and the volatiles were removed under reduced pressure. The residue was dissolved in DMF (3 mL) and cooled in an ice bath. Subsequently, DIPEA (0.71 mL) and TBTU (1.10 g) were added. The cooling bath was removed and the reaction was stirred at room temperature for 30 minutes. The solvent was removed under reduced pressure. The residue was dissolved in dichloromethane and washed with saturated aqueous sodium bicarbonate solution. The organic layer was separated, dried with Na₂SO₄ and concentrated. The crude product was purified by silica gel column chromatography (0-1.5 % MeOH in dichloromethane) to afford 350 mg of a white solid.

**Step 4. Compound 40-6:** A solution of (2S,3R)-1-{2-[3-acetyl-5-(2-methylpyrimidin-5-yl)-7-(prop-2-en-1-yl)indazol-1-yl]acetyl}-N-[(3-chloro-2-fluorophenyl)methyl]-3-(hept-6-en-1-yloxy)pyrrolidine-2-carboxamide (0.2 g, 0.16 mmol, 1 equiv.) in dichloromethane (34 mL) was degassed with argon. PtO₂ (18 mg) was added and the reaction was stirred at 40 °C overnight. Additional catalyst (18 mg) was added and the reaction was stirred for 8 hours at 40 °C. The reaction was adsorbed on silica gel and purified by silica gel column chromatography (eluent: 0-3 % MeOH in dichloromethane) to afford the desired compound as a brown solid that was a mixture of *cis* and *trans* isomers.

**Step 5. Compound 40:** A solution of **Compound 4-6** (5 mg, 0.007 mmol, 1 equiv.) in THF (1 mL)-EtOH (1 mL) was stirred under hydrogen atmosphere for 6 hours in presence of platinum oxide (1 mg). The reaction was filtered through celite and the filtrate was concentrated under reduced pressure. The crude product was purified by preparative TLC (solvent system: 5 % MeOH in chloroform) to afford **Compound 40** as a colorless solid. ¹H NMR (400 MHz, Chloroform-d, major rotamer) δ 0.86-0.89 (m, 2H), 1.26-1.63 (m, 8H), 1.71-1.79 (m, 1H), 1.91-1.99 (m, 1H), 2.27-2.37 (m, 2H), 2.72 (s, 3H), 2.87 (s, 3H), 2.87 (t, *J=* 8.5 Hz, 2H), 3.37-3.45 (m, 2H), 3.75 (t, *J* = 8.8 Hz, 1H), 3.92-3.96 (m, 1H), 4.32 (d, *J* =20.5 Hz, 2H), 4.42 (d, *J* = 5.8 Hz, 2H), 5.41 (d, *J =* 16.8 Hz, 1H), 5.51 (d, *J =* 16.9 Hz, 1H), 6.11-6.15 (m, 1H), 6.64 (t, *J* = 7.8 Hz, 1H), 7.07-7.11 (m, 2H), 7.27 (s, 1H), 8.49 (s, 1H), 8.91 (s, 2H). ¹⁹F NMR (CDCl₃) δ -121.1.

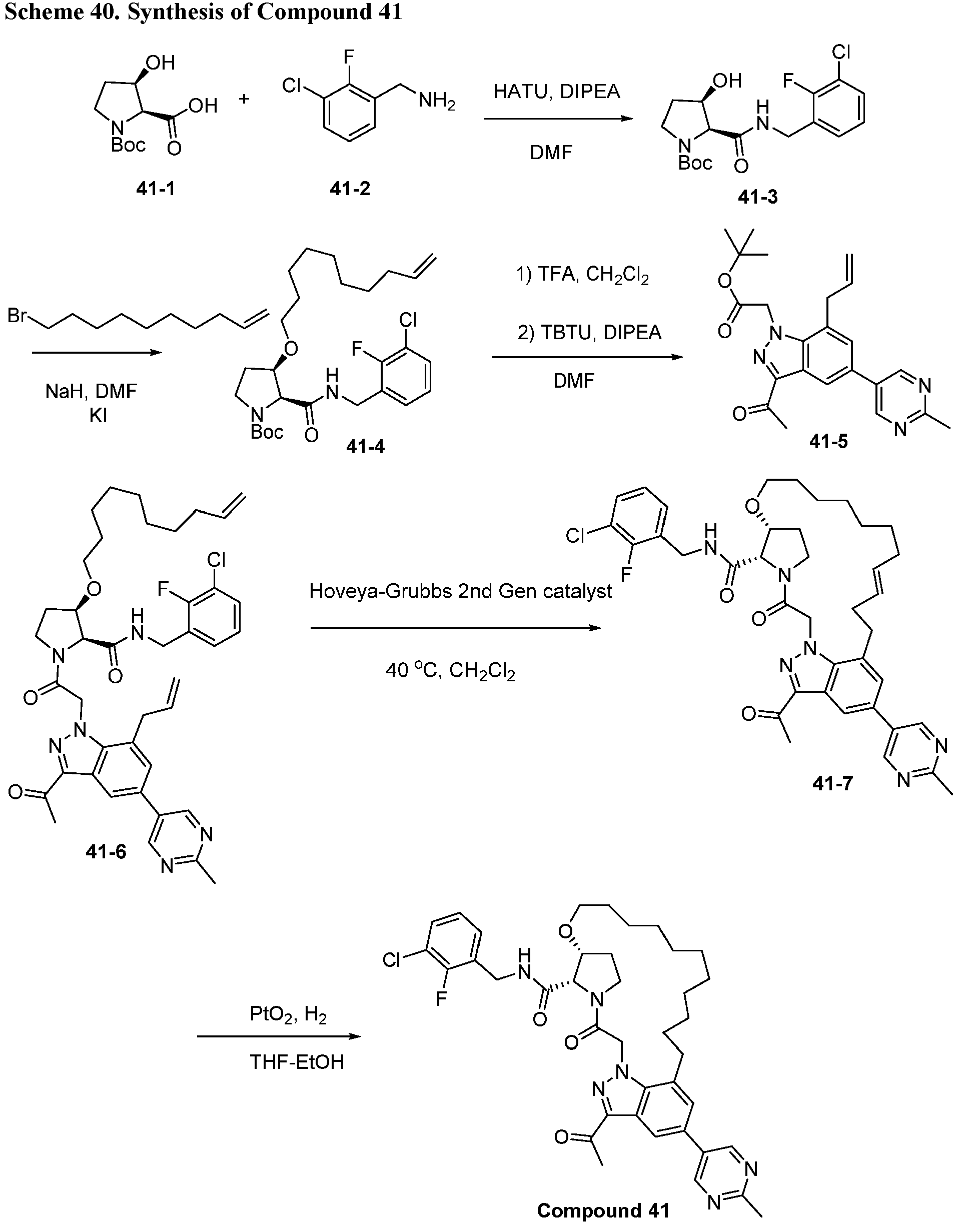

**Step 1: *tert*-Butyl (2*S*,3*R*)-2-((3-chloro-2-fluorobenzyl)carbamoyl)-3-hydroxypyrrolidine-1-carboxylate (41-3):** To an ice-cold solution of (2*S*,3*R*)-1-(*tert-*butoxycarbonyl)-3-hydroxypyrrolidine-2-carboxylic acid (1 g) in DMF (10 mL) were added HATU (1.97 g) and DIPEA (2.3 mL) followed by (3-chloro-2-fluorophenyl)methanamine (0.76 g). The cooling bath was removed and the reaction was stirred overnight at room temperature. The solvent was removed under reduced pressure and the residue was dissolved in chloroform. The resulting solution was washed successively with 1.0 M aqueous citric acid, water, and saturated aqueous NaHCO₃ solution. The organic layer was dried with Na₂SO₄ and concentrated. The crude product was purified by silica gel column chromatography (eluent: 0-2.5 % MeOH in dichloromethane) to afford a white solid.

**Step 2: *tert*-Butyl (2*S*,3*R*)-2-((3-chloro-2-fluorobenzyl)carbamoyl)-3-(dec-9-en-1-yloxy)pyrrolidine-1-carboxylate (41-4):** To a cooled solution of *tert*-butyl (2*S*,3*R*)-2-{[(3-chloro-2-fluorophenyl)methyl]carbamoyl}-3-hydroxypyrrolidine-1-carboxylate (1 g, 2.682 mmol, 0.498 equiv.) in DMF (10 mL) was added sodium hydride (0.16 g, 60 % in mineral oil) portion-wise. After the addition of NaH was complete, the reaction was stirred at 0 ° C for 30 minutes. 10-Bromodec-1-ene (1.18 g, 5.384 mmol, 1 equiv.) was added dropwise at 0 ° C and the reaction was stirred at 0 ° C for 2 hours and allowed to warm to room temperature and stir overnight. The reaction was quenched with the careful addition of water and the solvent was removed under reduced pressure. The residue was taken up in dichloromethane and washed with water. The organic layer was separated, dried with Na₂SO₄, and concentrated. The crude product was purified by silica gel column chromatography (eluent: 0-40 % EtOAc in hexanes) to afford 0.28 g of a colorless resin.

**Step 3: (2*S*,3*R*)-1-(2-(3-Acetyl-7-allyl-5-(2-methylpyrimidin-5-yl)-1*H*-indazol-1-yl)acetyl)-*N*-(3-chloro-2-fluorobenzyl)-3-(dec-9-en-1-yloxy)pyrrolidine-2-carboxamide (41-6):** *tert*-Butyl (2*S*,3*R*)-2-{[(3-chloro-2-fluorophenyl)methyl]carbamoyl}-3-(dec-9-en-1-yloxy)pyrrolidine-1-carboxylate (**41-4**, 0.28 g, 0.548 mmol, 1.012 equiv.) was stirred in a vial with TFA (2 mL) and dichloromethane (2 mL) for 15 minutes. In another vial, tert-butyl 2-[3-acetyl-5-(2-methylpyrimidin-5-yl)-7-(prop-2-en-1-yl)indazol-1-yl]acetate (**41-5**, 0.22 g, 0.541 mmol, 1 equiv.) was stirred with TFA (2 mL) and dichloromethane (1 mL) for 2 hours at room temperature. The contents of the two vials were combined and the volatiles were removed under reduced pressure. The residue was taken up in DMF (3 mL) and the vial was cooled in an ice-bath. DIPEA (0.477 mL) and TBTU (0.211 g, 0.657 mmol, 1.214 equiv.) were added and the cooling bath was removed. The reaction was stirred for 30 minutes at room temperature. The solvent was removed under reduced pressure. The residue was dissolved in dichloromethane and washed with saturated sodium bicarbonate solution. The organic layer was dried with Na₂SO₄ and concentrated. The crude product was purified by silica gel column chromatography (eluent:0-2 % MeOH in dichloromethane) to afford the title compound as a cream colored solid.

**Step 4: Compound 41-7:** A solution of (2*S*,3*R*)-1-{2-[3-acetyl-5-(2-methylpyrimidin-5-yl)-7-(prop-2-en-1-yl)indol-1-yl]acetyl }-*N*-[(3-chloro-2-fluorophenyl)methyl]-3-(dec-9-en-1-yloxy)pyrrolidine-2-carboxamide (0.13 g, 0.175 mmol, 1 equiv.) in dichloromethane (20 mL) was degassed with argon and Hoveyda-Grubbs 2^{nd} generation catalyst (10 mg) was added. The reaction mixture was capped and heated at 40 ° C overnight. The solvent was reduced by 50% and the reaction was adsorbed on silica and purified by silica gel column chromatography (eluent:0-1.5 % MeOH in dichloromethane) to afford the title compound as a mixture of *cis* and *trans* isomers.

**Step 5. Compound 41:** A mixture of Compound **41-7** (0.02 g, 0.028 mmol, 1 equiv.) and platinum oxide (2 mg) in THF (1 mL)-EtOH 91 mL) was stirred under hydrogen atmosphere overnight at room temperature. The reaction was then adsorbed on silica and purified by silica gel column chromatography (eluent: 0-1.5 % MeOH in dichloromethane) to afford the title compound as a white solid. ¹H NMR (400 MHz, Chloroform-*d*, major rotamer) δ 1.24-1.27 (m, 18 H), 1.99 - 2.04 (m, 1H), 2.27 - 2.37 (m, 1H), 2.70 (s, 3H), 2.65 - 2.81 (m, 1H), 2.78-2.81 (m, 1H), 2.81 (s, 3H), 3.43-3.51 (m, 2H), 3.74 - 3.92 (m, 2H), 4.29-4.32 (m, 1H), 4.39 - 4.59 (m, 3H), 5.41 (d, *J* = 17.3 Hz, 1H), 5.56 (d, *J* = 17.1 Hz, 1H), 5.90 (t, *J =* 6.3 Hz, 1H), 6.81 (t, *J =* 7.8 Hz, 1H), 7.17 - 7.24 (m, 1H), 7.20 - 7.41 (m, 2H), 8.47 (s, 1H), 8.89 (s, 2H). ¹⁹F δ -121.4.

### EXAMPLE 2. EXAMPLES OF COMPOUNDS OF THE PRESENT INVENTION

Tables 1 shows illustrative Factor D inhibitors with characaterizing data. The assay of Example 3 was used to determine the IC₅₀'s of the compounds. Other standard factor D inhibition assays are also available. Three ***s are used to denote compounds with an IC₅₀ less than 1 micromolar; two **s indicate compound with an IC₅₀ between 1 micromolar and 10 micromolar, and one * denotes compounds with an IC₅₀ greater than 10 micromolar.

**Table 1. Examples of Compounds of the Present Invention**

| Cmp No. | Structure | IC₅₀ (Stars) | RT min (Method A,B, C or D) | MS (M+1) |
|---|---|---|---|---|
| 2 | 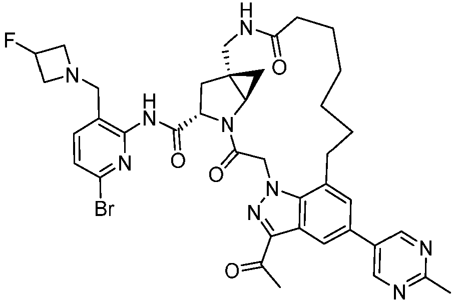 | *** | | |
| | (4¹R,4³S,4⁵R)-1³-acetyl-N-(6-bromo-3-((3-fluoroazetidin-1-yl)methyl)pyridin-2-yl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 3 | 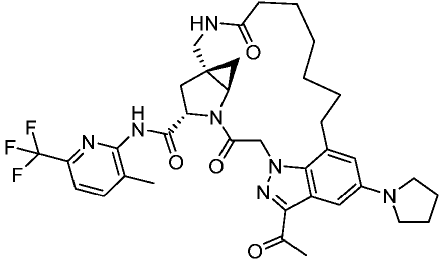 | *** | 3.51 (B) | 694 |
| | (4¹R,4³S,4⁵R)-1³-acetyl-N-(3-methyl-6-(trifluoromethyl)pyridin-2-yl)-3,7-dioxo-1⁵-(pyrrolidin-1-yl)-1¹H-4²,6-diaza-1(1,7)-indazola-4(2,5)-bicyclo[3.1.0]hexanacyclotridecaphane-4³-carboxamide | | | |
| 4 | 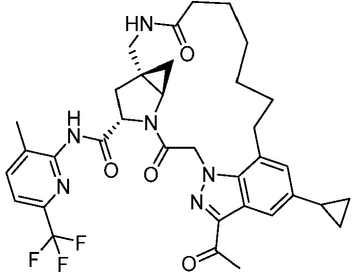 | *** | 3.8 (B) | 665 |
| | (4¹R,4³S,4⁵R)-1³-acetyl-1⁵-cyclopropyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 5 | 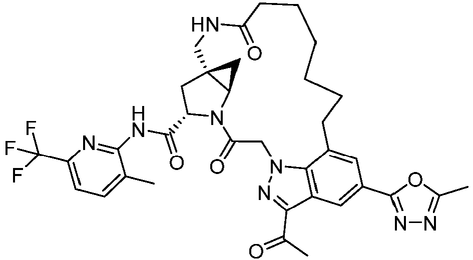 | *** | 3.52 (B) | 707 |
| | (4¹R,4³S,4⁵R)-1³-acetyl-1⁵-(5-methyl-1,3,4-oxadiazol-2-yl)-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 6 | 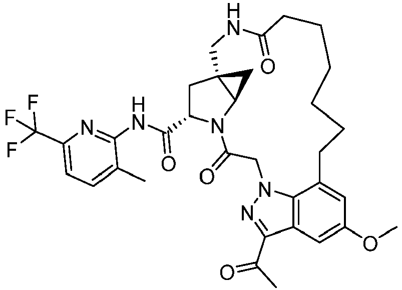 | *** | 3.81 (B) | 655 |
| | (4¹R,4³S,4⁵R)-1³-acetyl-1⁵-methoxy-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 7 | 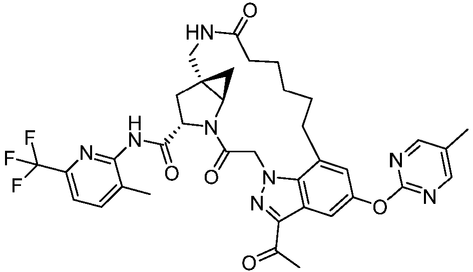 | *** | 3.01 (B) | 719 |
| | (4¹R,4³S,4⁵R)-1³-acetyl-N-(3-methyl-6-(trifluoromethyl)pyridin-2-yl)-1⁵-((5-methylpyrimidin-2-yl)oxy)-3,7-dioxo-1¹H-4²,6-diaza-1(1,7)-indazola-4(2,5)-bicyclo[3.1.0]hexanacyclododecaphane-4³-carboxamide | | | |
| 8 | 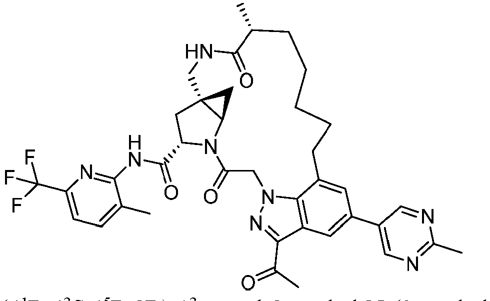 | *** | 3.43 (B) | 731 |
| | (4¹R,4³S,4⁵R,8R)-1³-acetyl-8-methyl-N-(3-methyl-6-(trifluoromethyl)pyridin-2-yl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 9 | 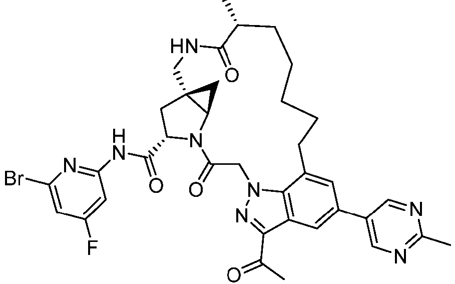 | *** | 3.96 (B) | 745 |
| | (4¹R,4³S,4⁵R,8R)-1³-acetyl-N-(6-bromo-4-fluoropyridin-2-yl)-8-methyl-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 10 | 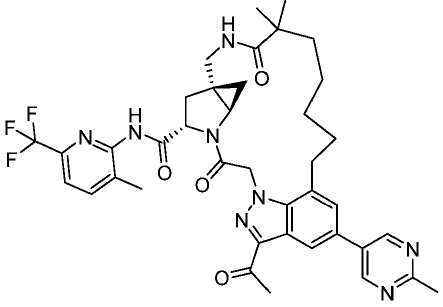 | *** | 4.04 (B) | 745 |
| | (4¹R,4³S,4⁵R)-1³-acetyl-8,8-dimethyl-N-(3-methyl-6-(trifluoromethyl)pyridin-2-yl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 11 | 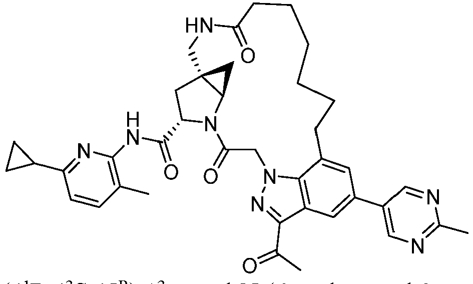 | *** | 2.6 (B) | 689 |
| | (4¹R,4³S,45^{R})-1³-acetyl-N-(6-cyclopropyl-3-methylpyridin-2-yl)-15-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 13 | 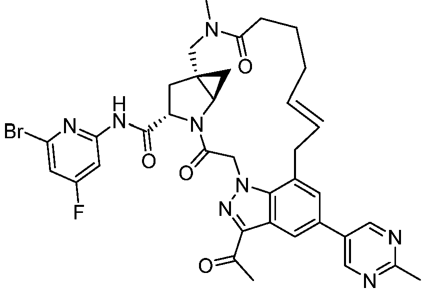 | *** | 3.82 (B) | 743 |
| | (4¹R,4³S,4⁵R,E)-1³-acetyl-N-(6-bromo-4-fluoropyridin-2-yl)-6-methyl-1⁵-(2-methylpyrimidin-5-yl)-3,7-dioxo-1¹H-4²,6-diaza-1(1,7)-indazola-4(2,5)- | | | |
| | bicyclo[3.1.0]hexanacyclotridecaphan-11-ene-4³-carboxamide | | | |
| 14 | 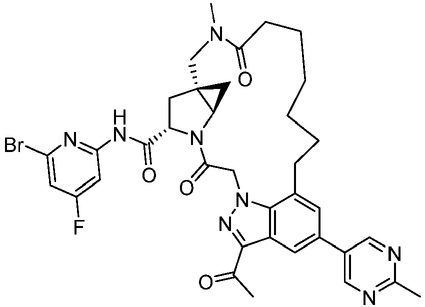 | *** | 3.96 (B) | 745 |
| | (4¹R,4³S,4⁵R)-1³-acetyl-N-(6-bromo-4-fluoropyridin-2-yl)-6-methyl-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 15 | 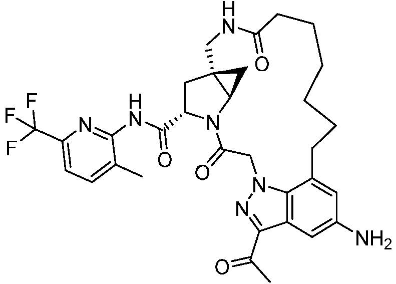 | *** | 2.04 (B) | 640 |
| | (4¹R,4³S,4⁵R)-1³-acetyl-1⁵-amino-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 16 | 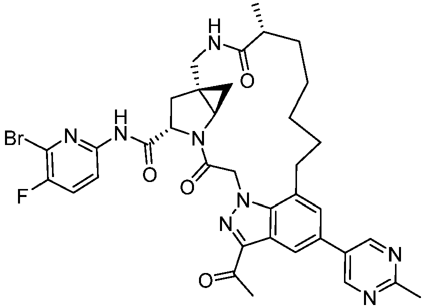 | *** | 3.68 (B) | 745 |
| | (4¹R,4³S,4⁵R,8R)-1³-acetyl-N-(6-bromo-5-fluoropyridin-2-yl)-8-methyl-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 17 | 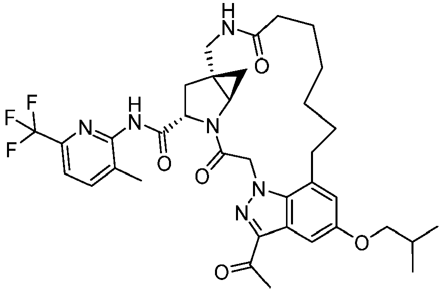 | *** | 4.49 (B) | 697 |
| | (4¹R,4³S,4⁵R)-1³-acetyl-1⁵-isobutoxy-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 18 | 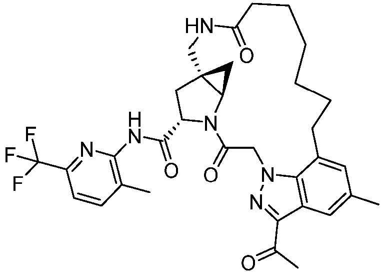 | *** | 3.88 (B) | 639 |
| | (4¹R,4³S,4⁵R)-1³-acetyl-1⁵-methyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 19 | 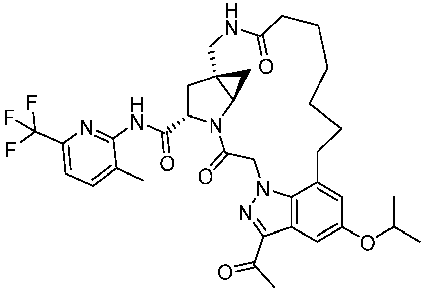 | *** | 4.06 (B) | 683 |
| | (4¹R,4³S,4⁵R)-1³-acetyl-1⁵-isopropoxy-N-(3-methyl-6-(trifluoromethyl)pyridin-2-yl)-3,7-dioxo-1¹H-4²6-diaza-1(1,7)-indazola-4(2,5)-bicyclo[3.1.0]hexanacyclotridecaphane-4³-carboxamide | | | |
| 20 | 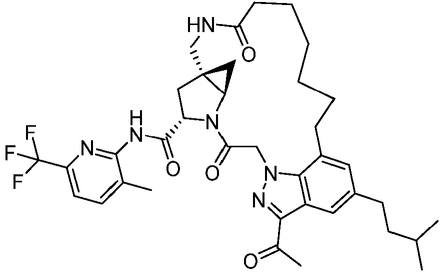 | *** | 4.55 (B) | 695 |
| | (4¹R,4³S,4⁵R)-1³-acetyl-1⁵-isopentyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 21 | 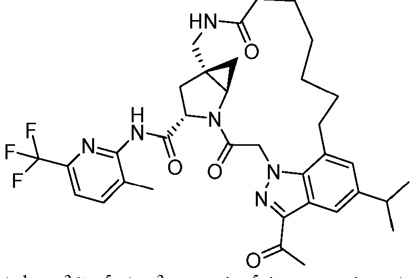 | *** | 4.14 (B) | 667 |
| | (4¹R,4³S,4⁵R)-1³-acetyl-1⁵-isopropyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 22 | 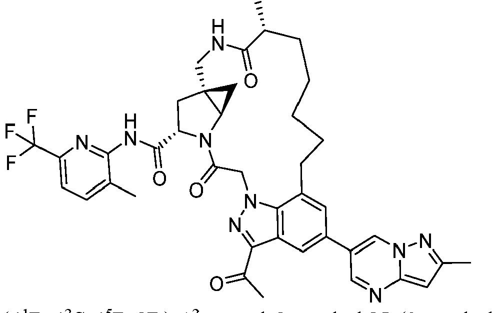 | *** | 3.77 (B) | 770 |
| | (4¹R,4³S,4⁵R,8R)-1³-acetyl-8-methyl-N-(3-methyl-6-(trifluoromethyl)pyridin-2-yl)-15-(2-methylpyrazolo[1,5-a]pyrimidin-6-yl)-3,7-dioxo-1¹H-4²,6-diaza-1(1,7)-indazola-4(2,5)-bicyclo[3.1.0]hexanacyclotridecaphane-4³-carboxamide | | | |
| 23 | 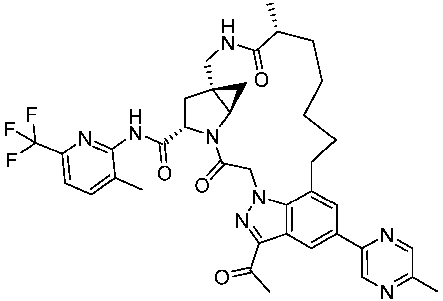 | *** | 3.68 (B) | 731 |
| | (4¹R,4³ S,4⁵R, 8R)-1³-acetyl-8-methyl-N-(3-methyl-6-(trifluoromethyl)pyridin-2-yl)-1⁵-(5-methylpyrazin-2-yl)-3,7-dioxo-1¹H-4²,6-diaza-1(1,7)-indazola-4(2,5)-bicyclo[3.1.0]hexanacyclotridecaphane-4³-carboxamide | | | |
| 24 | 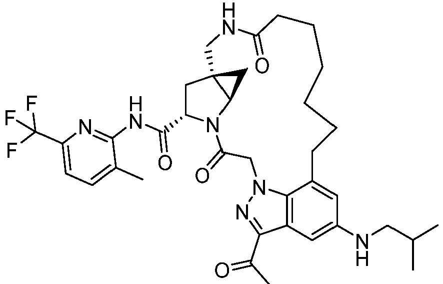 | *** | 2.72 (B) | 696 |
| | (4¹R,4³S,4⁵R)-1³-acetyl-15-(isobutylamino)-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 25 | 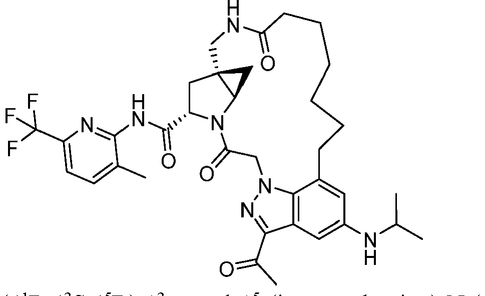 | *** | 2.16 (B) | 682 |
| | (4¹R,4³S,4⁵R)-1³-acetyl-1⁵-(isopropylamino)-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 26 | 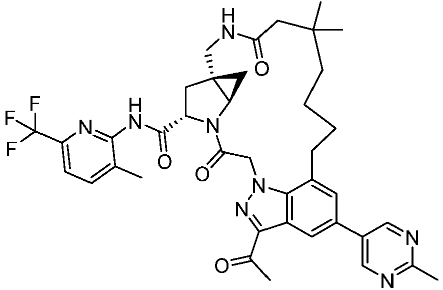 | *** | 3.85 (B) | 745 |
| | (4¹R,4³S,4⁵R)-1³-acetyl-9,9-dimethyl-N-(3-methyl-6-(trifluoromethyl)pyridin-2-yl)-15-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 27 | 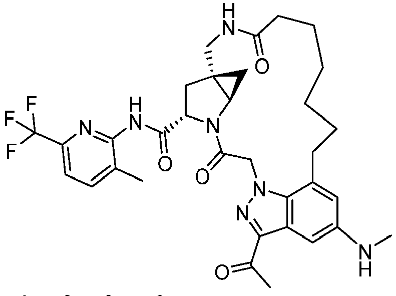 | *** | 1.89 (B) | 654 |
| | (4¹R,4³S,4⁵R)-1³-acetyl-N-(3-methyl-6-(trifluoromethyl)pyridin-2-yl)-1⁵-(methylamino)-3,7-dioxo-1¹H-4²,6-diaza-1(1,7)-indazola-4(2,5)-bicyclo[3.1.0]hexanacyclotridecaphane-4³-carboxamide | | | |
| 28 | 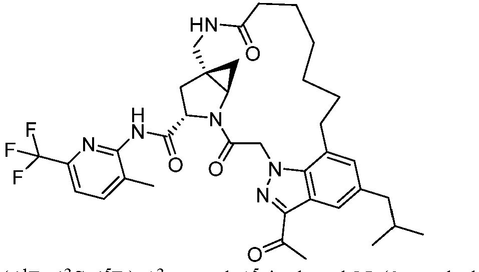 | *** | 4.22 (B) | 681 |
| | (4¹R,4³S,4⁵R)-1³-acetyl-1⁵-isobutyl-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 29 | 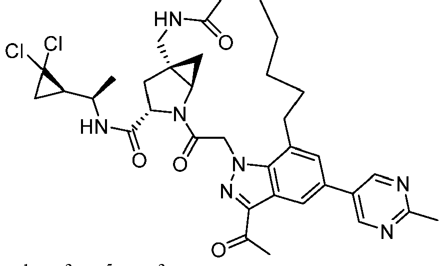 | *** | 3.7 (B) | 694 |
| | (4¹R,4³S,4⁵R)-1³-acetyl-N-((R)-1-((R)-2,2-dichlorocyclopropyl)ethyl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 30 | 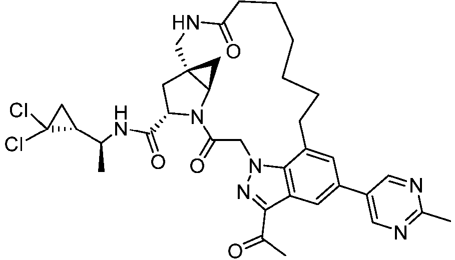 | *** | 3.34 (B) | 694 |
| | (4¹R,4³S,4⁵R)-1³-acetyl-N-((S)-1-((R)-2,2-dichlorocyclopropyl)ethyl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 31 | 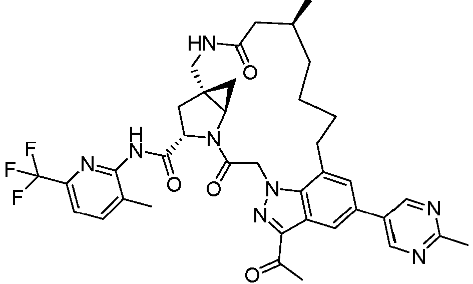 | *** | 3.34 (B) | 731 |
| | (4¹R,4³S,4⁵R,9S)-1³-acetyl-9-methyl-N-(3-methyl-6-(trifluoromethyl)pyridin-2-yl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 32 | 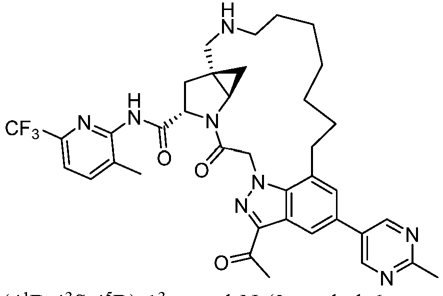 | *** | 3.18 (B) | 703 |
| | (4¹R,4³S,4⁵R)-1³-acetyl-N-(3-methyl-6-(trifluoromethyl)pyridin-2-yl)-1⁵-(2-methylpyrimidin-5-yl)-3-oxo-1¹H-4²,6-diaza-1(1,7)-indazola-4(2,5)-bicyclo[3.1.0]hexanacyclotridecaphane-4³-carboxamide | | | |
| 33 | 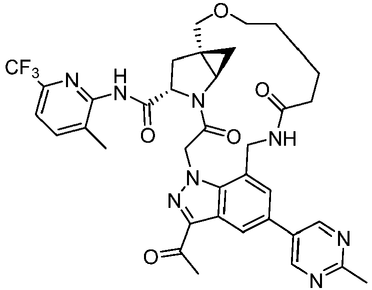 | *** | 1.75 (A) | 719 |
| | (4¹R,4³S,4⁵S)-1³-acetyl-N-(3-methyl-6-(trifluoromethyl)pyridin-2-yl)-1⁵-(2-methylpyrimidin-5-yl)-3,11-dioxo-1¹H-6-oxa-4²,12-diaza-1(1,7)-indazola-4(2,5)-bicyclo[3.1.0]hexanacyclotridecaphane-4³-carboxamide | | | |
| 34 | 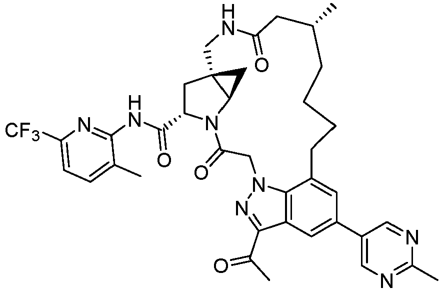 | *** | 3.86 (B) | 731 |
| | (4¹R,4³S,4⁵R,9R)-1³-acetyl-9-methyl-N-(3-methyl-6-(trifluoromethyl)pyridin-2-yl)-1⁵-(2-methylpyrimidin-5-yl)-3,7-dioxo-1¹H-4²,6-diaza-1(1,7)-indazola-4(2,5)-bicyclo[3.1.0]hexanacyclotridecaphane-4³-carboxamide | | | |
| 35 | 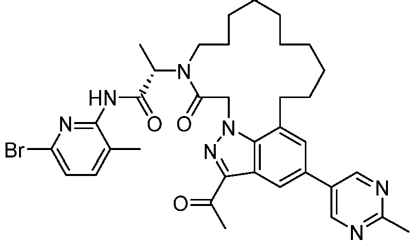 | ** | 4.01 (B) | 688 |
| | (S)-2-(2-acetyl-4-(2-methylpyrimidin-5-yl)-17-oxo-8,9,10,11,12,13,14,15,17,18-decahydro-6H-[1,4]diazacyclohexadecino[15,16,1-hi]indazol-16(7H)-yl)-N-(6-bromo-3-methylpyridin-2-yl)propanamide | | | |
| 36 | 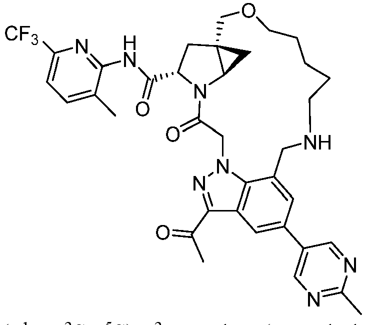 | *** | 1.59 (A) | 705 |
| | (4¹R,4³S,4⁵S)-1³-acetyl-N-(3-methyl-6-(trifluoromethyl)pyridin-2-yl)-1⁵-(2-methylpyrimidin-5-yl)-3-oxo-1¹H-6-oxa-4²,12-diaza-1(1,7)-indazola-4(2,5)-bicyclo[3.1.0]hexanacyclotridecaphane-4³-carboxamide | | | |
| 37 | 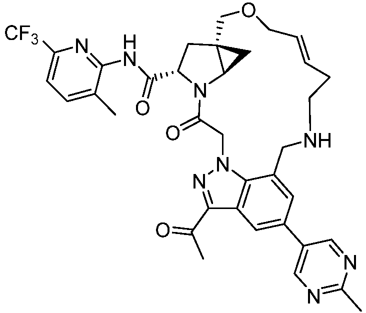 | *** | 1.61 (A) | 703 |
| | (4¹R,4³S,4⁵S,E)-1³-acetyl-N-(3-methyl-6-(trifluoromethyl)pyridin-2-yl)-1⁵-(2-methylpyrimidin-5-yl)-3-oxo-1¹H-6-oxa-4²,12-diaza-1(1,7)-indazola-4(2,5)-bicyclo[3.1.0]hexanacyclotridecaphan-8-ene-4³-carboxamide | | | |
| 39 | 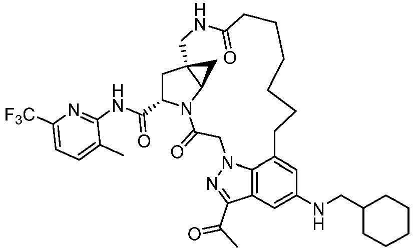 | *** | 3.31 (B) | 736 |
| | (4¹R,4³S,4⁵R)-1³-acetyl-1⁵-((cyclohexylmethyl)amino)-N-(3-methyl-6-(trifluoromethyl)pyridin-2-yl)-3,7-dioxo-1¹H-4²,6-diaza-1(1,7)-indazola-4(2,5)-bicyclo[3.1.0]hexanacyclotridecaphane-4³-carboxamide | | | |
| 40 | 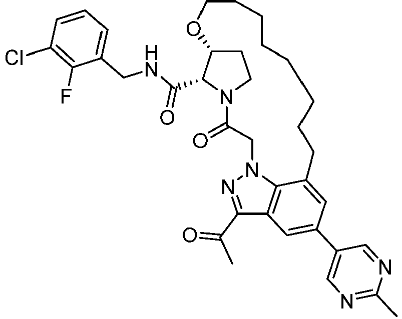 | *** | 2.97 (A) | 675 |
| | (4²S,4³R)-1³-acetyl-N-(3-chloro-2-fluorobenzyl)-1⁵-(2-methylpyrimidin-5-yl)-3-oxo-1¹H-5-oxa-1(1,7)-indazola-4(1,3)-pyrrolidinacyclotridecaphane-4²-carboxamide | | | |
| 41 | 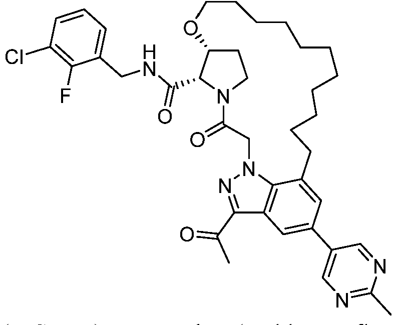 | | 3.40 (A) | 717 |
| | (42S,43R)-13-acetyl-N-(3-chloro-2-fluorobenzyl)-15-(2-methylpyrimidin-5-yl)-3-oxo-11H-5-oxa-1(1,7)-indazola-4(1,3)-pyrrolidinacyclohexadecaphane-42-carboxamide | | | |
| 42 | 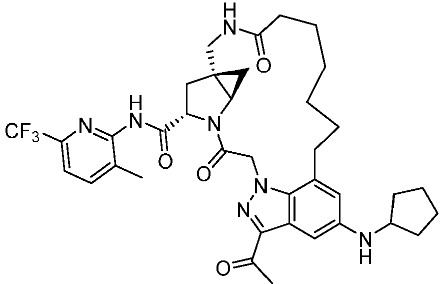 | | 2.8 (B) | 708 |
| | (41R,43S,45R)-13-acetyl-15-(cyclopentylamino)-N-(3-methyl-6-(trifluoromethyl)pyridin-2-yl)-3,7-dioxo-11H-42,6-diaza-1(1,7)-indazola-4(2,5)- | | | |
| 43 | 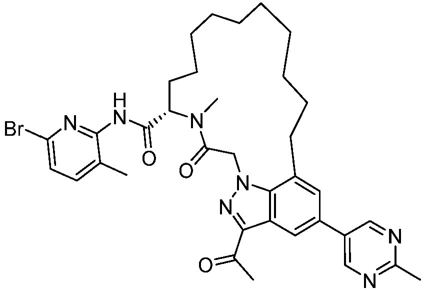 | | 3.52 (B) | 688 |
| | (S)-2-acetyl-N-(6-bromo-3-methylpyridin-2-yl)-17-methyl-4-(2-methylpyrimidin-5-yl)-18-oxo-6,7,8,9,10,11,12,13,14,15,16,17,18,19-tetradecahydro-[1,4]diazacycloheptadecino[16,17,1-hi]indazole-16-carboxamide | | | |
| 44 | 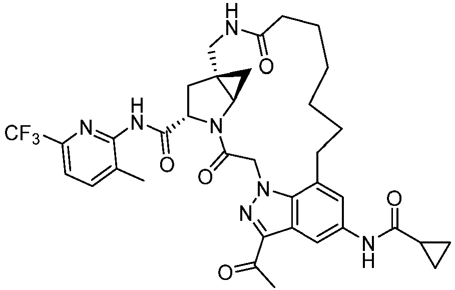 | | 3.05 (B) | 708 |
| | (41R,43S,45R)-13-acetyl-15-(cyclopropanecarboxamido)-N-(3-methyl-6- | | | |
| | (trifluoromethyl)pyridin-2-yl)-3,7-dioxo-11H-42,6-diaza-1(1,7)-indazola-4(2,5)- | | | |
| 46 | 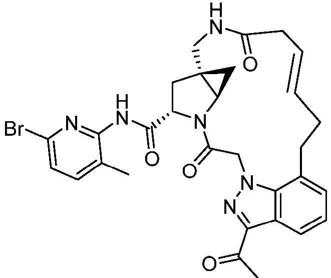 | *** | 3.28 (B) | 619 |
| | (41R,43S,45R,E)-13-acetyl-N-(6-bromo-3-methylpyridin-2-yl)-3,7-dioxo-11H-42,6-diaza-1(1,7)-indazola-4(2,5)-bicyclo[3.1.0]hexanacyclododecaphan-9-ene-43-carboxamide | | | |
| 47 | 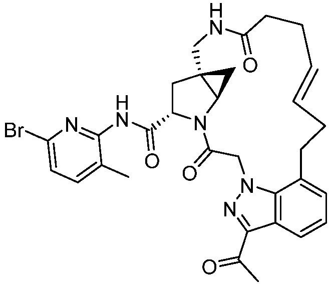 | *** | 3.40 (B) | 633 |
| | (41R,43S,45R,E)-13-acetyl-N-(6-bromo-3-methylpyridin-2-yl)-3,7-dioxo-11H-42,6-diaza-1(1,7)-indazola-4(2,5)-bicyclo[3.1.0]hexanacyclotridecaphan-10-ene-43-carboxamide | | | |
| 49 | 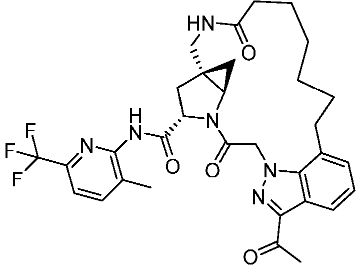 | | | |
| | (41R,43S,45R)-13-acetyl-N-(3-methyl-6-(trifluoromethyl)pyridin-2-yl)-3,7-dioxo-11H-42,6-diaza-1(1,7)-indazola-4(2,5)- | | | |
| 50 | 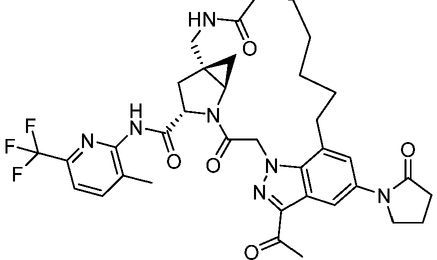 | | | |
| | (41R,43S,45R)-13-acetyl-N-(3-methyl-6-(trifluoromethyl)pyridin-2-yl)-3,7-dioxo-15-(2-oxopyrrolidin-1-yl)-11H-42,6-diaza-1(1,7)-indazola-4(2,5)-bicyclo[3.1.0]hexanacyclotridecaphane-43-carboxamide | | | |

### EXAMPLE 3. HUMAN FACTOR D ASSAY

Human Factor D (purified from human serum, Complement Technology, Inc.) at 80 nM final concentration is incubated with test compound at various concentrations for 5 min at room temperature in 50 mM Tris, 1M NaCl, pH 7.5. A synthetic substrate Z-L-Lys-SBzl and DTNB (Ellman's reagent) are added to final concentrations of 100 µM each. Absorbance at 405 nm (A₄₀₅) is recorded at 30 second intervals for 30 min using a microplate spectrophotometer. IC₅₀ values are calculated by nonlinear regression of complement Factor D reaction rates as a function of test compound concentration.

### EXAMPLE 4. HEMOLYSIS ASSAY

The hemolysis assay was previously described by G. Ruiz-Gomez, et al., J. Med. Chem. (2009) 52: 6042-6052. Prior to the assay, the optimum concentration of Normal Human Serum (NHS) needed to achieve 100% lysis of rabbit erythrocytes (RE) is determined by titration. In the assay, NHS (Complement Technology) is diluted in GVB⁰ Buffer (0.1 % gelatin, 5 mM Veronal, 145 mM NaCl, 0.025 % NaN₃, pH 7.3, Complement Technology) plus 10 mM Mg-EGTA and incubated with test compound at various concentrations for 15 min at 37°C. RE (Complement Technology) freshly suspended in GVB⁰ plus 10 mM Mg-EGTA are added to a final concentration of 1 x 10⁸ cells/mL and reactions are incubated for 30 min at 37°C. Positive control reactions (100% lysis) consist of GVB⁰ plus 10 mM Mg-EGTA with NHS and RE but without test compound; negative control reactions (0% lysis) consist of GVB⁰ plus 10 mM Mg-EGTA with RE only. Samples are centrifuged at 2000g for 3 min and supernatants collected. Absorbance at 405 nm (A₄₀₅) is recorded using a microplate spectrophotometer. IC₅₀ values are calculated by nonlinear regression from the percentage of hemolysis as a function of test compound concentration.

## Claims

1. A compound selected from: 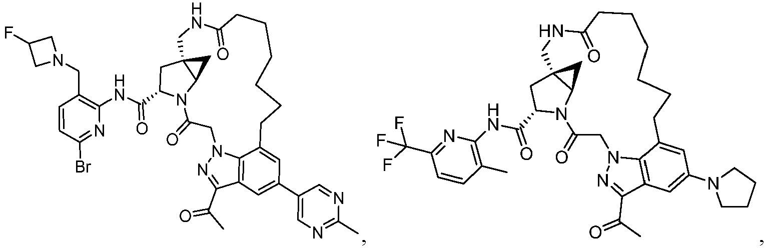 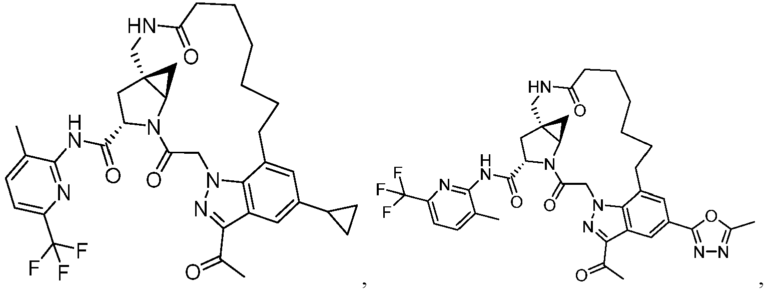 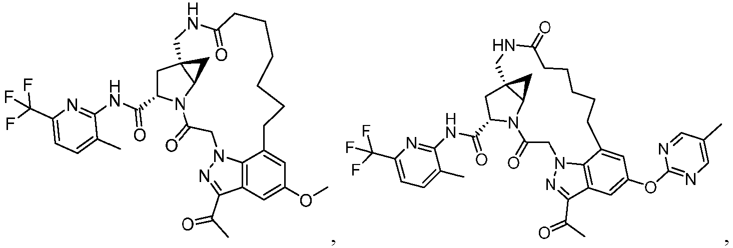 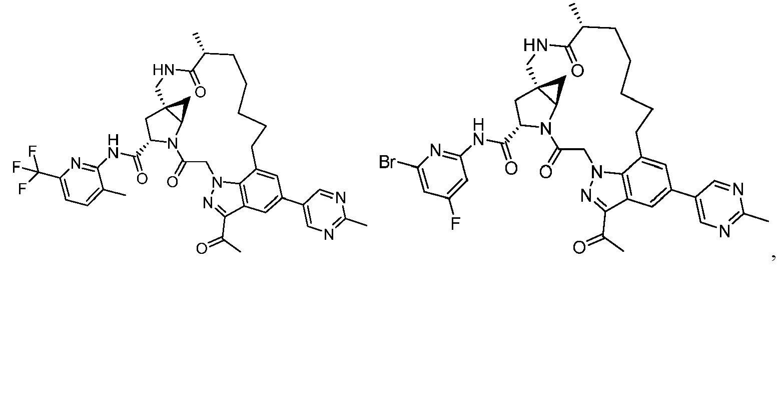 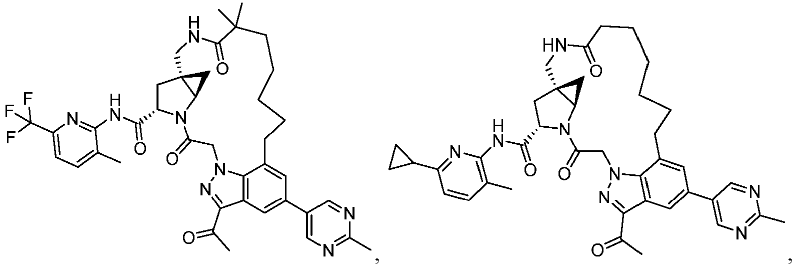 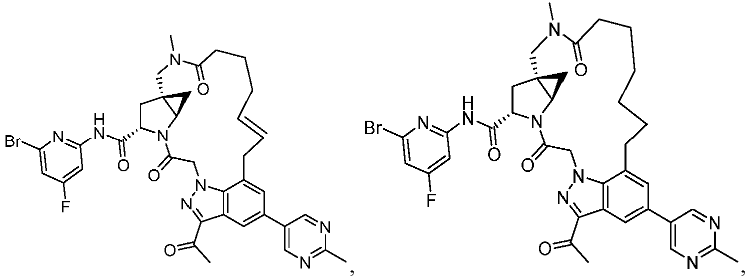 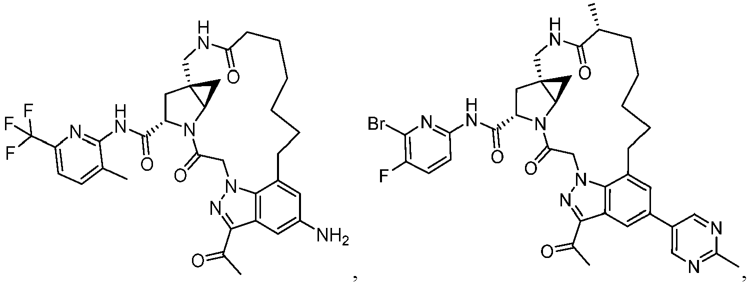 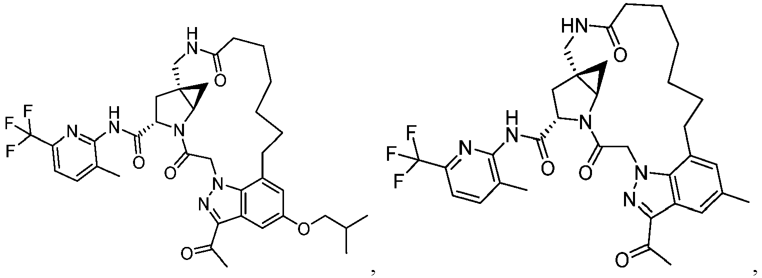 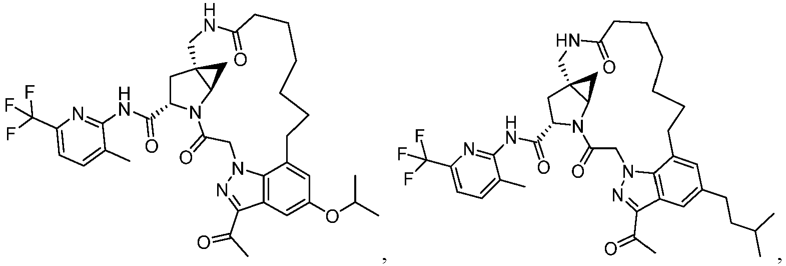 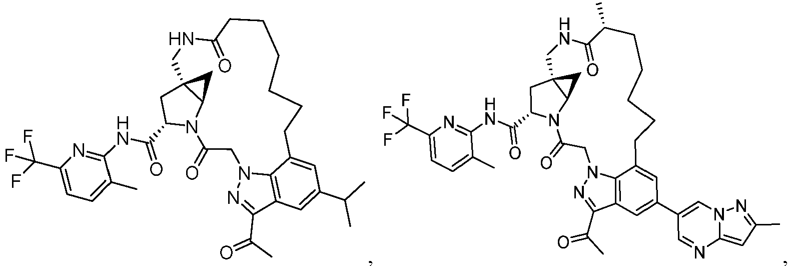 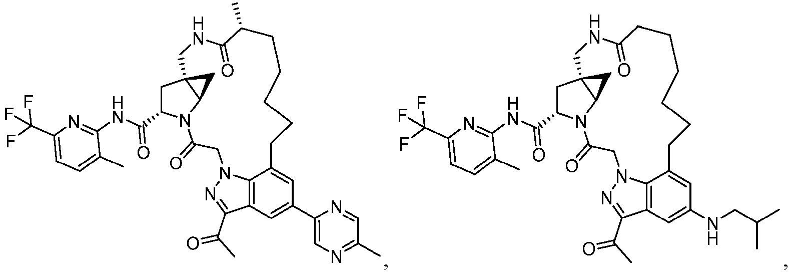 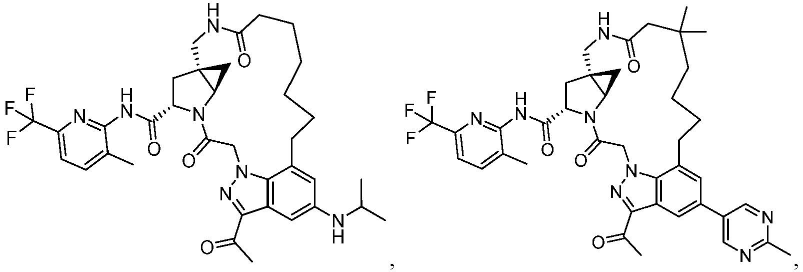 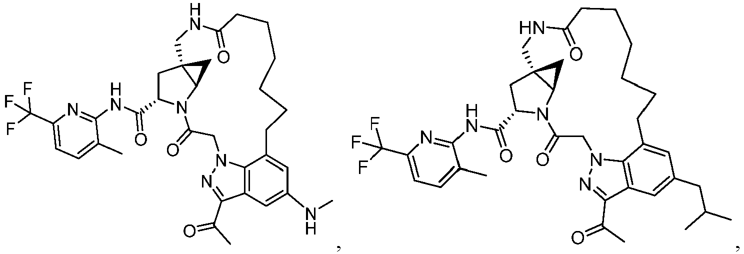 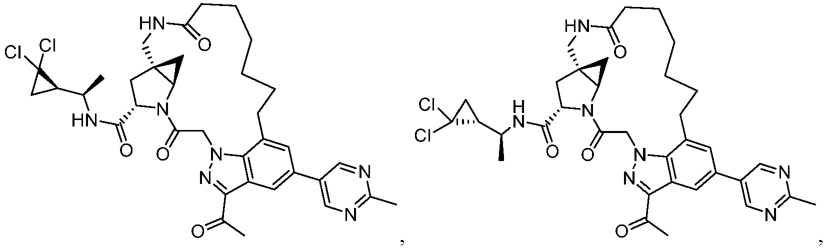 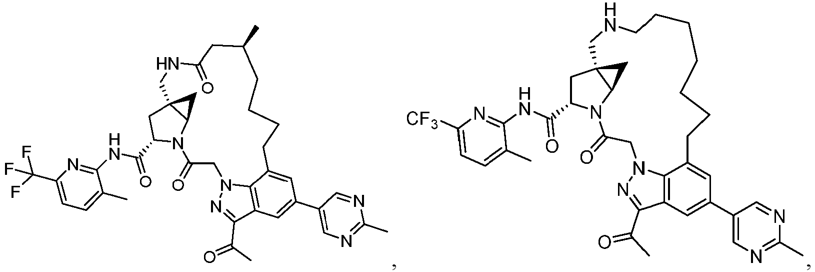 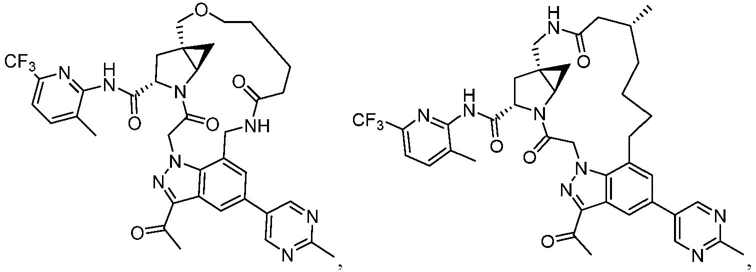 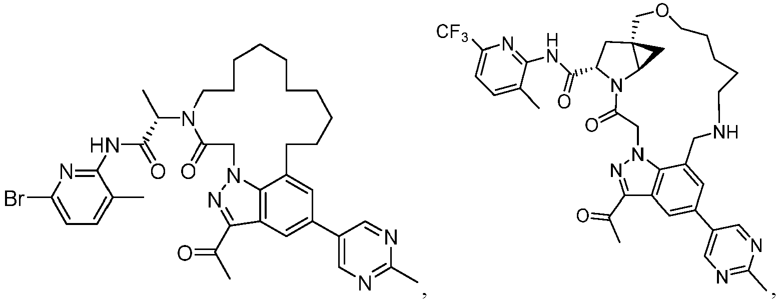 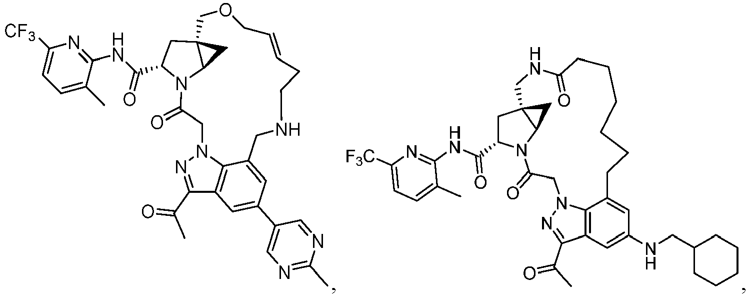 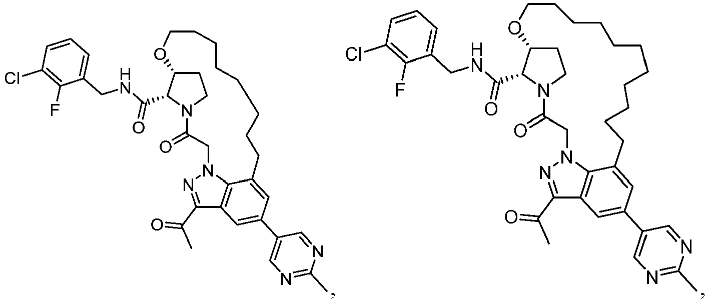 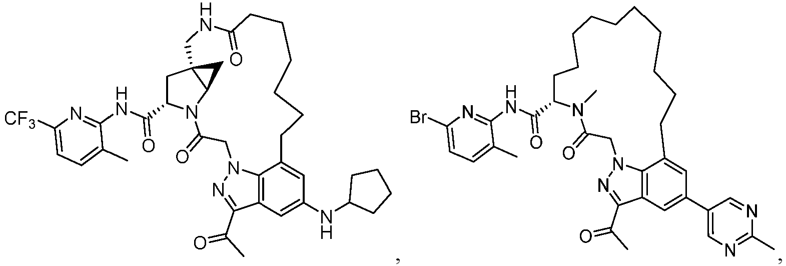 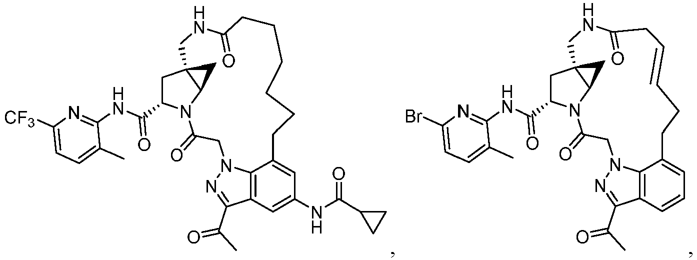 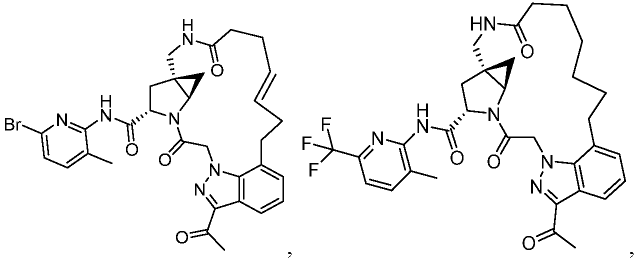 and 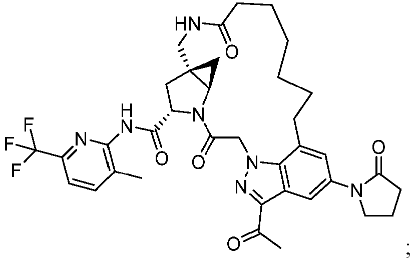 or a pharmaceutically acceptable salt thereof.

2. A pharmaceutical composition comprising a compound of claim 1 or a pharmaceutically acceptable salt thereof, and a pharmaceutically acceptable carrier.

3. A compound according to claim 1 or a pharmaceutically acceptable salt thereof, or a pharmaceutical composition according to claim 2, for use in treating a complement factor D mediated disorder in a subject in need thereof, wherein said use comprises administering a therapeutically effective amount of said compound or pharmaceutical composition to said subject;
wherein the disorder is an ophthalmic disorder, preferably geographic atrophy; C3 glomerulopathy; age related macular degeneration (AMD); paroxysmal nocturnal hemoglobinuria (PNH); C3 glomerulonephritis; dense deposit disease; atypical hemolytic uremic syndrome (aHUS); neuromyelitis optica (NMO); myasthenia gravis; IgA nephropathy; lupus nephitis; or sickle cell disease.

4. A compound for use or composition for use according to claim 3, wherein the subject is a human.

5. A compound for use or composition for use according to claim 3 or 4, wherein the disorder is an ophthalmic disorder, preferably geographic atrophy.

6. A compound for use or composition for use according to claim 3 or 4, wherein the disorder is C3 glomerulopathy, C3 glomerulonephritis, or dense deposit disease.

7. A compound for use or composition for use according to claim 3 or 4, wherein the disorder is AMD.

8. A compound for use or composition for use according to claim 3 or 4, wherein the disorder is PNH.

9. A compound for use or composition for use according to claim 3 or 4, wherein the disorder is myasthenia gravis.

10. A compound for use or compostion for use according to claim 3 or 4, wherein the disorder is aHUS.

11. A compound for use or compostion for use according to claim 3 or 4, wherein the disorder is NMO.

12. A compound for use or composition for use according to claim 3 or 4, wherein the disorder is IgA nephropathy.

13. A compound for use or composition for use according to claim 3 or 4, wherein the disorder is lupus nephritis.

14. A compound for use or composition for use according to claim 3 or 4, wherein the disorder is sickle cell disease.

## Patentansprüche

1. Verbindung, ausgewählt aus: 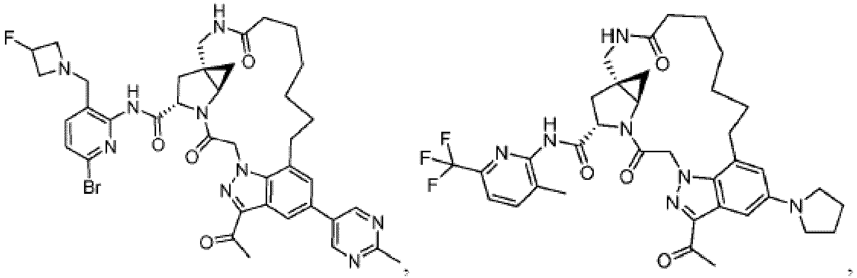 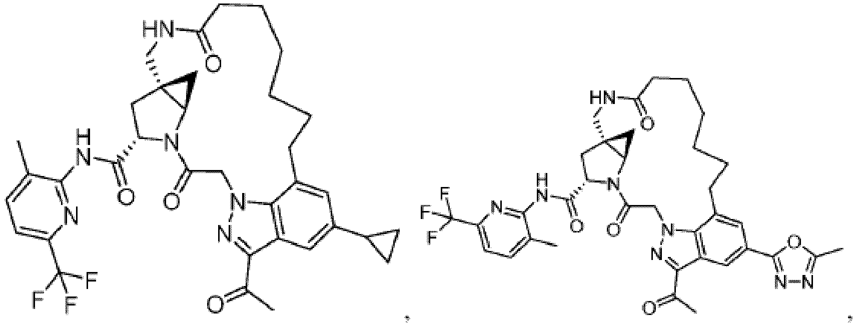 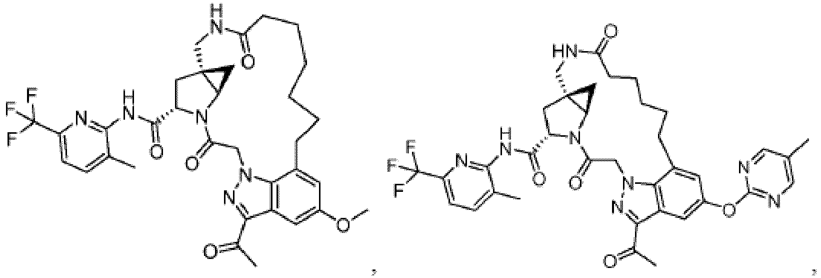 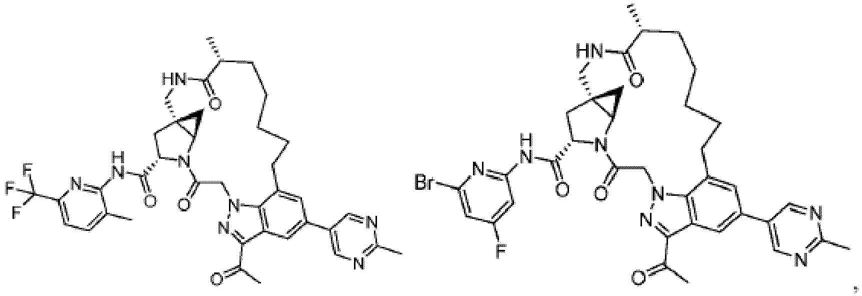 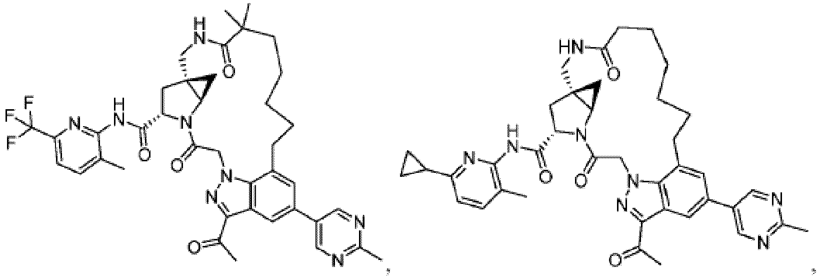 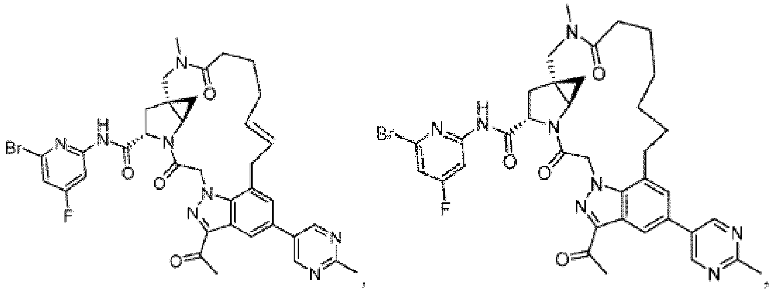 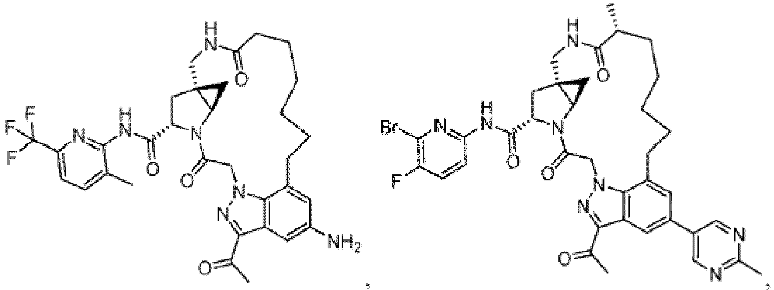 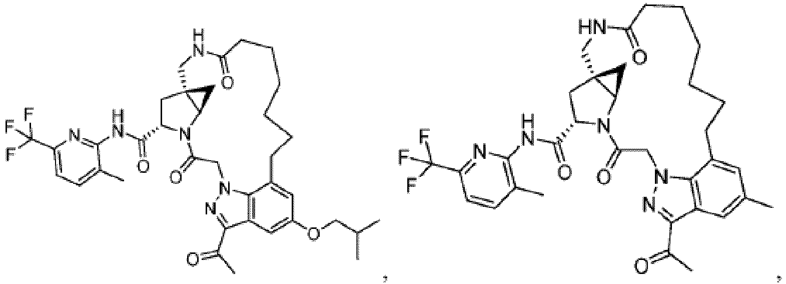 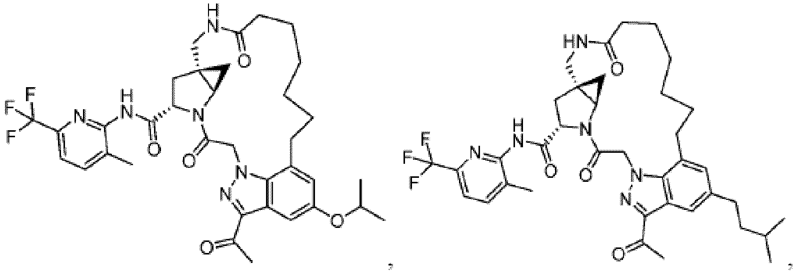 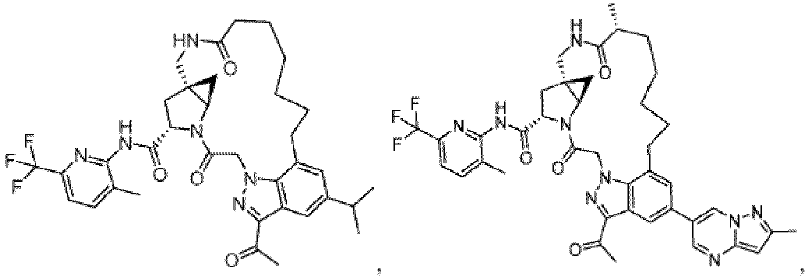 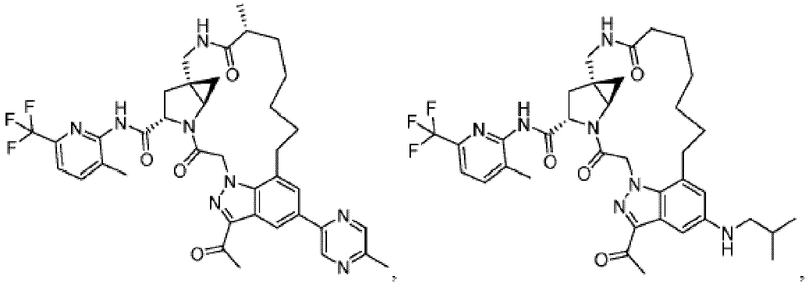 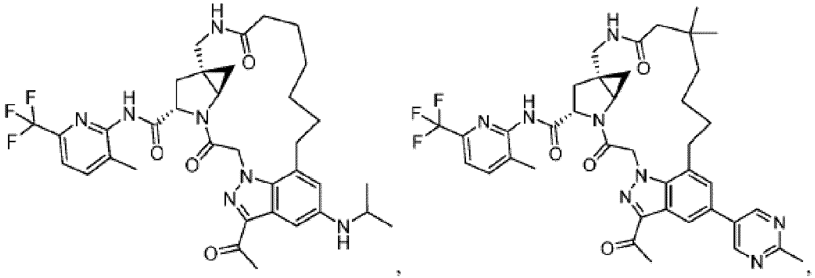 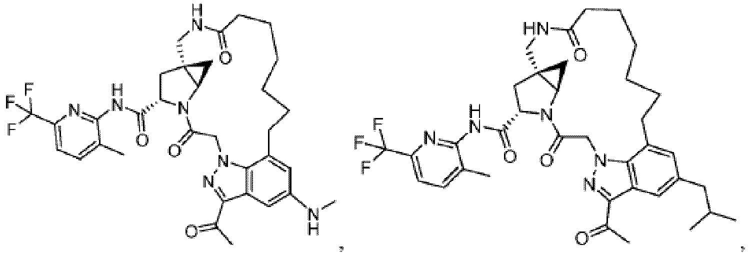 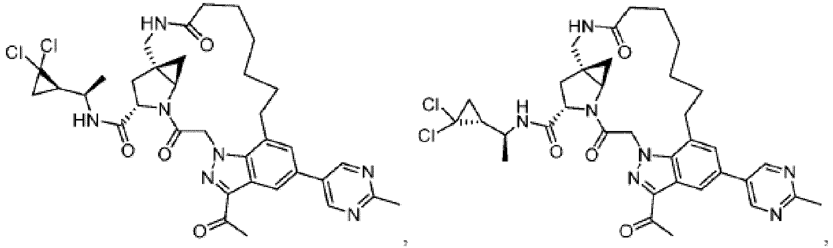 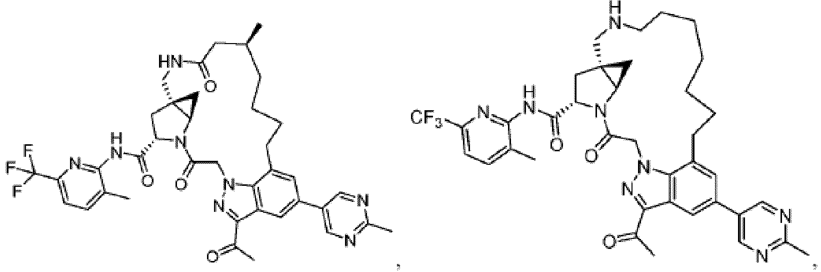 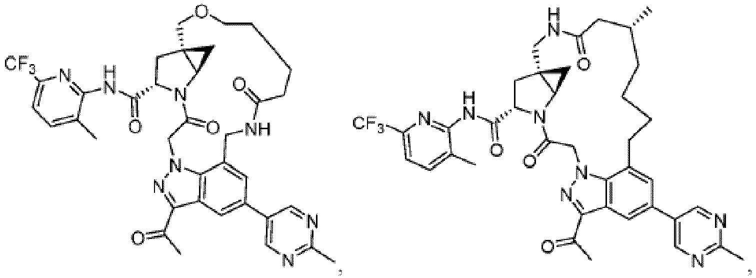 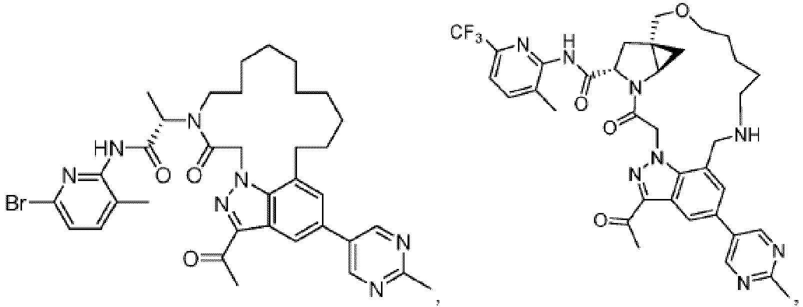 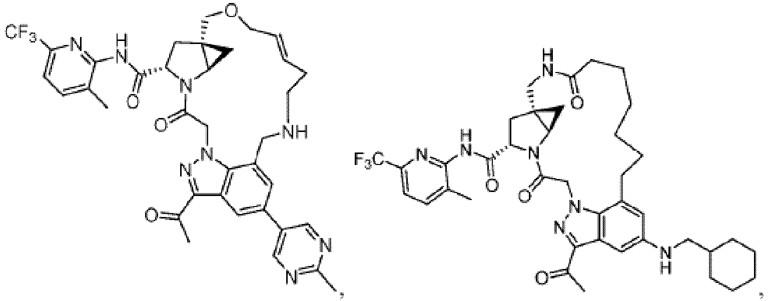 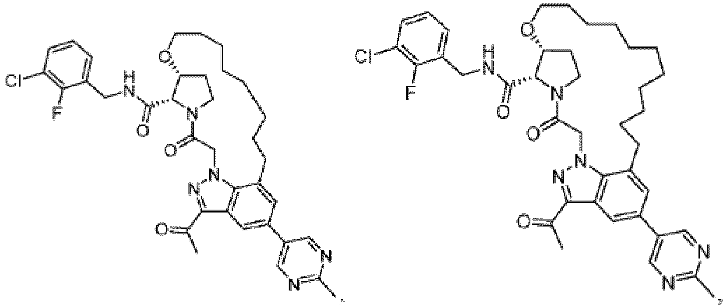 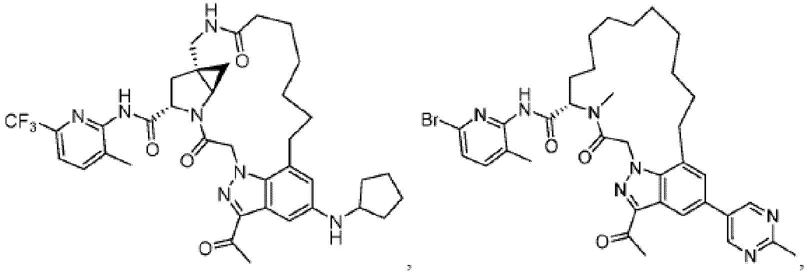 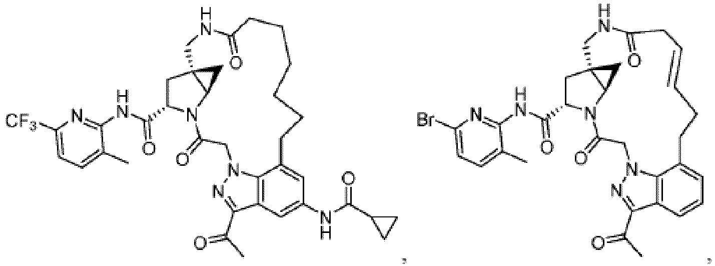 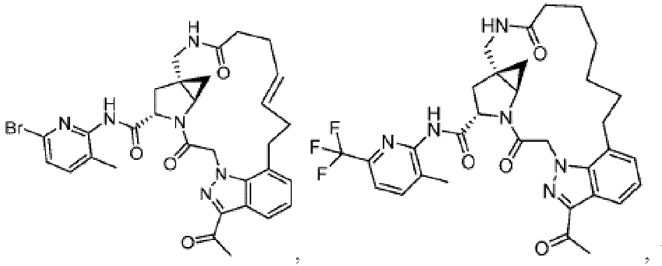 und 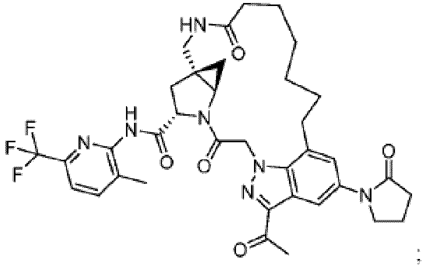 oder einem pharmazeutisch unbedenklichen Salz davon.

2. Pharmazeutische Zusammensetzung, umfassend eine Verbindung nach Anspruch 1 oder ein pharmazeutisch unbedenkliches Salz davon und einen pharmazeutisch unbedenklichen Träger.

3. Verbindung nach Anspruch 1 oder pharmazeutisch unbedenkliches Salz davon oder pharmazeutische Zusammensetzung nach Anspruch 2 für die Verwendung bei der Behandlung einer durch Komplementfaktor D vermittelten Erkrankung bei einem Subjekt, das dessen bedarf, wobei die Verwendung das Verabreichen einer therapeutisch wirksamen Menge der Verbindung oder der pharmazeutischen Zusammensetzung an das Subjekt umfasst;
wobei die Erkrankung eine Augenerkrankung ist, vorzugsweise eine geografische Atrophie; C3-Glomerulopathie; altersbedingte Makuladegeneration (AMD); paroxysmale nächtliche Hämoglobinurie (PNH); C3-Glomerulonephritis; Dense-Deposit-Krankheit; atypisches hämolytisch-urämisches Syndrom (aHUS); Neuromyelitis optica (NMO); Myasthenia gravis; IgA-Nephropathie; Lupus nephritis; oder Sichelzell-Anämie.

4. Verbindung für die Verwendung oder Zusammensetzung für die Verwendung nach Anspruch 3, wobei das Subjekt ein Mensch ist.

5. Verbindung für die Verwendung oder Zusammensetzung für die Verwendung nach Anspruch 3 oder 4, wobei die Erkrankung eine Augenerkrankung, vorzugsweise eine geografische Atrophie, ist.

6. Verbindung für die Verwendung oder Zusammensetzung für die Verwendung nach Anspruch 3 oder 4, wobei die Erkrankung eine C3-Glomerulopathie, eine C3-Glomerulonephritis oder eine Dense-Deposit-Krankheit ist.

7. Verbindung für die Verwendung oder Zusammensetzung für die Verwendung nach Anspruch 3 oder 4, wobei die Erkrankung AMD ist.

8. Verbindung für die Verwendung oder Zusammensetzung für die Verwendung nach Anspruch 3 oder 4, wobei die Erkrankung PNH ist.

9. Verbindung für die Verwendung oder Zusammensetzung für die Verwendung nach Anspruch 3 oder 4, wobei die Erkrankung Myasthenia gravis ist.

10. Verbindung für die Verwendung oder Zusammensetzung für die Verwendung nach Anspruch 3 oder 4, wobei die Erkrankung aHUS ist.

11. Verbindung für die Verwendung oder Zusammensetzung für die Verwendung nach Anspruch 3 oder 4, wobei die Erkrankung NMO ist.

12. Verbindung für die Verwendung oder Zusammensetzung für die Verwendung nach Anspruch 3 oder 4, wobei die Erkrankung IgA-Nephropathie ist.

13. Verbindung für die Verwendung oder Zusammensetzung für die Verwendung nach Anspruch 3 oder 4, wobei die Erkrankung Lupus nephritis ist.

14. Verbindung für die Verwendung oder Zusammensetzung für die Verwendung nach Anspruch 3 oder 4, wobei die Erkrankung Sichelzellkrankheit ist.

## Revendications

1. Composé choisi parmi : 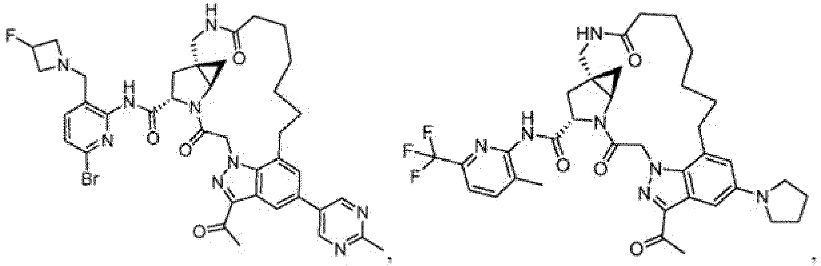 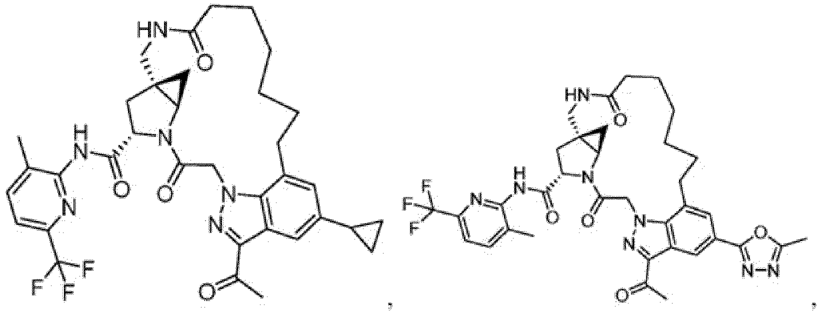 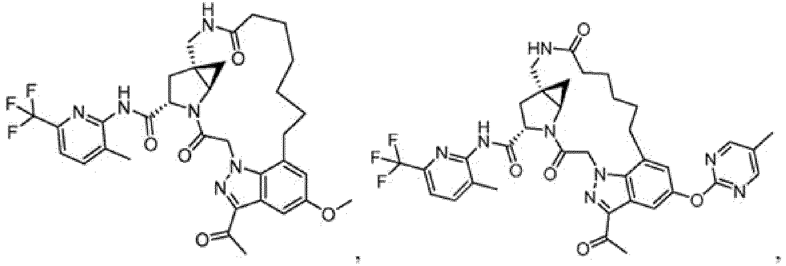 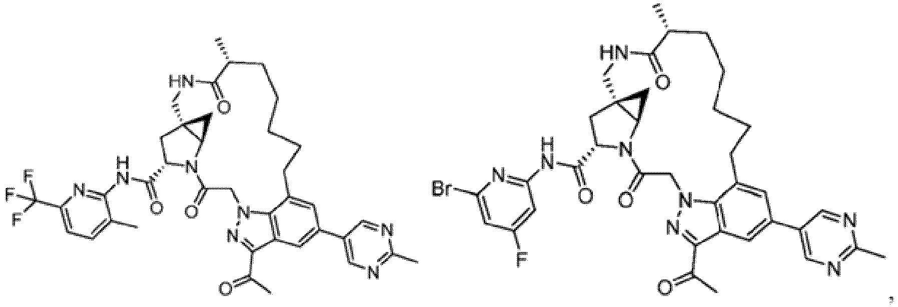 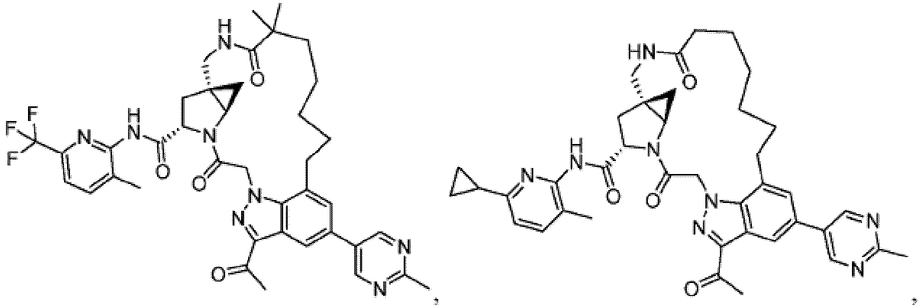 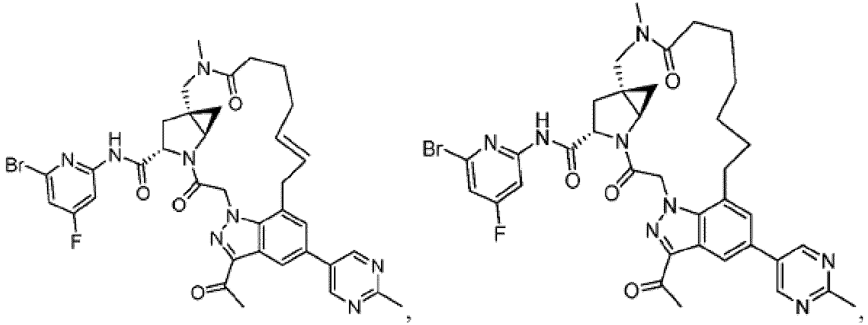 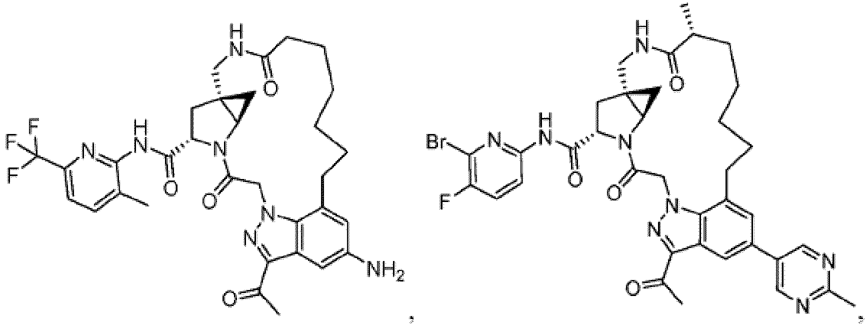 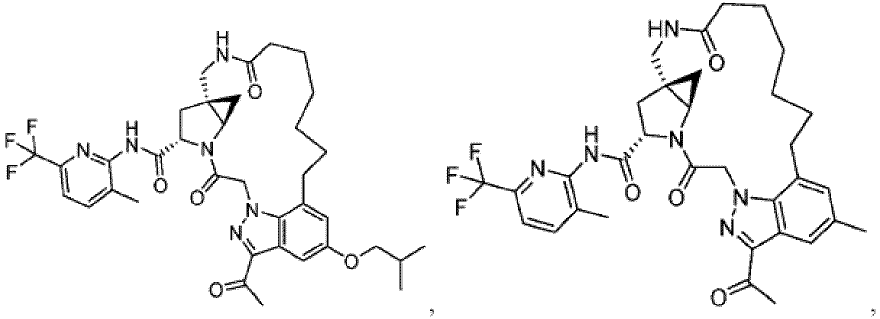 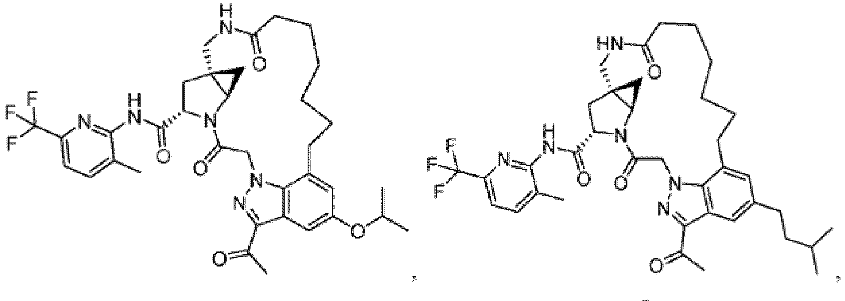 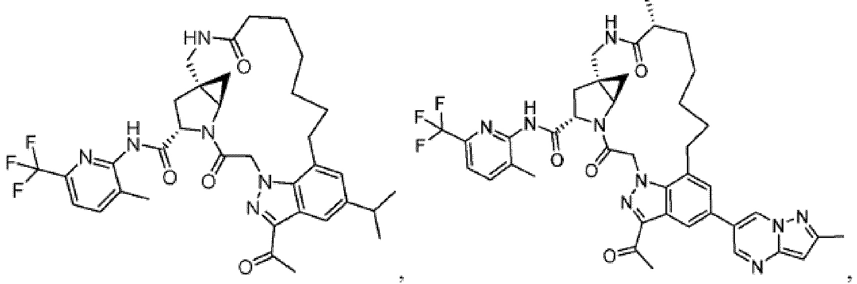 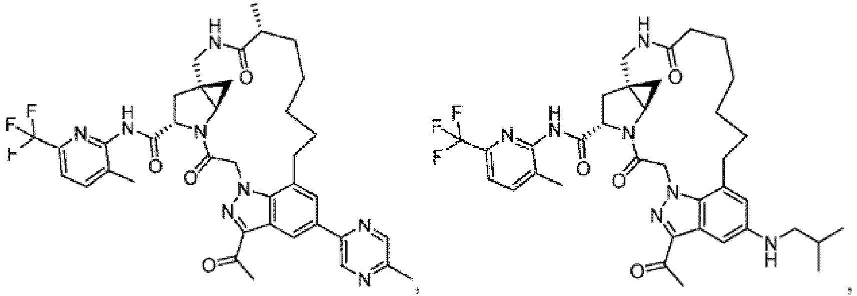 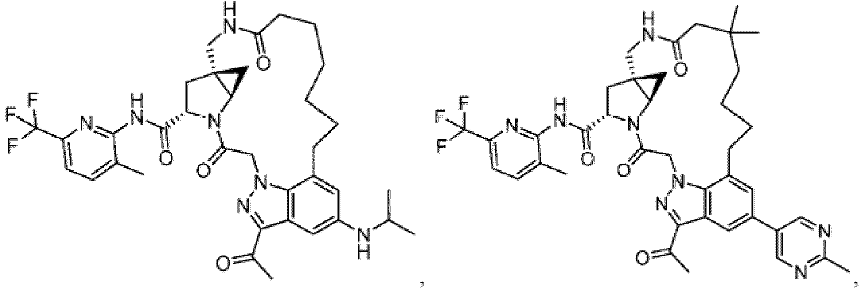 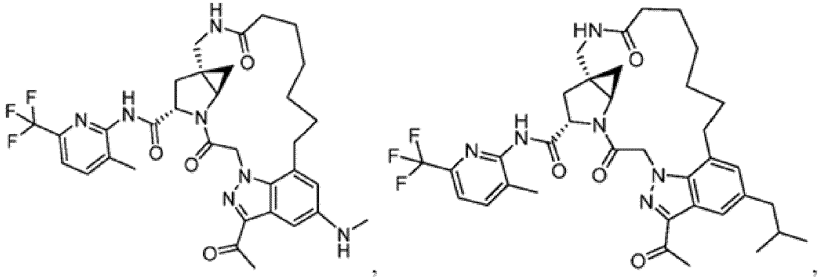 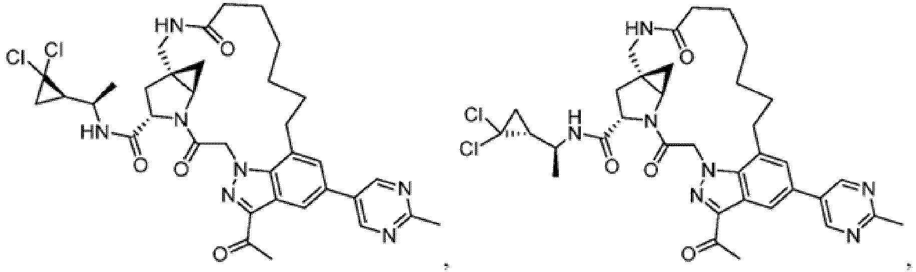 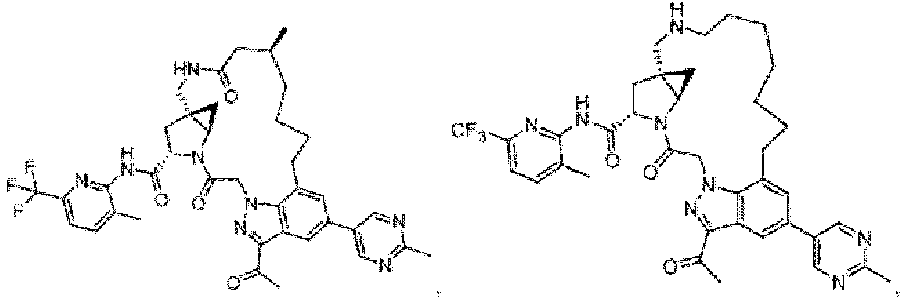 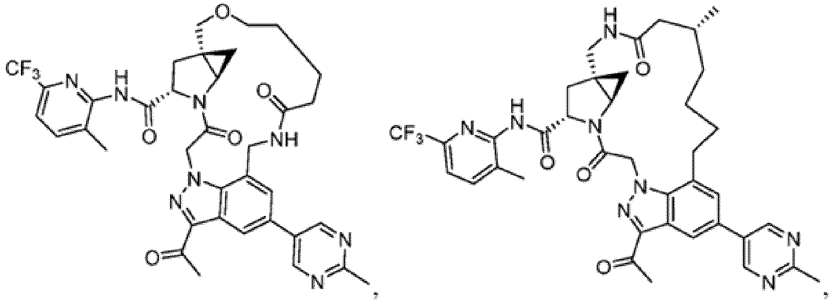 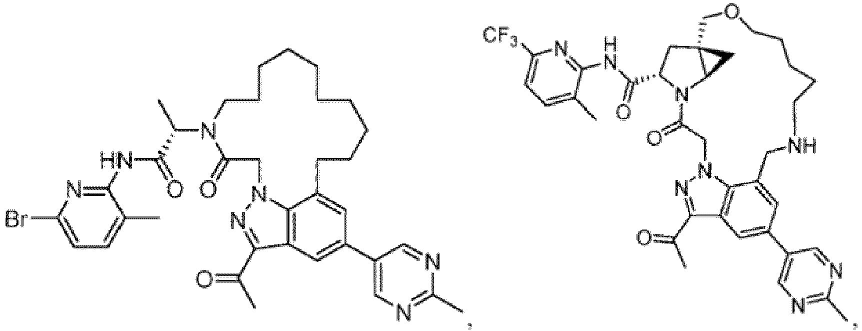 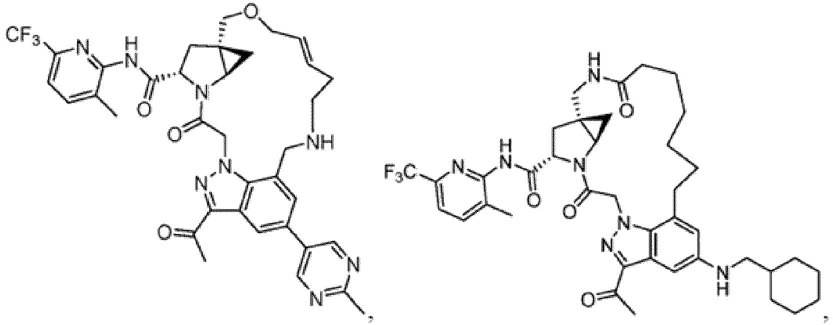 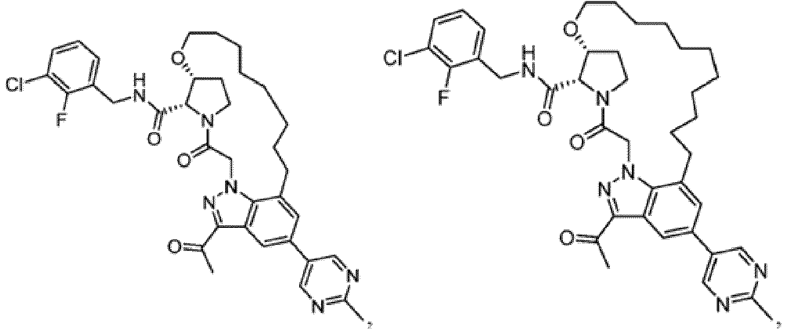 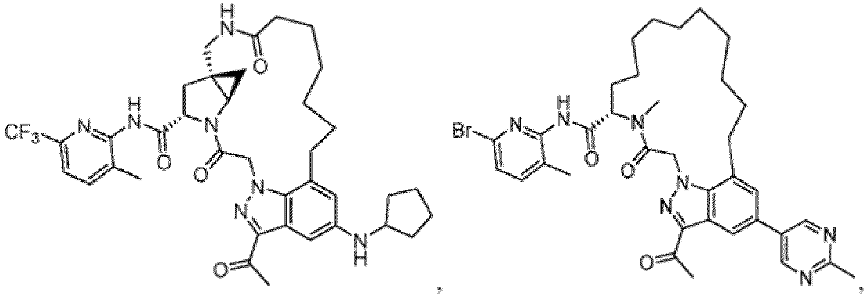 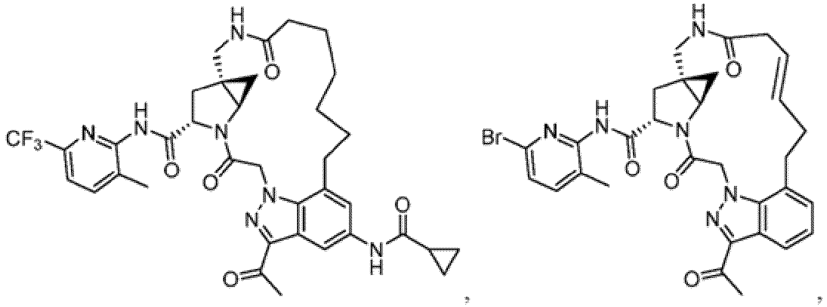 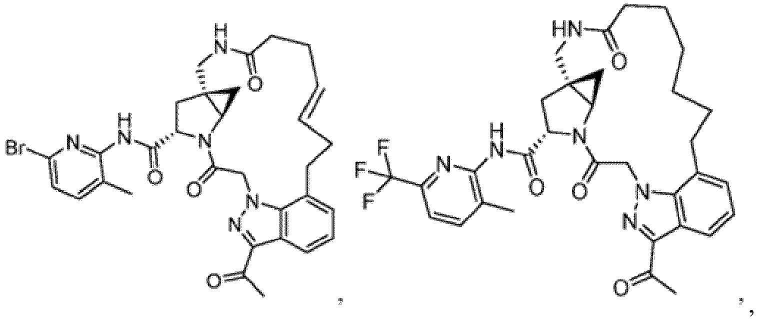 et 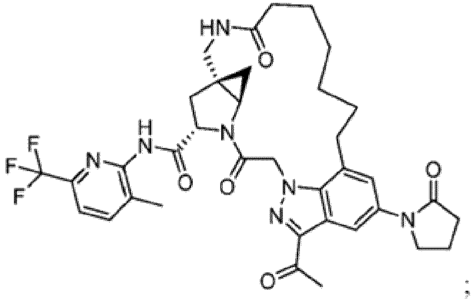 ou sel pharmaceutiquement acceptable de celui-ci.

2. Composition pharmaceutique comprenant un composé selon la revendication 1 ou un sel pharmaceutiquement acceptable de celui-ci et un vecteur pharmaceutiquement acceptable.

3. Composé selon la revendication 1 ou sel pharmaceutiquement acceptable de celui-ci, ou composition pharmaceutique selon la revendication 2, destinés à être utilisés dans le traitement d'un trouble médié par le facteur D du complément chez un sujet qui en a besoin, ladite utilisation comprenant l'administration d'une quantité thérapeutiquement efficace dudit composé ou de ladite composition pharmaceutique audit sujet,
ledit trouble étant un trouble ophtalmique, de préférence une atrophie géographique, une glomérulopathie C3, une dégénérescence maculaire liée à l'âge (DMLA), une hémoglobinurie paroxystique nocturne (HPN), une glomérulonéphrite C3, la maladie des dépôts denses, le syndrome hémolytique et urémique atypique (SHU atypique), la neuromyélite optique (NMO), la myasthénie, la néphropathie à IgA, la glomérulonéphrite lupique ou la drépanocytose.

4. Composé destiné à être utilisé ou composition destinée à être utilisée selon la revendication 3, le sujet étant un être humain.

5. Composé destiné à être utilisé ou composition destinée à être utilisée selon la revendication 3 ou 4, le trouble étant un trouble ophtalmique, de préférence une atrophie géographique.

6. Composé destiné à être utilisé ou composition destinée à être utilisée selon la revendication 3 ou 4, le trouble étant une glomérulopathie C3, une glomérulonéphrite C3 ou la maladie des dépôts denses.

7. Composé destiné à être utilisé ou composition destinée à être utilisée selon la revendication 3 ou 4, le trouble étant la DMLA.

8. Composé destiné à être utilisé ou composition destinée à être utilisée selon la revendication 3 ou 4, le trouble étant l'HPN.

9. Composé destiné à être utilisé ou composition destinée à être utilisée selon la revendication 3 ou 4, le trouble étant la myasthénie.

10. Composé destiné à être utilisé ou composition destinée à être utilisée selon la revendication 3 ou 4, le trouble étant le SHU atypique.

11. Composé destiné à être utilisé ou composition destinée à être utilisée selon la revendication 3 ou 4, le trouble étant la NMO.

12. Composé destiné à être utilisé ou composition destinée à être utilisée selon la revendication 3 ou 4, le trouble étant la néphropathie à IgA.

13. Composé destiné à être utilisé ou composition destinée à être utilisée selon la revendication 3 ou 4, le trouble étant la glomérulonéphrite lupique.

14. Composé destiné à être utilisé ou composition destinée à être utilisée selon la revendication 3 ou 4, le trouble étant la drépanocytose.
